# EUROPEAN PATENT APPLICATION

(11) **EP 1 944 315 A1**
(43) Date of publication of application: **16.07.2008**
(21) Application number: 07000521.0
(22) Date of filing: 11.01.2007
(51) Int. Cl.: C07K 14/47, C07K 16/18, A61K 39/00

(54) **Prophylaxis and therapy of alzheimer's disease and other neurodementing diseases**

(71) Applicant: Philipps-Universität Marburg, 35037 Marburg (DE); Universität Konstanz, 78464 Konstanz (DE)
(72) Inventor: Dodel, Richard, 35096 Weimar a.d. Lahn (DE); Bacher, Michael, 35091 Cölbe (DE); Przybylski, Michael, 65468 Trebur (DE); Stefanescu, Raluca, 78467 Konstanz (DE); Manea, Marilena, 78467 Konstanz (DE)
(74) Representative: Dehmel, Albrecht

(57) **Abstract**

The present inventions relates to polypeptides binding to a C-terminal epitope of Aβ-peptide, methods of obtaining such polypeptides, and use of said polypeptides in human and veterinary medicine, in particular for treatment and prophylaxis of Alzheimer's Disease and other neurodementing diseases.

## Description

### Field of the Invention

The present invention relates to polypeptides binding to a C-terminal epitope of Aβ-peptide, methods of obtaining such polypeptides, and use of said polypeptides in human and veterinary medicine, in particular for treatment and prophylaxis of Alzheimer's Disease and other neurodementing diseases.

### Description of Related Art

Alzheimer's disease (AD), the most common form of dementia among elderly population (prevalence: 1000/100,000; > 65 years), represents the fourth leading cause of death in the developed world. Cortical atrophy, neuronal loss, region-specific amyloid deposition, neuritic plaques, and neurofibrillary tangles are key neuropathological features in AD brain. These alterations are thought to be linked to cognitive decline which clinically defines AD. Within these markers, neuritic plaques are amyloid immunoreactive, thioflavin positive, and accompanied by astrogliosis, microgliosis, cytoskeletal changes, and synaptic loss. The degree of neuritic degeneration within plaques correlates with clinical parameters of dementia. Neuritic plaques are spherical, multicellular lesions that are usually found in moderate to large numbers in limbic structures and associated neocortices of the AD brain.
These plaques are comprised of extracellular deposits of amyloid-β peptide(s) (Aβ) that include abundant amyloid fibrils intermixed with non-fibrillary forms of the peptide. Such plaques also contain variable numbers of activated microglia that are often situated very near the fibrillar amyloid core, as well as reactive astrocytes surrounding the core.

The major constituent of the neuritic plaque, β-amyloid polypeptide (Aβ), arises from a larger precursor protein, the amyloid precursor protein (APP) (Kang, et al., 1987; Tanzi, et al., 1987). Aβ is produced by normal cells and can be detected as a circulating peptide in the plasma and cerebrospinal fluid (CSF) of healthy humans. Although the physiological role of the amyloid precursor protein (APP) in the brain is not well understood, missense mutations in APP confer autosomal dominant inheritance of AD (FAD), and shed light on potentially important pathogenic mechanism(s). The accumulation of Aβ, a 39-42 amino acid proteolytic product of APP, in neuritic plaques, structures which at autopsy fulfill the neuropathological criteria for a definitive diagnosis of AD, is thought to be causative for disease progression. A major Aβ cleavage product of APP is the Aβ(1-42) polypeptide, but Aβ peptides shorter at the C-terminus (39 to 41) are also produced by the proteolytic (y-secretase) cleavage in the membrane. The N-terminal part of Aβ(1-42) is localized in the extracellular region of APP, and the major C-terminal part of the Aβ peptide is contained within the transmembrane domain.

Missense mutations, in APP associated with FAD, occur in proximity to the Aβ domain and result in an increase in the production of the 4kDa Aβ peptide. In AD, it has been postulated that increased synthesis and/or a decreased clearance of Aβ may lead to amyloid plaque deposition and subsequently to the neuropathological changes associated with the disease. In vitro studies, using synthetic Aβ peptide(s), have shown that neurotoxicity is dependent on Aβ being fibrillar and predominantly in a β-pleated sheet conformation.

The accumulation of extracellular plaques containing the neurotoxic amyloid peptide fragment (Aβ) of β-amyloid precursor protein (APP), as the major product, is one of the characteristics of Alzheimer's disease (AD). Although APP has been recognised as a key molecule for AD, the molecular (patho-) physiological degradation and proteolytic pathways of APP, and cellular interactions and biochemical fate of Aβ peptide(s) are still unclear. Despite the lack of details on degradation pathways and cellular transport for the formation and deposition of Aβ-derived plaques, recent studies towards the development of immunisation methods of AD based on therapeutically active antibodies produced from Aβ(1-42) have yielded initial success in transgenic mouse models of Alzheimer's disease. Several reports have demonstrated that antibodies generated by immunisation with Aβ(1-42) are capable of inhibiting the formation of Aβ-plaques by disaggregating Aβ-fibrils, and improve the impairments in the spatial memory of mice. The transgenic APPV717F mouse (TG mouse) is a well characterized model of AD-like plaque pathology with age- and region-dependent deposits of Aβ(1-40) and Aβ(1-42) (Games, et al., 1995). Recently, Schenk et al. and others investigated alterations in the deposition of Aβ in APPV717F TG mouse following immunization with pre-aggregated Aβ(1-42) or administration of antibodies against Aβ (Bard, et al., 2000; Schenk, et al., 1999). Both immunization and administration of Aβ antibodies significantly attenuated amyloid plaque deposition, neuritic dystrophy, and astrogliosis. In these studies, increased titers of mouse anti-human Aβ-antibodies were necessary for the observed reduction in plaque burden. These findings raise the possibility that formation and clearance of an Aβ-antigen: antibody complex may decrease brain Aβ deposition either following antibody generation within the central nervous system or by peripheral antibody transport across the blood-brain-barrier (BBB). Furthermore, passive immunization appears to reduce brain Aβ burden by altering Aβ equilibrium between the CNS and plasma (DeMattos, et al., 2001). Remarkably, active or passive immunization significantly reverses cognitive and memory impairment in APPV717F mouse or other APP transgenic mice (Dodart, et al., 2002; Janus, et al., 2000; Morgan, et al., 2000). These results suggest that immunization may prevent memory deficits possibly by altering a soluble pool of Aβ. Thus, treatment of AD patients with active or passive immunization is one of several emerging therapeutic approaches targeting the production, clearance, and aggregation of the Aβ peptide.

Based on these results, a clinical trial using an active immunization procedure (Aβ(1-42) peptide and/or preaggregates thereof; Adjuvans: QS21) was initiated for treatment of patients with established AD. Unfortunately, severe side-effects developed ("meningoencephalitis") and the clinical trial was stopped. A subgroup of AD patients (n=30) treated with active immunization in this clinical trial, was analyzed (Hock, et al., 2002; Hock, et al., 2003). The authors demonstrated that (i), immunization induces the production of antibodies against Aβ(1-42) and (ii), in patients where a production of antibodies was observable, the cognitive decline was significantly reduced in comparison to the untreated control group. The authors concluded that immunization may be a therapeutic option for AD.

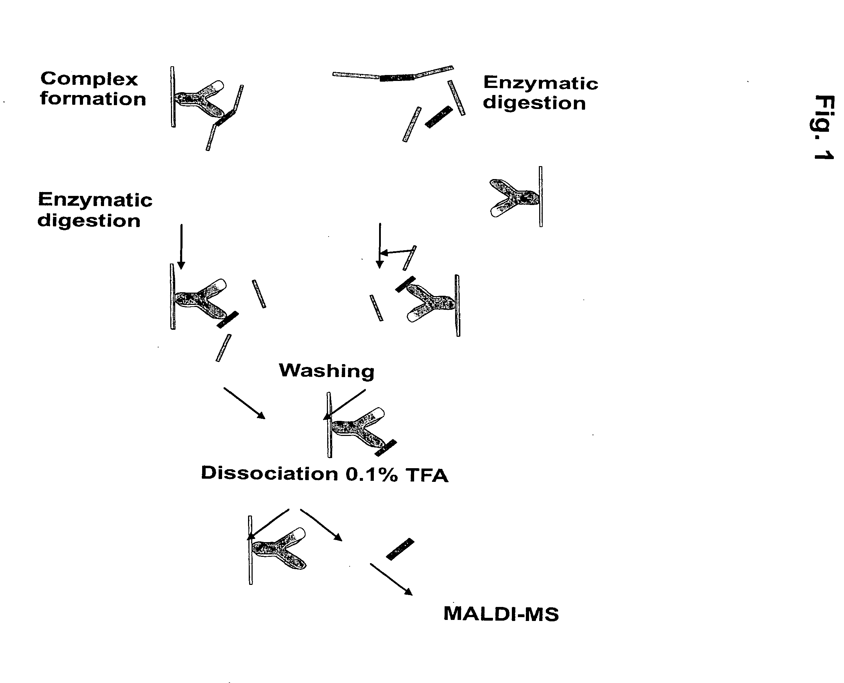

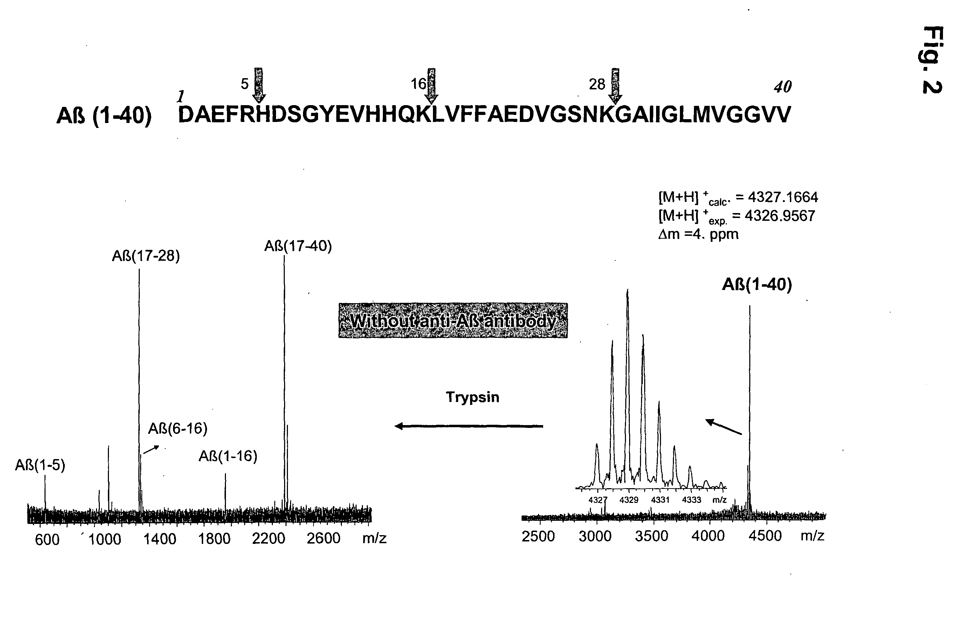

Recent studies elucidated in more detail the recognition properties of antibodies produced upon immunisation with Aβ(1-42). This work resulted in the identification of a specific Aβ-epitope recognised by the antibodies generated in transgenic AD mice (McLaurin et al., 2002; Przybylski et al., 2003). These results have been obtained by using selective proteolytic excision technologies (Epitope-Excision) in combination with high resolution mass spectrometry (FTICR-MS) as bioanalytical tools of high sensitivity and specificity for the identification of antigen epitopes (Macht et al 1996; Suckau et al 1992; Macht et al. 2004; see Figures 1, 2)). Using mass spectrometric epitope excision of the immobilised Aβ-antigen-immune complex, the epitope was identified to consist of the residues (4-10) (FRHDSGY) of Aβ(1-42). The selectivity of this recognition structure was ascertained by elucidation of the identical epitope from AD plaques, Aβ42 extracts from Aβ-protofibrils, chemically synthesised Aβ42, and other (Aβ-independent) polypeptides comprising the N-terminal Aβ sequence (Przybylski et al. 2003).

Anti-Aβ antibodies were identified by Du et al. in both the blood and the CSF from non-immunized humans (Du, et al., 2001). These antibodies specifically recognize human Aβ as has been shown by immunoprecipitation (Du, et al., 2001) and ELISA as well as mass spectrometric characterization of the immunoprecipitation product. Furthermore, the antibodies readily recognize synthetic Aβ(1-40) as well as human Aβ deposited in the brain of PDAPP transgenic mice. In addition, fibrillation/oligomerization and neurotoxicity of Aβ-peptides were reduced in the presence of Aβ-autoantibodies (Du, et al., 2003).

Furthermore, it has been investigated whether there is a difference of the Aβ-autoantibody concentration in patients with Alzheimer's disease compared to controls. Interestingly, a significant difference among the two groups was found, resulting in a substantially (approximately 15 - 20-fold) decreased titer of antibodies against Aβ in patients with Alzheimer's disease. These results have been confirmed recently by other groups (Weksler et al., 2004). Antibodies against Aβ can also be detected in commercially available intravenous IgG preparations (IVIgG). The treatment of patients with different neurological diseases with these intravenous immunoglobulin preparations led to the reduction of Aβ concentration in the CSF (Dodel, et al., 2002). The substantial effect of the Aβ-autoantibodies in preventing, and protecting for Aβ-plaque deposition was also established in young (4 months) APP-transgenic (TgCRND8) mice. Additionally, in a pilot trial with 5 patients with AD, utilizing, total Aβ was reduced significantly upon delivery of IVIgG in the CSF and increased in the serum (Dodel, et al., 2004). In the five investigated patients no cognitive deterioration was observed during the six months observation period.

However, administering IVIgG to a patient with AD is not convenient and associated with high costs, as the fraction of therapeutic Aβ autoantibodies is low. The vast majority of IgG in this preparation is not Aβ specific and may provide for undesirable effects. Furthermore the sources for IVIgG are limited which is in view of the quantity of patients with Alzheimer's disease an unacceptable disadvantage.

Thus, the inventors faced the problem to provide a reliable tool with constant quality for therapy and prophylaxis of Alzheimer's disease and other neurodementing diseases.

The inventors have solved the problem by way of the subject matter set forth in the claims.

The following figures form part of the present specification and are included to further demonstrate certain aspects of the present invention. The invention may be better understood by reference to one or more of these figures in combination with the detailed description of specific embodiments presented herein.
**Figure 1****:** Principle of epitope-excision and epitope extraction for mass spectrometric epitope identification. Antibody immunoglobulin with native, disulfide-bonded is generally highly resistant to proteolytic digestion by endoproteases (e.g., trypsin, chymotrypsin, AspN-protease), and the epitope region of antigen polypeptides comprising the epitope-paratope interaction structure is generally protected from proteolytic degradation in the immune complex, while the free nonbinding regions are amenable to digestion. Thus, the epitope sequence remaining bound to the antibody after proteolytic removal and washing away nonbinding structures is then dissociated from the antibody and identified by mass spectrometry. Both electrospray-ionisation (ESI) and matrix-assisted laser desorption-ionisation (MALDI) have been found useful mass spectrometric methods, and have been applied successfully for epitope identifications. "TFA" means trifluoroacetic acid. "MALDI-MS" stands for matrix-assisted laser desorption-ionisation mass spectrometry.
**Figure 2****:** Mass spectrometric identification of proteolytic peptide fragments of free soluble Aβ peptide. Without the complexation by antibody binding, digestion of Aβ(1-40) by trypsin leads to formation of all peptide fragments expected according to the proteolytic cleavage specificity (Aβ(1-5), Aβ(6-16), Aβ(17-28), Aβ(17-40), Aβ(1-16)). Mass spectrometric analysis is performed by high resolution MALDI-Fouriertransform-ion cyclotron resonance (MALDI-FTICR-MS), which provides spectra at approximately 100,000 mass resolution with complete isotope resolution of ions and mass determination accuracies of typically 1-5 ppm. All FTICR-MS spectra were obtained with a Bruker (Bruker Daltonik, Bremen, Germany) Apex II 7T FT-ICR mass spectrometer equipped with an Apollo II electrospray/nanoelectrospray multiport ion source and an external Scout 100 fully-automated X-Y target stage MALDI source with pulsed collision gas. The pulsed nitrogen laser is operated at 337 nm. Ions generated by laser shots were accumulated in the hexapole for 0.5-1 sec at 15 V and extracted at -7 V into the analyzer cell. A 100 mg/ml solution of 2,5-dihydroxybenzoic acid (DHB, Aldrich, Germany) in acetonitrile: 0.1% TFA in water (2:1) was used as the matrix. 0.5 µl of sample solution were mixed on the stainless-steel MALDI sample target and allowed to dry. Typical ESI conditions were ~2 kV needle voltage and 100 nA spray current. Ions were accumulated in a hexapole for 2 sec and then transferred into the cylindrical ICR cell. "ppm" stands for parts per million. "m/z" indicates the mass-to-charge-ratio.

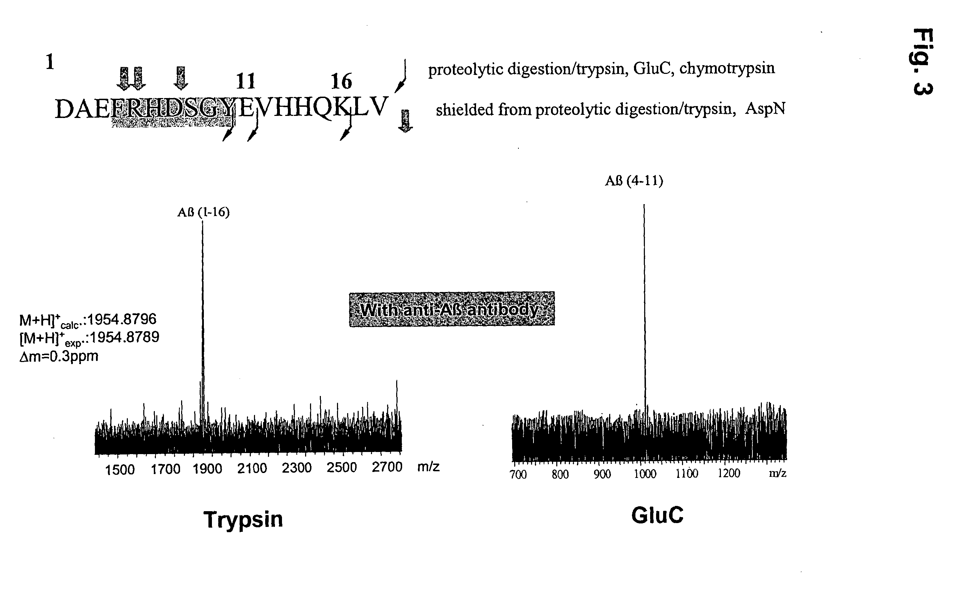

**Figure 3****:** Epitope Identification of Amyloid Plaque Specific Antibody by MALDI-FTICR-MS: Mass spectrometric identification of N-terminal Aβ-epitope recognised by plaque-specific antibody produced upon active immunisation of transgenic mice with Aβ(1-42) or Aβ(1-42)-derived aggregates. The immobilised, purified antibody was incubated with Aβ(1-40), Aβ(1-42), and the immune complex subjected to epitope excision by proteases trypsin, chymotrypsin, Glu-C protease and AspN-protease. The left spectrum shows the fragment, Aβ(1-16) remaining bound after trypsin digestion, the right spectrum shows Aβ(1-11) after epitope excision using GluC-protease. Black small arrows in the Aβ sequence shown denote cleavages identified by epitope excision, fat grey arrows denote substrate cleavage sites on Aβ that were found shielded upon antibody binding. Identical Aβ(4-10) epitope sequences were identified with soluble Aβ-plaques and -protofibrils bound as antigens, and from a mouse anti-Aβ(1-16) peptide monoclonal antibody (Bachem-Peninsula Laboratories, San Francisco).
**Figure 4****:** Toxicity of Aβ-oligomers for human neuroblastoma cells (SH-Sy5y) in absence or presence of Aβ autoantibody. OD: optical density.

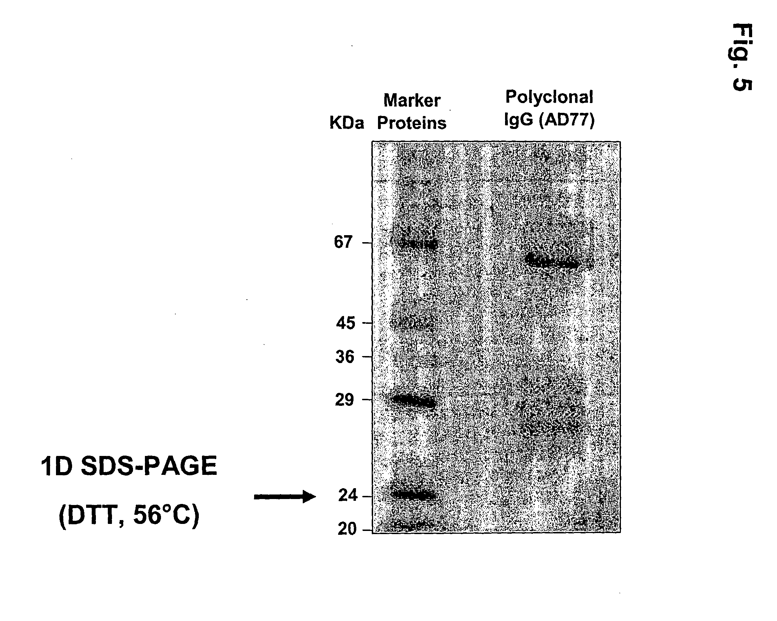

**Figure 5****:** 1D-Gel electrophoretic separation of polyclonal serum Aβ-autoantibody from an AD patient (AD77), isolated by Aβ(12-40) epitope-specific affinity chromatography. KDa: Molecular Weight in Kilodalton. IgG: Immunoglobulin G. DTT: Dithiothreitol.

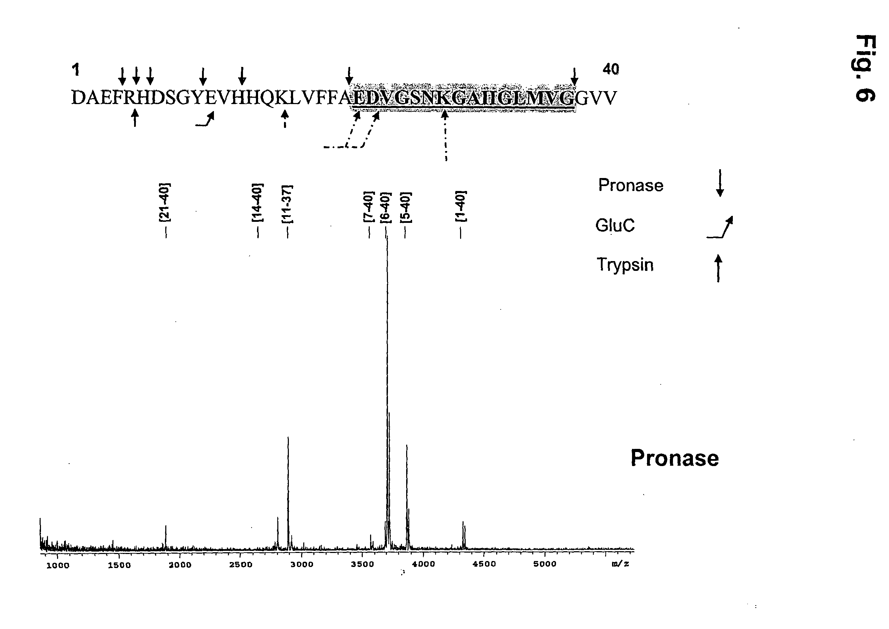

**Figure 6****:** Identification of Aβ (21-37) as the epitope recognised by human Aβ-autoantibodies by epitope excision-mass spectrometry. The upper graph shows the sequence of Aβ(1-40), with cleavages by different proteases indicated by black arrows. Peptide fragments denoted by solid black arrows above the Aβ-sequence were identified after epitope excision using pronase; peptide fragments denoted by black dotted arrows underneath the Aβ sequence were found by epitope excision using trypsin and GluC-protease (R5, E11, K16); note that Arg-5 is completely shielded in the immune complex with the plaque-specific antibody, while completely amenable to cleavage in the immune complex with the Aβ-autoantibody. Cleavage positions, observed in free Aβ, indicated by broken arrows were found shielded after binding of Aβ-autoantibody (GluC: E22, D23; trypsin: K28). The MALDI-MS analysis upon partial digestion (2 hrs) with pronase is shown here for illustration.

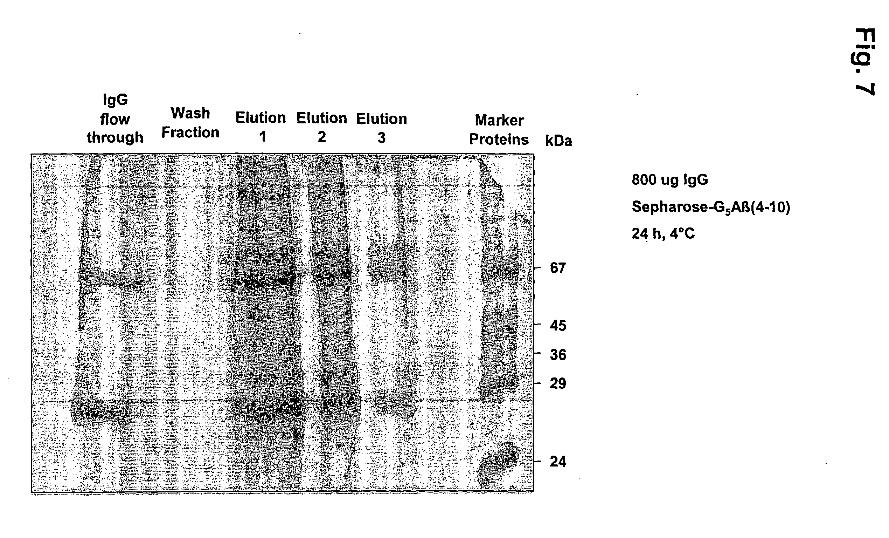

**Figure 7****:** Isolation of "plaque-specific" antibodies recognising the N-terminal Aβ(4-10) epitope from the serum Aβ-autoantibodies of an Alzheimer patient, isolated by Aβ(4-10)-epitope specific chromatography. IgG stands for immunoglobulin G. AD signifies Alzheimer's Disease. Affinity column: G5Aβ(4-10). kDa: Molecular weight in kilodalton.

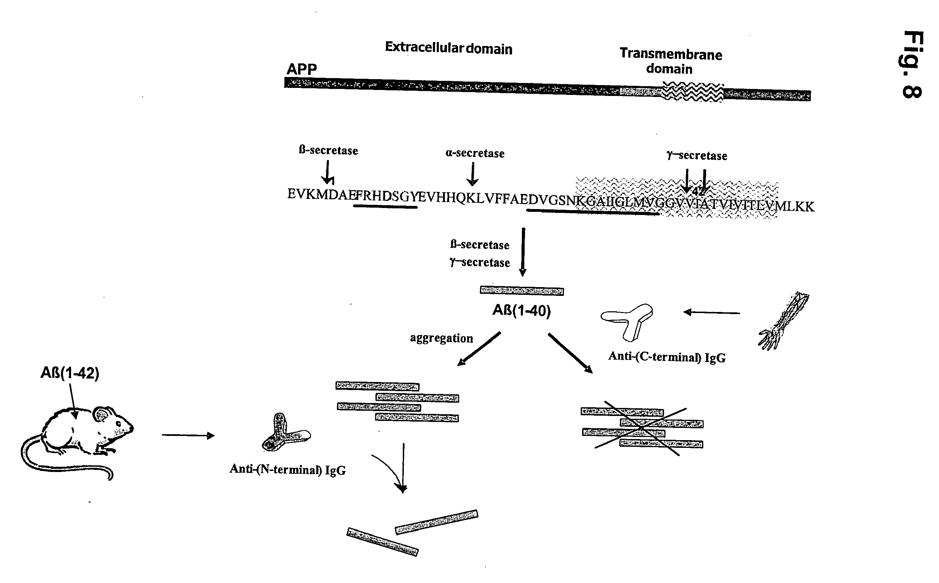

**Figure 8****:** Molecular recognition mechanism of plaque-specific, plaque-disaggregating Aβ-antibodies recognising the Aβ(4-10) epitope, and the Aβ-autoantibodies recognising the Aβ(21-3) carboxyterminal epitope.

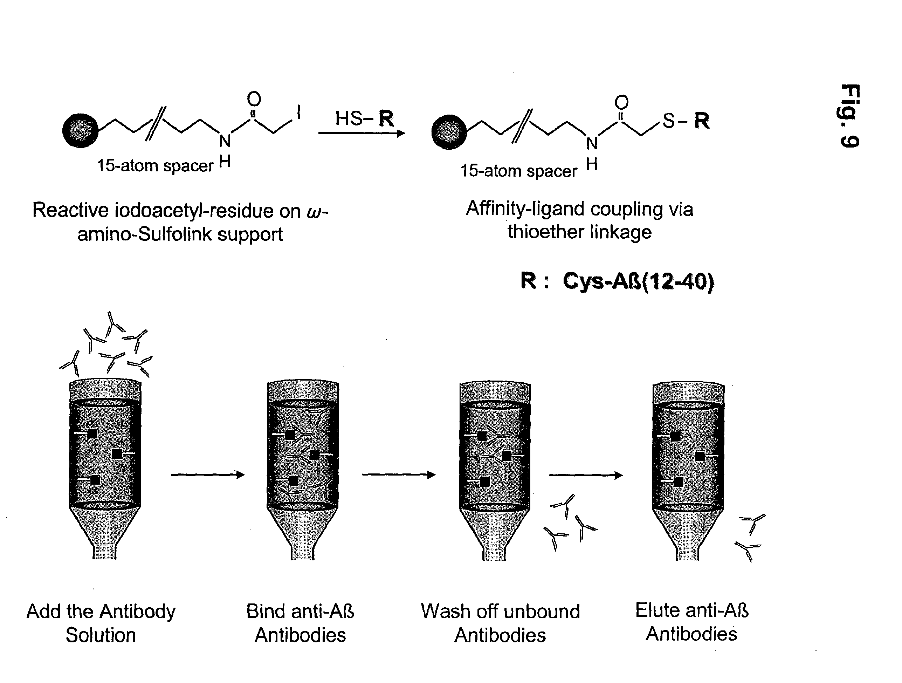

**Figure 9****:** Structure of Aβ(12-40) epitope-specific affinity column and experimental procedure for isolation of Aβ-autoantibodies. Cys-Aβ(12-40): Cysteine coupled Aβ(12-40) peptide.
**Figure 10****:** Analytical scheme and experimental procedures employed for sequence determination of affinity- isolated Aβ-autoantibodies: N-terminal protein sequence analysis; 2D-electrophoretic separation, in-gel proteolytic digestion and high resolution FTICR-MS identification of constant region sequences; proteolytic digestion and HPLC separation of peptide fragments, followed by a) Edman sequence determinations; b) LC-MS/MS sequence determination; c) MALDI-TOFMS identification of constant region partial sequences; MALDI-FTICR-MS identification of constant/variable partial sequences.

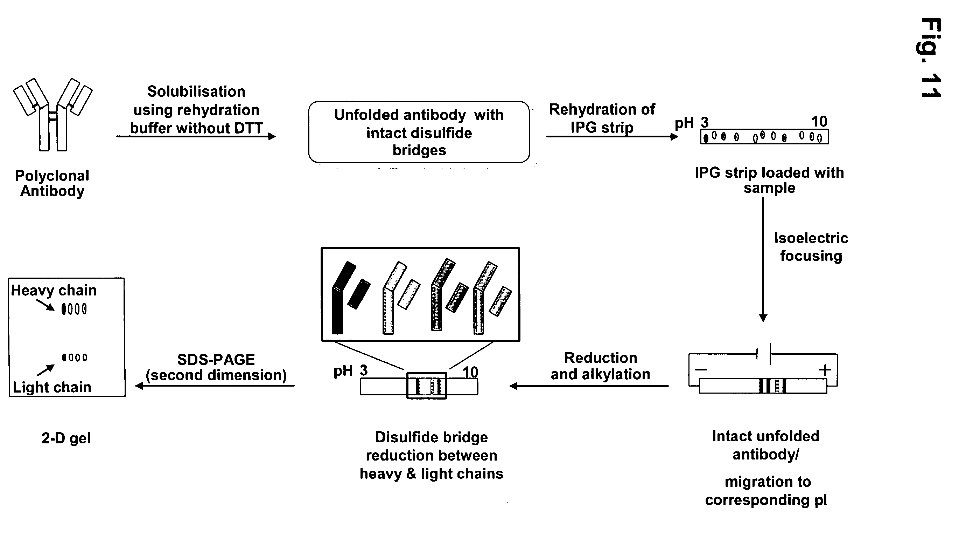

**Figure 11****:** Analytical scheme of experimental procedure employed for assignment of heavy-and light chain sequence pairs of serum-IVIgG Aβ-autoantibodies.

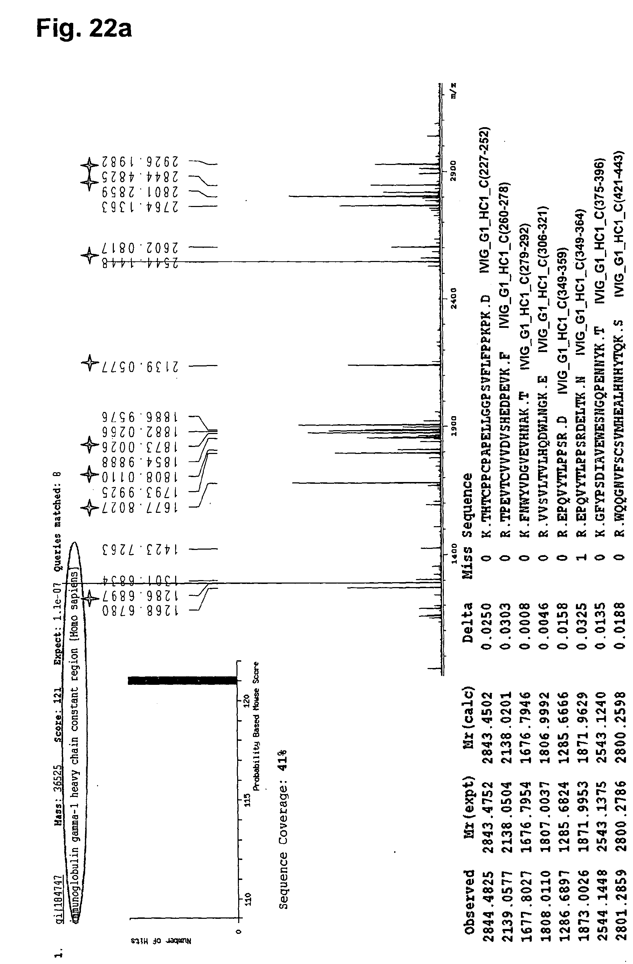

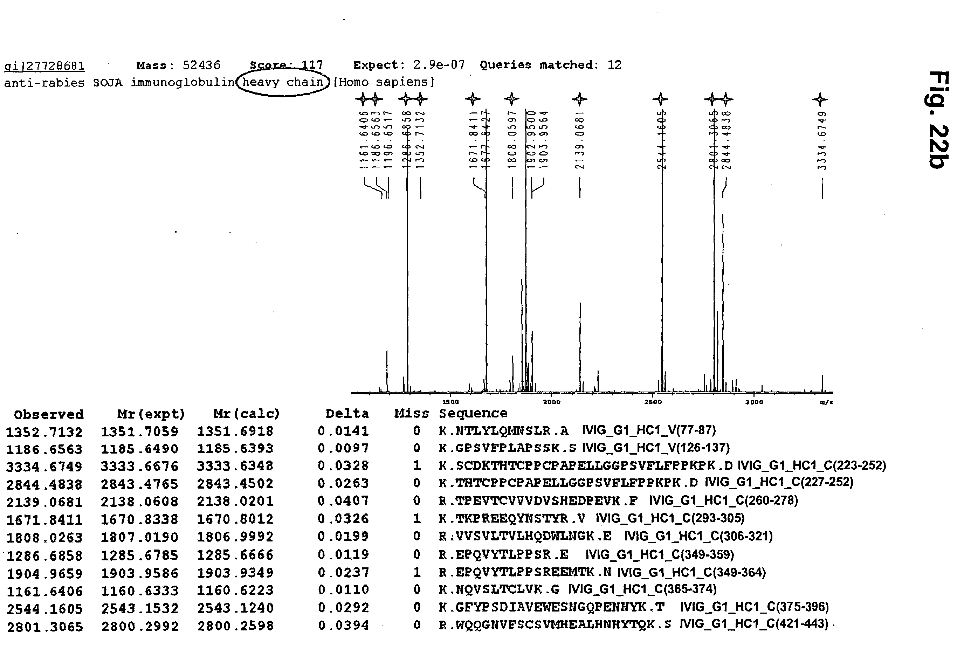

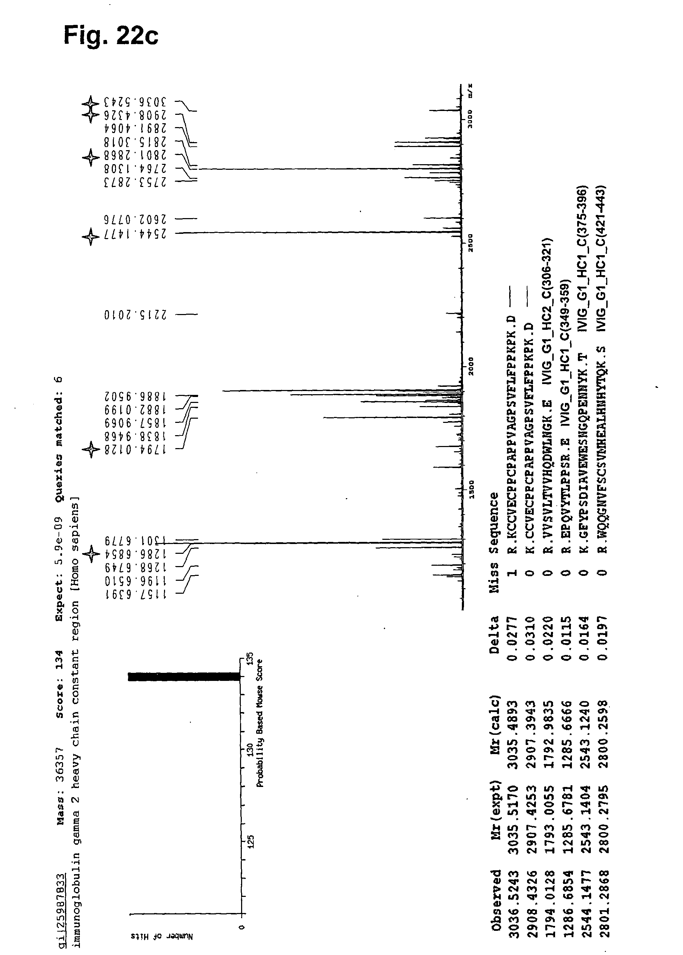

**Figure 12****:** 2-Dimensional SDS-gel electrophoretic separation of polyclonal Aβ-autoantibodies isolated from IVIgG; see Figure 22 (a-c) for identification and sequence determination of Aβ-antibody isoforms. DTT: Dithiotreitol. CHAPS: 3-[(3-Cholamidopropyl)-dimethyl-ammonio]- 1 -propanesulfonate.
**Figure 13****:** 1D-SDS-PAGE isolation of heavy and light chains of serum IVIgG Aβ-autoantibody. a) Reduction (10000x DTT); b) Alkylation (3x iodoacetamide/ DTT). LMW: low molecular weight protein standard.

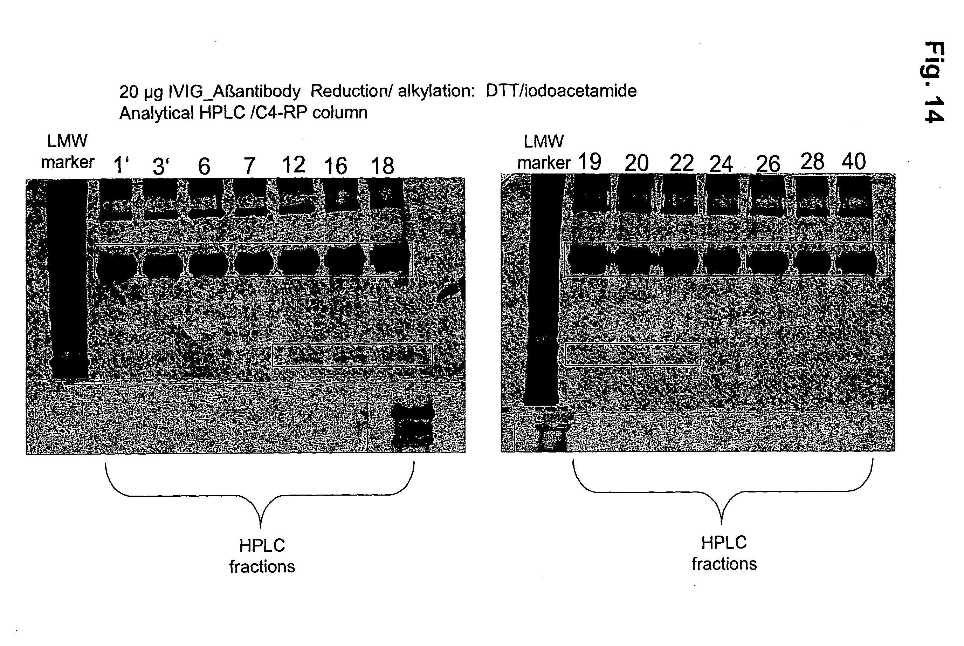

**Figure 14****:** 1D-Gel electrophoretic separation of HPLC-isolated heavy and light chains of Aβ-autoantibodies (serum-IVIgG) used for Edman sequence determinations. LMW: low molecular weight protein standard.

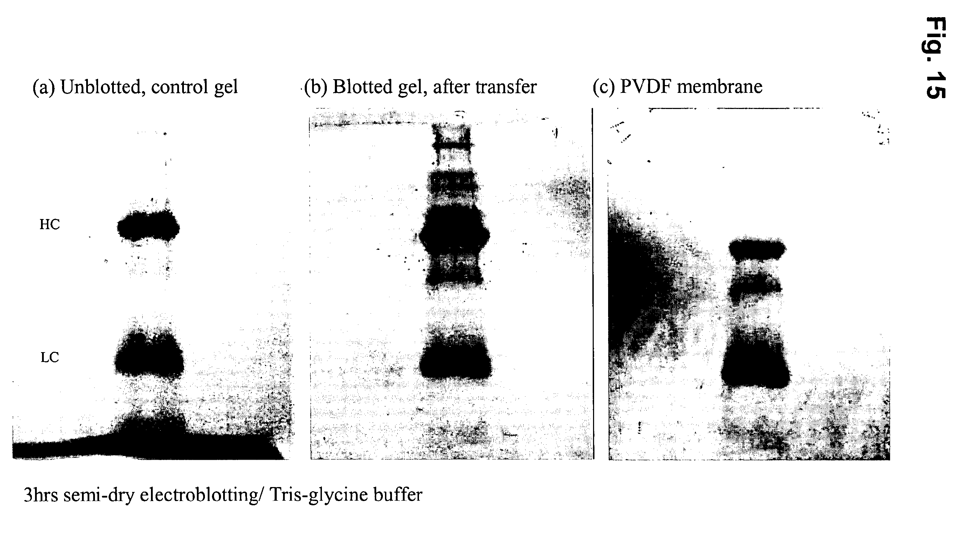

**Figure 15****:** 1D-Gel electrophoretic separation and blotting of serum IVIgG Aβ-autoantibody heavy and light chains on PVDF membranes for Edman sequence determination.

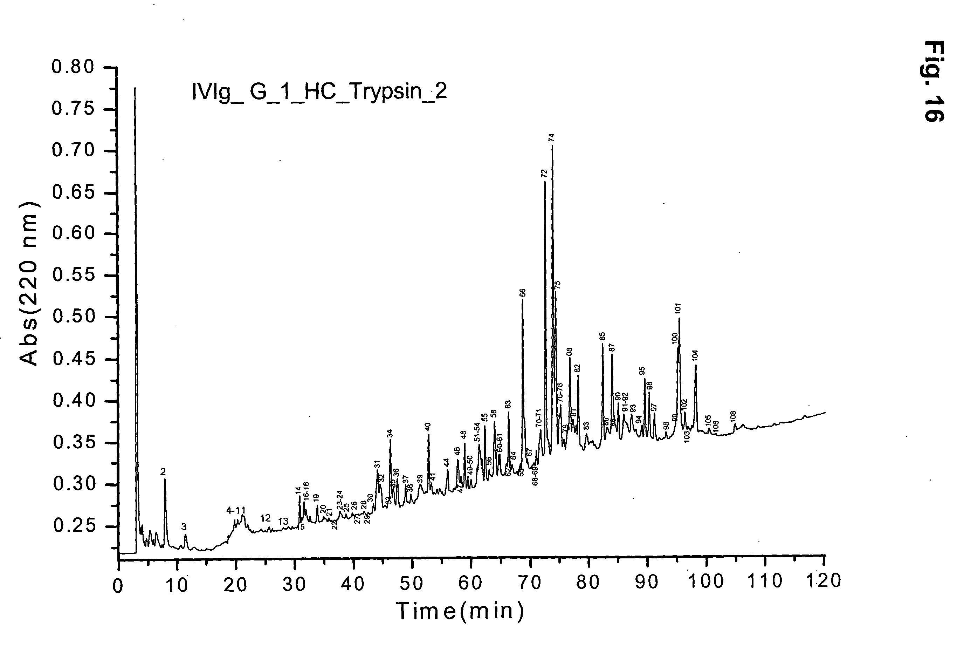

**Figure 16****:** HPLC separation of heavy chain tryptic peptides. Isolated peptide fractions were subjected to a) Edman sequence analysis, b) LC-MS/MS sequence determination, c), d) direct MALDI-TOF-MS and MALDI-FTICR-MS analysis.

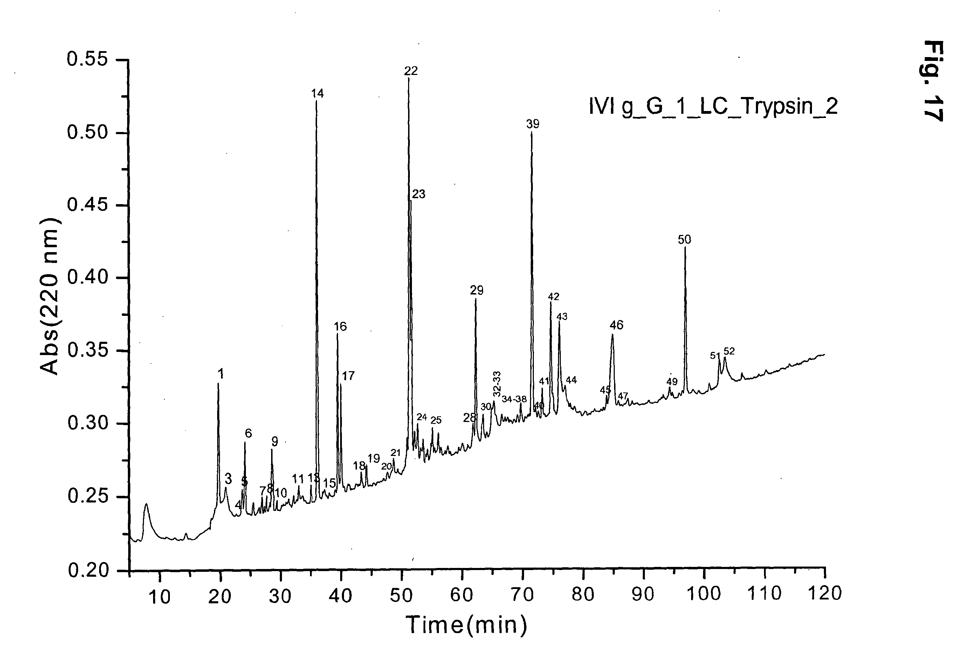

**Figure 17****:** HPLC separation of light- chain tryptic peptides. Isolated peptide fractions were subjected to a) Edman sequence analysis, b) LC-MS/MS sequence determination, c), d) direct MALDI-TOF-MS and MALDI-FTICR-MS analysis.

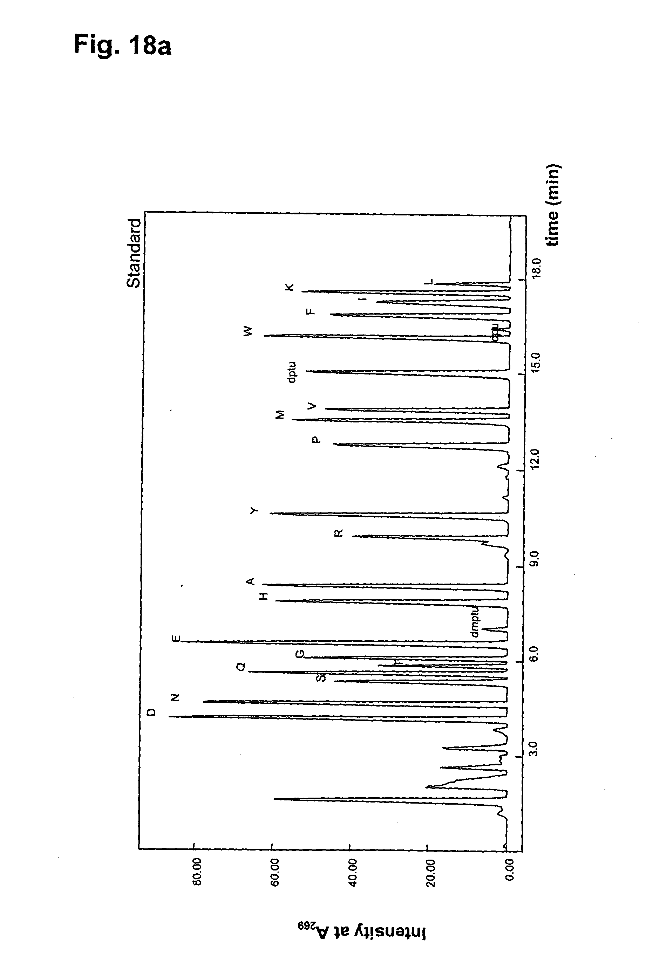

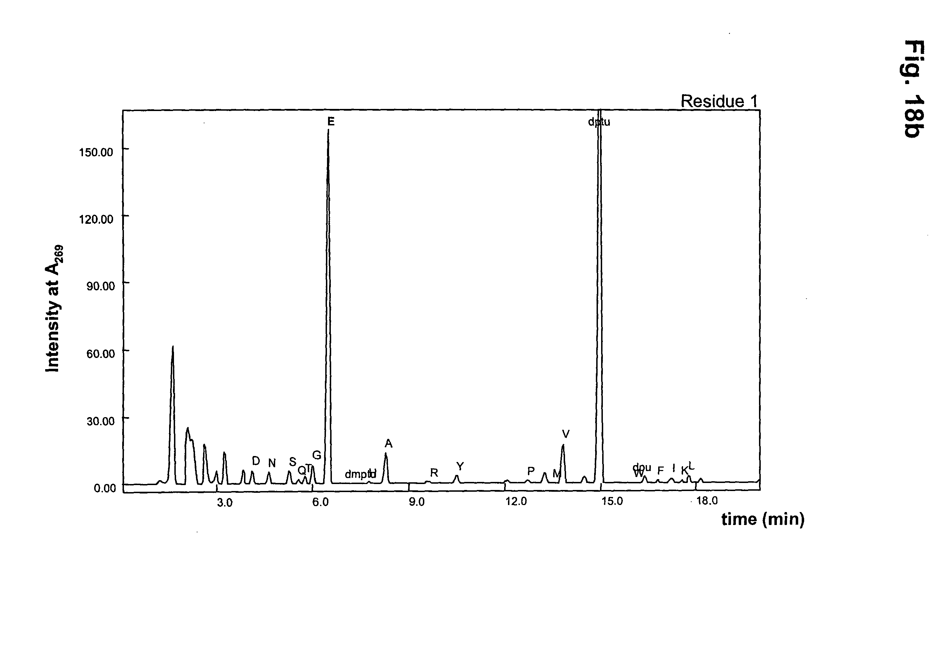

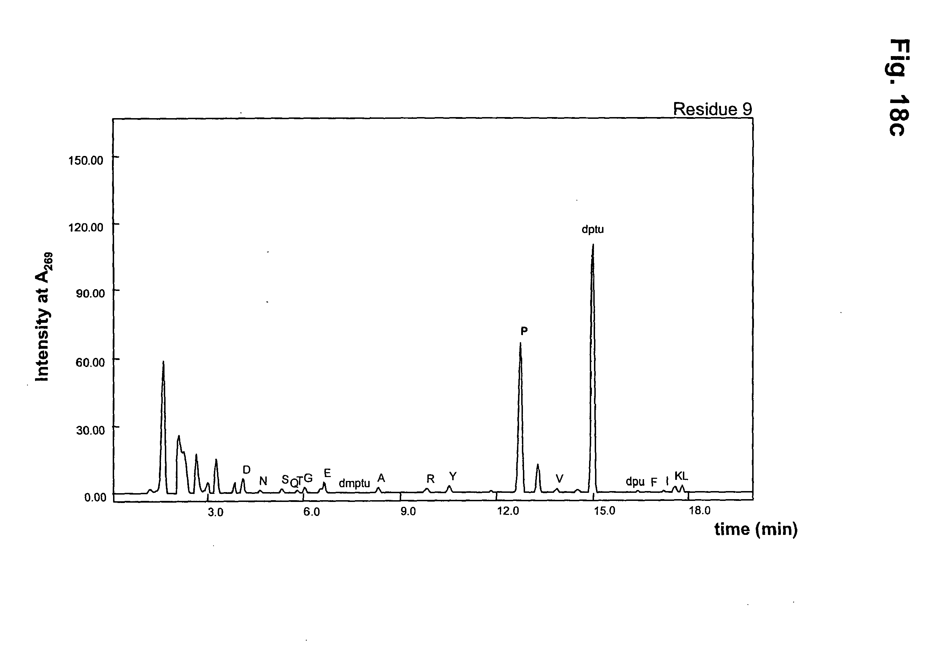

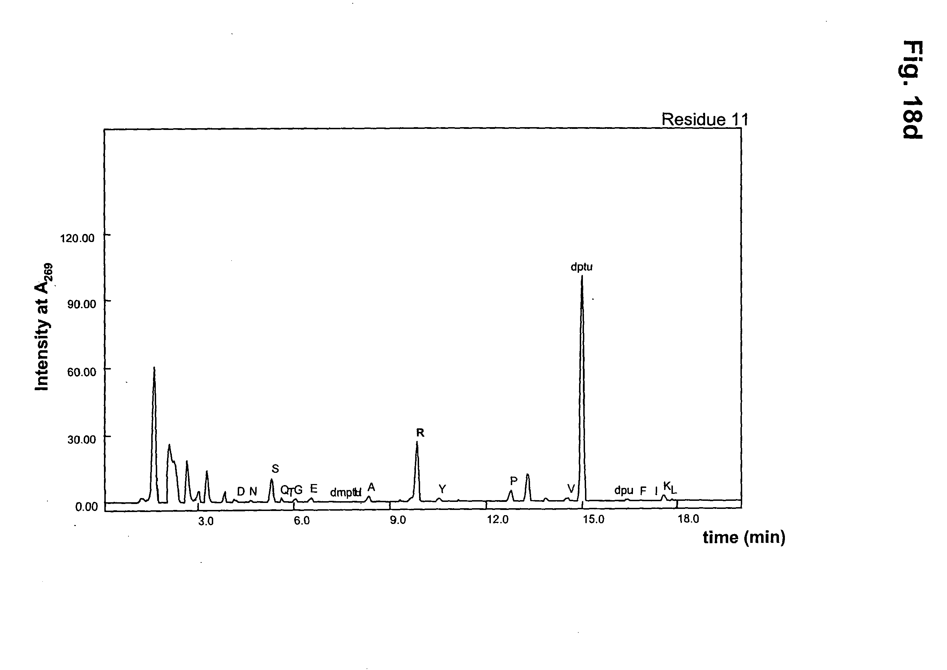

**Figure 18** **(a-d):** Edman sequence determination of HPLC-isolated heavy-chain tryptic peptide, Serum_IVIG_Gl_HC(1)_1 c(348-359; EPQVYTLPPSR). 18a: Standard. 18b: Residue 1. 18c: Residue 9. 18d: Residue 11.

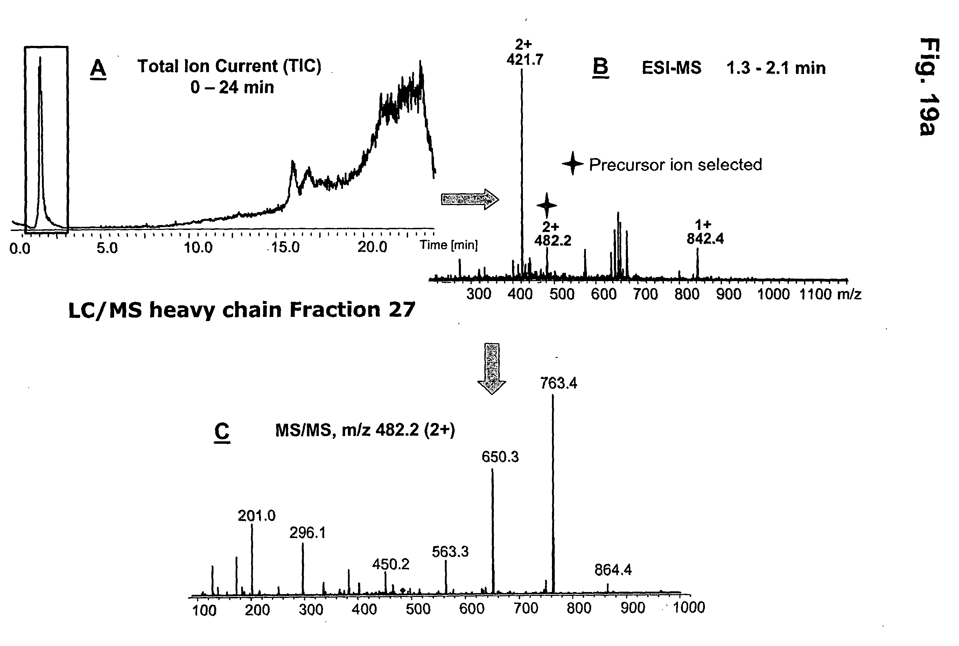

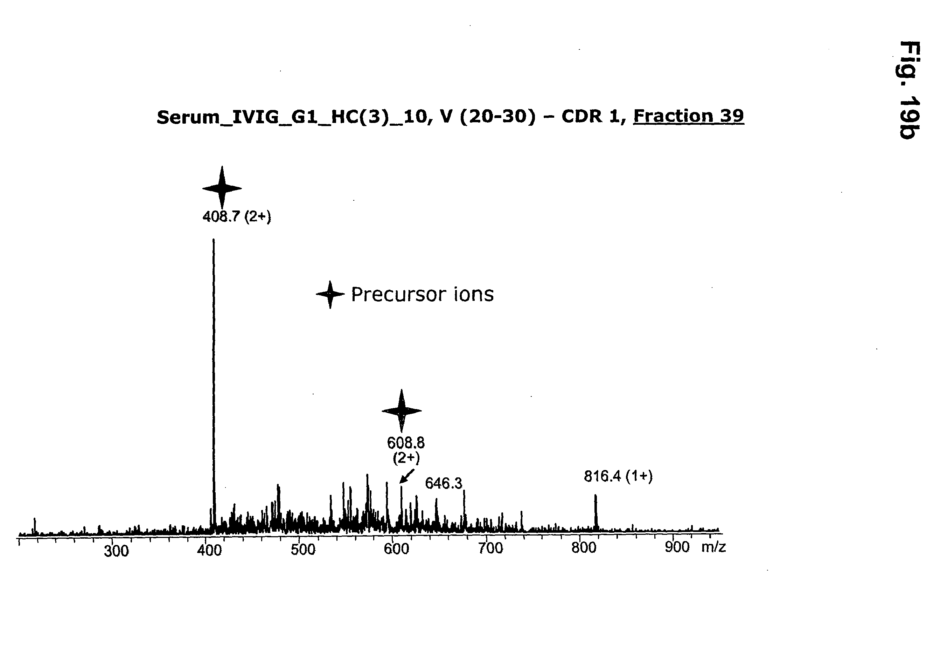

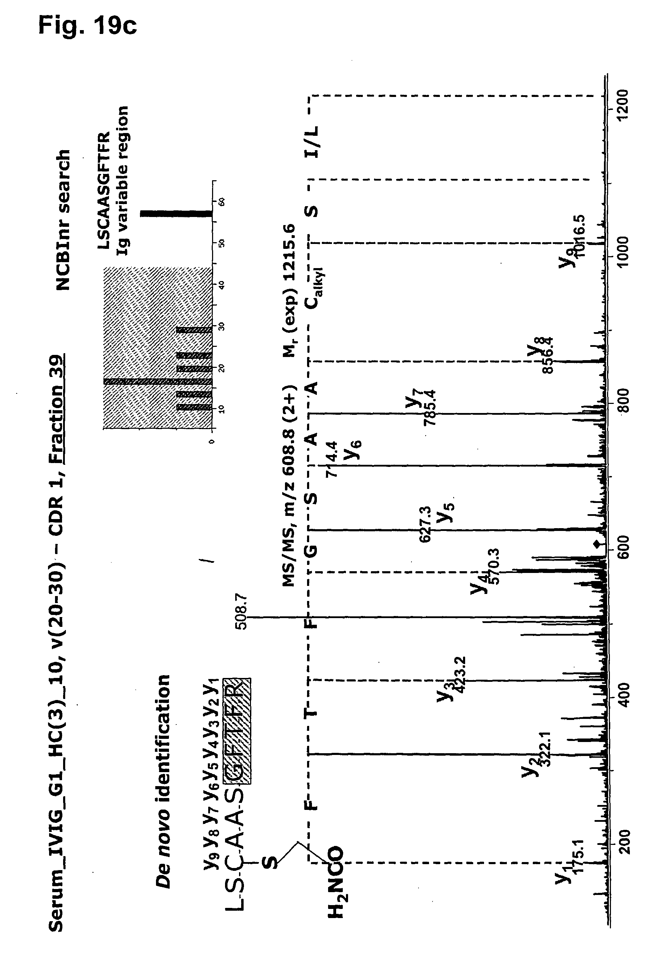

**Figure 19** **(a-c):** LC-MS/MS sequence determination of heavy chain tryptic HPLC peptides, fraction 27 (a) and HPLC fraction 39, heavy chain CDR1 peptide v(20-30). Fig. 19a shows the total ion chromatogram, the peptide fraction isolated at 1.3 - 2.1 min elution time is encircled in red. (b) ESI- mass spectrum of the peptide fraction isolated at 1.3 - 2.1 min; (c) MS/MS fragment ion analysis of the doubly charged precursor ion selected, m/z 482.2.

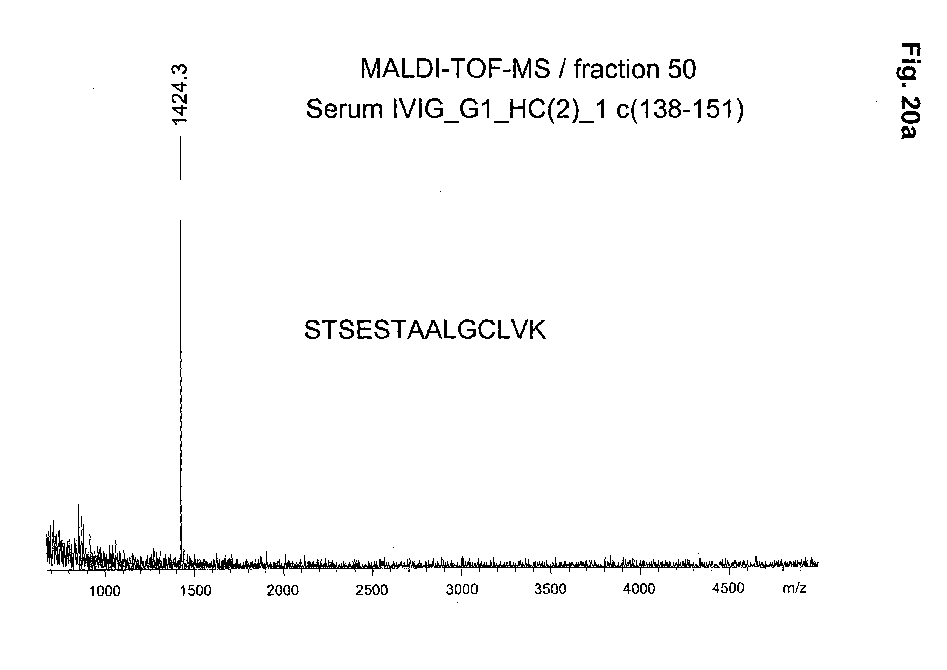

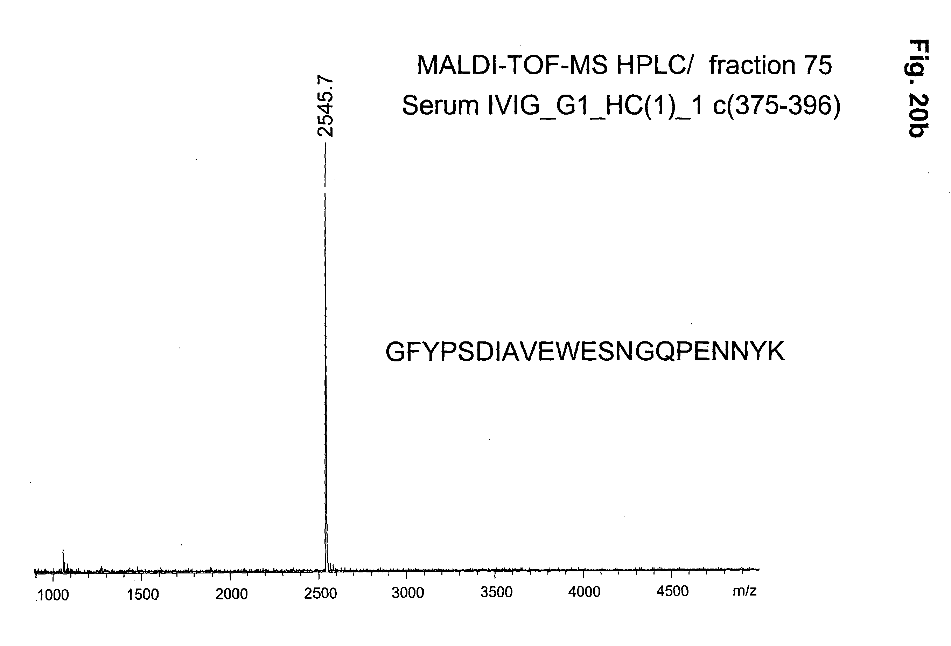

**Figure 20** **(a, b):** MALDI-TOF-MS Identification of tryptic HPLC peptides. a) identification of tryptic peptide, fraction 50, heavy chain (138-151), mol. mass 1423; b) identification of peptide isolated in fraction 75, heavy chain (375-396), mol. mass 2544 Da.

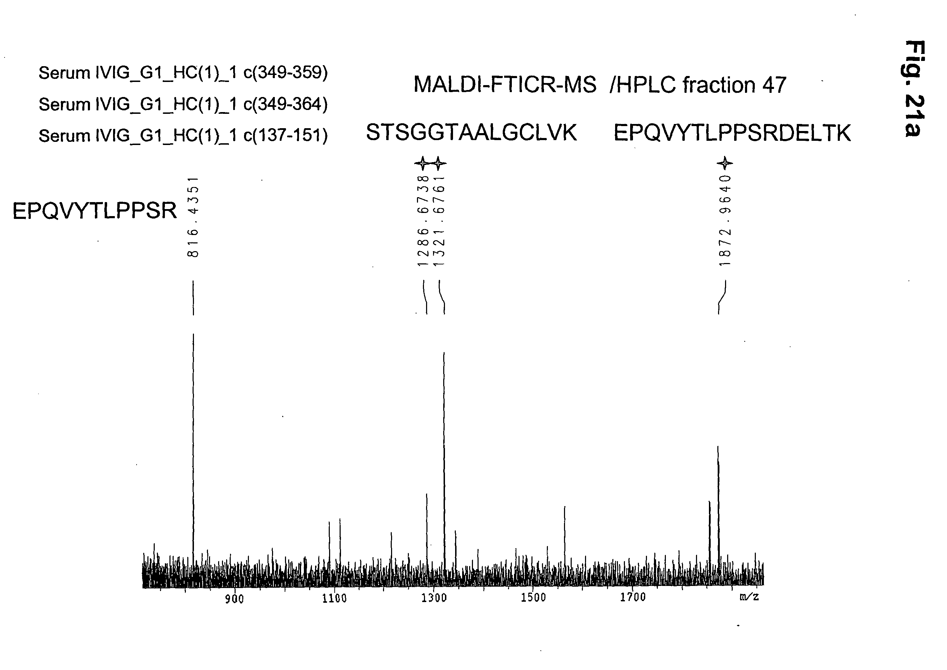

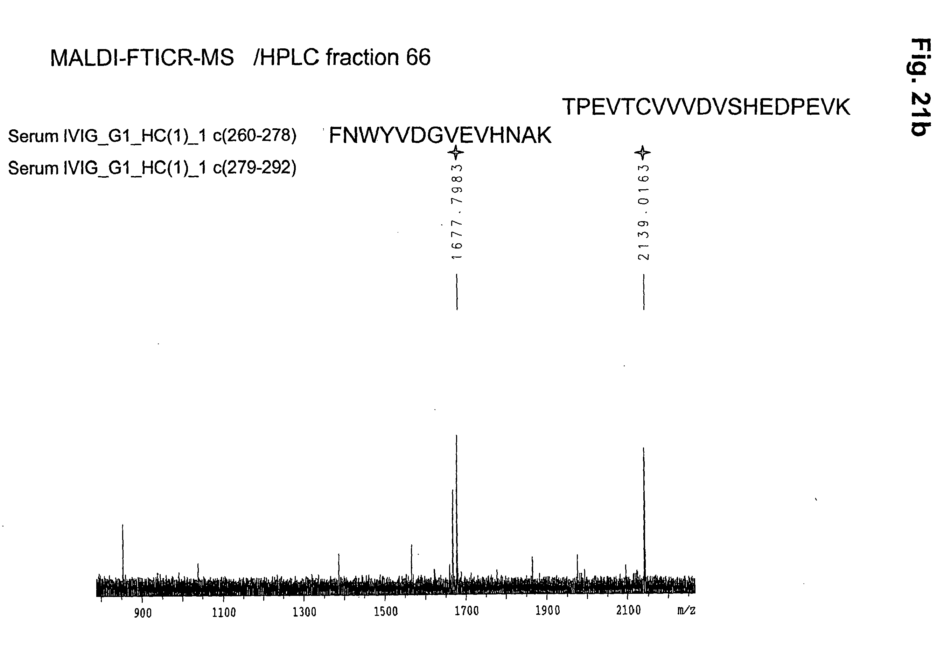

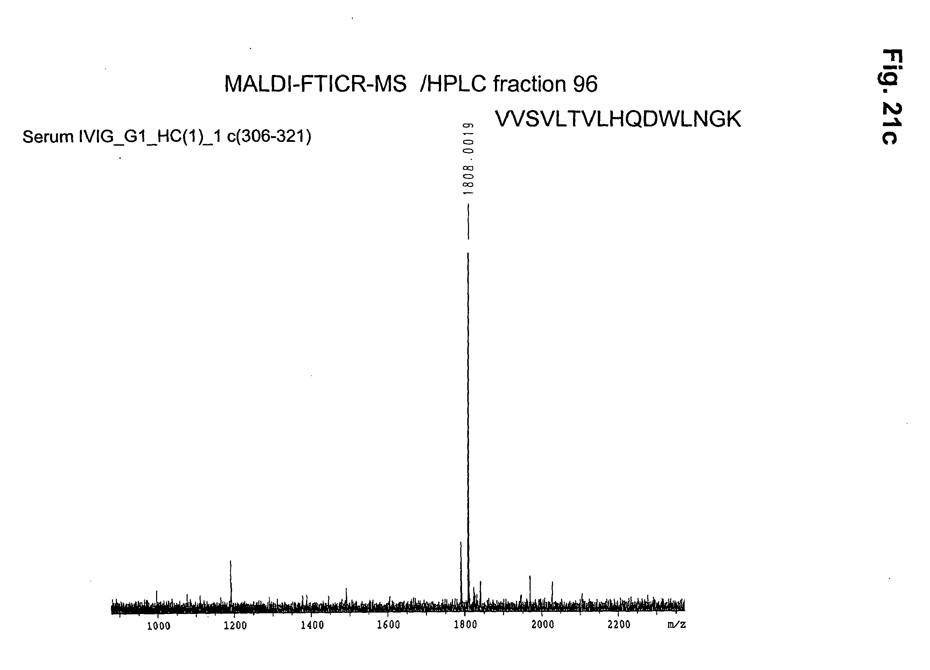

**Figure 21** **(a-c):** MALDI-FTICR-Mass spectrometric identification of heavy chain constant region tryptic peptides from HPLC fractions 47, 66, and 96. (a) identification of 3 peptides isolated in fraction 47 denoted on the molecular ion peaks, (349-359); (349-364), (137-151); (b) identification of 2 peptides in fraction 66, (260-278) and 279-292); (c) identification of peptide (306-321) in fraction 96.
**Figure 22** **(a-c):** MALDI-FT-ICR Identification of Sequences comprising the serum IVIgG1 heavy chain constant regions, isolated from 2D-gel bands subjected to in-gel tryptic digestion; spot 4 heavy chain (22a), spot 12 heavy chain (22b) and spot 13 heavy chain (22c) as illustrated in Figure 12. Sequence determinations were performed using the NCBI data base, at a mass accuracy threshold of 5-10 ppm.

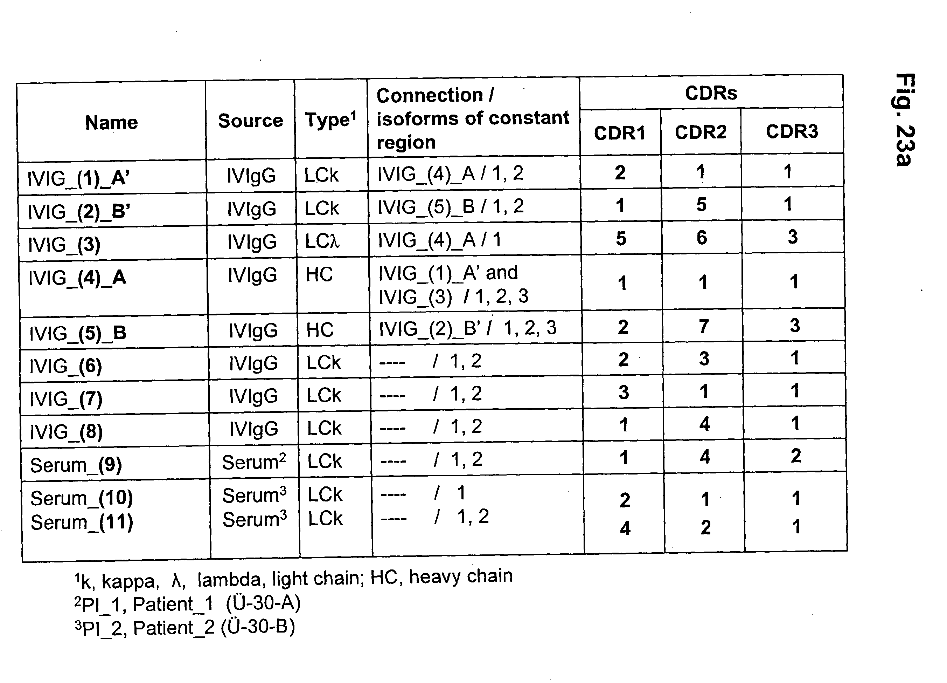

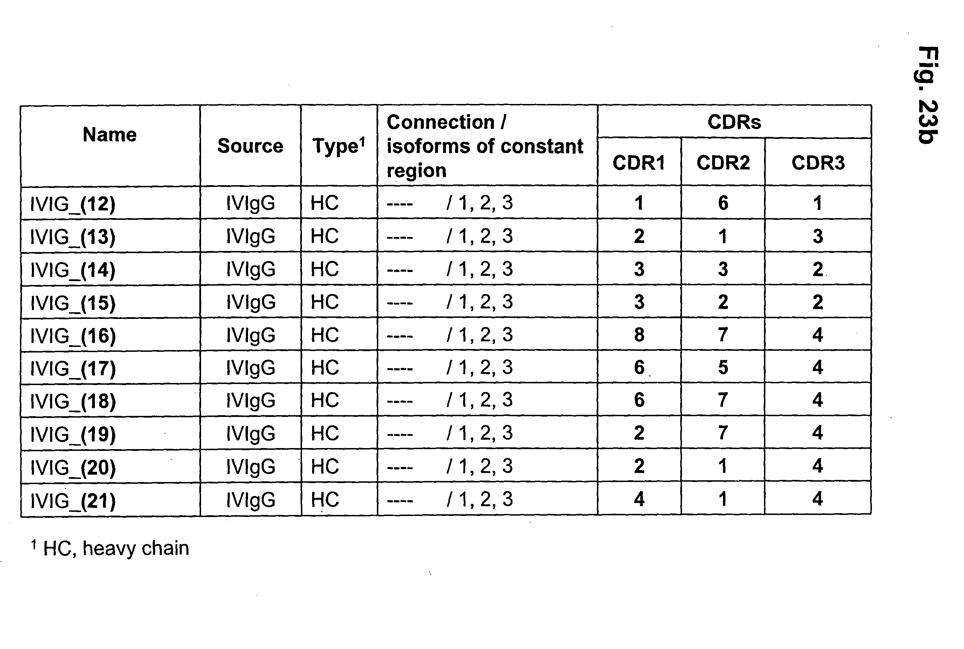

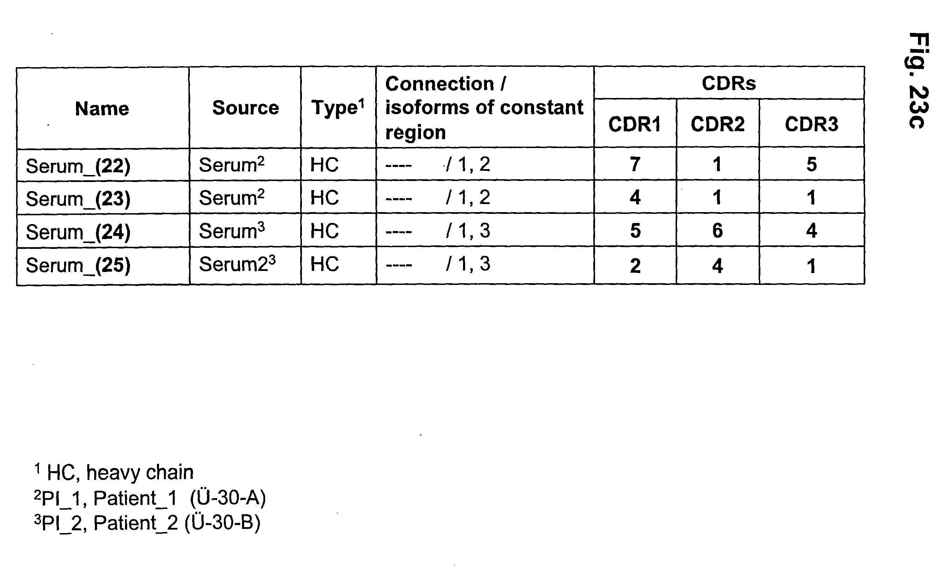

**Figure 23a** **to c:** Table giving an overview about the identified and sequenced antibodies specific for the C-terminal half of Aβ-peptide, in particular specific for Aβ(21-37). The table provides the name of the antibody chain sample, the source of the sample, the type of immunoglobulin chain sequenced, verified interactions of light and heavy chains (indicated is the name of the partner chain as connection) as well as confirmed isoforms of the respective immunoglobulin chain and the type of CDR sequence identified for CDR1, CDR2 and CDR3. Fig. 23a: IVIG_(1)_A'; IVIG_(2)_B'; IVIG_(3); IVIG_(4)_A; IVIG_(5)_B; IVIG_(6); IVIG_(7); IVIG_(8); Serum_(9); Serum_(10) ; Serum_(11); Fig. 23b: IVIG_(12); IVIG_(13); IVIG_(14); IVIG_(15); IVIG_(16); IVIG_(17); IVIG_(18); IVIG_(19); IVIG_(20); IVIG_(21). Fig. 23c: Serum_(22); Serum_(23); Serum_(24); Serum_(25).
**Figure 24a** **to d:** Table indicating the identified CDR types and corresponding consensus sequences for all sequenced antibody chains. The CDR numbers correspond with the numbering as used in Fig. 23 for CDRs. Fig. 24a CDRs of heavy chains; Fig 24b: CDRs of light chains (lambda chain as well as kappa chain); Fig. 24c: CDR consensus sequences for CDR1, CDR2 and CDR3 of the heavy chains; Fig. 24d: CDR consensus sequences for CDR1, CDR2 and CDR3 of the kappa light chain.
**Figure 25** **a to i:** Amino acid sequences of light chain variable region sequences of Aβ-autoantibodies from serum-IVIgG and individual serum autoantibodies (see Fig. 23 for sequence overview). Annotation of codes for sequencing methods employed (Edman; Edman-protein N-terminal; MALDI-TOF-MS; MALDI-FTICR-MS, LC-MS/MS), and of CDR sequences is indicated on the bottom of each sequence. CDRs are indicated by boxes.
   Fig. 25a: amino acid sequence of light chain kappa variable region of sample IVIG_(1)_A' (SEQ ID NO:47).
   Fig. 25b: amino acid sequence of light chain kappa variable region of sample IVIG_(2)_B' (SEQ ID NO:48).
   Fig. 25c: amino acid sequence of light chain lambda variable region of sample IVIG_(3) (SEQ ID NO:49).
   Fig. 25d: amino acid sequence of light chain kappa variable region of sample IVIG_(6) (SEQ ID NO:50).
   Fig. 25e: amino acid sequence of light chain kappa variable region of sample IVIG_(7) (SEQ ID NO:51).
   Fig. 25f: amino acid sequence of light chain kappa variable region of sample IVIG_(8) (SEQ ID NO: 52).
   Fig. 25g: amino acid sequence of light chain kappa variable region of sample Serum_(9) (SEQ ID NO:53).
   Fig. 25h: amino acid sequence of light chain kappa variable region of sample Serum_(10) (SEQ ID NO:54).
   Fig. 25i: amino acid sequence of light chain kappa variable region of sample Serum_(11) (SEQ ID NO:55).
**Figure 26** **a to p:** Amino acid sequences of heavy chain variable region sequences of Aβ-autoantibodies from serum-IVIgG and individual serum autoantibodies (see Fig. 23, sequence overview). Annotation of codes for sequencing methods employed (Edman; Edman-protein N-terminal; MALDI-TOF-MS; MALDI-FTICR-MS, LC-MS/MS), and of CDR sequences is indicated on the bottom of each sequence. CDRs are indicated by boxes.
   Fig. 26a: amino acid sequence of heavy chain variable region of sample IVIG_(4)_A (SEQ ID NO:56).
   Fig. 26b: amino acid sequence of heavy chain variable region of sample IVIG_(5)_B (SEQ ID NO:57).
   Fig. 26c: amino acid sequence of heavy chain variable region of sample IVIG_(12) (SEQ ID NO:58).
   Fig. 26d: amino acid sequence of heavy chain variable region of sample IVIG_(13) (SEQ ID NO:59).
   Fig. 26e: amino acid sequence of heavy chain variable region of sample IVIG_(14) (SEQ ID NO:60).
   Fig. 26f: amino acid sequence of heavy chain variable region of sample IVIG_(15) (SEQ ID NO:61).
   Fig. 26g: amino acid sequence of heavy chain variable region of sample IVIG_(16) (SEQ ID NO:62).
   Fig. 26h: amino acid sequence of heavy chain variable region of sample IVIG_(17) (SEQ ID NO:63).
   Fig. 26i: amino acid sequence of heavy chain variable region of sample IVIG_(18) (SEQ ID NO:64).
   Fig. 26j: amino acid sequence of heavy chain variable region of sample IVIG_(19) (SEQ ID NO:65).
   Fig. 26k: amino acid sequence of heavy chain variable region of sample IVIG (20) (SEQ ID NO:66).
   Fig. 26l: amino acid sequence of heavy chain variable region of sample IVIG_(21) (SEQ ID NO:67).
   Fig. 26m: amino acid sequence of heavy chain variable region of sample Serum_(22) (SEQ ID NO: 68).
   Fig. 26n: amino acid sequence of heavy chain variable region of sample Serum_(23) (SEQ ID NO:69).
   Fig. 26o: amino acid sequence of heavy chain variable region of sample Serum_(24) (SEQ ID NO:70).
   Fig. 26p: amino acid sequence of heavy chain variable region of sample Serum_(25) (SEQ ID NO:71).
**Figure 27a** **to c:** Amino acid sequences of the constant region of kappa and lambda light chains of Aβ-autoantibody chains. The mutation of the LCkappa-constant region sequence at V192L is indicated in bold letters.

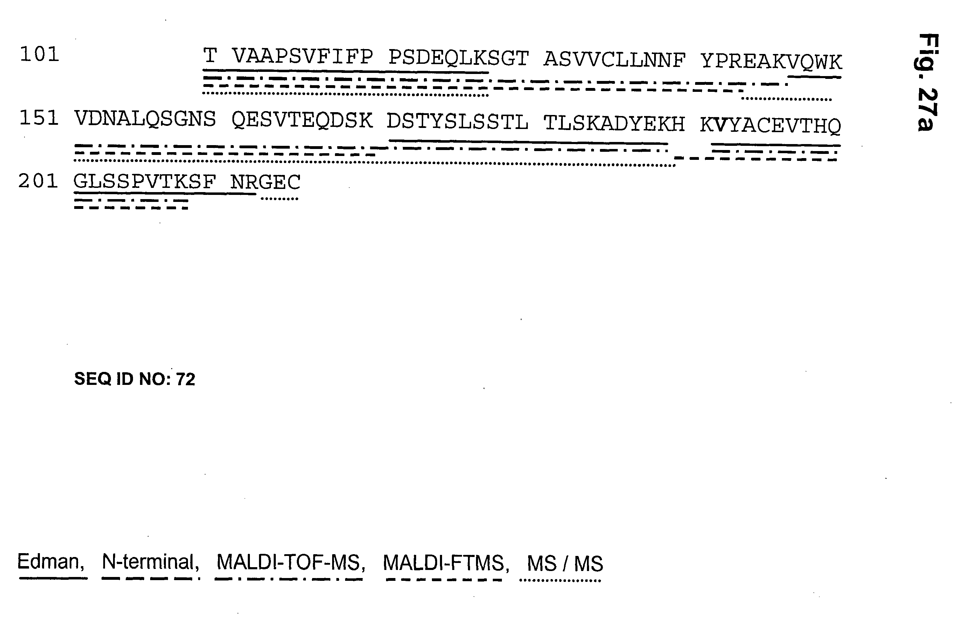

   Fig. 27a: Complete amino acid sequence of constant region light chain kappa isoform 1 (SEQ ID NO:72).

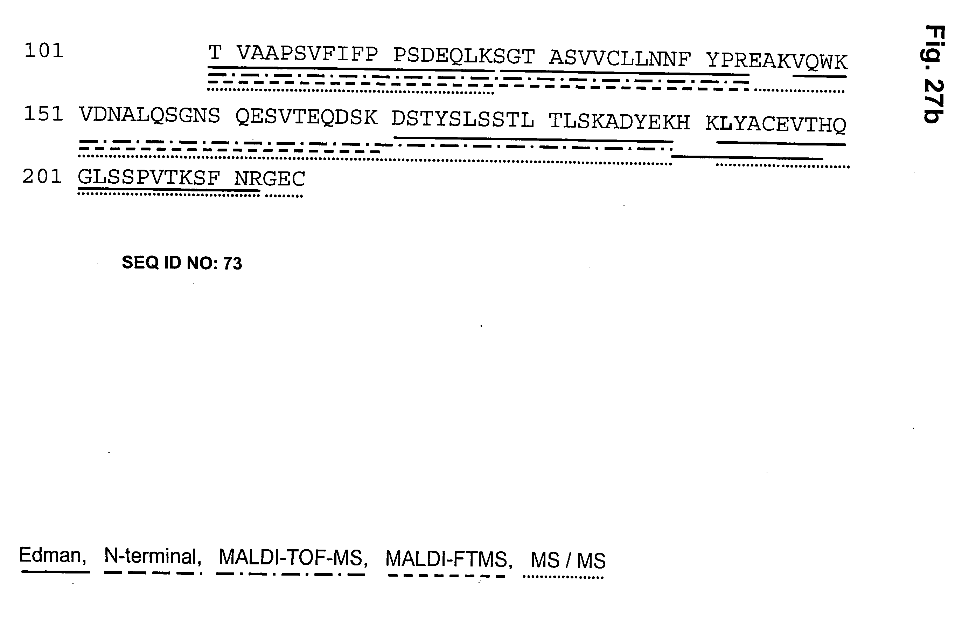

   Fig. 27b: Complete amino acid sequence of constant region light chain kappa of isoform 2 (SEQ ID NO:73).

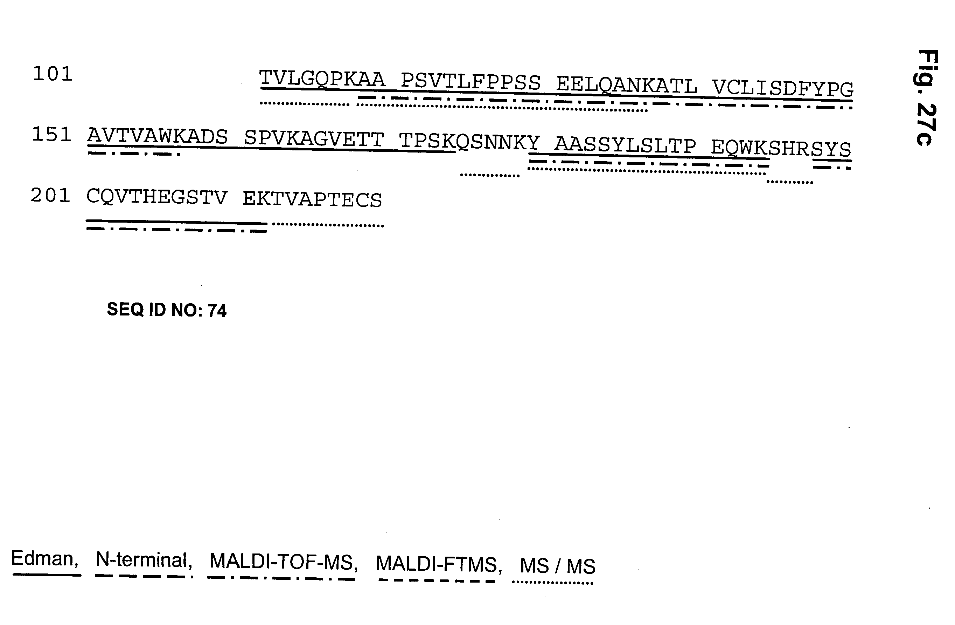

   Fig. 27c: Complete amino acid sequence of constant region light chain lambda (SEQ ID NO:74).

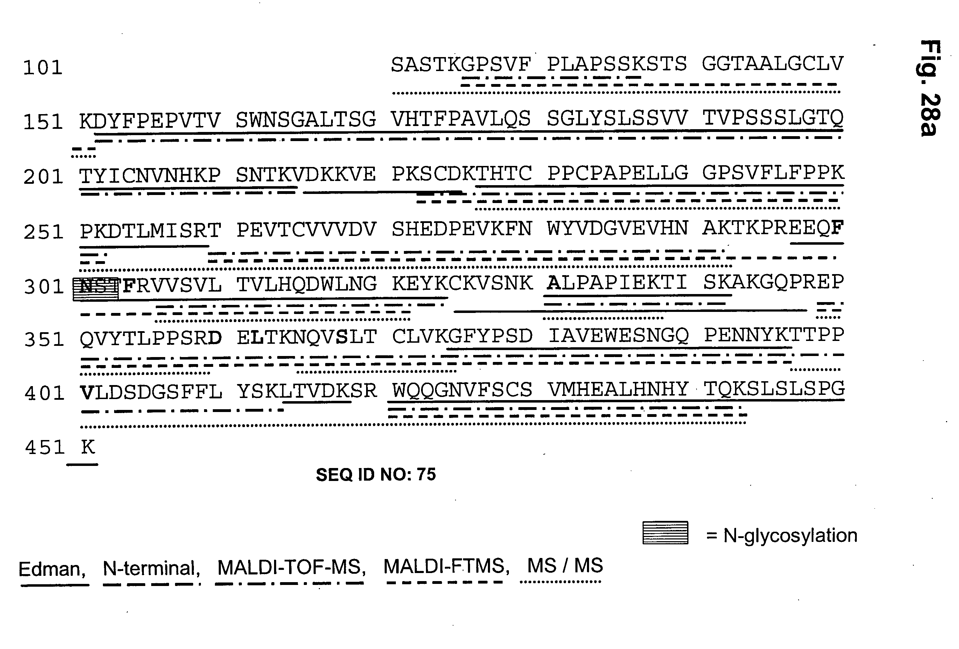

**Figure 28a** **to c:** Amino acid sequences and sequence isoforms of the constant region identified for IVIgG- Aβ-autoantibody heavy chains. Amino acid mutations identified at F300Y, N301A (N-glycosylation site), F304Y, G331A, D360E, L362M, S368T, and V401M are indicated by bold letters. The N-glycosylation consensus sequence and site at, N-301^{ST}, are indicated by shaded box and bold grey letter.
   Fig. 28a: Complete amino acid sequence of constant region heavy chain of isoform 1 (SEQ ID NO:75).

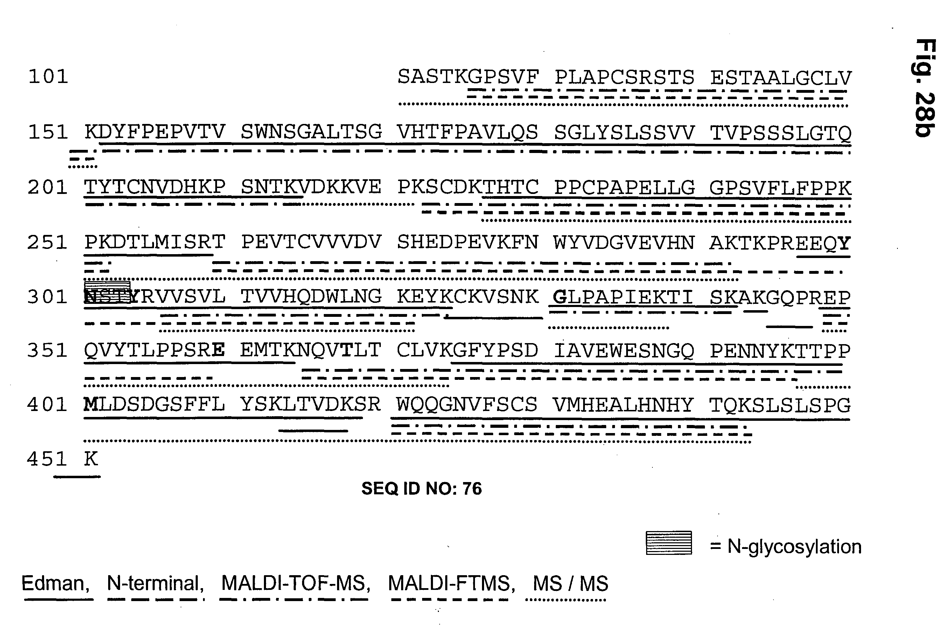

   Fig. 28b: Complete amino acid sequence of constant region heavy chain of isoform 2 (SEQ ID NO: 76).

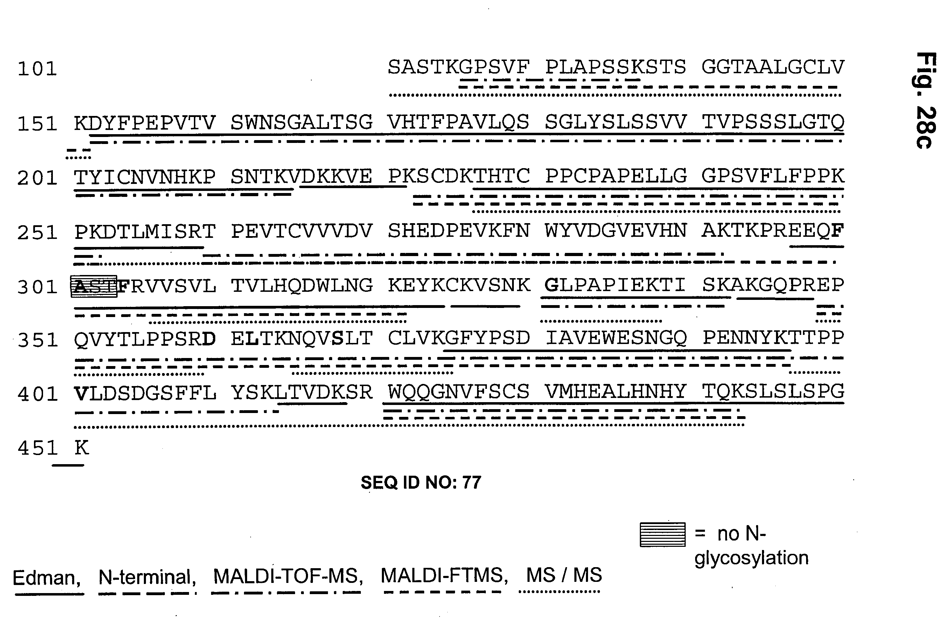

   Fig. 28c: Complete amino acid sequence of constant region heavy chain isoform 3 (SEQ ID NO:77).

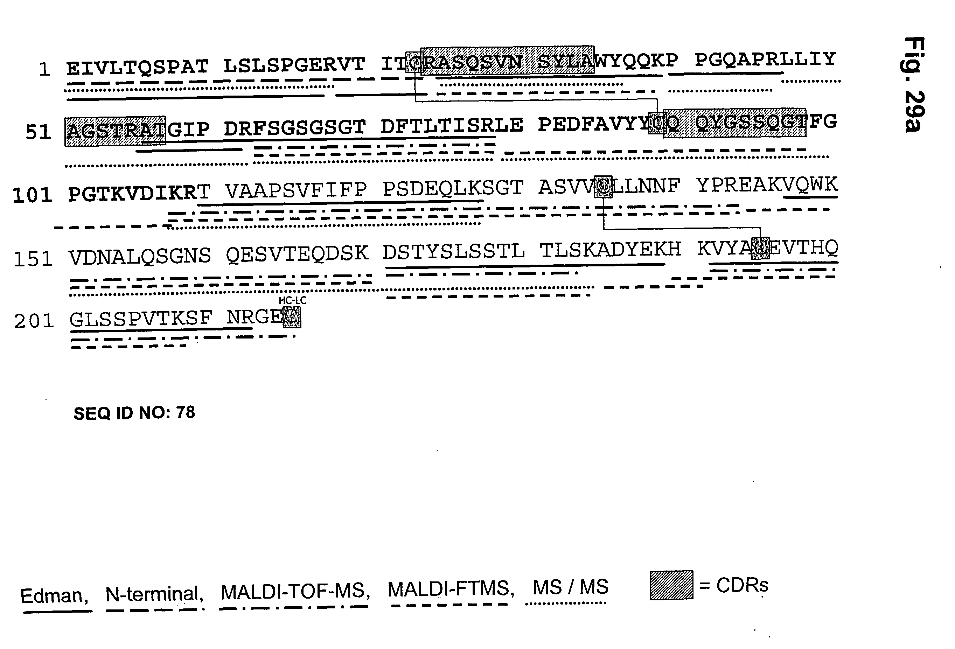

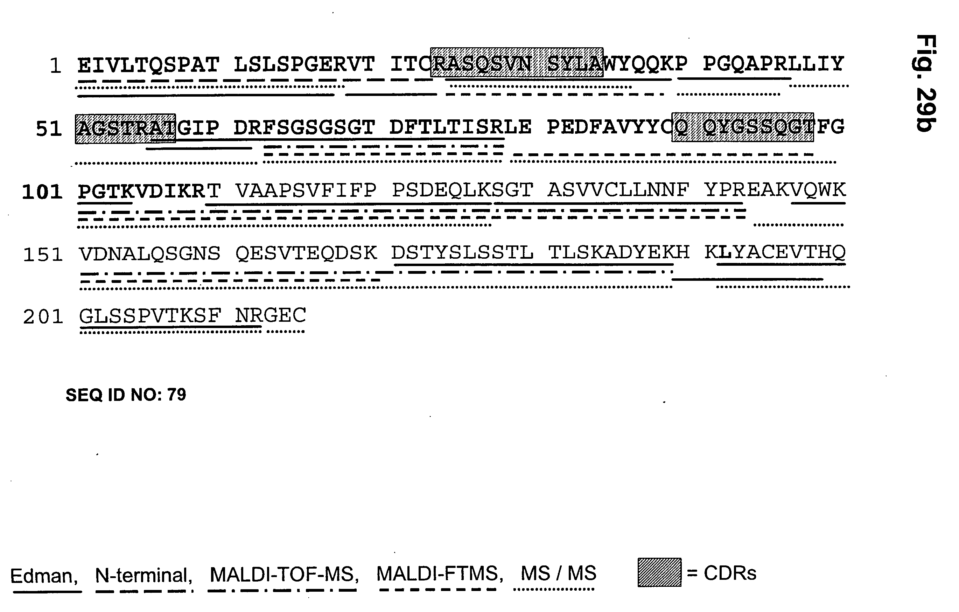

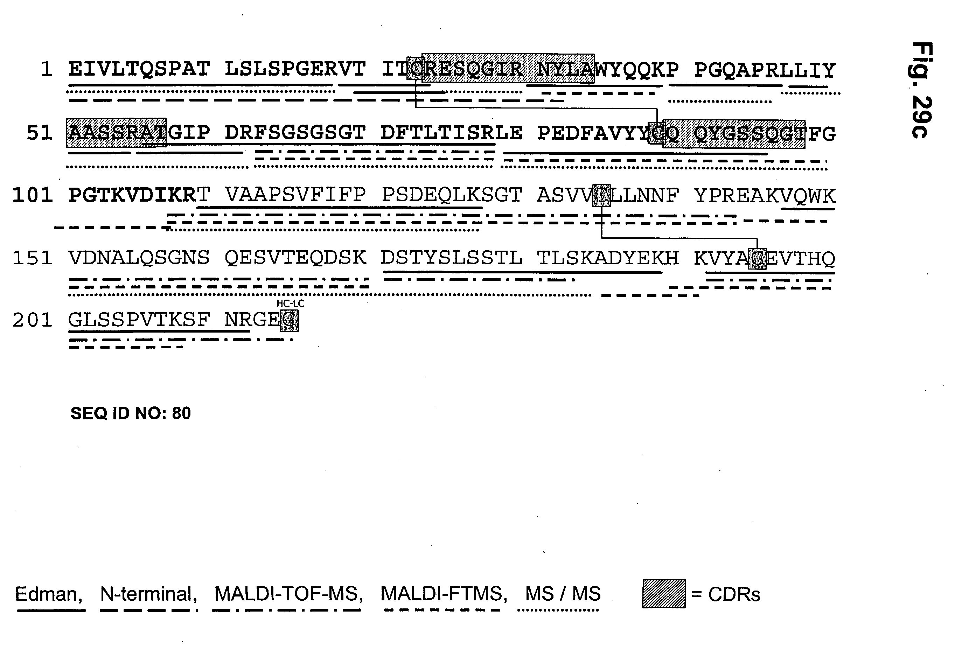

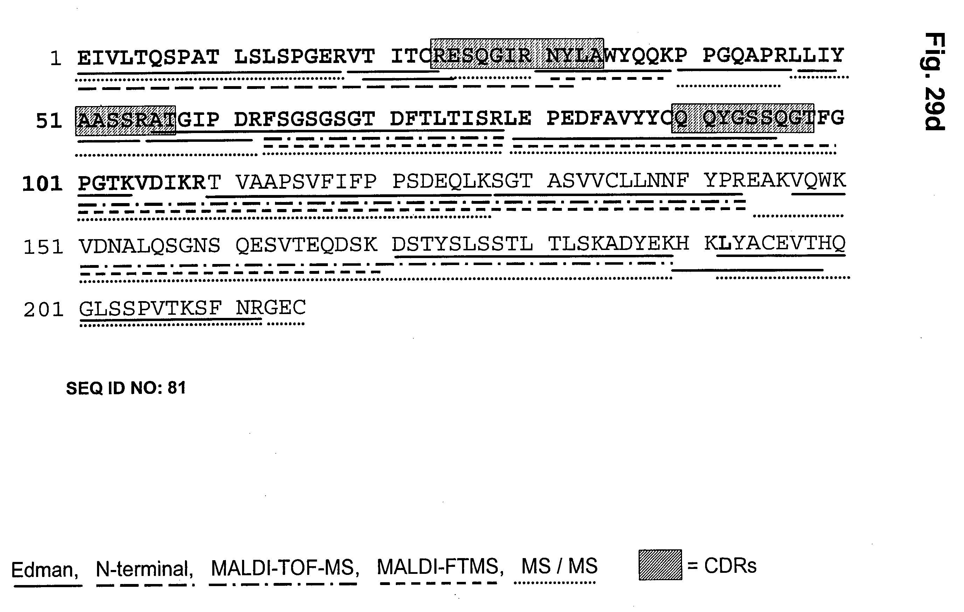

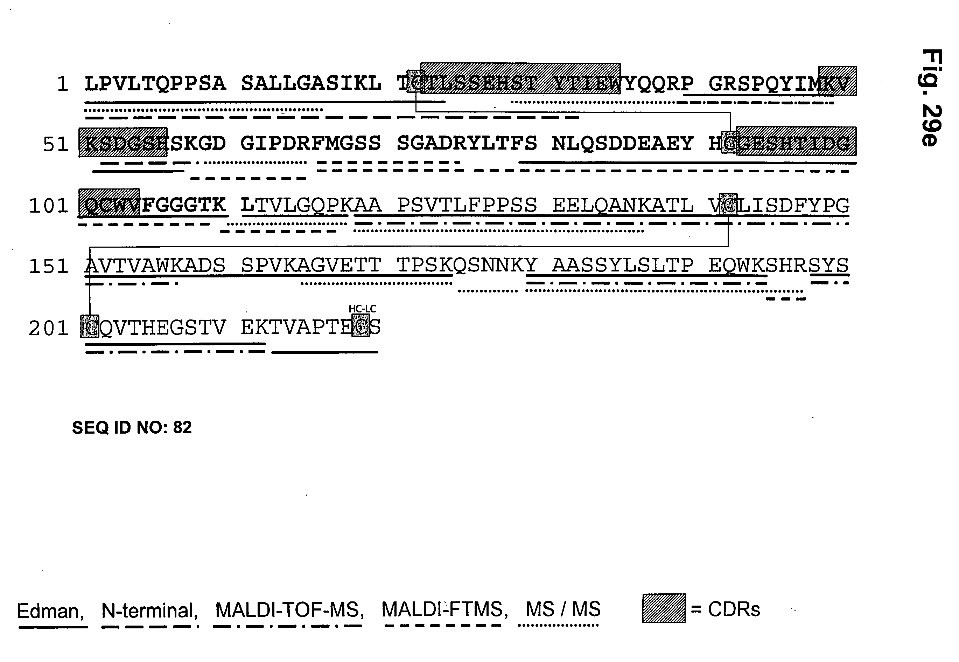

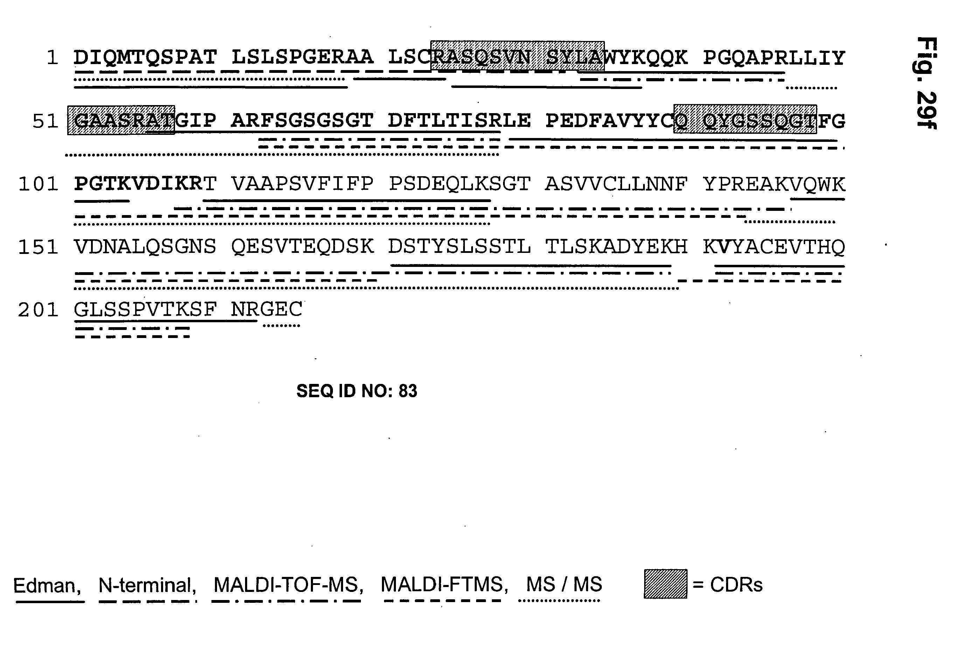

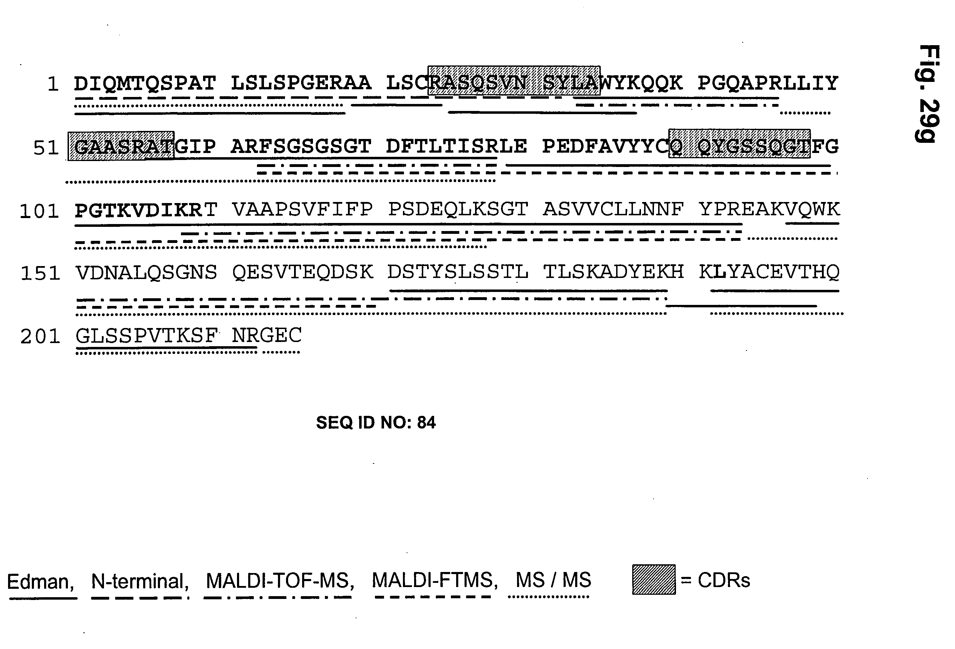

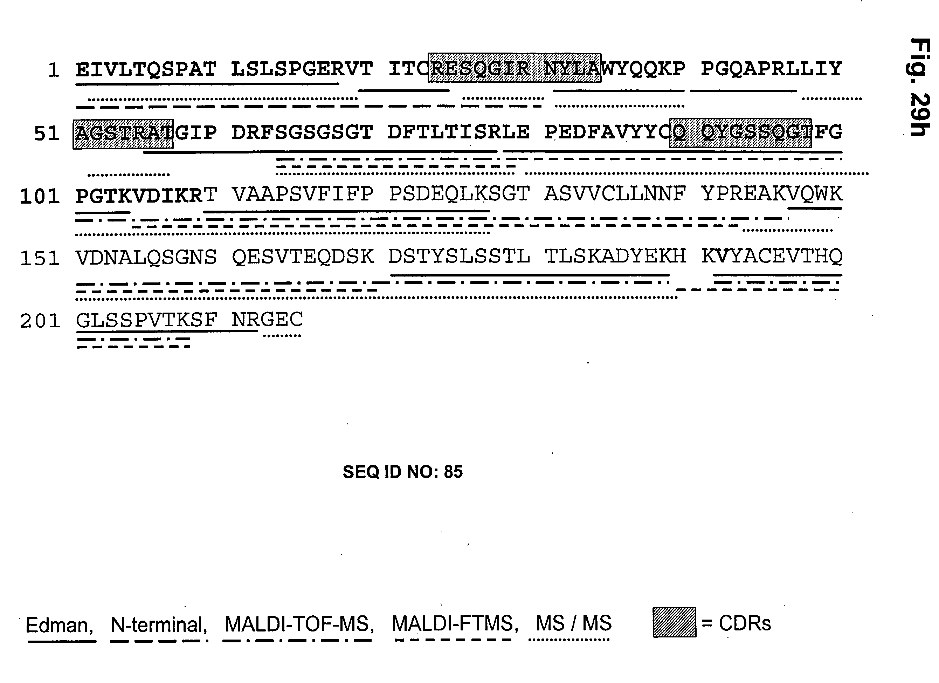

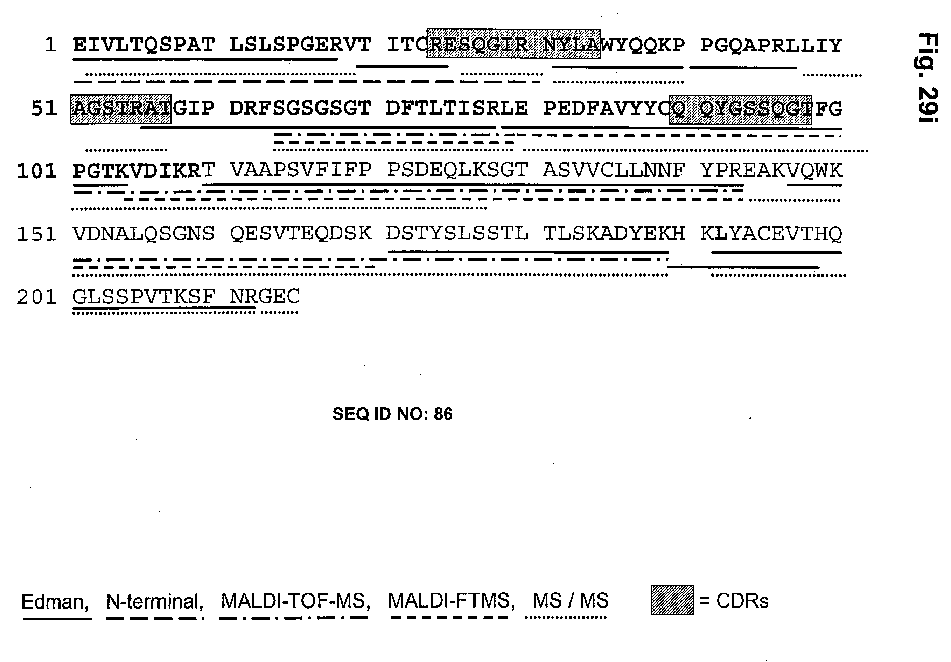

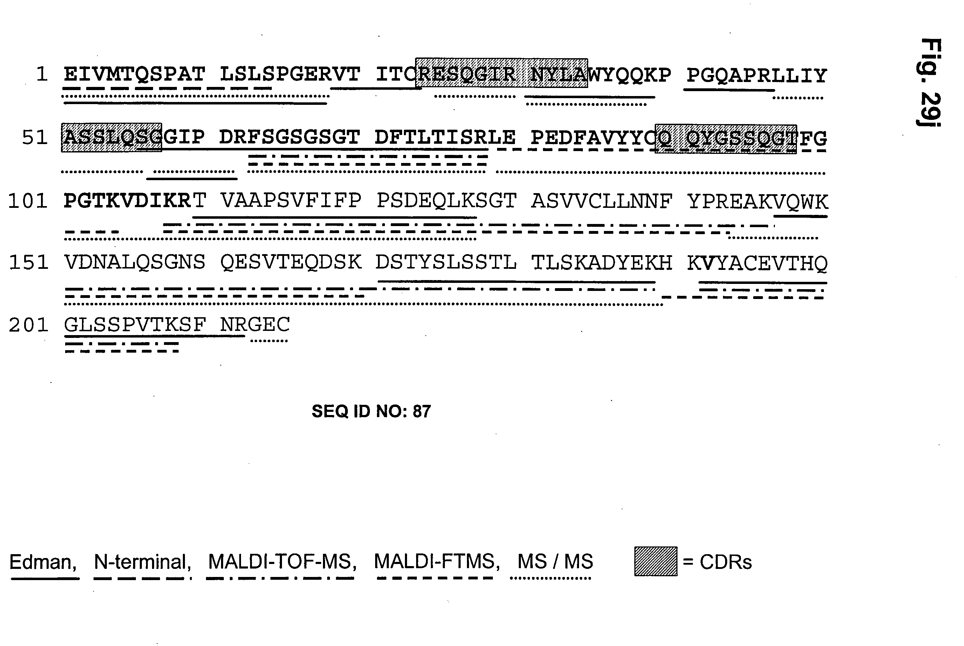

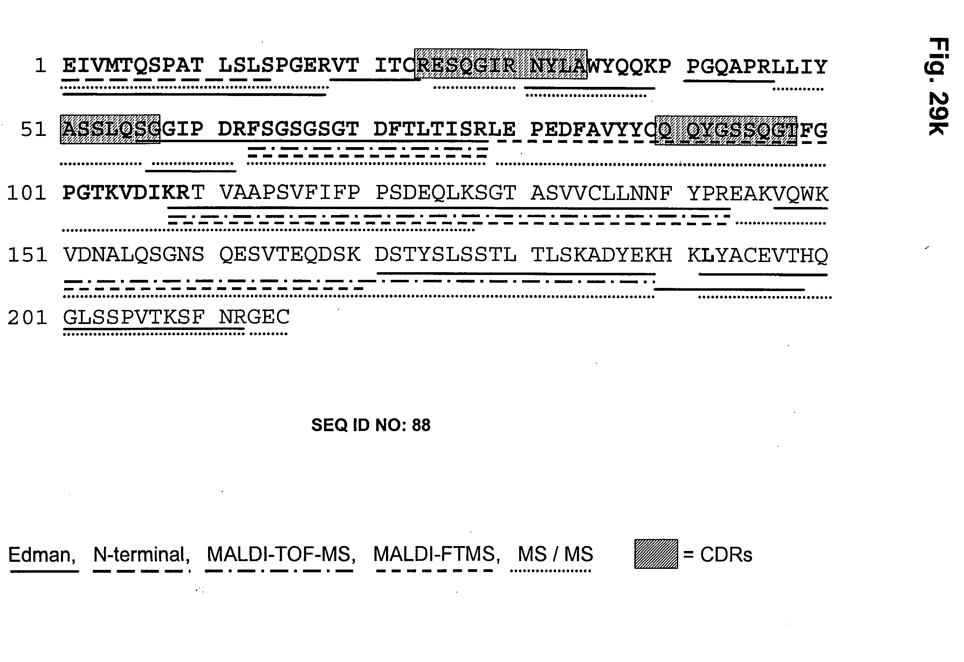

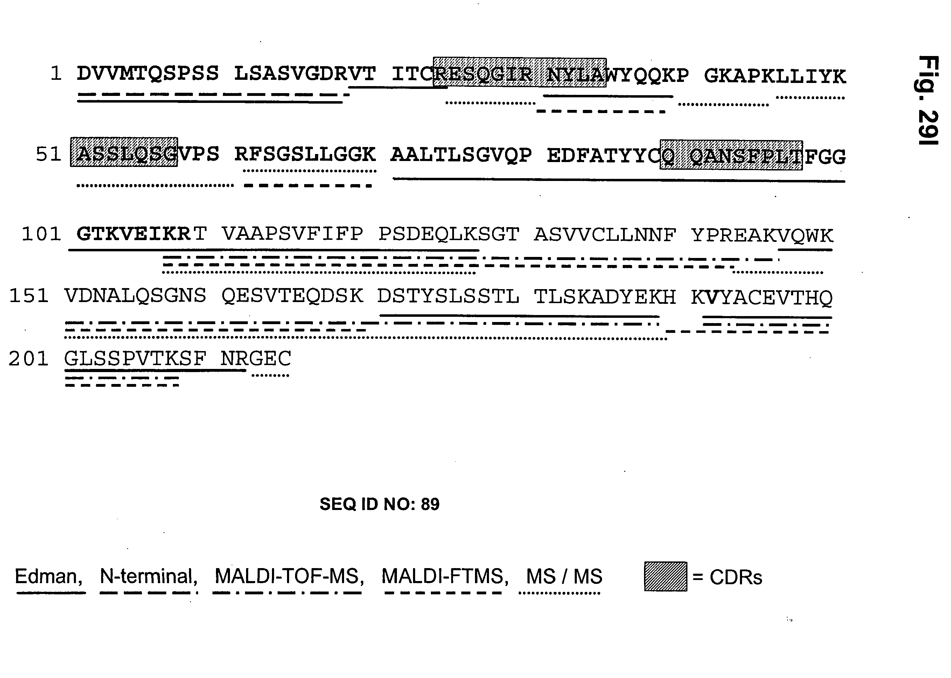

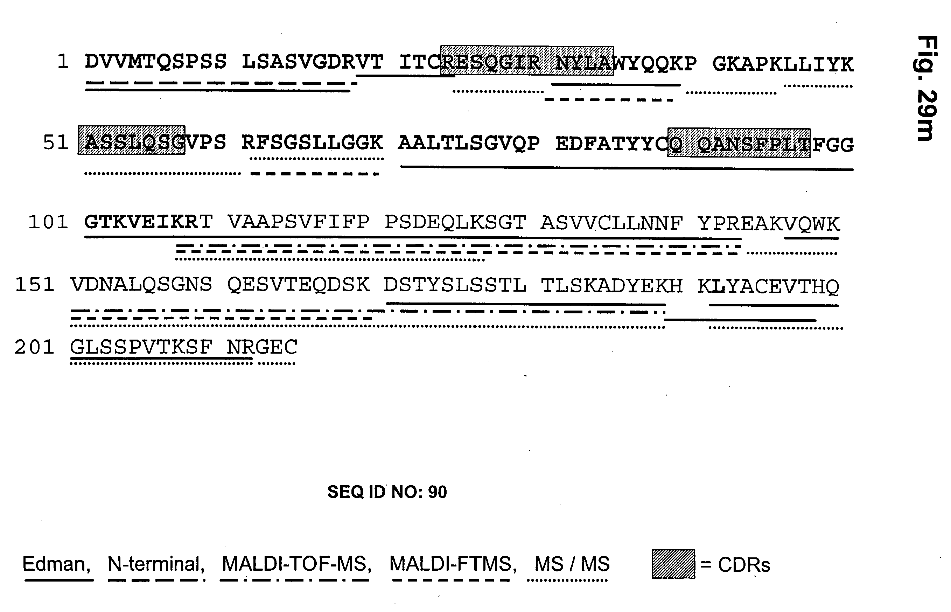

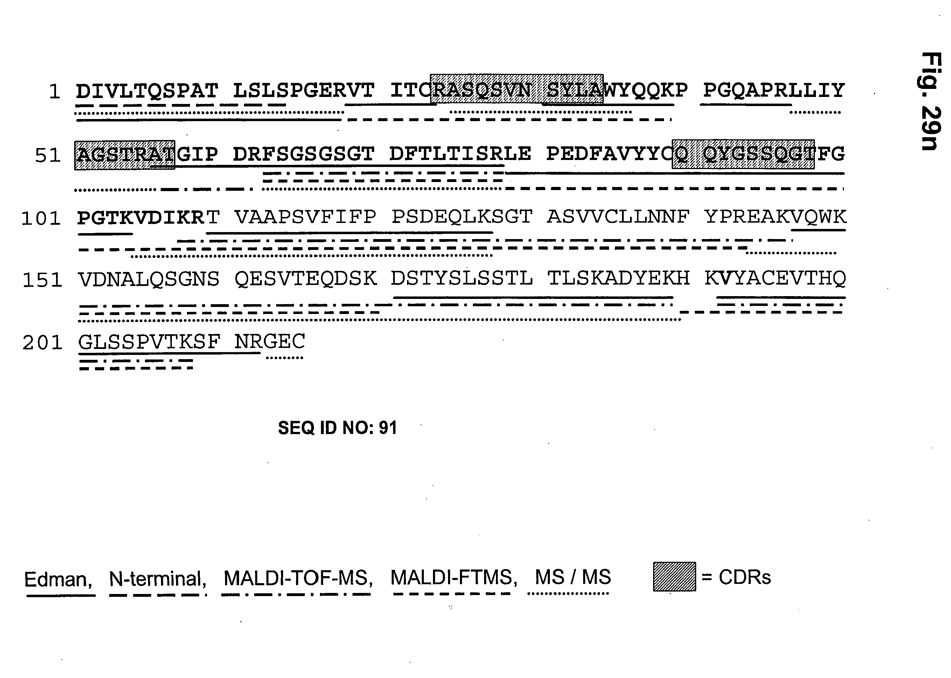

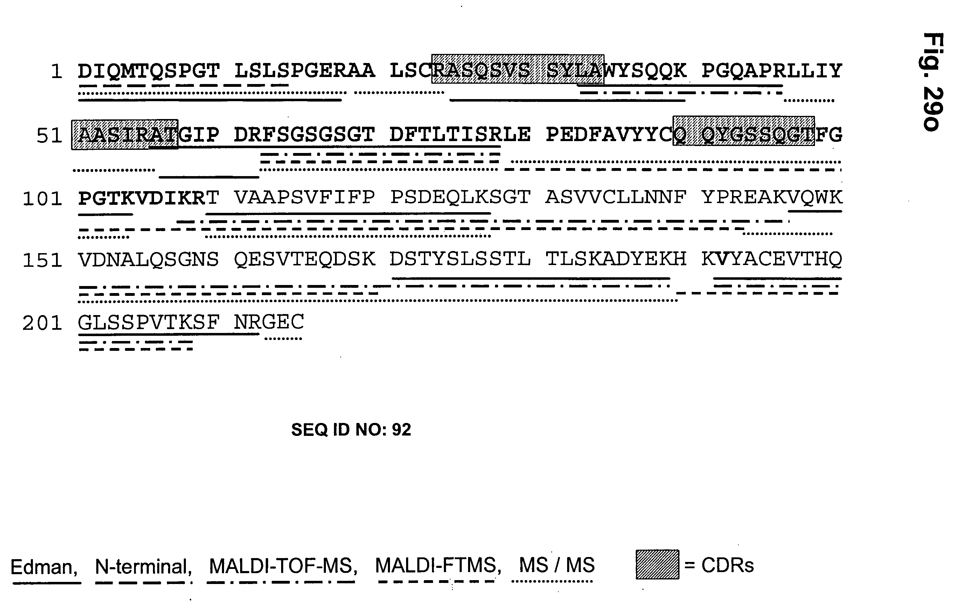

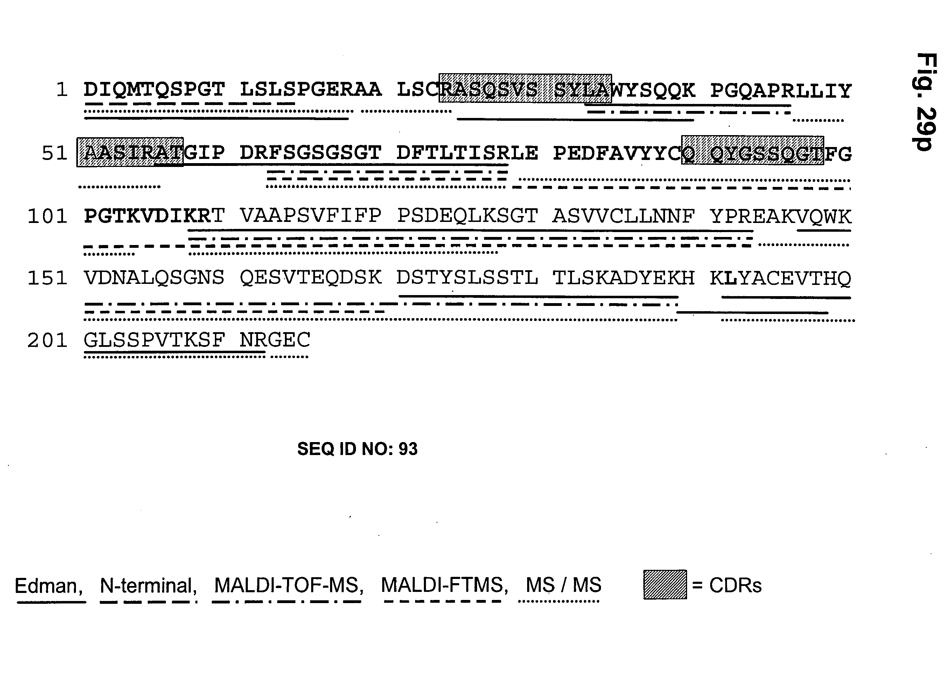

**Figure 29** **a to p:** Complete amino acid sequences of light chains of Aβ-autoantibodies from serum-IVIgG and individual serum (see Fig. 23 for sample overview). Annotation of codes for sequencing methods employed (Edman; Edman-protein N-terminal; MALDI-TOF-MS; MALDI-FTICR-MS, LC-MS/MS), and of CDR sequences is indicated on the bottom of each sequence. CDRs are indicated by boxes. Variable sequence domains, and single amino acid residues in the constant region sequences found with single site mutations are indicated in bold letters.
   Fig. 29a: complete amino acid sequence of light chain kappa of sample IVIG_(1)_A', constant region isoform 1 (SEQ ID NO:78).
   Fig. 29b: complete amino acid sequence of light chain kappa of sample IVIG_(1)_A', constant region isoform 2 (SEQ ID NO:79).
   Fig. 29c: complete amino acid sequence of light chain kappa of sample IVIG_(2)_B', constant region isoform 1 (SEQ ID NO: 80).
   Fig. 29d: complete amino acid sequence of light chain kappa of sample IVIG_(2)_B', constant region isoform 2 (SEQ ID NO:81).
   Fig. 29e: complete amino acid sequence of light chain lambda of sample IVIG_(3) (SEQ ID NO:82).
   Fig. 29f: complete amino acid sequence of light chain kappa of sample IVIG_(6), constant region isoform 1 (SEQ ID NO: 83).
   Fig. 29g: complete amino acid sequence of light chain kappa of sample IVIG_(6), constant region isoform 2 (SEQ ID NO:84).
   Fig. 29h: complete amino acid sequence of light chain kappa of sample IVIG_(7), constant region isoform 1 (SEQ ID NO:85).
   Fig. 29i: complete amino acid sequence of light chain kappa of sample IVIG_(7), constant region isoform 2 (SEQ ID NO:86).
   Fig. 29j: complete amino acid sequence of light chain kappa of sample IVIG_(8), constant region isoform 1 (SEQ ID NO:87).
   Fig. 29k: complete amino acid sequence of light chain kappa of sample IVIG_(8), constant region isoform 2 (SEQ ID NO:88).
   Fig. 29l: complete amino acid sequence of light chain kappa of sample IVIG_(9), constant region isoform 1 (SEQ ID NO:89).
   Fig. 29m: complete amino acid sequence of light chain kappa of sample IVIG_(9), constant region isoform 2 (SEQ ID NO:90).
   Fig. 29n: complete amino acid sequence of light chain kappa of sample IVIG_(10) (SEQ ID
   NO:91).
   Fig. 29o: complete amino acid sequence of light chain kappa of sample IVIG_(11), constant region isoform 1 (SEQ ID NO:92).
   Fig. 29p: complete amino acid sequence of light chain kappa of sample IVIG_(11), constant region isoform 2 (SEQ ID NO:93).

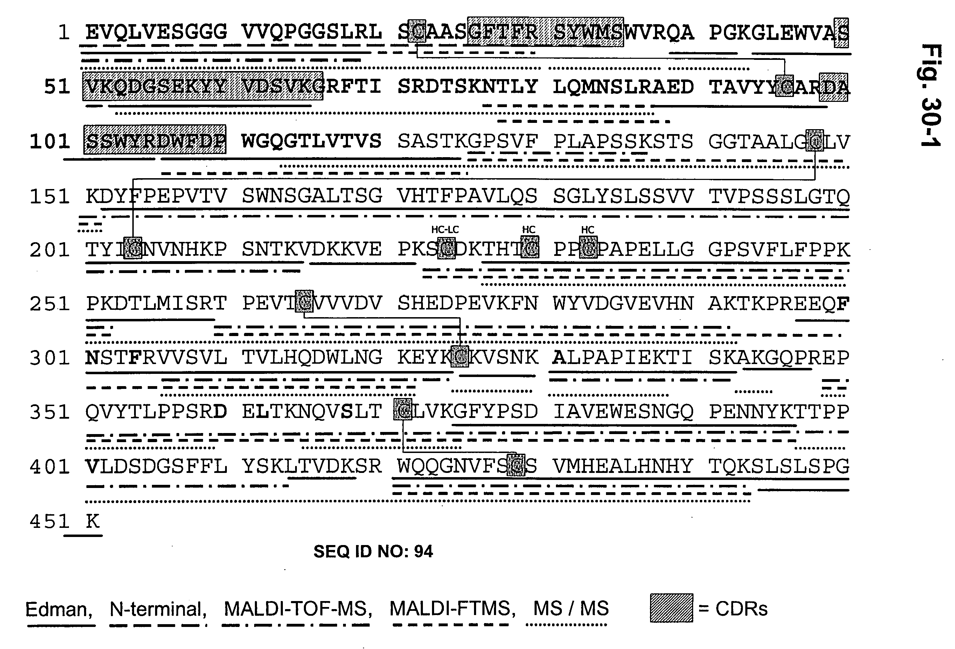

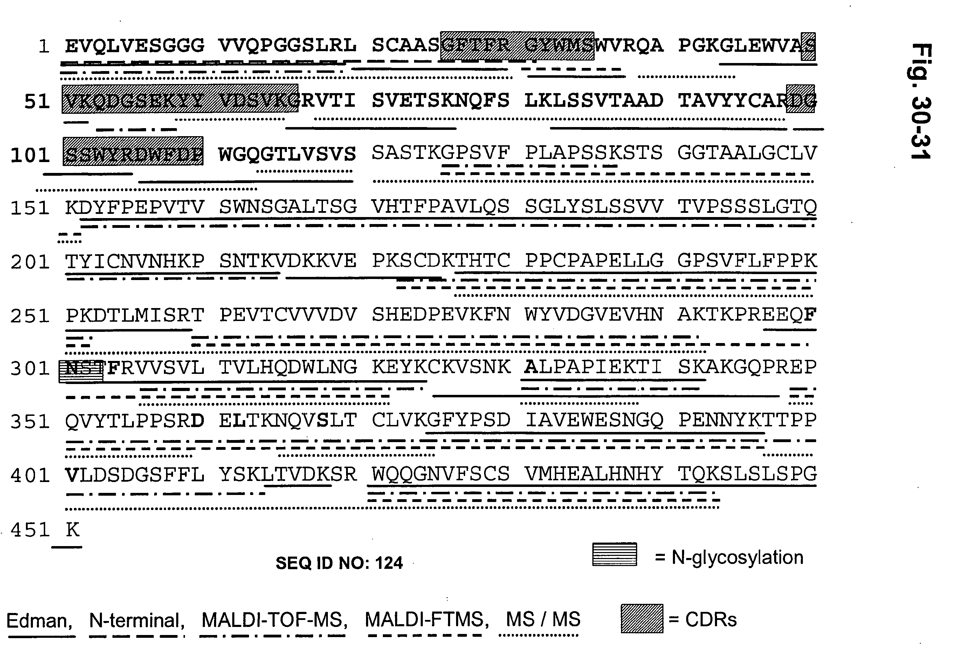

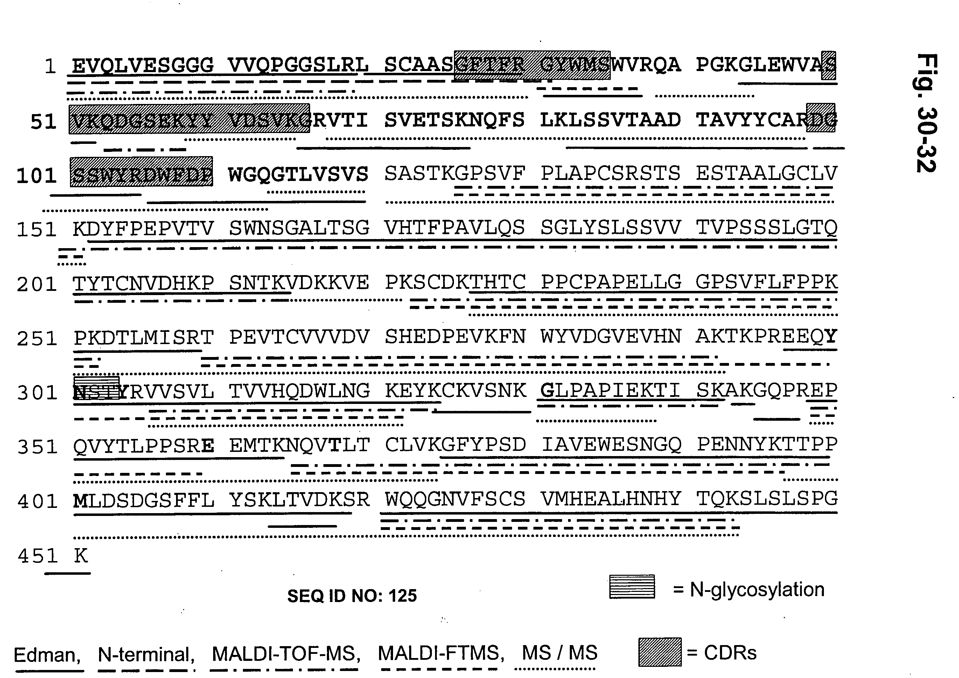

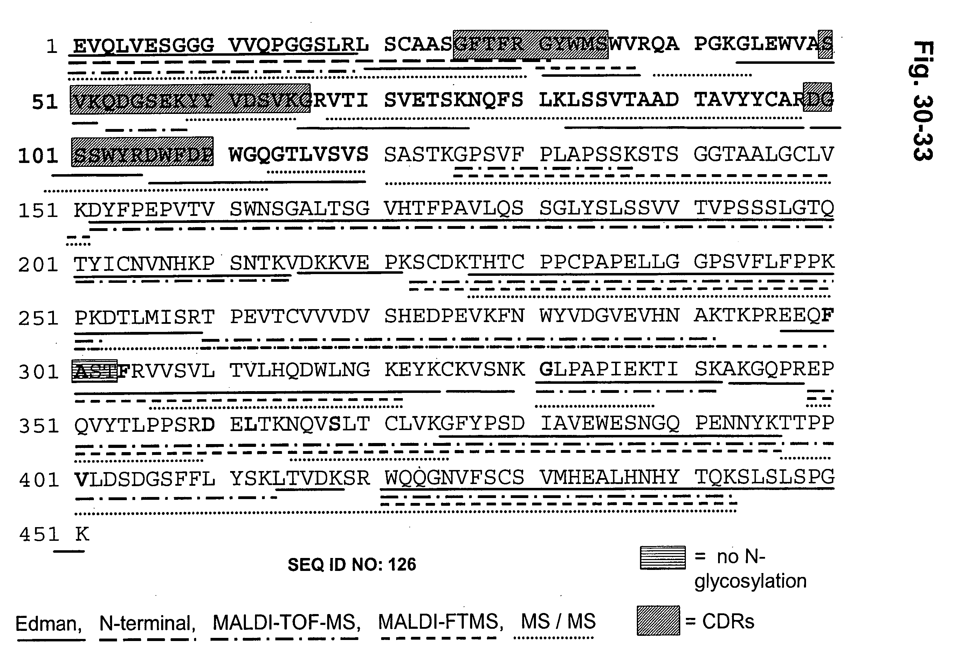

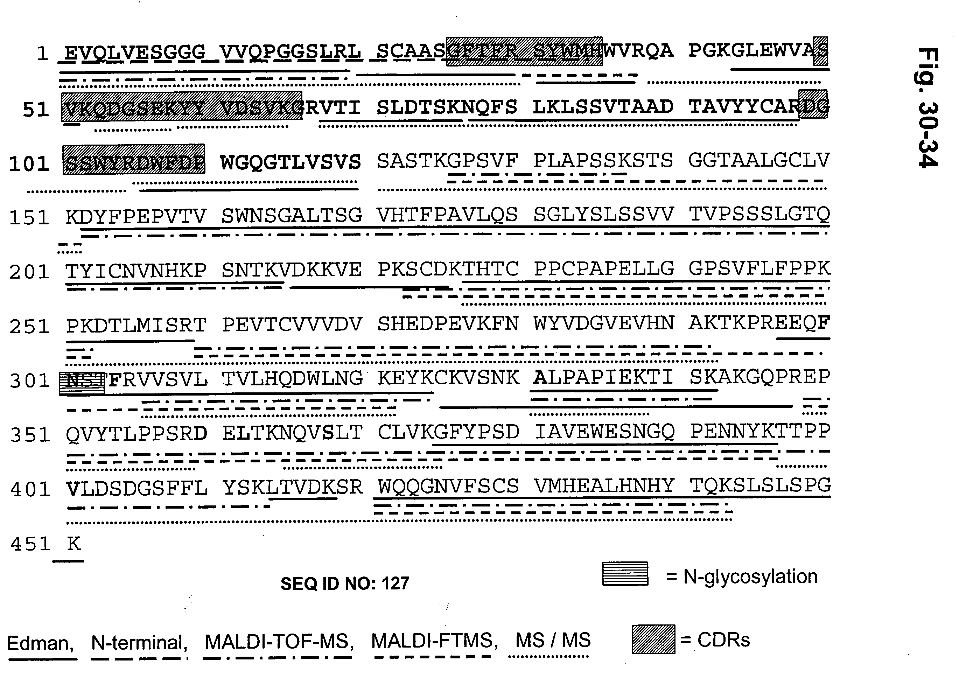

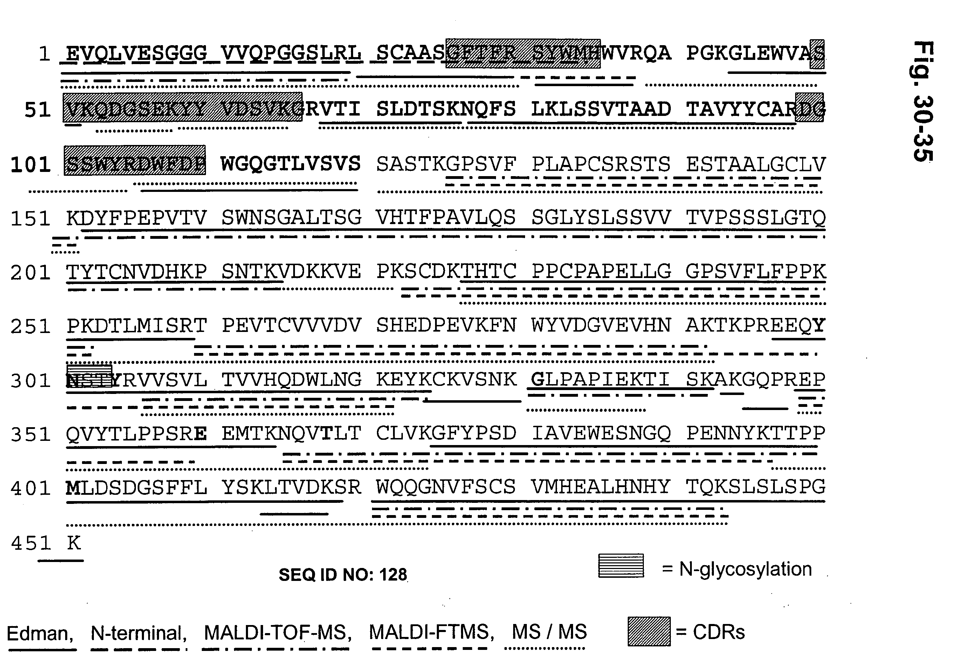

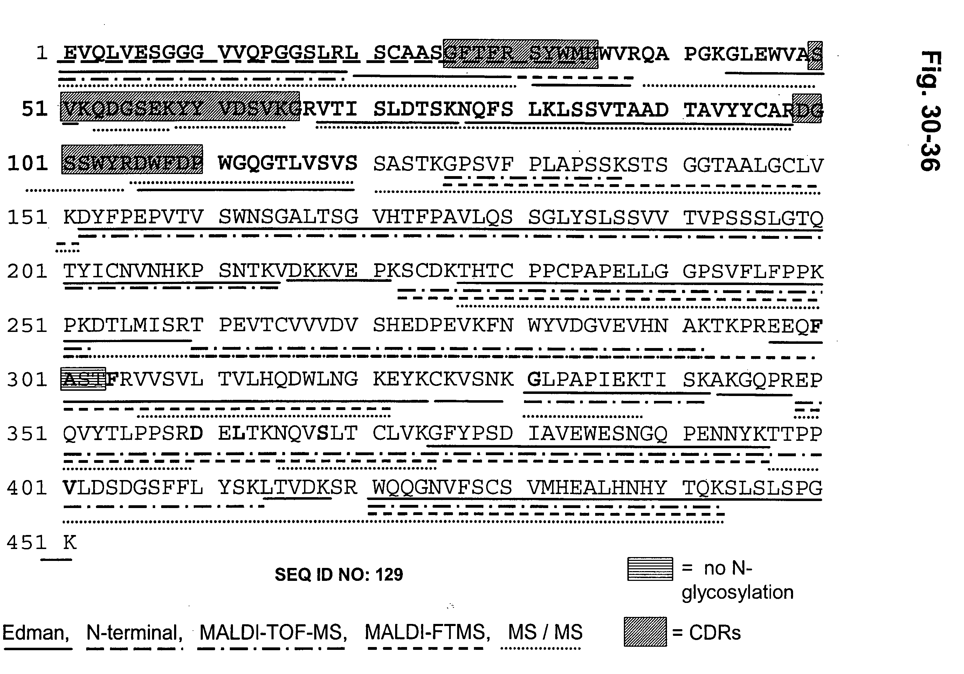

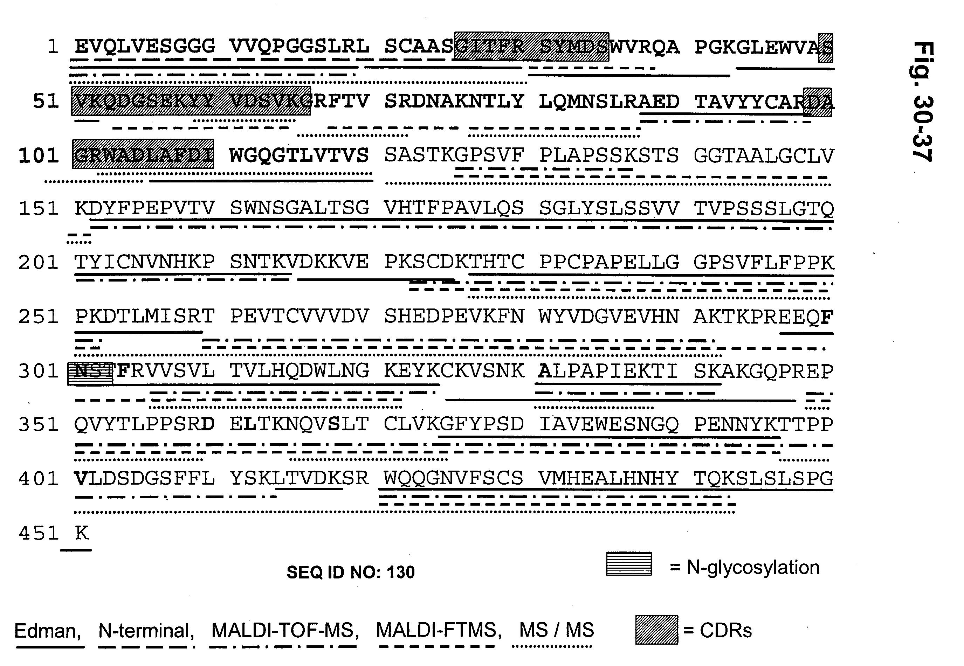

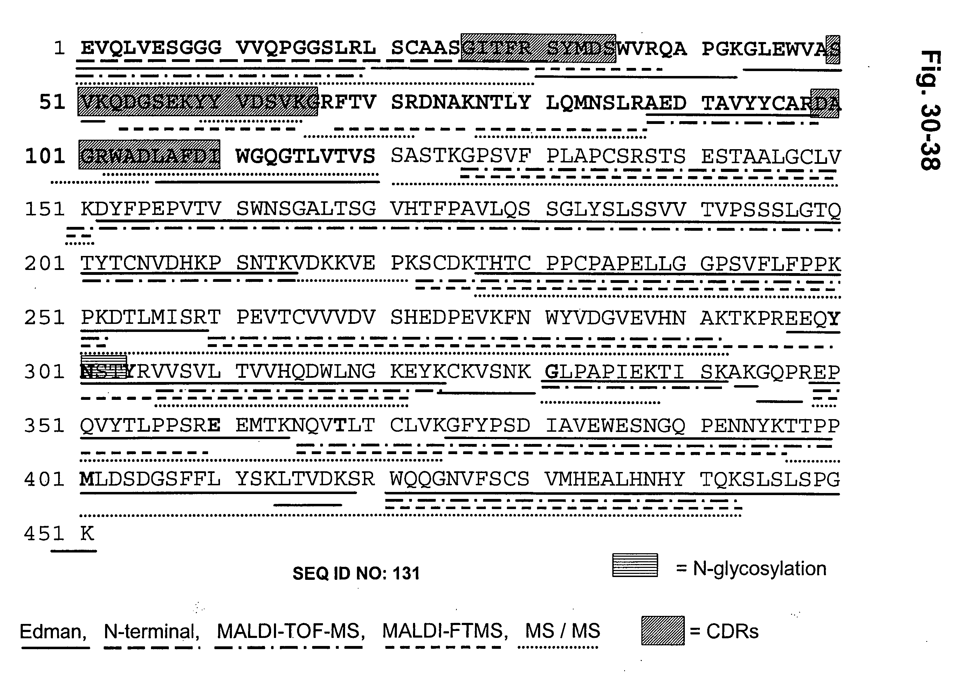

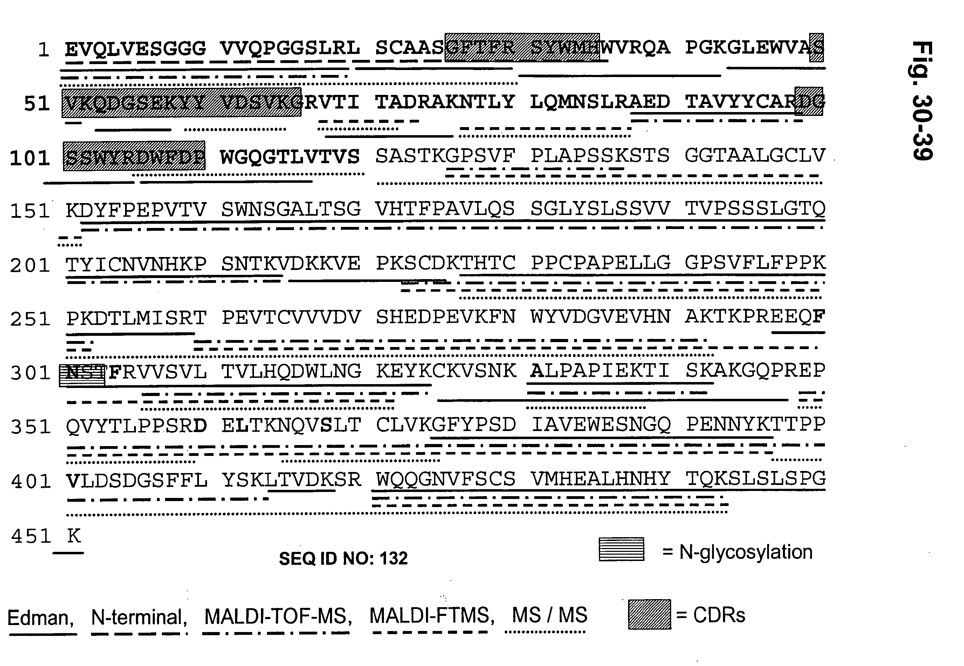

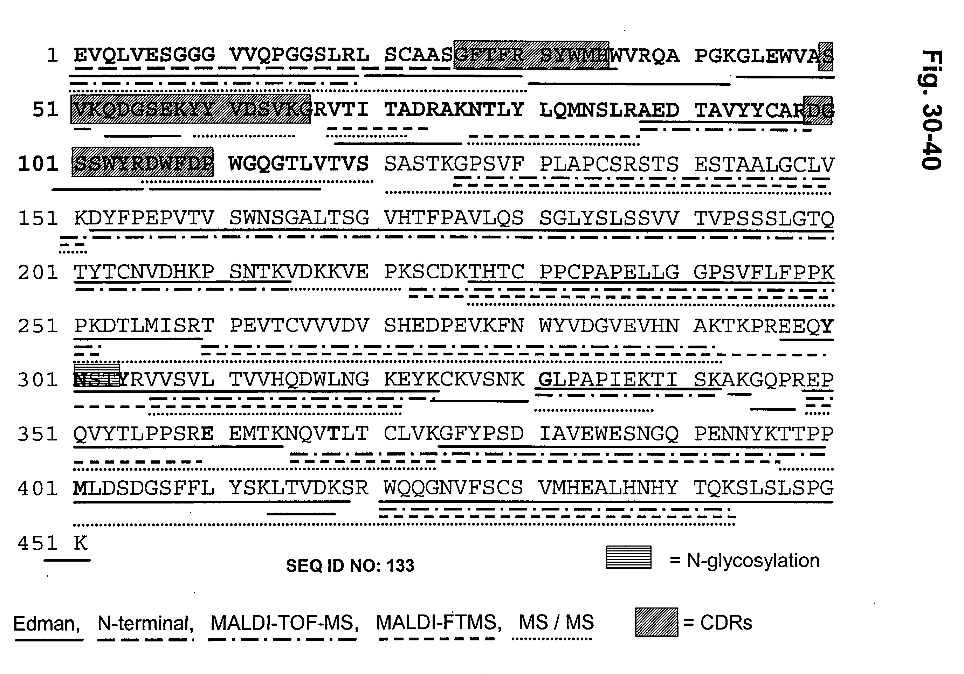

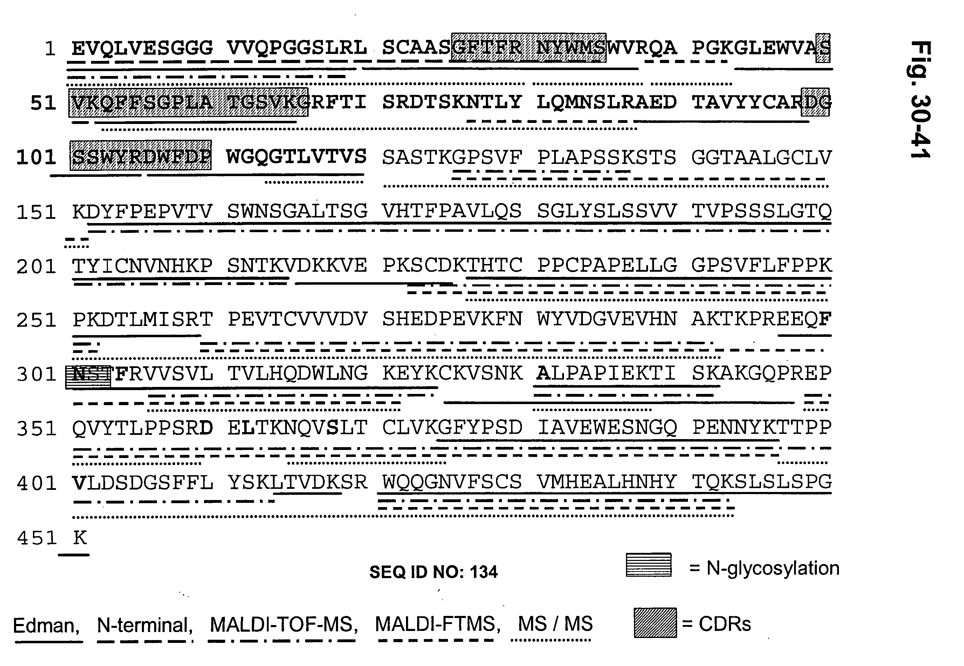

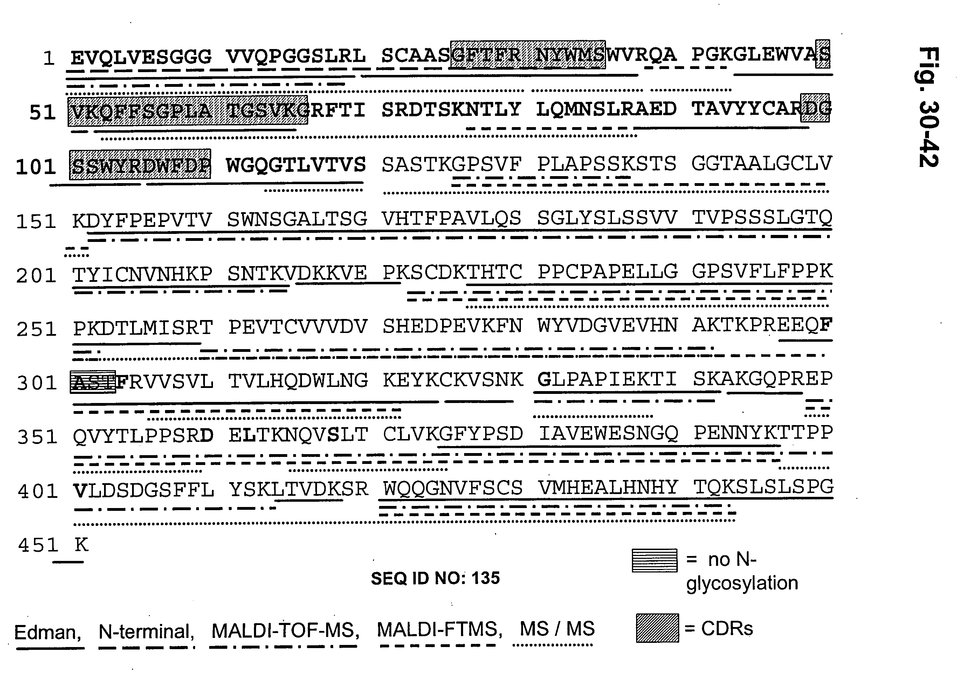

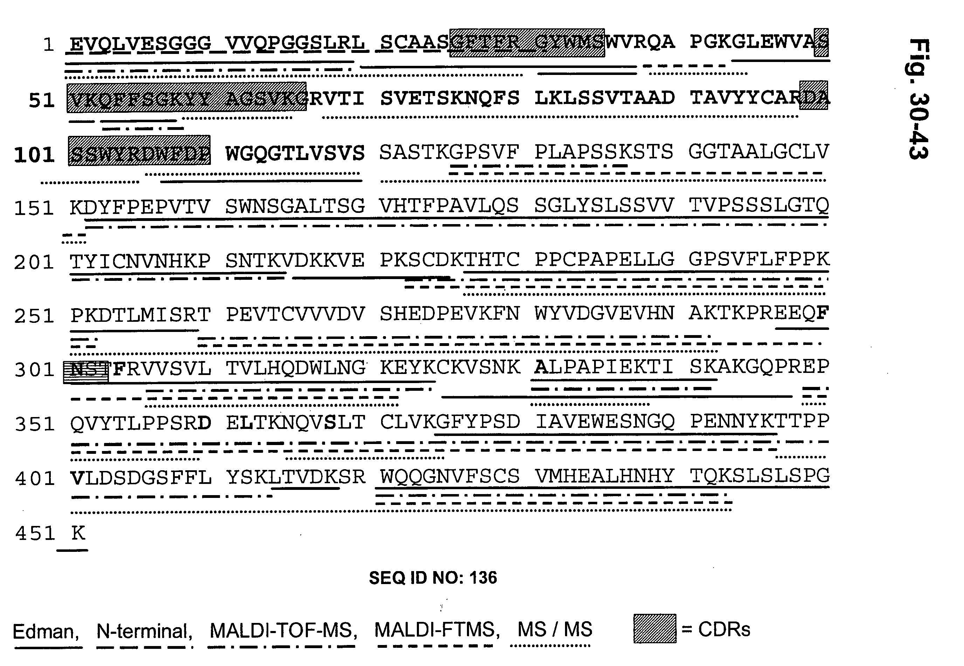

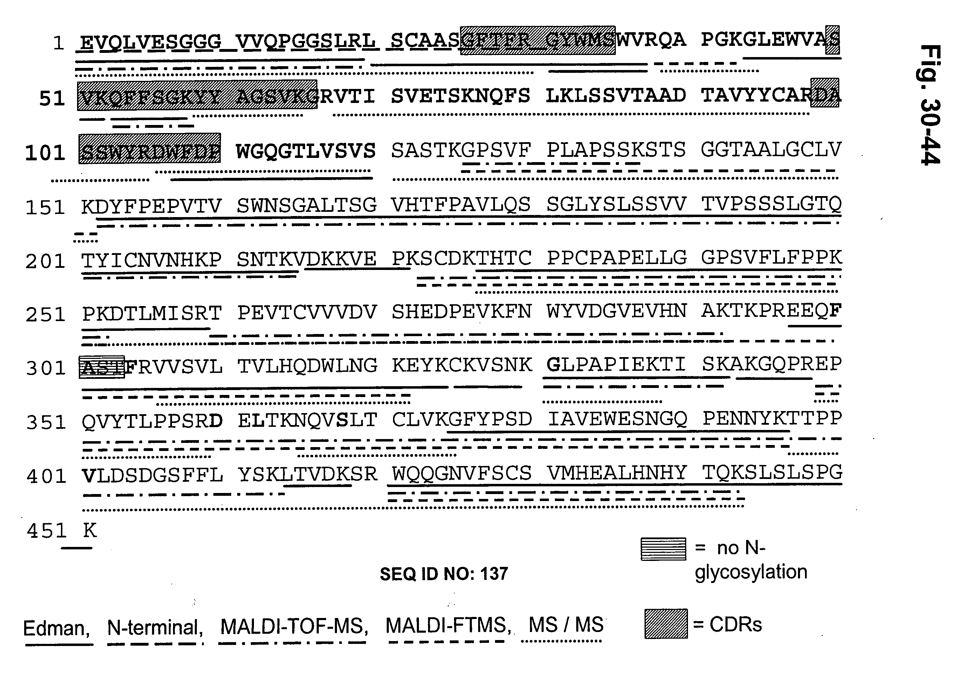

Figure 30-1 to 30-44: Complete amino acid sequences of heavy chains of Aβ-autoantibodies from serum-IVIgG and individual serum (see Fig. 23 for sample overview). Annotation of codes for sequencing methods employed (Edman; Edman-protein N-terminal; MALDI-TOF-MS; MALDI-FTICR-MS, LC-MS/MS), and of CDR sequences is indicated on the bottom of each sequence. CDRs are indicated by boxes. Variable sequence domains, and single amino acid residues in the constant region sequences found with single site mutations are indicated in bold letters. The N-glycosylation site, N-301 is indicated in bold, grey letter.
   Fig. 30-1: complete amino acid sequence of heavy chain of sample IVIG_(4)_A, constant region isoform 1 (SEQ ID NO:94).

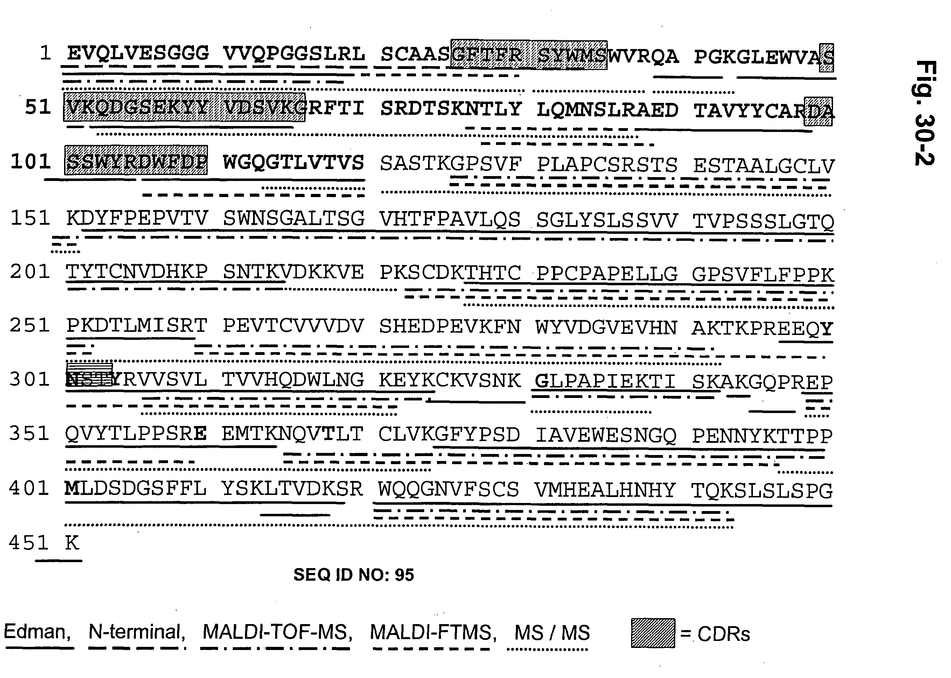

   Fig. 30-2: complete amino acid sequence of heavy chain of sample IVIG_(4)_A, constant region isoform 2 (SEQ ID NO:95).

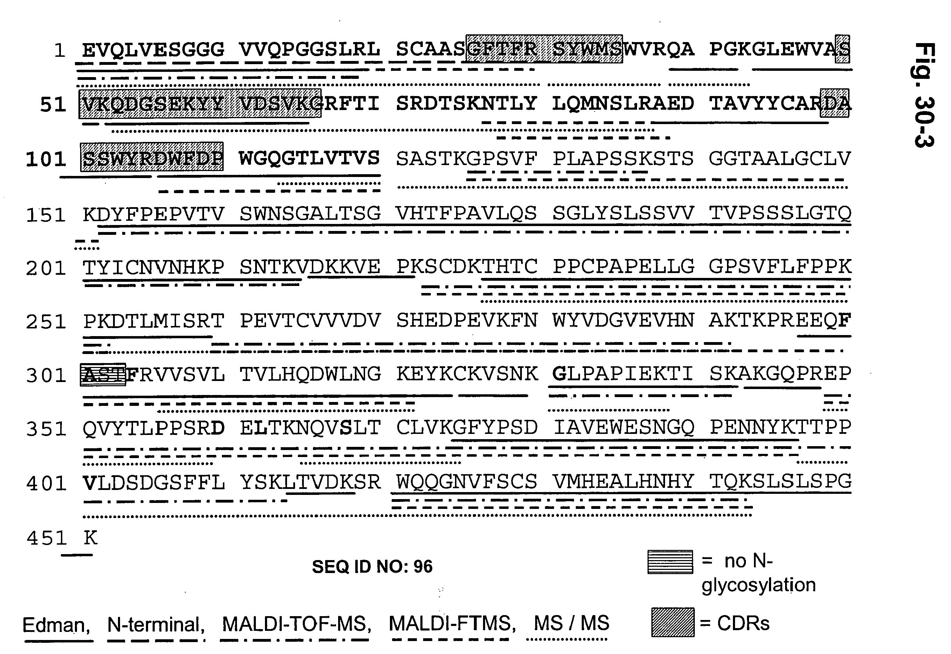

   Fig. 30-3: complete amino acid sequence of heavy chain of sample IVIG_(4)_A, constant region isoform 3 (SEQ ID NO:96).

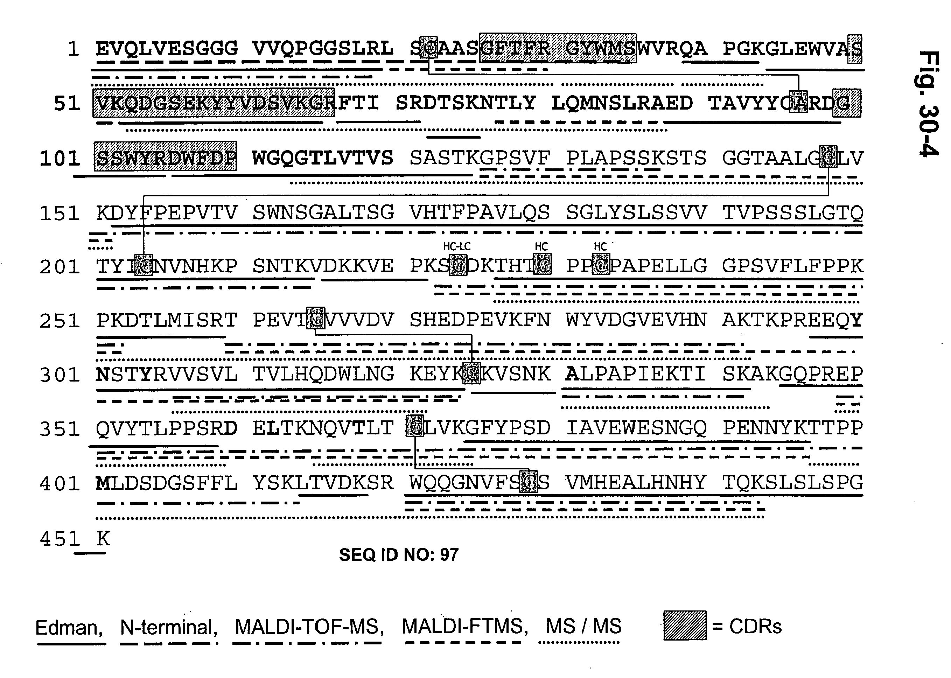

   Fig. 30-4: complete amino acid sequence of heavy chain of sample IVIG_(5)_B, constant region isoform 1 (SEQ ID NO:97).

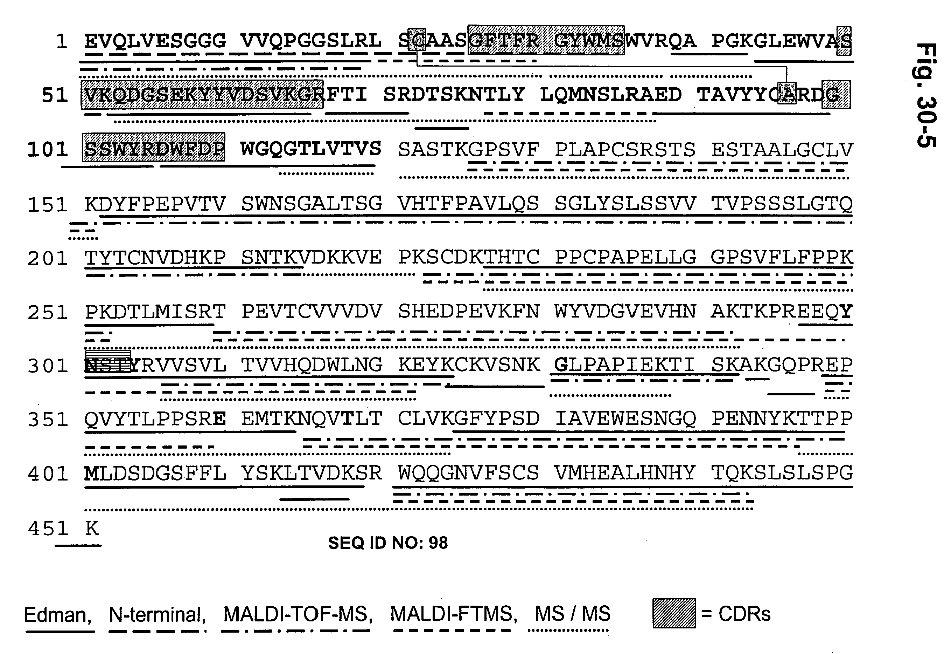

   Fig. 30-5: complete amino acid sequence of heavy chain of sample IVIG_(5)_B, constant region isoform 2 (SEQ ID NO:98).

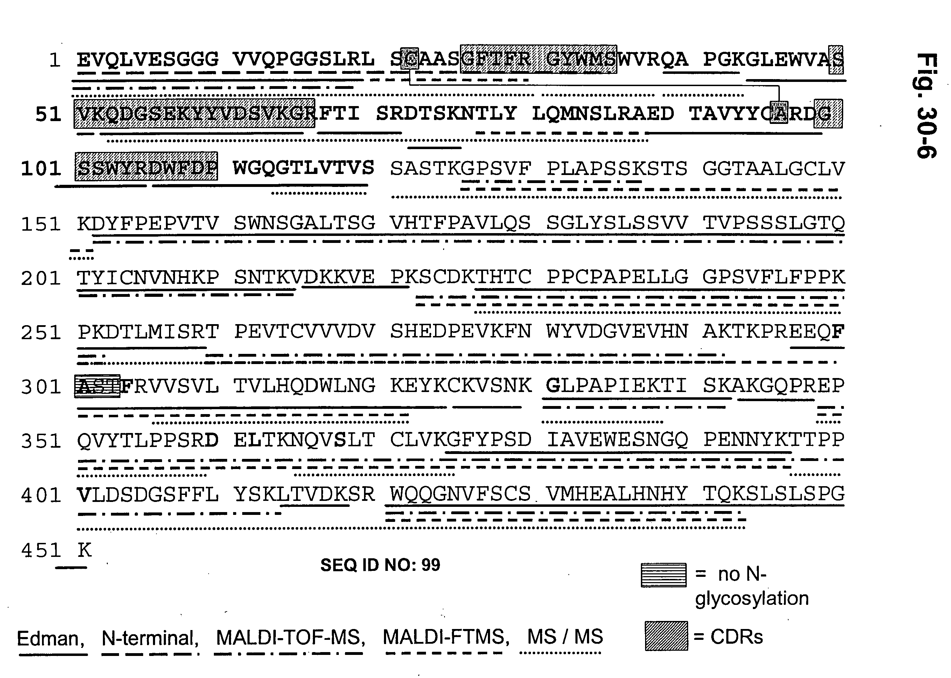

   Fig. 30-6: complete amino acid sequence of heavy chain of sample IVIG_(5)_B, constant region isoform 3 (SEQ ID NO:99).

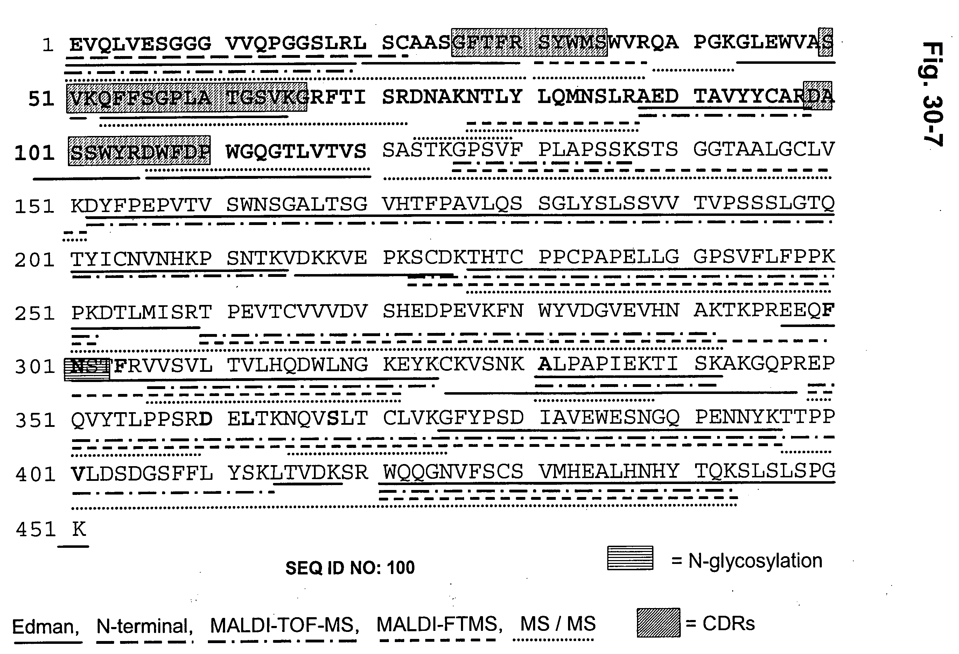

   Fig. 30-7: complete amino acid sequence of heavy chain of sample IVIG_(12)_B, constant region isoform 1 (SEQ ID NO: 100).

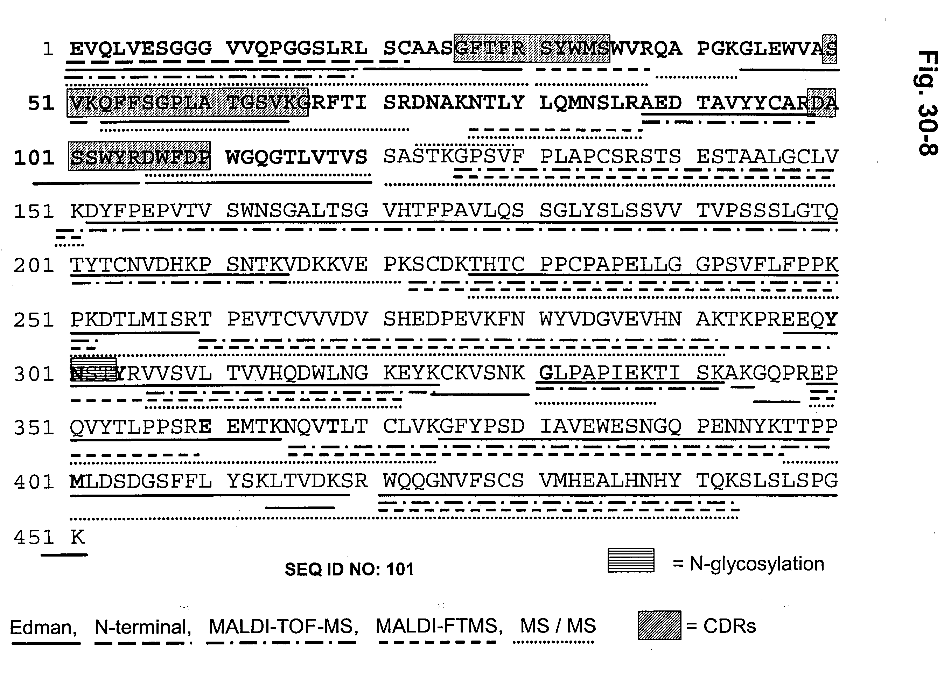

   Fig. 30-8: complete amino acid sequence of heavy chain of sample IVIG_(12)_B, constant region isoform 2 (SEQ ID NO:101).

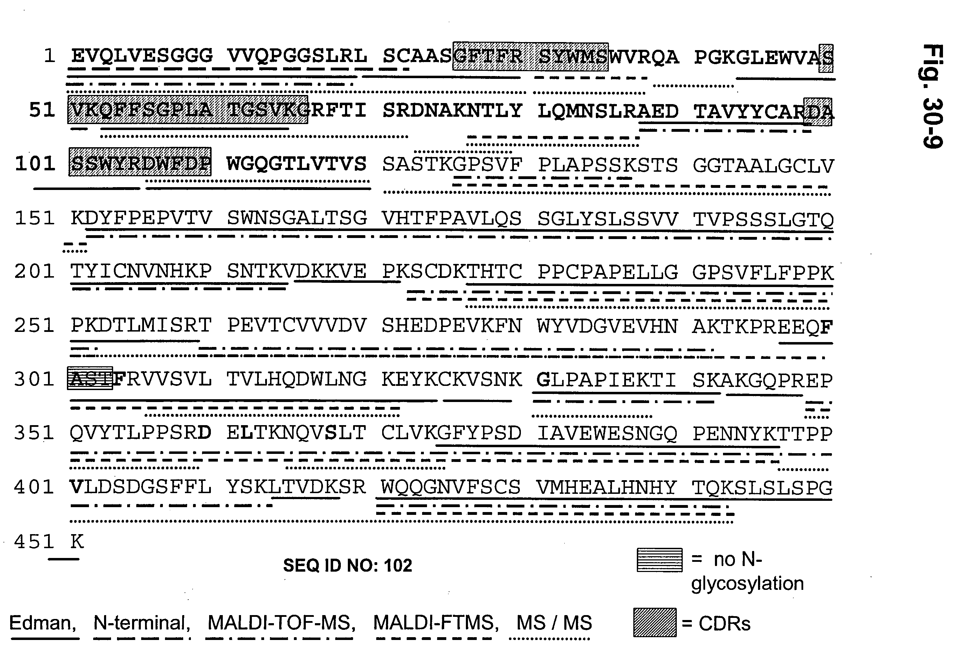

   Fig. 30-9: complete amino acid sequence of heavy chain of sample IVIG_(12)_B, constant region isoform 3 (SEQ ID NO: 102).

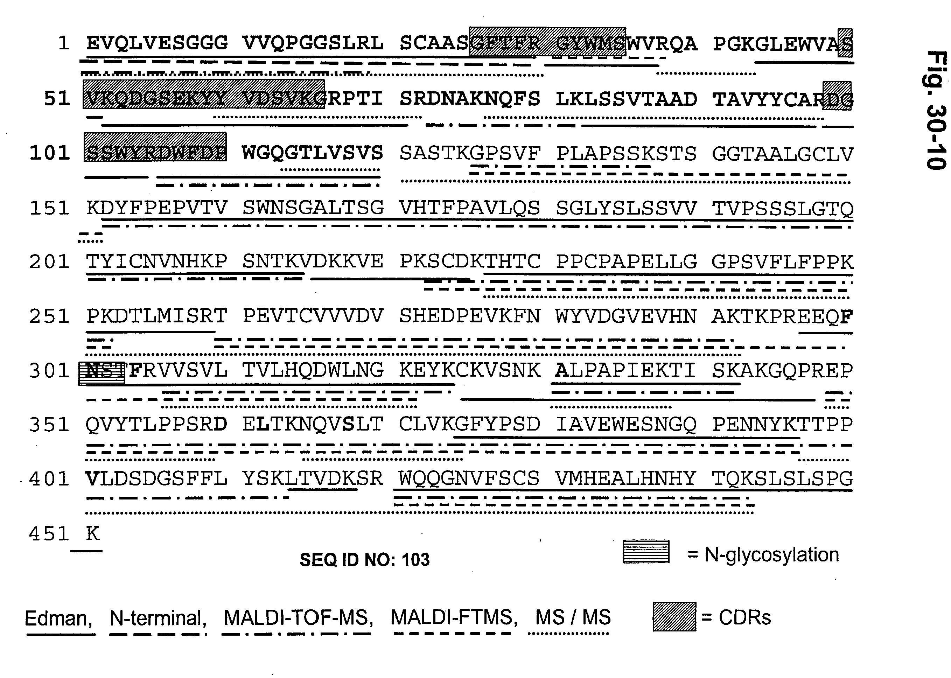

   Fig. 30-10: complete amino acid sequence of heavy chain of sample IVIG_(13), constant region isoform 1 (SEQ ID NO:103).

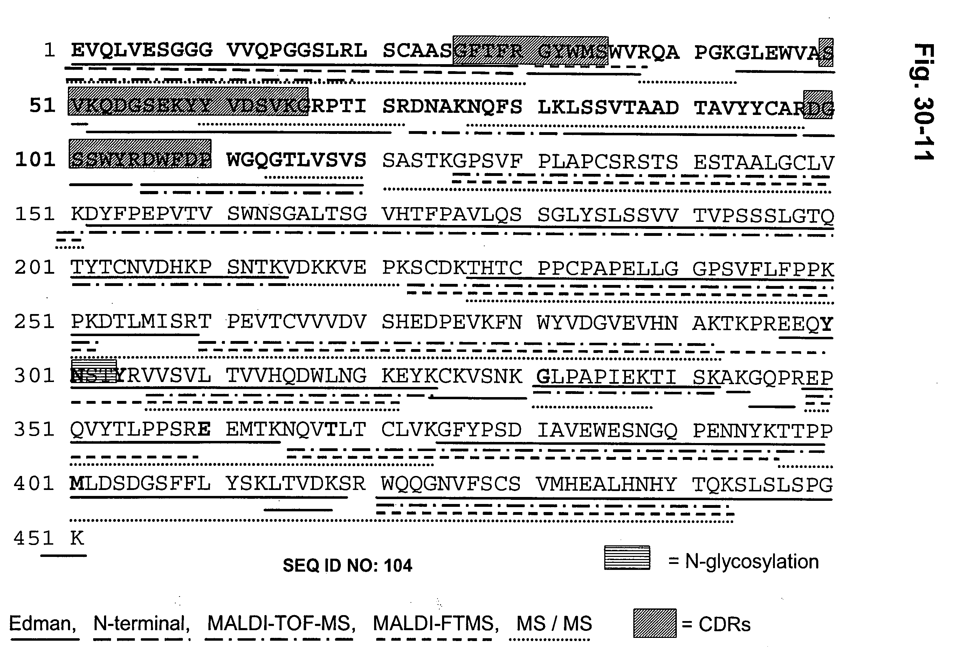

   Fig. 30-11: complete amino acid sequence of heavy chain of sample IVIG_(13), constant region isoform 2 (SEQ ID NO: 104).

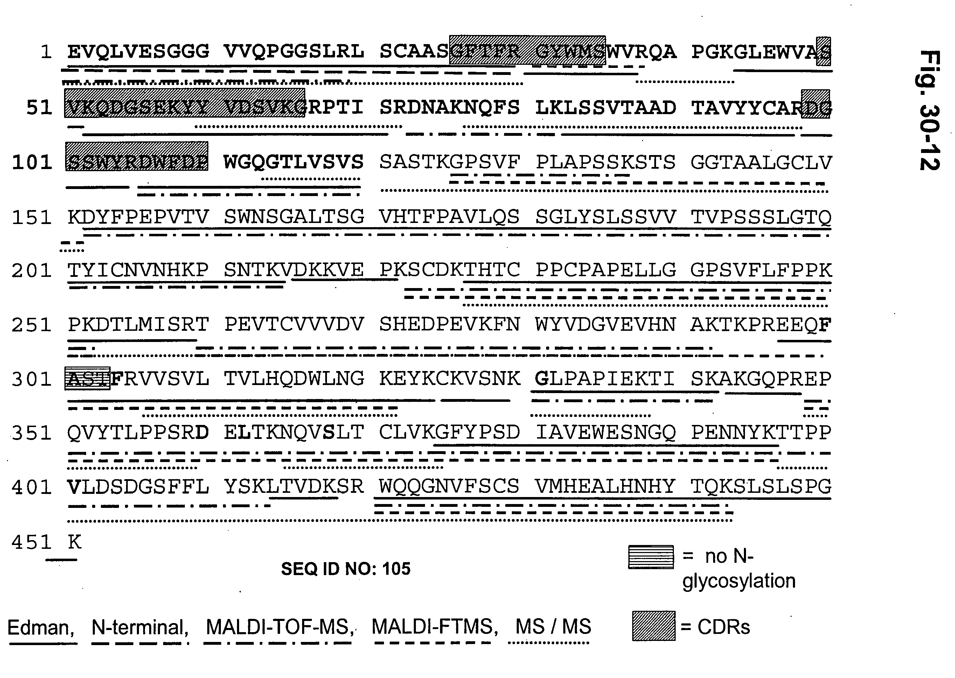

   Fig. 30-12: complete amino acid sequence of heavy chain of sample IVIG_(13), constant region isoform 3 (SEQ ID NO:105).

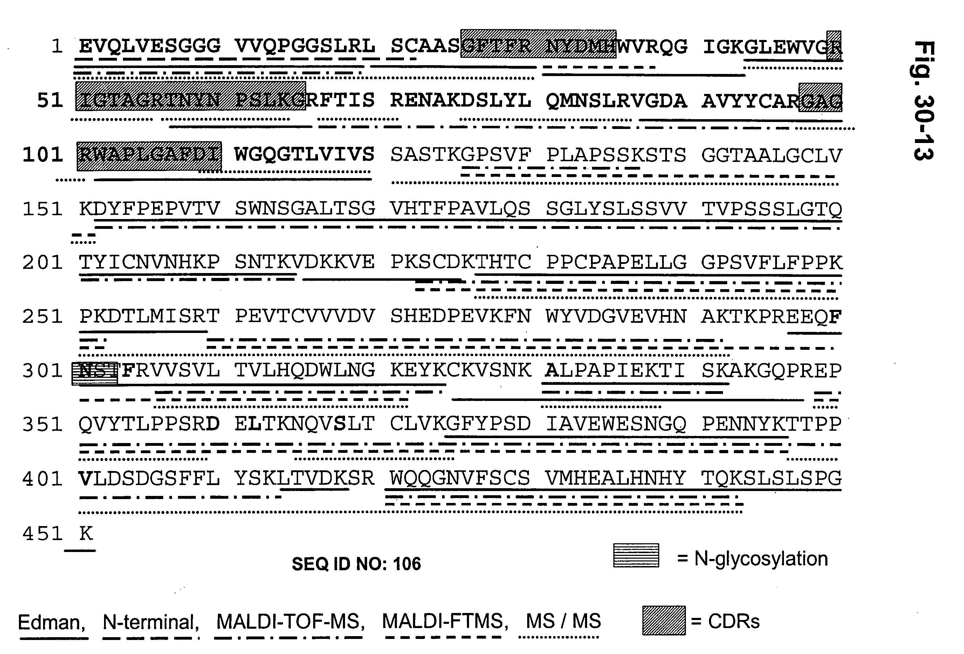

   Fig. 30-13: complete amino acid sequence of heavy chain of sample IVIG_(14), constant region isoform 1 (SEQ ID NO: 106).
   Fig: 30-14: complete amino acid sequence of heavy chain of sample IVIG_(14), constant region isoform 2 (SEQ ID NO: 107).

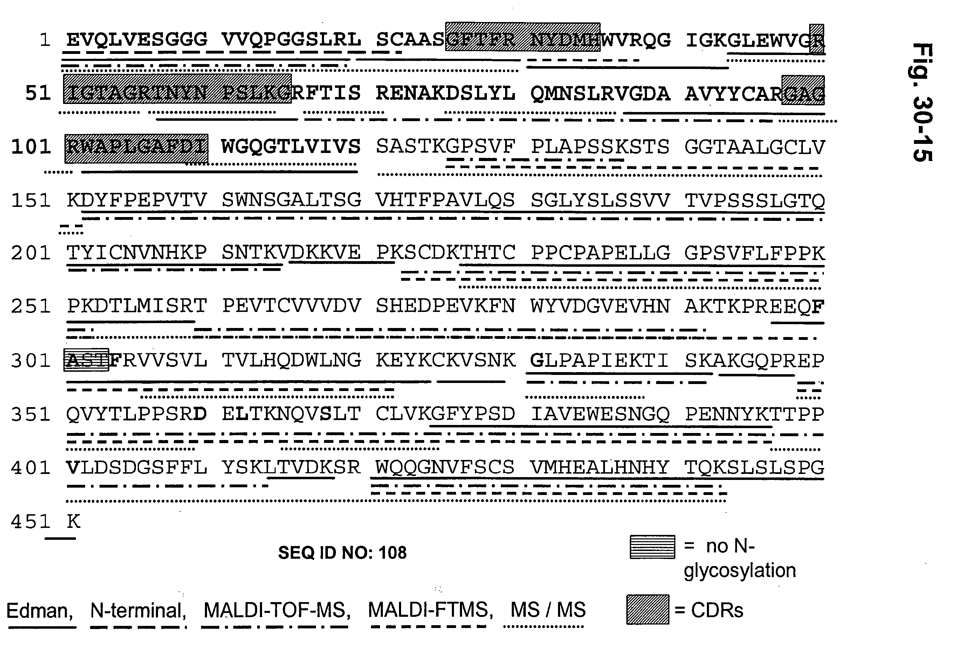

   Fig. 30-15: complete amino acid sequence of heavy chain of sample IVIG_(14), constant region isoform 3 (SEQ ID NO:108).

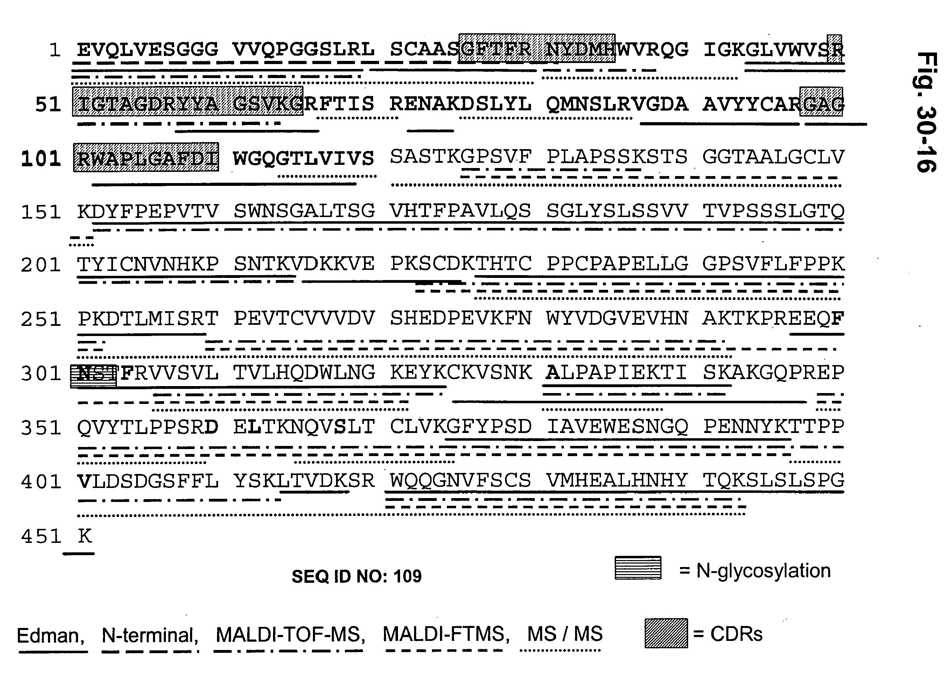

   Fig. 30-16: complete amino acid sequence of heavy chain of sample IVIG_(15), constant region isoform 1 (SEQ ID NO: 109).

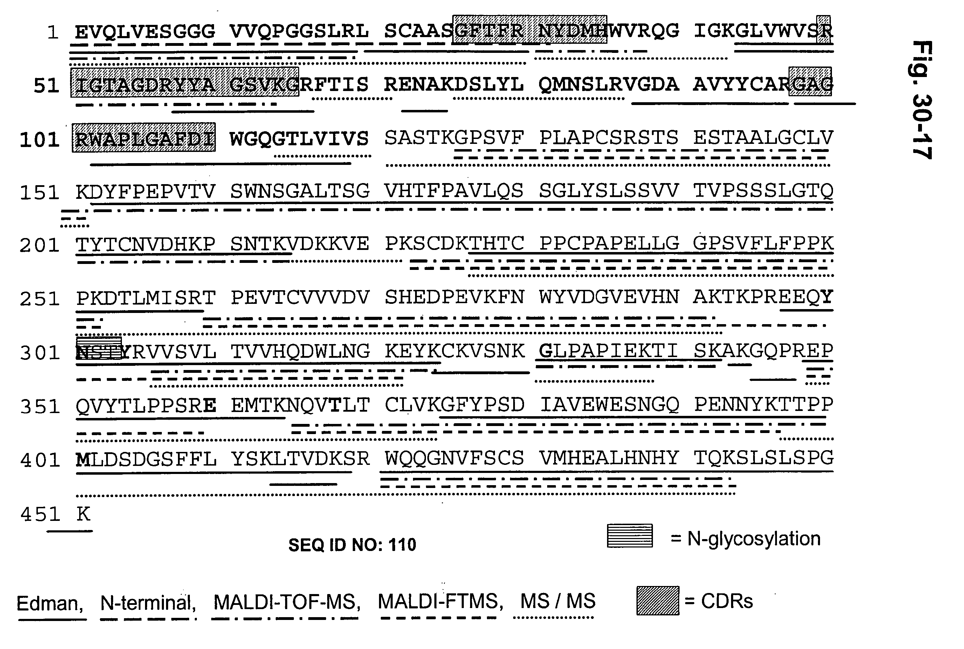

   Fig. 30-17: complete amino acid sequence of heavy chain of sample IVIG_(15), constant region isoform 2 (SEQ ID NO: 110).

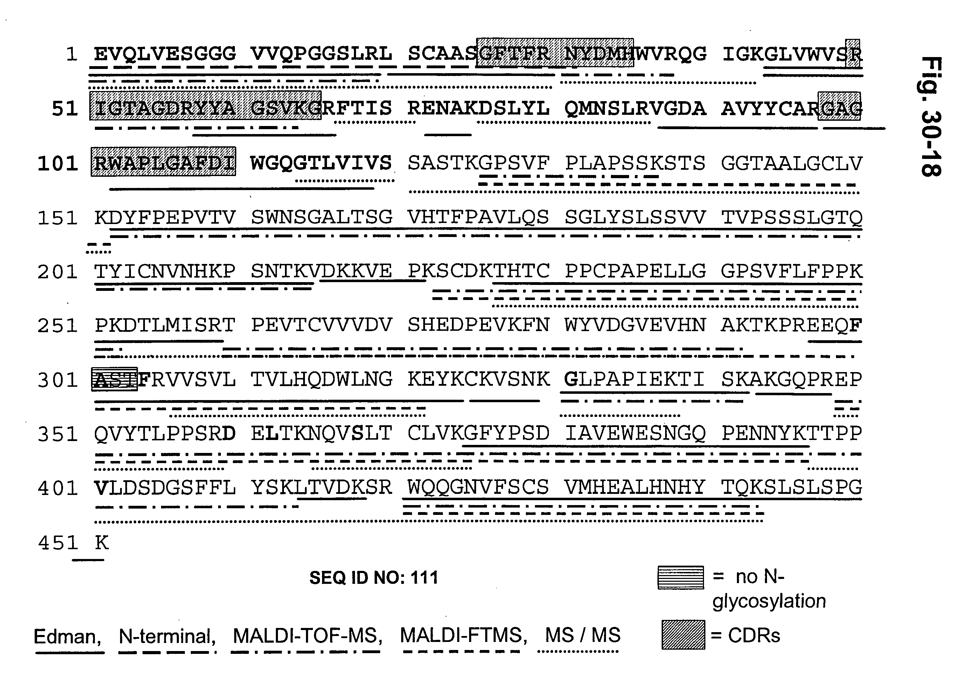

   Fig. 30-18: complete amino acid sequence of heavy chain of sample IVIG_(15), constant region isoform 3 (SEQ ID NO:111).

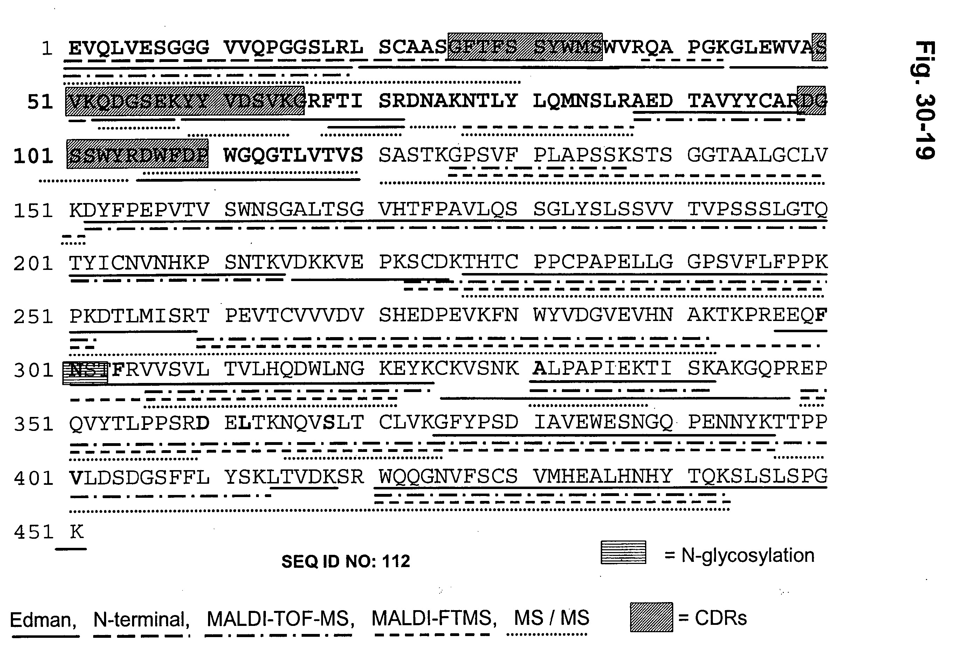

   Fig. 30-19: complete amino acid sequence of heavy chain of sample IVIG_(16), constant region isoform 1 (SEQ ID NO: 112).

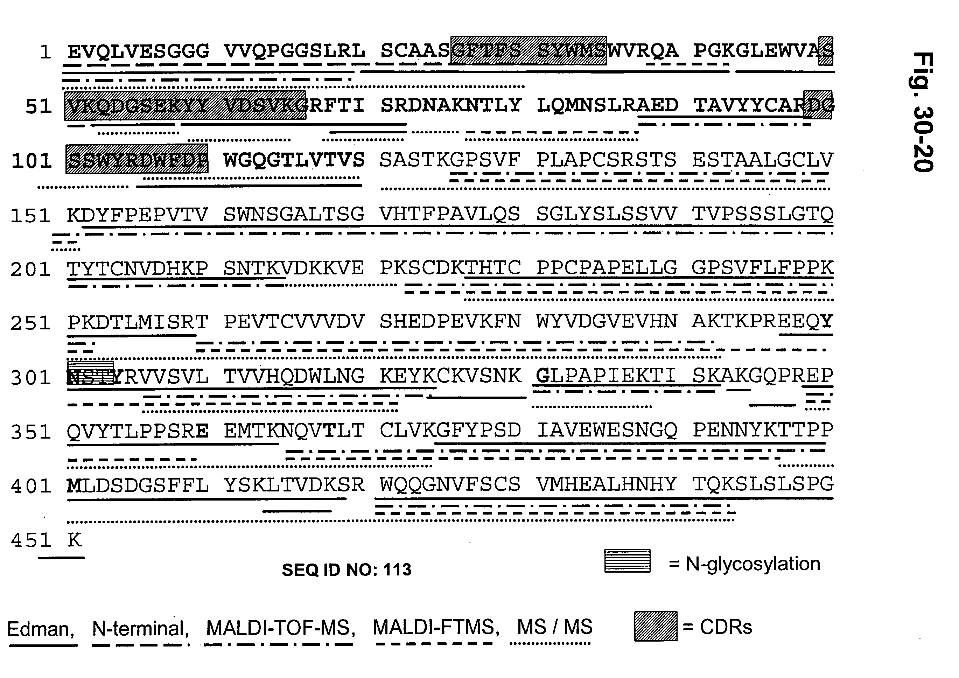

   Fig. 30-20: complete amino acid sequence of heavy chain of sample IVIG_(16), constant region isoform 2 (SEQ ID NO: 113).

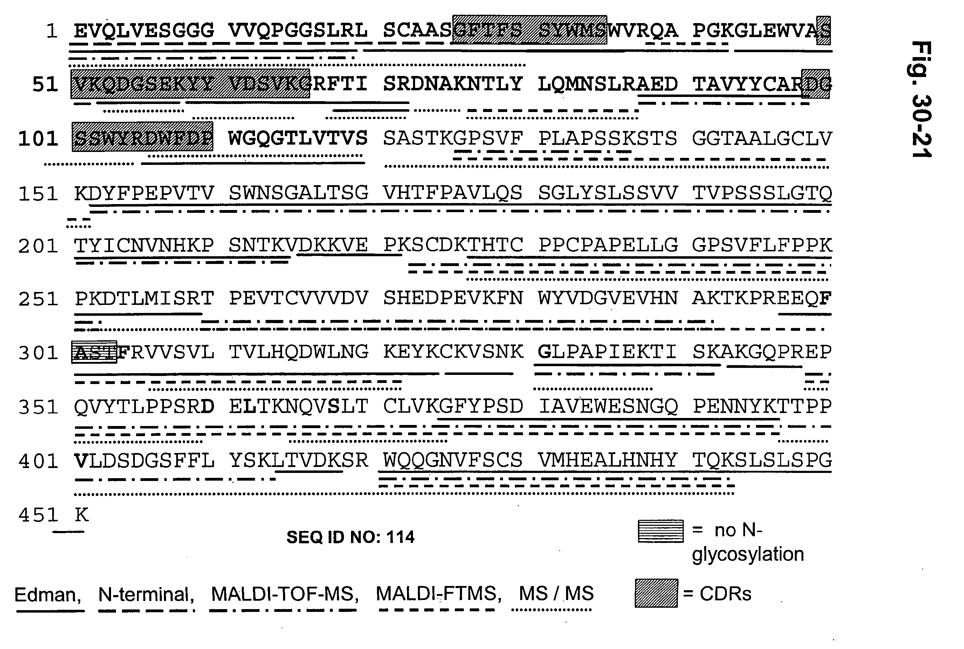

   Fig. 30-21: complete amino acid sequence of heavy chain of sample IVIG_(16), constant region isoform 3 (SEQ ID NO: 114).

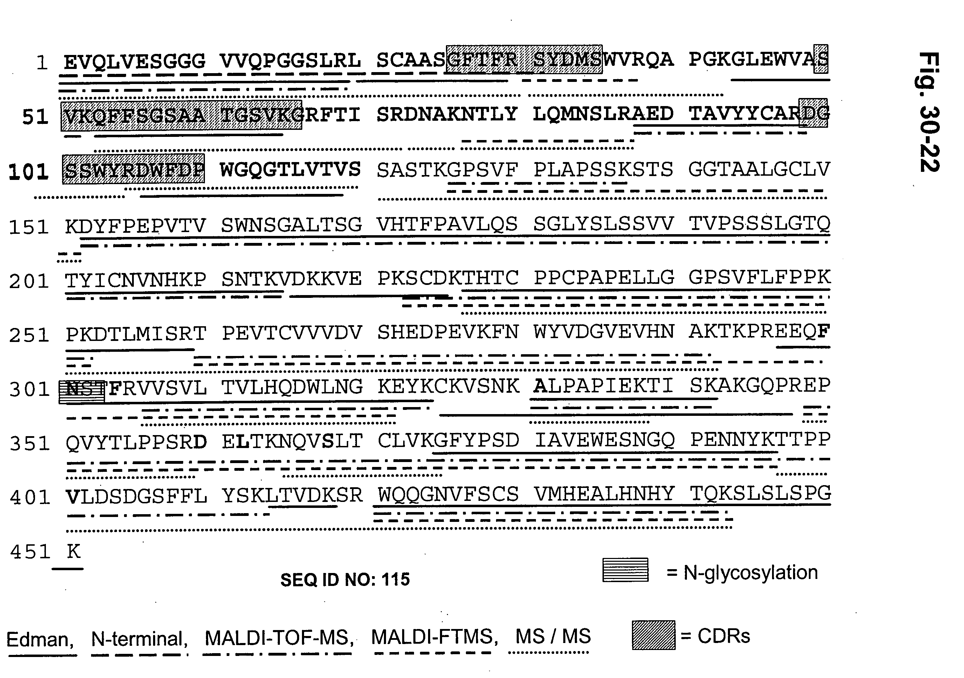

   Fig. 30-22: complete amino acid sequence of heavy chain of sample IVIG_(17), constant region isoform 1 (SEQ 1D NO:115).

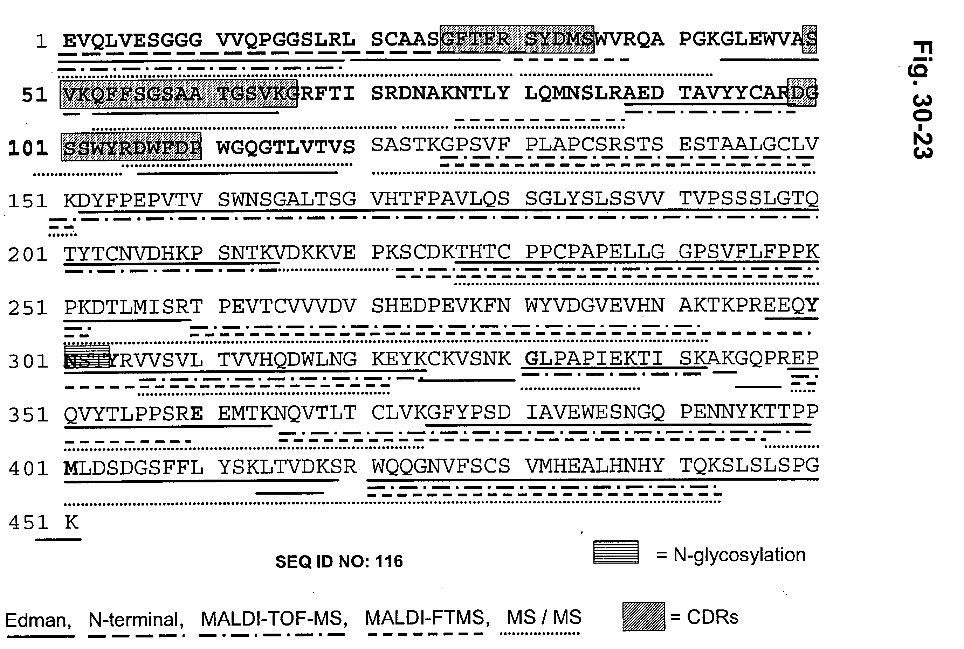

   Fig. 30-23: complete amino acid sequence of heavy chain of sample IVIG_(17), constant region isoform 2 (SEQ ID NO: 116).

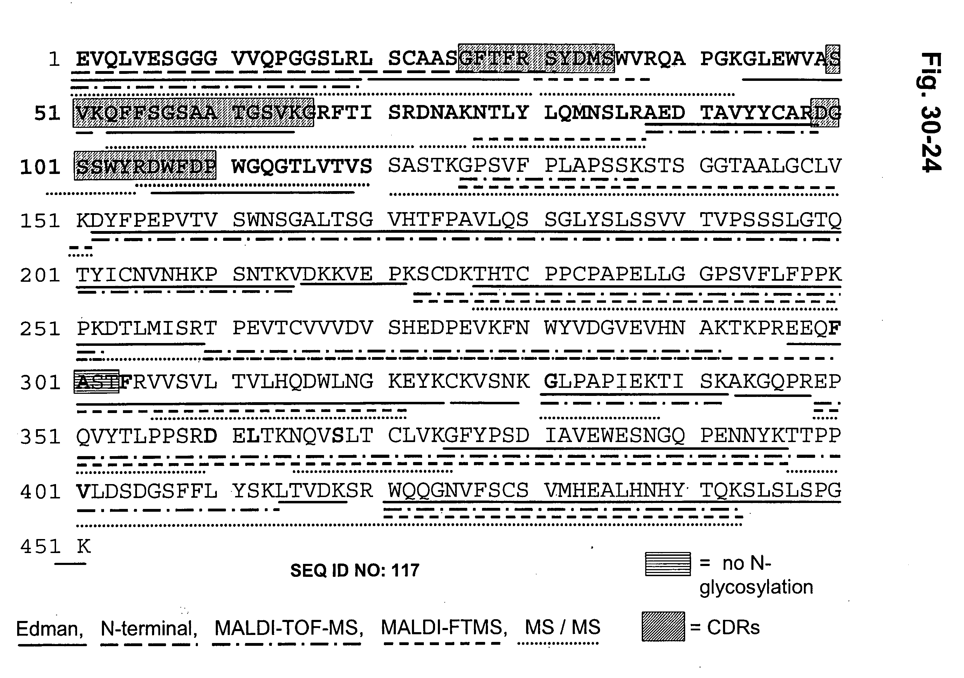

   Fig. 30-24: complete amino acid sequence of heavy chain of sample IVIG_(17), constant region isoform 3 (SEQ ID NO: 117).

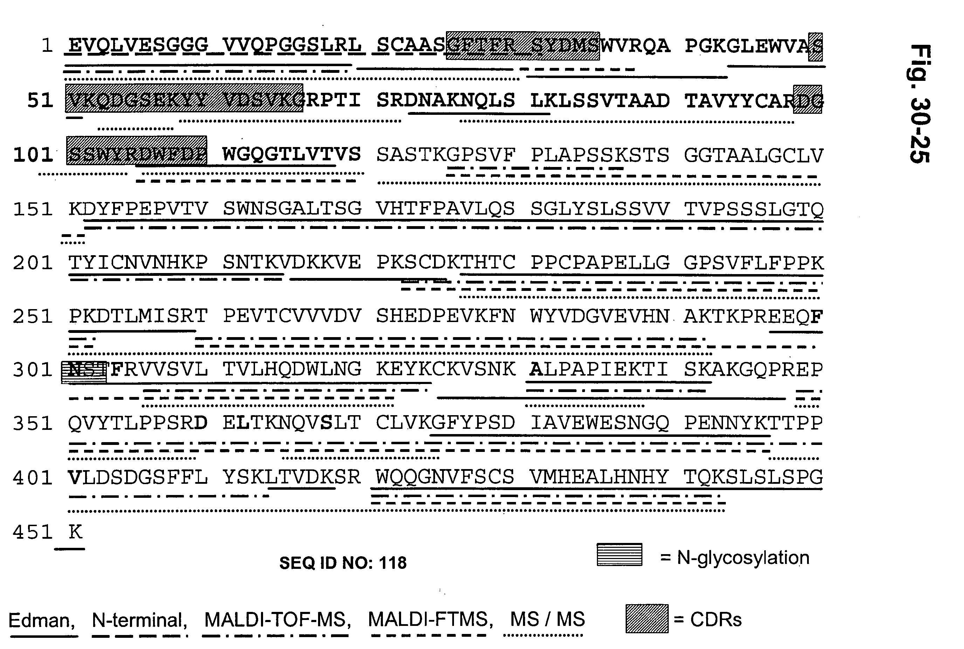

   Fig. 30-25: complete amino acid sequence of heavy chain of sample IVIG_(18), constant region isoform 1 (SEQ ID NO: 118).

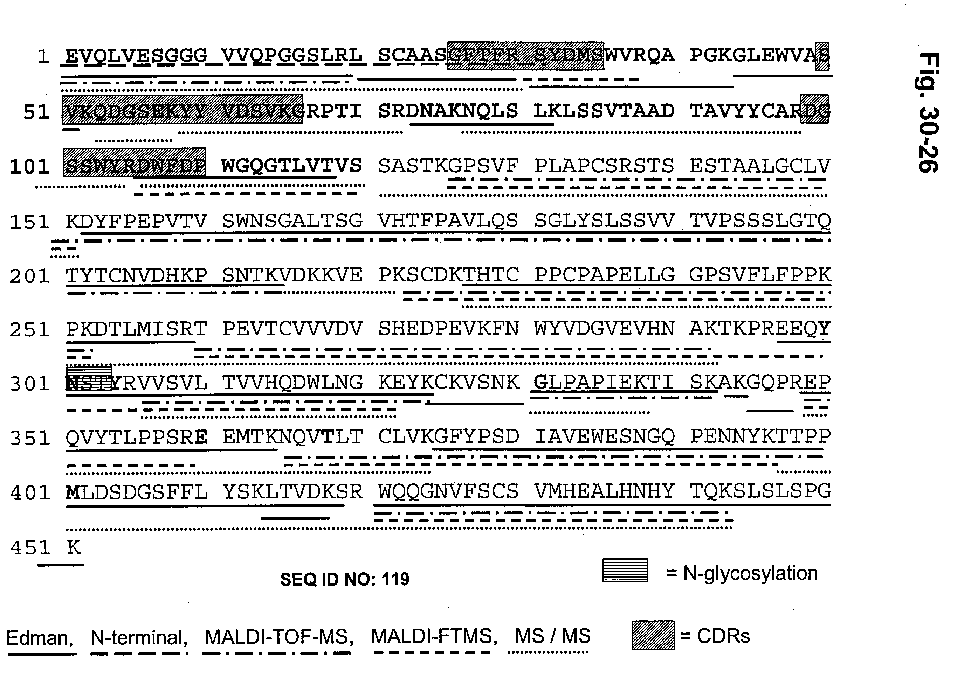

   Fig. 30-26: complete amino acid sequence of heavy chain of sample IVIG_(18), constant region isoform 2 (SEQ ID NO: 119).

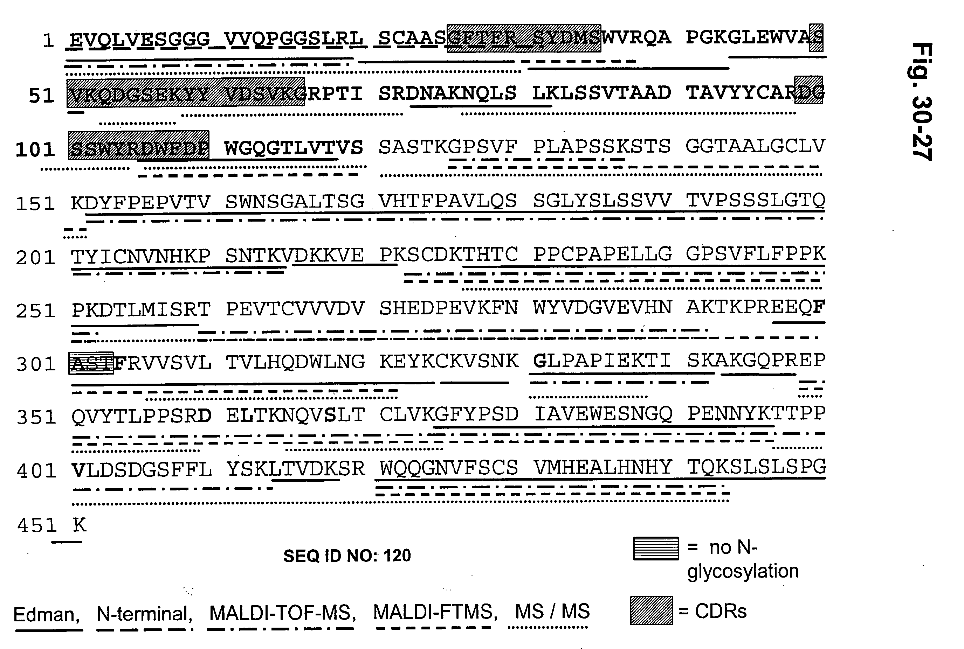

   Fig. 30-27: complete amino acid sequence of heavy chain of sample IVIG_(18), constant region isoform 3 (SEQ ID NO: 120).

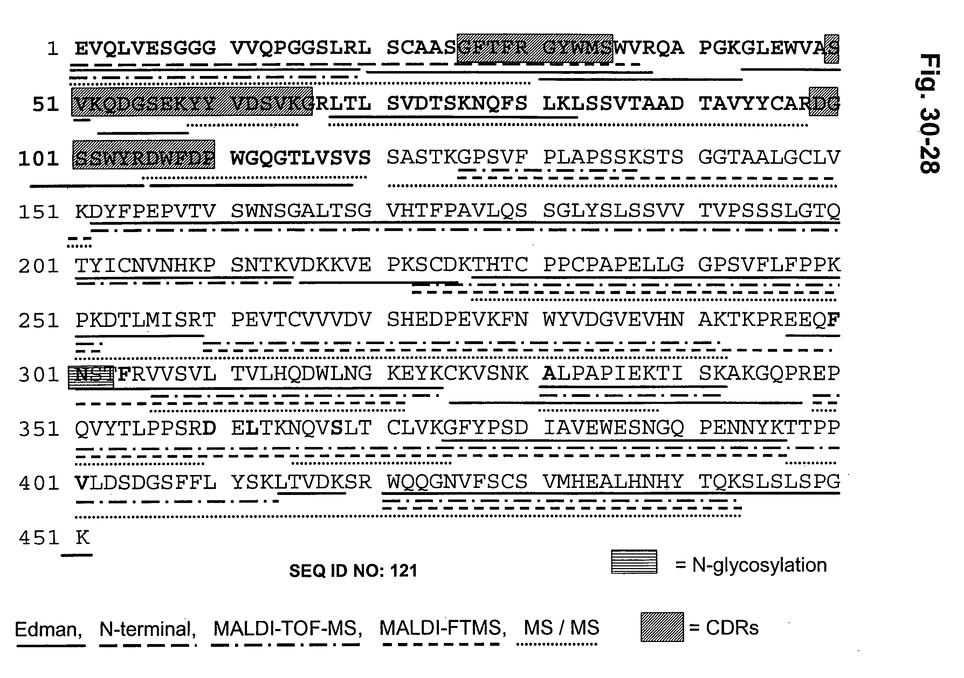

   Fig. 30-28: complete amino acid sequence of heavy chain of sample IVIG_(19), constant region isoform 1 (SEQ ID NO:121).

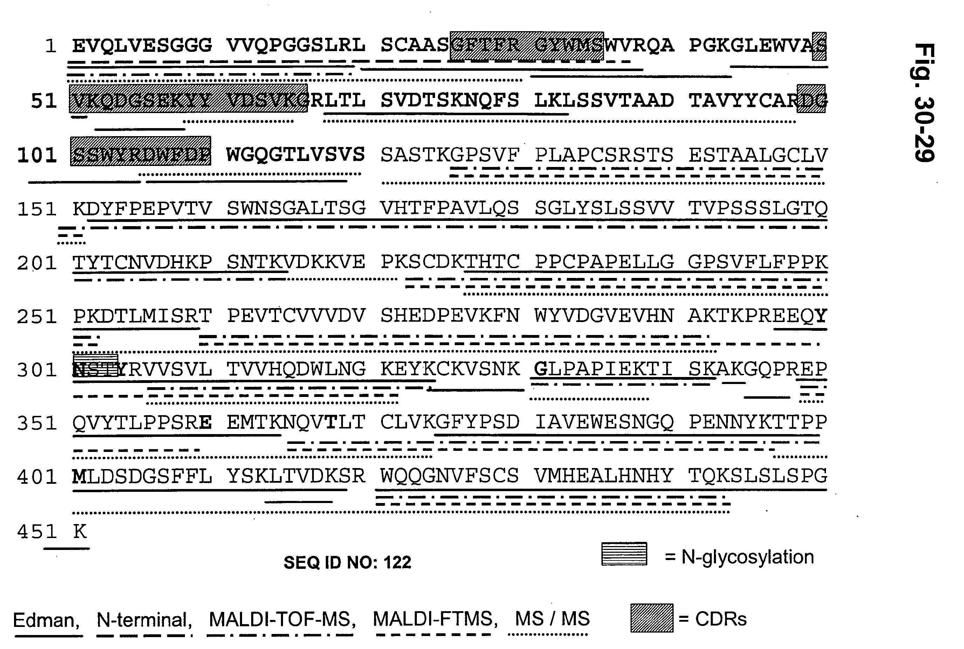

   Fig. 30-29: complete amino acid sequence of heavy chain of sample IVIG_(19), constant region isoform 2 (SEQ ID NO: 122).

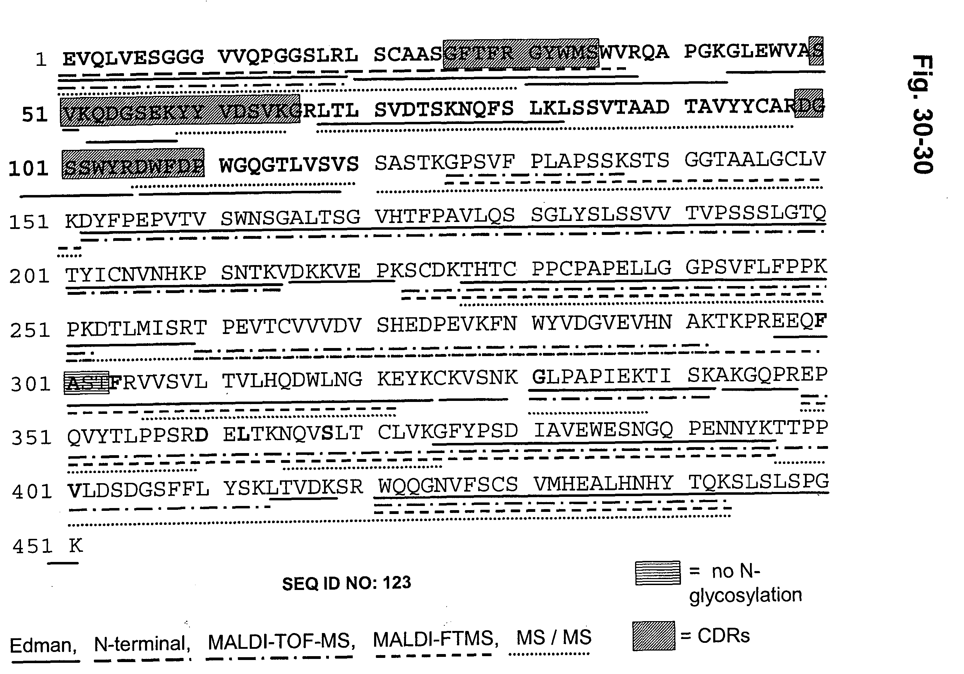

   Fig. 30-30: complete amino acid sequence of heavy chain of sample IVIG_(19), constant region isoform 3 (SEQ ID NO:123).

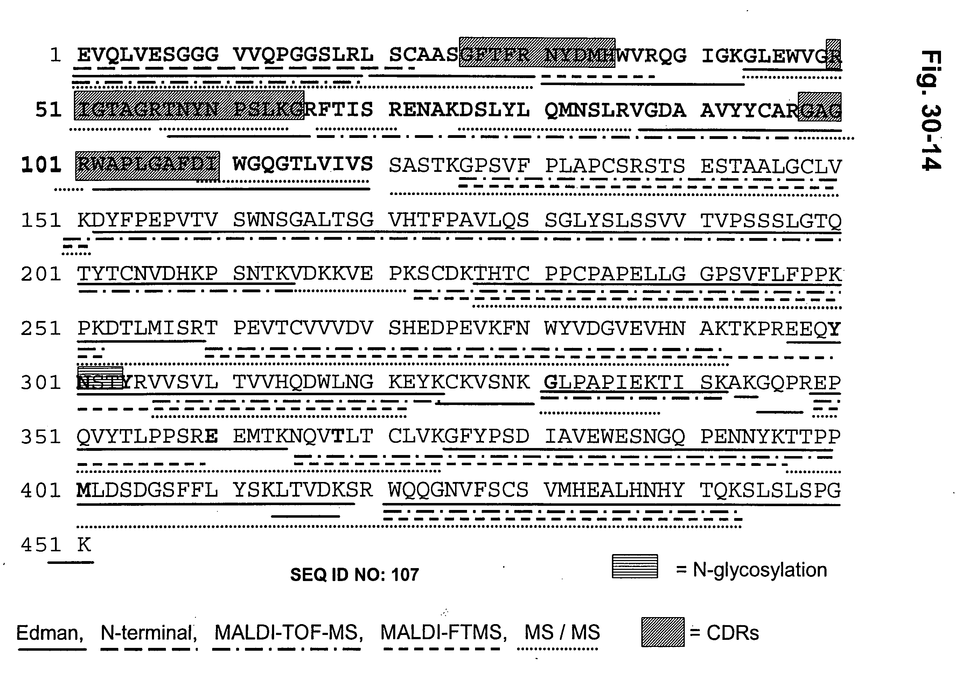

   Fig. 30-31: complete amino acid sequence of heavy chain of sample IVIG_(20), constant region isoform 1 (SEQ ID NO: 124).
   Fig. 30-32: complete amino acid sequence of heavy chain of sample IVIG_(20), constant region isoform 2 (SEQ ID NO:125).
   Fig. 30-33: complete amino acid sequence of heavy chain of sample IVIG_(20), constant region isoform 3 (SEQ ID NO:126).
   Fig. 30-34: complete amino acid sequence of heavy chain of sample IVIG_(21), constant region isoform 1 (SEQ ID NO: 127).
   Fig. 30-35: complete amino acid sequence of heavy chain of sample IVIG_(21), constant region isoform 2 (SEQ ID NO:128).
   Fig. 30-36: complete amino acid sequence of heavy chain of sample IVIG_(21), constant region isoform 3 (SEQ ID NO: 129).
   Fig. 30-37: complete amino acid sequence of heavy chain of sample Serum_(22), constant region isoform 1 (SEQ ID NO: 130).
   Fig. 30-38: complete amino acid sequence of heavy chain of sample Serum_(22), constant region isoform 2 (SEQ ID NO:131).
   Fig. 30-39: complete amino acid sequence of heavy chain of sample Serum_(23), constant region isoform 1 (SEQ ID NO:132).
   Fig. 30-40: complete amino acid sequence of heavy chain of sample Serum_(23), constant region isoform 2 (SEQ ID NO: 133).
   Fig. 30-41 complete amino acid sequence of heavy chain of sample Serum_(24), constant region isoform 1 (SEQ ID NO: 134).
   Fig. 30-42: complete amino acid sequence of heavy chain of sample Serum-(24), constant region isoform 3 (SEQ ID NO: 1 35).
   Fig. 30-43 complete amino acid sequence of heavy chain of sample Serum_(24), constant region isoform 1 (SEQ ID NO: 136).
   Fig. 30-44: complete amino acid sequence of heavy chain of sample Serum_(24), constant region isoform 3 (SEQ ID NO: 137).

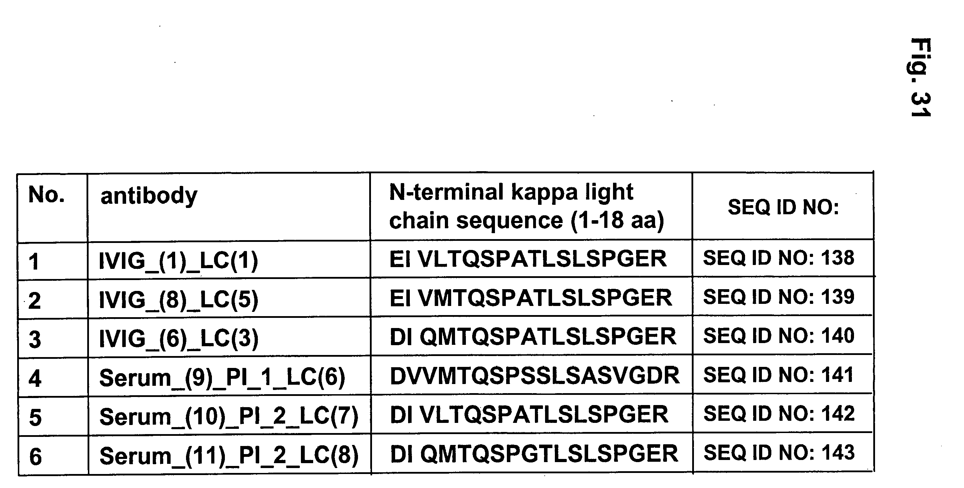

**Figure 31****:** Table illustrating the conserved nature of the N-terminus of kappa light chain of the antibodies sequenced for the present invention. Indicated are the 6 types of N-terminal sequences, consisting of 18 amino acid residues, which were identified in the kappa light chain sequences of the antibodies of the present invention.

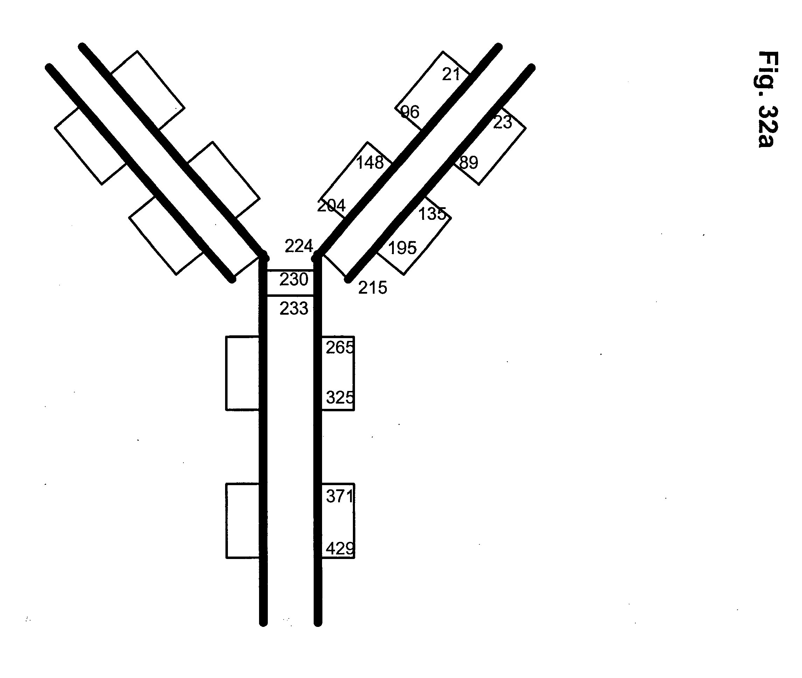

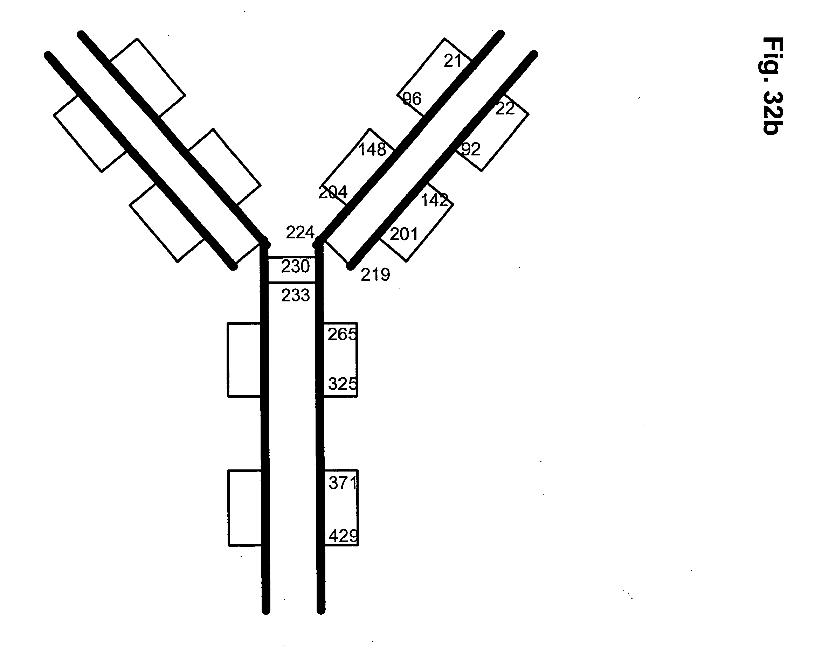

**Figure 32a** **and b:** Scheme of intra- and inter-disulfide linkages ofAβ-autoantibodies for HC-LCkappa and HC-LClambda connections. HC Intradisulfide linkages are C21 - C96, C 148 - C204, C265 - C325, C371 - C429; LCkappa-intradisulfide linkages are C23 - C89, C135 - C195; LCIambda-intradisulfide linkages are C22 - C92, C142 - C201; HC-HC interdisulfide linkages are C230 - C230, C233 - C33; HC - LCkappa- and HC- LClambda- interdisulfide linkages are C224 - C215 and C224 - C219, respectively. 32a: IVIgG_LC(1)_HC(1); 32b: IVIG_HC(1)_LCλ(3).

### Definitions:

The term "polypeptide", as used herein, refers to a polypeptide chain of at least 5 amino acid residues. The term also refers to an assembly of more than one polypeptide chain, e.g. an assembly of four polypeptide chains, such as an IgG antibody.

The term "polypeptide according to the present invention", as used herein, refers to any polypeptide capable of binding specifically to the epitope Aβ(21-37) (SEQ ID NO 2) of amyloid beta, in particular under physiological conditions, e.g. pH about 7.4, salt concentrations about 50 to about 150 mM in PBS. Preferably, the specific binding has under in vitro conditions a relative dissociation constant (relative K_{D}; reflecting *in vitro* results but not necessarily identical values under *in vivo* conditions) of at least about 10⁻⁵ M or higher, more preferably about 10⁻⁶ M, about 10⁻⁷ M, about 10⁻⁸, about 10⁻⁹ M, about 10⁻¹⁰ M, about 10⁻¹¹ M, about 10⁻¹² M, or even higher. In a particularly preferred embodiment the relative dissociation constant of the binding of said polypeptide to said epitope is in between about 10⁻⁸ M to about 10⁻¹² M, in particular around 1 to 50 x 10⁻⁹ M.

The term "antibody", as used herein, comprises also derivatives and/or fragments of antibodies. Such derivatives or fragments of antibodies retain the antigen binding properties of the intact antibody, but which lack some sequences of the intact antibody, for example the Fc-domain. Examples for such derivatives or fragments are for example Fab or F(ab')₂ fragments, which are obtainable via enzymatical digest of antibodies with papain or pepsin protease, respectively. Another example for a fragment of an antibody is a single chain variable fragment (scFv) of an antibody.

The term "autoantibody" or "autoantibodies", as used herein, refers in general to antibodies which are directed against epitopes on proteins of the human body and which can be found in the blood or cerebrospinal fluid of a human subject without prior immunisation with the respective antigen. The term herein particularly refers to antibodies binding to the C-terminal half of Aβ polypeptide, in particular to Aβ(21-37)(SEQ ID NO:2).

The term "epitope" or "epitope peptide", as used herein, generally refers to a polypeptide comprising the molecular recognition peptide sequence or structure derived from an antigen, that is bound by a specific antibody. It is an immunological determinant group of an antigen which is specifically recognized by the antibody. An epitope may comprise at least 5, preferably at least 8 amino acids in a spatial or discontinuous conformation. An epitope may also comprise a single segment of a polypeptide chain comprising a continuous linear amino acid sequence with a minimal length of approx. 5 amino acids.

The term "plaque-specific" antibody, as used herein, refers to an antibody directed against the Aβ(4-10) epitope of Aβ polypeptide.

The terms "neurodementing diseases", "dementing disorders" or "neurodementia diseases of AD-type", as used herein, refer to diseases selected from Alzheimer's disease, Down's syndrome, Dementia with Lewy bodies, fronto-temporal dementia as well as to disorders such as cerebral amyloid angiopathy and amyloidoses.

The term "paratope" or "paratope peptide", as used herein, generally defines a molecular recognition peptide sequence derived from a specific monoclonal or polyclonal antibody. This recognition peptide sequence exerts specific binding properties to an antigen epitope, and may comprise variable and/or constant region partial sequences of an antibody.

The term "CDR", as used herein, refers to Complementarity Determining Regions. Usually 3 of such CDR-regions (CDR1, CDR2, CDR3) can be found in the variable region on the light chain as well as on the heavy chain of an antibody. Each of these six hypervariable loops contributes significantly to the antigen specificity of the antibody. However, if used herein, the term CDR does not imply that the molecule referred to is in fact an antibody. Rather, the term is considered to designate a sequence contributing to the specific binding of an polypeptide according to the invention to the C-terminal half of Aβ polypeptide, in particular contributing to the binding to Aβ(21-37) polypeptide. Consequently, also derivatives of antibodies or other polypeptides engineered for binding to said epitope can exhibit CDRs.

In one aspect the present invention relates to a polypeptide binding specifically to the epitope Aβ(21-37) (SEQ ID NO 2).

A polypeptide according to the present invention can be for example an antibody, an antibody fragment, or any other polypeptide, binding to the C-terminal portion of the Aβ polypeptide, in particular to the epitope denoted in Aβ(21-37) (SEQ ID NO:2).

In particular, a polypeptide according to the present invention can comprise a CDR sequence stretch having a sequence as denoted in any of SEQ ID NOs: 6 to 8. SEQ ID NOs: 6 to 8 represent consensus sequences for CDR regions on the heavy chain of an antibody having the ability to bind to SEQ ID NO:2. The consensus sequences are derived from the sequence information derived from the antibodies, which have been identified by the inventors to bind specifically to SEQ ID NO:2.

SEQ ID NO:6 represents a CDR1 consensus sequence for heavy chain, wherein X at position 2 of SEQ ID NO:6 can be Phe or IIe, X at position 5 of SEQ ID NO:6. can be Arg or Ser, X at position 6 of SEQ ID NO:6 can be Ser, Gly or Asn, X at position 8 of SEQ ID NO:6 can be Trp, Asp or Met, and/or wherein X at position 10 of SEQ ID N0:6 can be Ser or His.

SEQ ID NO:7 represents a CDR2 consensus sequence for heavy chain, wherein X at position 5 of SEQ ID NO:7 can be Asp, Thr or Phe, X at position 6 of SEQ ID NO:7 can be Gly, Ala or Phe, X at position 8 of SEQ ID NO:7 can be Ser or Gly, X at position 9 of SEQ ID NO:7 can be Glu, Asp, Arg or Gly, X at position 10 of SEQ ID NO:7 can be Lys, Pro, Ser or Thr, X at position 11 of SEQ ID NO:7 can be Ala, Leu, Tyr or Asn, X at position 12 of SEQ ID NO:7 can be Tyr or Ala, and/or wherein X at position 13 of SEQ ID NO:7 can be Asp, Gly or Pro.

SEQ ID NO:8 represents a CDR3 consensus sequence for heavy chain, wherein X at position 3 of SEQ ID NO:8 can be Ala or Gly, X at position 4 of SEQ ID NO:8 can be Ser, Arg or Gly, X at position 5 of SEQ ID NO:8 can be Ser, Arg or Trp, X at position 7 of SEQ ID NO:8 can be Tyr or Ala, X at position 8 of SEQ ID N0:8 can be Arg, Asp or Leu, X at position 9 of SEQ ID NO:8 can be Asp, Leu or Gly, X at position 10 of SEQ ID NO:8 can be Ala or Trp, and/or wherein X at position 13 of SEQ ID NO:8 can be Pro or Ile.

In one embodiment, the polypeptide according to the invention comprises as CDRs all three respective consensus CDR sequences as denoted in SEQ ID NO:6 to 8.

In another embodiment, the polypeptide according to the invention comprises a CDR sequence stretch having a sequence as denoted in any of SEQ ID NOs: 9 to 12.

SEQ ID NOs: 9 to 12 represent consensus sequences for CDR regions on the light chain of an antibody having the ability to bind to SEQ ID NO:2. The consensus sequences are derived from the sequence information derived from the antibodies, which have been identified by the inventors to bind specifically to SEQ ID NO:2.

SEQ ID NO:9 represents a CDR1 consensus sequence for kappa light chain immunoglobulin CDR1 region, wherein X at position 2 of SEQ ID NO:9 can be Glu or Ala, X at position 5 of SEQ ID NO:9 can be Gly or Ser, X at position 6 of SEQ ID NO:9 can be Ile or Val, X at position 7 of SEQ ID NO:9 can be Arg, Asn or Ser, and/or wherein X at position 8 of SEQ ID NO:9 can be Ser or Asn.

SEQ ID NO:10 represents a CDR2 consensus sequence for kappa light chain immunoglobulin CDR2 region, wherein X at position 2 of SEQ ID NO: 1 0 can be Gly, Ala or Ser, X at position 3 of SEQ ID NO: 1 0 can be Ser or Ala, and/or wherein X at position 4 of SEQ ID NO: 1 0 can be Thr, Ile, Ser or Leu.

SEQ ID NO:11 represents an additional CDR2 consensus sequence for kappa light chain immunoglobulin CDR2 region, wherein X at position 2 of SEQ ID NO:11 can be Gly, Ala or Ser, X at position 3 of SEQ ID NO:11 can be Ser or Ala, and/or wherein X at position 4 of SEQ ID NO:11 can be Thr, Ile, Ser or Leu.

SEQ ID NO:12 represents an additional CDR3 consensus sequence for kappa light chain immunoglobulin CDR3 region, wherein X at position 3 of SEQ ID NO:12 can be Tyr or Ala, X at position 4 of SEQ ID NO:12 can be Gly or Asn, X at position 6 of SEQ ID NO:12 can be Ser or Phe, X at position 7 of SEQ ID NO:12 can be Gln or Pro, and/or wherein X at position 8 of SEQ ID NO:12 can be Gly or Leu.

In one embodiment, an the polypeptide according to the invention comprises as CDRs for the light chain all three respective consensus CDR sequences as denoted in SEQ ID NO:9 to 12. Even more preferred is a polypeptide comprising as CDR sequences one, two or three sequences selected from the consensus CDR sequences denoted for the light chain (SEQ ID NO:9 to 12) and/or one, two or three of the consensus CDR sequences denoted for the heavy chain (SEQ ID NO: 6 to 8).

In a further embodiment, the polypeptide according to the invention comprises as CDR1 on the heavy chain one of the sequences as denoted in SEQ ID NOs: 13 to 20, as CDR2 on the heavy chain one of the sequence as denoted in SEQ ID NOs: 21 to 27, and/or as CDR3 on the heavy chain one of the sequences as denoted in SEQ ID NOs: 28 to 32.

SEQ ID NOs 13 to 20 represent the CDR1 sequences, SEQ ID NOs 21 to 27 represent the CDR2 sequences and SEQ ID NOs 28 to 32 represent the CDR3 sequences found by the inventors to be present on the heavy chain of antibodies binding to the C-terminal half of Aβ(1-40), in particular to Aβ(21-37).

In a further embodiment, the polypeptide according to the present invention comprises as CDR1 on the light chain one of the sequences as denoted in SEQ ID NOs: 33 to 37, as CDR2 on the light chain one of the sequence as denoted in SEQ ID NOs: 38 to 43, and/or as CDR3 on the light chain one of the sequences as denoted in SEQ ID NOs: 44 to 46.

SEQ ID NOs 33 to 37 represent the CDR1 sequences, SEQ ID NOs 38 to 43 represent the CDR2 sequences and SEQ ID NOs 44 to 46 represent the CDR3 sequences found by the inventors to be present on the light chain of antibodies binding to the C-terminal half of A(1-40), in particular to Aβ(21-37).

In a preferred embodiment, a polypeptide according to the present invention comprises on the heavy chain a CDR1 sequence selected from SEQ m NOs: 13 to 20, a CDR2 sequence selected from SEQ ID NOs: 21 to 27, and/or a CDR3 sequence selected from SEQ m NOs:

28 to 32, and on the light chain a CDR1 sequence selected from SEQ ID NOs: 33 to 37, a CDR2 sequence selected from SEQ ID NOs: 38 to 43 and/or a CDR3 sequence selected from SEQ ID NOs: 44 to 46.

In particular, a polypeptide according to the present invention can comprise on the light chain:
a) as CDR1 SEQ ID NO:34, as CDR2 SEQ ID NO:38 and as CDR3 SEQ ID NO:44, or
b) as CDR1 SEQ ID NO:33, as CDR2 SEQ ID NO:42 and as CDR3 SEQ ID NO:44, or
c) as CDR1 SEQ ID NO:37, as CDR2 SEQ ID NO:43 and as CDR3 SEQ ID NO:46, or
d) as CDR1 SEQ ID NO:34, as CDR2 SEQ ID NO:40 and as CDR3 SEQ ID NO:44, or
e) as CDR1 SEQ ID NO:35, as CDR2 SEQ ID NO:38 and as CDR3 SEQ ID NO:44, or
f) as CDR1 SEQ ID NO:33, as CDR2 SEQ ID NO:41 and as CDR3 SEQ ID NO:44, or
g) as CDR1 SEQ ID NO:33, as CDR2 SEQ ID NO:41 and as CDR3 SEQ ID NO:45, or
h) as CDR1 SEQ ID NO:34, as CDR2 SEQ ID NO:38 and as CDR3 SEQ ID NO:44, or
i) as CDR1 SEQ ID NO:36, as CDR2 SEQ ID NO:39 and as CDR3 SEQ ID NO:44.

In particular, a polypeptide according to the present invention can also comprise on the heavy chain:
a) as CDR1 SEQ ID NO:13, as CDR2 SEQ ID NO:21 and as CDR3 SEQ ID NO:28, or
b) as CDR1 SEQ ID NO: 14, as CDR2 SEQ ID NO:27 and as CDR3 SEQ ID NO:30, or
c) as CDR1 SEQ ID NO:13, as CDR2 SEQ ID NO:26 and as CDR3 SEQ ID NO:28, or
d) as CDR1 SEQ ID NO:14, as CDR2 SEQ ID NO:21 and as CDR3 SEQ ID NO:30, or
e) as CDR1 SEQ ID NO:15, as CDR2 SEQ ID NO:23 and as CDR3 SEQ ID NO:29, or
f) as CDR1 SEQ ID NO:15, as CDR2 SEQ ID NO:22 and as CDR3 SEQ ID NO:29, or
g) as CDR1 SEQ ID NO:20, as CDR2 SEQ ID NO:27 and as CDR3 SEQ ID NO:31, or
h) as CDR1 SEQ ID NO:18, as CDR2 SEQ ID NO:25 and as CDR3 SEQ ID NO:31, or
i) as CDR1 SEQ ID NO:18, as CDR2 SEQ ID NO:27 and as CDR3 SEQ ID NO:31, or
j) as CDR1 SEQ ID NO: 14, as CDR2 SEQ ID NO:27 and as CDR3 SEQ ID NO:31, or
k) as CDR1 SEQ ID NO:14, as CDR2 SEQ ID NO:21 and as CDR3 SEQ ID NO:31, or
l) as CDR1 SEQ ID NO: 16, as CDR2 SEQ ID NO:21 and as CDR3 SEQ ID NO:31, or
m) as CDR1 SEQ ID NO:19, as CDR2 SEQ ID NO:21 and as CDR3 SEQ ID NO:32, or
n) as CDR1 SEQ ID NO:16, as CDR2 SEQ ID NO:21 and as CDR3 SEQ ID NO:28, or
o) as CDR1 SEQ ID NO:17, as CDR2 SEQ ID NO:26 and as CDR3 SEQ ID NO:31, or
p) as CDR1 SEQ ID NO:14, as CDR2 SEQ ID NO:24 and as CDR3 SEQ ID NO:28.

Even more preferred is a polypeptide according to the present invention comprising:
a) on the light chain as CDR1 SEQ ID NO:34, as CDR2 SEQ ID NO:38 and as CDR3 SEQ ID NO:44, and on the heavy chain as CDR1 SEQ ID NO:13, as CDR2 SEQ ID NO:21 and as CDR3 SEQ ID NO:28, or
b) on the light chain as CDR1 SEQ ID NO:33, as CDR2 SEQ ID NO:42 and as CDR3 SEQ ID NO:44, and on the heavy chain as CDR1 SEQ ID NO:14, as CDR2 SEQ ID NO:27 and as CDR3 SEQ ID NO:30.

SEQ ID NOs: 47 to 55 denote variable regions of the light chain and SEQ ID NOs: 56 to 71 denote variable regions of the heavy chain of antibodies having the ability to bind specifically to the C-terminal half of Aβ(1-40), in particular to Aβ(21-37), as determined by the inventors. The variable regions of the heavy and light chain are responsible for antigen specificity. Therefore, in a further embodiment, the polypeptide according to the present invention comprises a sequence selected from SEQ ID NOs: 47 to 55 and/or a sequence selected from SEQ ID NOs: 56 to 71. In a particularly preferred embodiment the polypeptide according to the present invention comprises SEQ ID NO:47 and SEQ ID NO:56 or SEQ ID NO:48 and SEQ ID NO:57.

In addition, SEQ ID NOs. 72 to 74 denote constant regions of the light chain and SEQ ID NOs. 75 to 77 denote constant regions of the heavy chain of antibodies having the ability to bind specifically to the C-terminal half of Aβ(1-40), in particular to Aβ(21-37). Therefore, in a further embodiment, the polypeptide according to the present invention can comprise a sequence selected from SEQ ID NOs: 72 to 74 and/or a sequence selected from SEQ ID NOs: 75 to 77. In particular, the polypeptide according to the present invention can comprise a sequence selected from SEQ ID NO:72 or 73 and a sequence selected from SEQ ID NOs. 75 to 77.

The complete sequences, consisting of variable region and constant region, for the light chain of antibodies having the ability to bind specifically to the C-terminal half of Aβ(1-40), in particular to Aβ(21-37), are denoted in SEQ ID NOs: 78 to 93.

Therefore, in another embodiment of the present invention, the polypeptide according to the present invention comprises a sequence selected from SEQ ID NOs: 78 to 93.

The complete sequences, consisting of variable region and constant region, for the heavy chain of antibodies having the ability to bind specifically to the C-terminal half of Aβ(1-40), in particular to Aβ(21-37), are denoted in SEQ ID NOs: 94 to 137. Therefore, in another embodiment of the present invention, the polypeptide according to the present invention comprises a sequence selected from SEQ ID NOs: 94 to 137.

In an even more preferred embodiment, the polypeptide according to the present invention comprises a sequence as denoted in SEQ ID NO:78 and a sequence as denoted in SEQ ID NO:94 or a sequence as denoted in SEQ ID NO:80 and a sequence as denoted in SEQ ID NO:97. The same applies to the analogous isoforms of respective chains and combinations thereof (as can be taken from Fig. 23a).

It has to be noted that the inventors found, that the N-terminal sequence, about 18 amino acid residues, of kappa light chain of antibodies having the ability to bind specifically to the C-terminal half of Aβ(1-40), in particular to Aβ(21-37), is well conserved. This applies for antibodies derived from IvIgG preparations as well as for antibodies derived from patient serum. In Fig. 31 said sequences are depicted, additionally also denoted as SEQ ID NOs: 138 to 143. It is contemplated, that the conservative nature of the N-Terminus of kappa light chain of these antibodies might contribute to antigen specificity and/or prevention of plaque formation when bound to Aβ peptide. Thus, in a preferred embodiment, the polypeptide according to the present invention comprises a sequence as denoted in the consensus sequence of SEQ ED NO: 44, in particular a sequence as denoted in SEQ ID NOs: 138 to 143.

In a preferred embodiment, where the polypeptide according to the invention is an antibody, said antibody may be a monoclonal antibody. Monoclonal antibodies have the advantage, that they exhibit less cross reactivity.

In another preferred embodiment the polypeptides according to the present invention comprises derivatives and/or fragments of antibodies binding to Aβ(21-37)(SEQ ID NO:2). It is well known in the art, that antibodies can be treated enzymatically, e.g. with proteases in order to obtain fragments of antibodies which retain the antigen binding properties of the intact antibody, but which lack the Fc-domain. Such fragments are for example Fab or F(ab')₂ fragments, which are obtainable via enzymatical digest of antibodies with papain or pepsin protease, respectively. Another fragment of an antibody is a single chain variable fragment (scFv) of an antibody, i.e. a fusion of the variable regions of the heavy and light chains of an antibody via a short flexible linker (usually serine, glycine). Normally, such a chimeric molecule retains the specificity of the original antibody, despite removal of the constant regions and the introduction of a linker peptide. Single chain variable fragments can be obtained by genetical engineering of a respective nucleic acid encoding for such a fusion protein. The advantage of such a fragment is that it consists only of a single polypeptide chain which is more easily expressed and properly folded in artificial expression systems than the whole antibody, which comprises at least 4 polypeptide chains which need a correct assembly in order to function adequately.

If the polypeptide according the invention is an antibody, a human antibody is preferred due to its low antigenic properties. However, the antibody or fragment thereof can be derived from any species suitable for antibody production. Non-human antibodies can be derived in particular from mouse, chicken, rabbit, rat, donkey, camel, dromedary, shark and llamas. Antibodies of camel, dromedary, shark and llamas have the advantage that these animals have antibodies which consist only of a single polypeptide chain. Thus, such polypeptides have similar advantages in expression and assembly as described above for single chain variable fragments.

In another preferred embodiment the antibody, derivatives or fragments thereof is a humanized antibody, derivative or fragment thereof, i.e. while the antigen binding domain or parts thereof is/are of non-human origin, the rest of the antibody, derivative or fragment thereof is of human origin. In another preferred embodiment the antibody, derivative or fragment thereof is chimeric, i.e. while the variable domain or parts thereof is/are of non-human origin the constant domain is of human origin. Both embodiments serve the purpose to reduce negative side effects due to immunogenic properties of protein domains of non-human origin. In an even more preferred embodiment, the polypeptide binding to epitope Aβ(21-37) (SEQ ID NO:2) of amyloid-beta peptide (1-40) (SEQ ID NO:1) is an antibody derivative or fragment which comprises only the paratope of an antibody binding to said epitope. Example for such a paratope is, for example, a polypeptide comprising the amino acid sequences for the respective CDR domains of heavy and light chain connected via the intervening sequences or via synthetic linkers.

In another aspect the present invention relates to the use of the above mentioned polypeptides according to the present invention for use in human and veterinary medicine.

In particular, the polypeptides according to the present invention can be used for the manufacture of a medicament in order to treat and/or prevent the progression of Alzheimer's disease, Down's syndrome, Dementia with Lewy bodies, fronto-temporal dementia, cerebral amyloid angiopathy, and/or amyloidoses.

Furthermore, the present invention relates to the use of a polypeptide comprising an amino acid sequence of an Aβ peptide, wherein the Aβ peptide has at least the sequence according to SEQ ID NO:2 and at most the sequence according to SEQ ID NO:4 for use in human and veterinary medicine, in particular for the manufacture of a medicament for the treatment and/or prevention of Alzheimer's disease, Down's syndrome, Dementia with Lewy bodies, fronto-temporal dementia, cerebral amyloid angiopathy, and amyloidoses.

In this invention, the inventors identified two Aβ epitope sequences recognized by Aβ-autoantibodies isolated from serum of AD patients as well as of healthy control individuals. The Aβ-autoantibodies of healthy control individuals were found to specifically recognize the C-terminal part of the Aβ sequence, namely Aβ(21-37) (SEQ ID NO:2). Furthermore, AD patients have an increased fraction of antibodies recognizing the Aβ(4-10) part of the Aβ polypeptide (SEQ ID NO:3), while having a decreased fraction of the antibodies recognizing the Aβ(21-37) part of Aβ polypeptide. Additionally, the inventors found that in healthy individuals not suffering from AD, the ratio of autoantibodies directed against a specific C-terminal epitope of Aβ, namely Aβ(21-37), is much higher than in AD patients. The binding to this epitope seems to inhibit the formation of plaques and therefore delay the onset or progression of AD. This provides the basis for a new, therapeutic approach by administration of agents to a human subject or animal, which agents bind to said epitope and thereby prevents the aggregation of Aβ peptide. This delays the onset and/or the progression of Alzheimer's disease and provides consequently a valuable therapeutic/prophylactic pharmaceutical.

Thus, the present invention also relates to the use of the above.mentioned polypeptides for the manufacture of a medicament in order to treat and/or prevent the progression of a neurodementing disease, Alzheimer's disease, Down's syndrome, Dementia with Lewy bodies, fronto-temporal dementia, cerebral amyloid angiopathy, and amyloidoses. The treatment/prevention of the above mentioned diseases is provided by prevention of Aβ plaque formation. Depending on the stage of the respective disease this leads to a prevention of the disease (no onset yet) or to a treatment (after onset of the disease), e.g. by preventing a further formation of plaques. In a preferred embodiment the medicament is formulated for the treatment and/or prevention of plaques in the brain of a patient.

Alternatively, as already indicated above, the present invention relates also to the use of a polypeptide comprising an amino acid sequence of an Aβ polypeptide, wherein the Aβ polypeptide has at least the sequence according to SEQ ID NO:2 and at most the sequence according to SEQ ID NO:4, for the manufacture of a medicament for the treatment of Alzheimer's disease, Down's syndrome, Dementia with Lewy bodies, fronto-temporal dementia, cerebral amyloid angiopathy, and/or amyloidoses. In such a scenario, this epitope serves for the active immunisation of a human or animal subject in order to enhance endogenous antibody production against Aβ(21-37). Such immunisation approaches can also utilize DNA vaccines, which have the benefit of avoiding the administration of Aβ protein fragments. Therefore, the present invention also relates to a nucleic acid molecule encoding for a polypeptide comprising an amino acid sequence of an Aβ polypeptide, wherein the Aβ polypeptide has at least the sequence according to SEQ ID NO:2 and at most the sequence according to SEQ ID NO:4. Consequently the present invention also relates to the use of this nucleic acid sequence for the manufacture of a medicament for the treatment and/or prevention of Alzheimer's disease, Down's syndrome, Dementia with Lewy bodies, fronto-temporal dementia, cerebral amyloid angiopathy, and amyloidoses.

Administration of medicaments according to the present invention can be achieved via any common route. Although, the intravenous route is a preferred embodiment, other routes of administration are contemplated. This includes oral, nasal, buccal, rectal, vaginal or topical. Alternatively, administration may be by orthotopic, intradermal, subcutaneous, intramuscular or intraperitoneal.

In another preferred embodiment the medicament according to the present invention comprising a polypeptide according to the invention is formulated for a combined administration with a second medicament for the respective disease. Examples for such therapies would be inhibitors of acetylcholine esterase or NMDA (N-methyl-D-aspartate) receptor antagonists. The administration of the medicament according to the invention can be prior to, simultaneously with or after administration of the second medicament.

In a further aspect the present invention relates to a method of treatment and/or prevention of Alzheimer's disease, Down's syndrome, Dementia with Lewy bodies, fronto-temporal dementia, cerebral amyloid angiopathy, and amyloidoses comprising administering one or more of the above mentioned polypeptides of the invention to a subject in need thereof.

The present invention also relates to the use of a polypeptide comprising an amino acid sequence of an Aβ polypeptide, wherein the Aβ polypeptide has at least the sequence according to SEQ ID NO:2, i.e. Aβ(21-37) and at most the sequence according to SEQ ID NO:4, i.e. Aβ(12-40) for isolation and separation of a polypeptide according to the present invention from a sample.

The present invention also relates to a method of isolation and separation of a polypeptide according to the invention from a sample, the method comprising the following steps:
a) incubating a polypeptide comprising an amino acid sequence of an Aβ polypeptide, wherein the Aβ polypeptide has at least the sequence according to SEQ ID NO:2, i.e. Aβ(21-37) and at most the sequence according to SEQ ID NO:4, i.e. Aβ(12-40), which polypeptide is immobilized on a carrier, with a sample,
b) separating said sample from the carrier.

c) separating polypeptides according to the invention bound to the polypeptide of step a) from the carrier.

In an alternative approach, a polypeptide according to the invention can be obtained by incubating the sample with the polypeptide comprising an amino acid sequence of an Aβ polypeptide, wherein the Aβ polypeptide has at least the sequence according to SEQ ID NO:2 and at most the sequence according to SEQ ID NO:4 prior to incubation with the carrier. Thus, the present invention also relates to a method of separation of a polypeptide according to the invention from a sample, the method comprising the following steps:
a) incubating a polypeptide, comprising an amino acid sequence of an Aβ polypeptide, wherein the Aβ polypeptide has at least the sequence according to SEQ ID NO:2 and at most the sequence according to SEQ ID NO:4, with a sample and subsequently
b) incubating the sample with a carrier having a binding affinity for the polypeptide of step a), and
c) separating said sample from the carrier, and,
d) separating polypeptides according to the invention bound to the polypeptide of step a) from the carrier.

Strategies and techniques are well known in the art to obtain the above mentioned polypeptides, i.e. via genetic engineering and expression of the respective polypeptide in a cell line of interest. Antibody fragments according to the invention do not have to be physically derived from an intact antibody but can also be genetically engineered by conventional means.

The above mentioned polypeptides can be obtained for example by screening antibodies derived from the blood of a human subject for the capacity of binding to amino acid 21 to 37 of SEQ ID NO 1 (i.e. SEQ ID NO: 2).

A person skilled in the art with the information of this patent application at hand will readily know how to perform the methods according to the invention. One example would be to coat a sepharose column with a polypeptide having the sequence of SEQ ID NO: 2, followed by incubation with an immunoglobulin fraction (antibody fraction) derived from the sample, performing a washing step and subsequent elution of the Aβ (21-37) specific antibodies bound to the column. For the methods of separation according to the invention the polypeptide, comprising an amino acid sequence of an Aβ polypeptide, wherein the Aβ polypeptide has at least the sequence according to SEQ ID NO:2 and at most the sequence according to SEQ ID NO:4, additionally comprises moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids. This markers can provide for an facilitated binding of this polypeptide to a carrier. Elution, i.e. separation of polypeptides according to the present invention form the carrier can, for instance, be achieved by applying a short-term pH change to pH 2, by adding excess of a polypeptide comprising the sequence according to SEQ ID NO:2 or by increasing the salt content of the elution buffer. Further, non-limiting examples for isolation of polypeptides from the serum of a subject or from commercially available IvIgG preparations are given in more detail in the examples section of this application.

Antibodies can not only be derived from a human subject but also from an animal. Besides isolating pre-existing autoantibodies from the blood of such an animal in analogy to the methods described above, such animals can be additionally immunized with a polypeptide comprising an amino acid sequence of an Aβ polypeptide, wherein the Aβ polypeptide has at least the sequence according to SEQ ID NO:2 and at most the sequence according to SEQ ID NO:4. After several rounds of immunisation the corresponding Aβ-specific antibody producing B-cells can be obtained from the blood of the animal by routine methods.

If desired, such B cell clones (of human or animal origin) can be converted into a cell line, for example by isolating cells from the spleen of an animal and immortalizing them by transfection with Epstein Barr- Virus or by fusing the cells with myeloma cells (hybridoma technology). The latter is especially useful for producing antibodies in large quantities.

Alternatively, the polypeptides of the present invention can be, if suitable, be directly synthesized, either by conventional polypeptide synthesis, by in vitro translation or by any other means for synthesizing polypeptides and proteins. A person skilled in the art will be familiar with a multitude of appropriate techniques and will be readily able to apply them to the subject-matter of the present invention.

Additionally, the polypeptide binding to the epitope Aβ(21-37) (SEQ ID NO:2) of amyloid-beta (1-40) (SEQ ID NO: 1) can originate from other proteins than antibodies. As an example, anticalins can be engineered to bind to certain epitopes i.e. to the epitope as denoted in SEQ ID NO: 2. Anticalins are a class of engineered ligand-binding proteins that are based on the lipocalin scaffold. Using targeted mutagenesis of the loop region and biochemical selection techniques, variants with novel ligand specificities, both for low-molecular weight substances and for macromolecular protein targets, can be generated (see DE 199 26 068; Schlehuber et al., J. Mol. Biol. (2000),297 (5) p. 1105-1120; Expert Opin Biol Ther. 2005, 5(11), p. 1453-62). Binding of such polypeptide to the Aβ C-terminal half, in particular to Aβ (21-37) can also provide for an inhibition of polymerization of Aβ peptide and thus provide an efficient means for treatment and or prophylaxis of Alzheimer's Disease and other neurodementing diseases.

The polypeptides according to the invention can also be obtained via a recombinant expression system. In order to express a polypeptide according to the invention a respective nucleic acid expression construct has to be generated. Therefore, the present invention relates also to a nucleic acid having a sequence encoding for a polypeptide according to the present invention, in particular encoding the light chain or the heavy chain of one of the above mentioned antibodies, derivatives or fragments thereof or encoding for another protein according to the invention such as an anticalin binding to Aβ (21-37). Such a nucleic acid can be for instance obtained by identifying the amino acid sequence of one of the peptides mentioned above, for instance via mass spectrometry means, via Edman sequencing or any other method for protein sequencing known to the skilled artisan. Following the genetic code a nucleic acid sequence can be derived from the amino acid sequence. Preferably, the generated nucleic acid sequence is optimised in regard of the codon usage of the respective expression system of interest. Alternatively, cells expressing such an antibody can be isolated (see above) and the genomic loci or mRNA encoding for the heavy and light chain of the antibody specific for the C-Terminus of Aβ are sequenced. For certain expression systems this nucleic acid sequence might need to be adapted in order to provide for an optimal codon usage. A person skilled in the art with the above mentioned nucleic acid sequences at hand will be readily capable of generating expression constructs for use in a suitable expression system. Therefore the present inventions also relates to an expression construct providing for the expression of the polypeptides of the invention and to an isolated cell, which expresses a polypeptide or a fragment thereof according to the invention. Expression constructs, i.e. vectors include plasmids, cosmids, viruses (bacteriophage, animal viruses, and plant viruses), episomes and artificial chromosomes (e.g., YACs). One of skill in the art would be able to construct a vector by standard recombinant techniques. Said cell can be a for example a myeloma cell, a chinese hamster ovary cell, a syrian hamster ovary cell, a human embryonic kidney cell, insect cell (baculovirus system), or a transgenic plant cell, which is transformed with an expression vector according to the invention (see Schillberg et al., Cell Mol Life Sci. 2003 60(3): p. 433-445) or a cell, which expresses endogenously a polypeptide according to the invention (hybridoma). The expression of recombinant polypeptides of the invention is not restricted to eukaryotic cells but can also be expressed in procaryotes. Recombinant polypeptides according to the invention can be obtained from any such cell by purification means which are well known in the art.

In a preferred embodiment, the polypeptide according to the invention is an antibody or a fragment thereof and is optionally encoded by two expression vectors, i.e. one expression vector for the light chain and one for the heavy chain.

In order to bind within the polypeptide fragment Aβ (21-37), an antibody or a fragment thereof requires primarily an intact antigen binding domain (variable domain). The constant region of such an antibody is usually not critical for antigen binding. Thus, it is clear for a person skilled in the art, that if the polypeptide according to the invention is an antibody or a fragment thereof, this antibody/fragment may have any given isotype selected from the group consisting of IgG, IgM, IgA, IgD and IgE, including all respective subclasses of these isotypes. If the polypeptide according to the present invention is expressed as antibody in an immune cell or hybridoma cell, the isotype can be switched by additional expression or administration of activation induced cytidine deaminase and administration of stimulating factors known to the skilled artisan.

In one embodiment the polypeptides according to the invention are chemically coupled or fused with substances which prevent plaque aggregation and/or lead to the disintegration of toxic Aβ oligomers. Such a substance could be for example a protease cleaving Amyloid beta polypeptide as used for the experimental characterisation of the epitope according to the invention, i.e. Serin-proteases like Trypsin, Chymotrypsin; or Lys-C-protease, Arg-C-protease, Asp-N-proteases, also unspecific proteasen such as proteinase-K, thermolysine, subtilisin.

The following examples explain the invention but are not considered to be limiting the scope of the invention. Unless indicated otherwise, molecular biological standard methods were used, as e.g., described by Sambrock and Russel, 2001, Molecular cloning: A Laboratory Manual, 3. edition, Cold Spring Harbor Laboratory Press, Cold Spring Harbor, New York.

### Examples:

### Example 1: Isolation of Aβ-antibody from an AD patient

Immunoisolation of the serum Aβ-antibody from an AD patient by epitope-specific affinity-chromatography was performed on a Sepharose-G5Aβ(4-10) affinity matrix column. The Sepharose-G5Aβ(4-10) affinity matrix was washed with 10 ml of PBS (5 mmol L⁻¹ Na₂HPO₄, 150 mmol L⁻¹ NaCl, pH 7.5) and transferred into a 1.7 ml vial using 300 µl of PBS. 800 µl (1 µg/µl) of two different autoantibodies (isolated from the sera of Alzheimer patients) were added and the sample was slowly rotated overnight at 4°C. The suspension was transferred to a 0.8 ml micro-column (Mobitec, Gottingen, Germany) providing the possibility of extensive washing without significant loss of material. The first 2 ml were collected as flow through fraction. The column was washed with 20 ml of PBS and the last 1 ml was collected for one-dimensional electrophoresis. The affinity bound IgG was eluted with 6× 0.5 ml 0.1% TFA; the column was shaken gently for 15' and the released antibody molecules collected in a microreaction cup. The samples were lyophilised and stored until 1D-SDS-PAGE analysis.

Serum samples from healthy controls from all age ranges investigated were tested for the presence of plaque-antibodies (N-terminal epitope), using the Aβ(4-10) epitope column. In all investigated samples, non-AD control samples were devoid of detectable plaque-specific antibody.

### Example 2: Detailed description of amino acid sequences of Aβ-autoantibodies and experimental procedures for sequence determinations

### 1) Affinity isolation and purification of Aβ-autoantibodies

The Aβ-autoantibodies were isolated from (i), serum immunoglobulin (serum IVIgG; obtainable for instance from Octapharma GmbH, Germany) and (ii) from serum of healthy individuals (HI). Isolation of antibodies was performed by Aβ-epitope-specific affinity chromatography by a procedure that employed a N-cysteinyl- Aβ(12-40) column which was immobilized on Ultralink-iodoacetyl- solid phase carrier as described below.

N-Cysteinyl -Aβ(12-40) (H-CVHHQKLVFFAEDVGSNKGAIIGLMVGGW-COOH) was synthesized by solid phase peptide synthesis using 9-fluorenylmethoxycarbonyl/t-buthyl (Fmoc/tBu) chemistry on a NovaSyn TGR resin (0.23 mmole/g coupling capacity) on a semi-automated Peptide Synthesizer EPS-221 (INTAVIS, Langenfeld, Germany). The following side-chain protected amino acid derivatives were used: Fmoc-Lys(Boc)-OH, Fmoc-Asn(Trt)-OH, Fmoc-Ser(tBu)-OH, Fmoc-Asp(OtBu)-OH, Fmoc-Glu(OtBu)-OH, Fmoc-Gln(Trt)-OH, Fmoc-His(Trt)-OH, Fmoc-Cys(Trt)-OH. The synthesis was performed according to the following protocol: (i) DMF washing; (ii) Fmoc deprotection with 2 % DBU, 2 % piperidine in DMF (5 + 10 min), (iii) DMF washing, (iv) coupling of 5 equiv of Fmoc amino acid : PyBOP : NMM in DMF (40 min), (v) coupling of 5 mol-equivalents of Fmoc amino acid : PyBOP : NMM in DMF (40 min) (vi) DMF washing (3 x 1 min). Due to the hydrophobic character of the C-terminal sequence of Aβ, double coupling of each amino acid was employed throughout the synthesis. After completion of the synthesis cycles, the peptide was cleaved from the resin for 3 h using a mixture containing 95 % TFA, 2.5 % triisopropylsilan and 2.5 % deionized water. The crude product was precipitated with cold tert-butylmethylether, washed three times with diethyl ether and solubilized in 10% acetic acid (aqueous solution) prior to freeze-drying. Purification of the peptides was performed by semipreparative HPLC; subsequent characterisation by HPLC and MALDI-TOF mass spectrometric analysis ensured molecular homogeneity of the peptide.

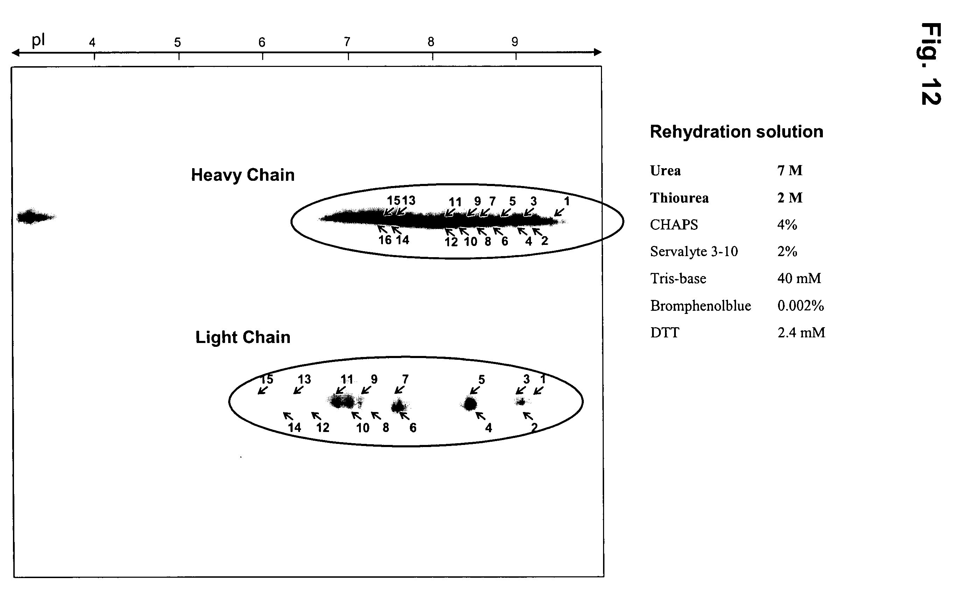

*Immobilisation of CysAβ(12-40) on Ultralinklodoacetyl Gel.-* Since the Aβ(12-40) sequence contains two internal lysine residues which might lead to side reactions in immobilisation procedures using amino groups, a specific affinity column was prepared using a cysteine residue attached to the Aβ- N-terminus, to ensure homogeneous orientation of peptide molecules on the column support by immobilisation through cysteinyl-S-thioether linkage. The azlactone-activated support contains an iodoacetyl group (UltraLink; Perbio, Bonn, Germany) at the end of a hexadecyl-spacer group, which was reacted with the cysteinyl-sulfhydryl group to yield a stable thioether linkage, in order to reduce steric hindrance and provide maximum binding capacity of the antibodies. For covalent attachment of the Cys-Aβ(12-40), 3.7 mg of peptide were dissolved in 50 mM Tris, 5 mM EDTA-Na coupling buffer (pH 8.5) to a final concentration of 0.37 mg/ml. The solution was added to 1 ml of drained Ultralink- Iodoacetyl gel and the coupling reaction was performed for 1 hr at 25°C under gentle mixing, followed by 30 min reaction time without mixing. An aliquot of 0.5 ml of the Cys-Aβ(12-40) coupled support was packed into a column (2.5 ml, MoBiTec, Göttingen, Germany) allowing the solution to drain. The column was washed with 3 ml of coupling buffer, and non-specific binding sites on the gel were blocked for 2 x 45 min by reaction with 1 ml of 50 mM L-Cysteine•HCl in coupling buffer. Subsequently the column was washed with 5 ml of 1 M NaCl and 5 ml of 0.1 M Na-phosphate, 0.15 M NaCl (pH 7.2) and stored at 4°C. The gel support (0.5 ml) was transferred into a 15 ml Falcon vial using 5 ml PBS and mixed with 5 ml IVIgG. After gentle shaking overnight at 4 °C, the suspension was transferred to the column using the effluent to completely rinse the matrix back into the column. The column was washed eight times with 10 ml of PBS followed by 2 wash cycles with 10 ml ultrapure water. The affinity-bound antibodies were eluted from the column with 10 x 0.5 ml 0.1 % trifluoroacetic acid (TFA). Subsequent isolation and preparation of IgG for structural characterization and affinity studies was performed using two different protocols:
(i) The first procedure involved adjustment to neutral pH for each fraction collected using 0.5 M NaH₂PO₄ (pH 8) in order to maintain integrity of the antibodies for use in affinity studies. The bound antibodies were eluted from the column with 10 x 0.5 ml 0.1 M glycine buffer, pH 2.8. Each fraction was collected in a microreaction tube containing 35 µl 1M Tris-HCl, pH 9. To maintain integrity of the antibodies neutral pH was adjusted immediately after elution by adding the appropriate amount of Tris-HCl or glycine buffer. To regenerate the column for further use, the column was washed once with 10 ml 10 mM sodium-phosphate buffer pH 6.8, followed by two wash cycles with 10 ml of PBS containing 1M sodium chloride and finally two wash cycles with 10 ml PBS. Protein concentrations were determined by the BCA method (Pierce; Perbio, Bonn, Germany). This procedure yielded the elution of single, defined antibody.
(ii) The bound antibodies were eluted from the column with 10 x 0.5 ml 0.1 M glycine buffer, pH 2.8. For separation by gel electrophoresis the elution of affinity-bound antibodies was not performed by subsequent pH adjustment, in order to reduce the salt content of the samples subjected to isoelectric focusing. Gel electrophoretic separations provided a set of defined bands of antibodies (see numbering in Fig. 12).

*Antibody quantification.* Antibody concentrations in the elution fractions were determined by the Micro BCA^{™} Protein Assay Kit method (Pierce; Perbio, Bonn, Germany). The stock solution of 2 mg/ml of bovine albumin supplied within the Micro BCA^{™} Kit was used to prepare fresh standard dilutions within the range 40 - 0.5 µg/ml. The antibodies eluted between fractions 1 to 6, with highest concentrations in fractions 1 and 3. For quantification of each set of 10 elution fractions, fresh albumin standard dilutions were prepared. Results were read at 562 nm with the ELISA reader.

### 2) Determination of the epitope recognized by the Aβ autoantibodies via epitope excision

Antibody immobilisation was performed with a solution of 100 µg Aβ-IgG in 500 µl 0.2 M NaHC0₃/0.5 M NaCl (pH 8.3), which was added to n-hydroxysuccinimidyl (NHS)-activated 6-aminohexanoic acid-coupled sepharose (Sigma, St Louis, USA) and allowed to bind for 60 min at 20°C before transferring onto a microcapillary (MoBiTec, Goettingen, Germany). The column was washed two times alternately with blocking (ethanolamine/NaCl) and washing buffers (NaAc/NaCl). Epitope excision was performed by application of 2-5 µg Aβ- antigen and Aβ-antigen-containing material to the antibody microcolumn for 60 min at 20°C. After washing, digestion was performed on the column for 2 h at 37°C with 0.2 µg protease in 200 µl PBS. Unbound peptides were removed and the epitope was dissociated from the antibody using 500 µl 0.1 % trifluoroacetic acid. After incubation for 15 min at 20°C, the epitope eluate was lyophilized and reconstituted in 10 µl 0.1 % TFA for mass spectrometric analysis. Epitope extraction was performed in an analogous manner, however proteolytic digestion was performed first with the unbound antigen and the proteolytic digest was applied directly to the antibody column. Upon antibody complexation and epitope excision, relevant amino acid residues, e.g. F4, R5, D7, E11 (see Fig. 6), were found accessible to digestion while these residues are all shielded in the immune complex with the plaque-specific Aβ(4-10) antibody (s. Figure 3). In all non-AD control sera, the carboxy-terminal (Aβ21-37) sequence was found to be specifically recognised (proteolytically shielded), while N-terminal residues of Aβ were accessible for cleavage. The extracted Epitope bound by the Aβ-autoantibody isolated from the healthy individuals exhibited thus the amino acid sequence of Aβ(21-37).

The structure and conformational properties, binding affinity and specificity of the Aβ-autoantibody epitope were further characterised by investigation of synthetic peptides comprising the Aβ(21-37) epitope sequence, and by fine-structure mapping using Alanine sequence mutations, H-D exchange and high resolution mass spectrometry, ELISA studies and CD spectroscopic conformational analysis in different solvents.. Biotinylated Aβ(21-37) peptides and peptides derivative flanked with oligo-Glycine and -(D-Ala) spacer groups were synthesized by solid-phase peptide synthesis according to previously described procedures for Aβ-peptides, and were purified by reversed-phase HPLC and characterised by MALDI- and ESI- mass spectrometry for molecular homogeneity (Manea et al., 2004; Mezo et al., 2004). Comparative binding studies were performed with Aβ-epitope peptides comprising different C-terminal sequence lengths, using an ELISA system (see below, 3). These results established an essential function for antibody affinity of the carboxyterminal sequence end of Aβ, comprising residues 30-37; this partial sequence is critically involved in β-sheet formation and aggregation of Aβ. Thus, full binding affinity is obtained in Aβ(20-37) peptide (K_{D} approx. 80 nmol), as well as in peptides with extended C-terminal sequences (e.g., Aβ(12-40). In contrast the shortened Aβ(20-30) peptide showed almost completely abolished affinity. Mass spectrometric studies of peptides upon H-D equilibrium exchange showed rapid deuterium incorporation of peptide backbone hydrogens only for the Aβ sequence (20-30), but only little backbone deuteration for residues (30-37), suggesting increased shielding in this part due to conformational or aggregation effects. Control binding studies of the epitope peptide Aβ(20-37) with antibodies that recognized the N-terminal, Aβ(1-16) peptide (plaque-specific mono- and polyclonal antibodies) did not show any binding affinity.

### 3) Affinity evaluation of purified Aβ-autoantibodies

To assess the quality of the affinity purified antibodies ELISA was performed using the IVIgG flow through as a control. The 96-well plate was incubated with 200 ng/well of Aβ(1-40) in PBS buffer for 2 hrs at 20°C. The plate was washed 4 times with 200 µl of PBS containing 0.05 % Tween-20 and blocked for 2 hours with 5 % BSA containing 0.05 % Tween-20 in PBS buffer. The plate was then incubated for 2 hrs at 20°C under gentle shaking with the affinity-purified antibodies in 5 % BSA, 0.05 % Tween-20 using the IVIgG flow through as a control. After washing, anti-human horse-radish peroxidae (HRP) conjugated antibodies were added to the wells and incubated for 1 hr. After adding the substrate Aβ(1-40) the optical density was determined at 450 nm. Affinities were determined by competitive ELISA, with K_{d}-values ranging between about 8 to about 15 x 10⁻⁹ M.

### 4) Electrophoretic separation for sequence determinations of Aβ-autoantibodies

Electrophoretic separation and isolation by isoelectric focussing of the Aβ-autoantibodies was carried out by ID- and 2D-SDS-PAGE. Samples were equilibrated for 30 min in 6 M urea, 30 % glycerol, 2 % w/v SDS, 0.05 M Tris-HCl (pH 8.8), 1 % DTT and a trace of bromophenol blue, then for 30 min in the same solution except that DTT was replaced by 4.5 % (w/v) iodoacetamide. Isoelectric focusing (IEF) was carried out with a Multiphor II horizontal electrophoresis system (Amersham Pharmacia Biotech) using 17 cm immobilised pH gradient (IPG) strips (pH range 3-10. linear). The second-dimensional separation was carried out with a Bio-Rad Protean II xi cell vertical electrophoresis system using 10 % SDS-PAGE gels of 1.5 mm thickness. The IPG strips were rehydrated overnight in a solution containing about 100 µg lyophilized Aβ-autoantibody for Coomassie and 30 µg for silver staining solubilised in 7 M urea, 2 M thiourea, 4 % CHAPS, 0.3 % DTT, 2 % Servalyt pH 3-10 and a trace of bromophenol blue. The samples were applied using the in-gel rehydration method. Rehydrated strips containing the sample were run in the first dimension for about 30 kVh at 20°C.

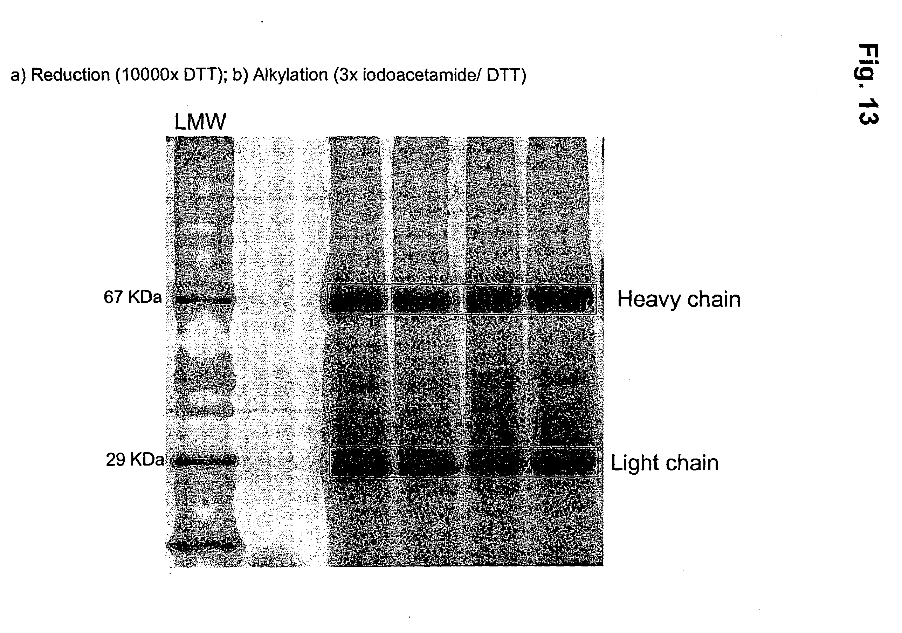

Strips placed on the vertical gels were overlayed with 1 % agarose in SDS running buffer (25 mM Tris-HCl, 192 mM glycine and 0.1 % w/v SDS) and subjected to electrophoresis at 25 mA/gel for 30 min and 40 mA/gel until the tacking dye reached the anodic end of gels. After separation in SDS-PAGE gels, the proteins were visualised by silver staining or by sensitive colloidal Coomassie staining and scanned using a GS-710 Calibrated Imaging Densitometer (Bio-Rad) (see Fig. 12 and 13).

*Heavy and light chain isolation. -* Isolation of light chains and heavy chains of antibodies was made using 1D gel electrophoresis. The samples (50 - 200 µg) were dissolved in sample buffer (4 % SDS, 25 % glycerol, 50 mM Tris-buffer, 0.02 % Coomassie-blue, 6 M urea, pH 6.8) with repeated agitation, sonication and centrifugation to ensure maximum solubilisation of the antibodies. Reduction of the disulfide bridges was performed by reaction with dithiotreitol (DTT) at a 1000-x molar excess for 90 min at 20 °C. Subsequently, alkylation of reduced cysteinyl-sulfhydryl groups was performed by reaction with a 3-x molar excess of iodacetamide (IAA)/DTT concentration for 60 min at 20 °C. 1D-SDS-PAGE isolation of heavy and light chain bands was performed on a 12 % acrylamide gel, using a BIO-RAD Protean-(II) Electrophoresis cell, by application of approximately 20 µg antibody pro band. The PDQuest software from Bio-Rad was employed for imaging and analyzing 1-D and 2-D gels. After the gels had been stained and scanned, separate algorithms of the PDQuest software were used to reduce background noise levels, gel artifacts, and horizontal or vertical streaking from the image. The PDQuest software was then used to automatically detect the protein spots separated on 2-D gels, and for comparison of different gels. Approximately 20 bands were detected as discerned spots, of which 16 heavy chain and 15 light chain spots were analysed and identified by mass spectrometric analysis.

### 5) Overview of analytical strategy and methods for sequence determination of Aβ-autoantibodies

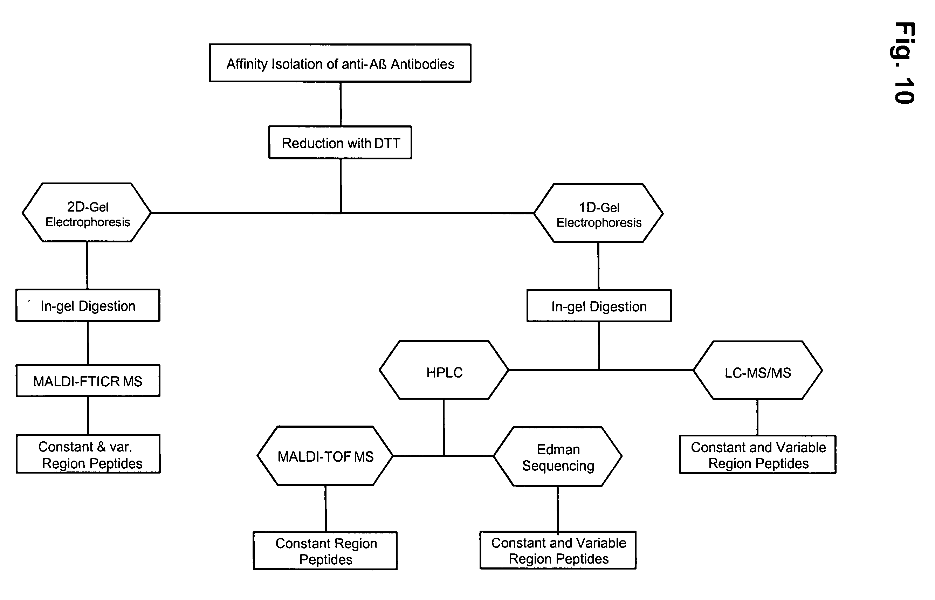

The primary structure determinations of antibodies, encompassing amino acid sequences of heavy and light chains, determination and multiplicity of CDR sequences, disulfide linkages, and N-terminal sequences and their variations were performed by a combination of the following complementary and partially overlapping methods (overview in Fig. 10):
(i) Direct Edman N-terminal sequence determinations of intact proteins, heavy and light chains following 1D-electrophoretic isolation and blotting of antibody bands (N-terminus and CDR1 variable domains);
(ii) ID-Electrophoretic separation of heavy and light chains, followed by tryptic digestion of isolated protein bands, HPLC isolation of tryptic peptides, and Edman sequence determinations of peptide fragments (variable sequences and CDR regions);
(iii) LC-ESI-MS/MS sequence determinations of proteolytic peptides isolated by HPLC, using a combination of *de-novo* sequencing and sequence determination from NCBI data base search procedures (variable sequences and CDR regions);
(iv) Mass spectrometric sequence assignments of proteolytic digest peptides from 2D-gel electrophoretic separations of antibody- isoforms, using high resolution MALDI-FTICR-MS in conjunction with database search; by performing two or more data base search procedures (e.g., Mascot, Profound search engines); constant domains and partially variable sequence domains;
(v) Mass spectrometric analysis of HPLC- isolated proteolytic peptides, using MALDI-TOF-MS (constant region sequences); in addition, MALDI-MS (MALDI-TOF and MALDI-FTICR-MS) of proteolytic peptide fragments without and with reduction of disulfide bridges was used for assignment and confirmation of correct disulfide linkages;
(vi) assignments of the heavy and light chain connectivities of antibody-isoforms were performed by MALDI-MS (MALDI-TOF- and MALDI-FTICR-MS) of proteolytic peptide mixtures following 2D-gel electrophoretic separation, in which the dithiotreitol (DTT) reduction step was initially omitted, providing intact disulfide-linked antibodies during the isoelectric focussing step. Disulfide reduction and alkylation was then performed in the second electrophoresis step.

### 6) Detailed description of sequences of Aβ-autoantibodies

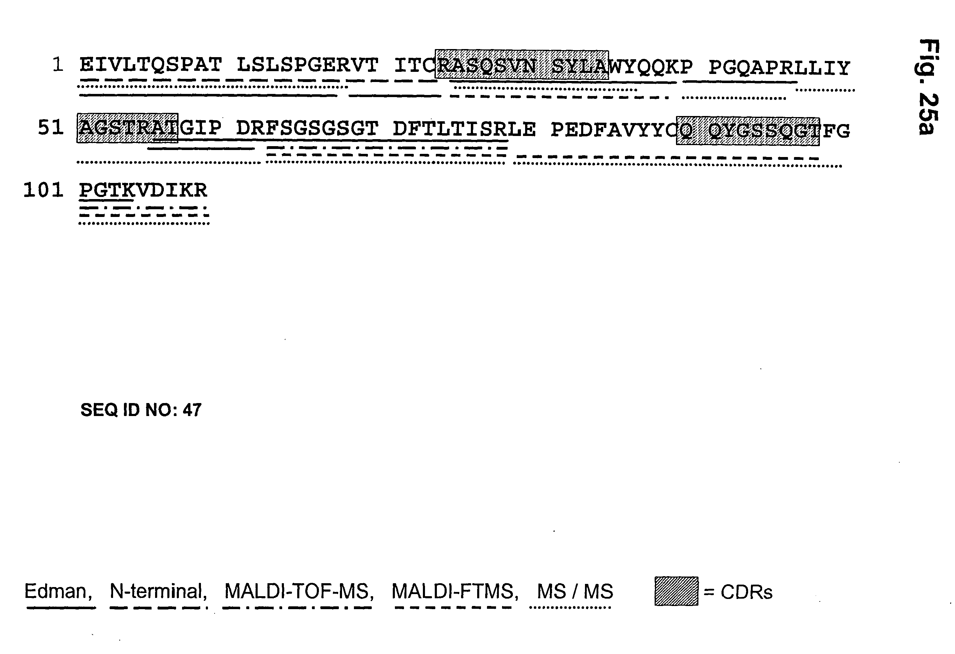

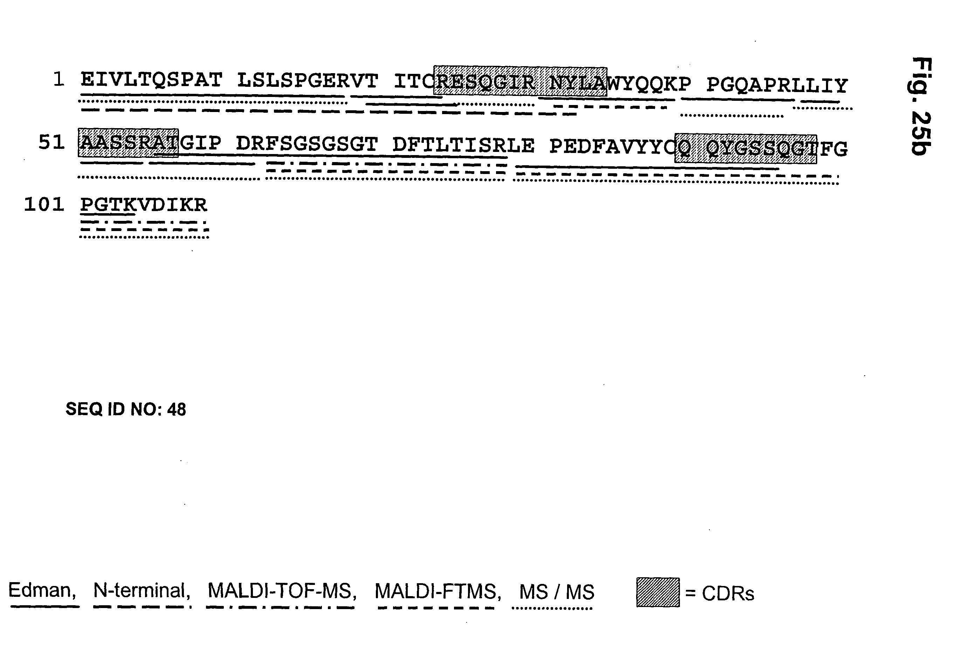

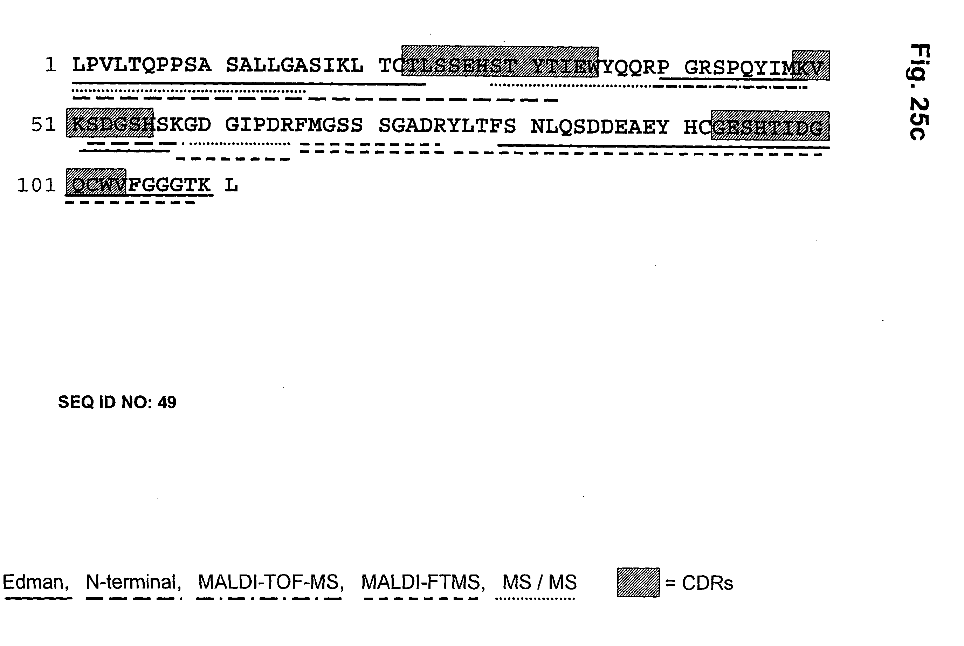

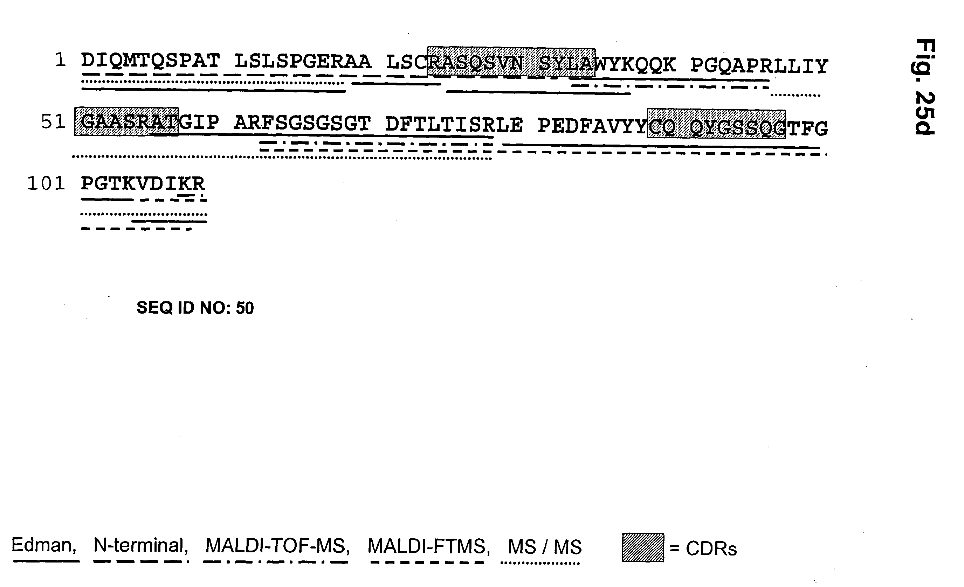

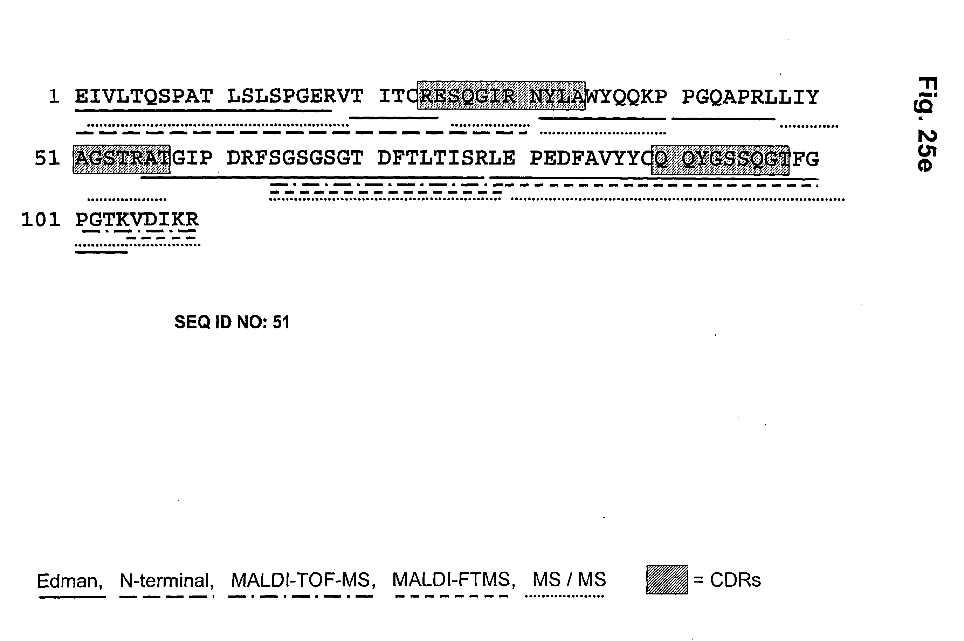

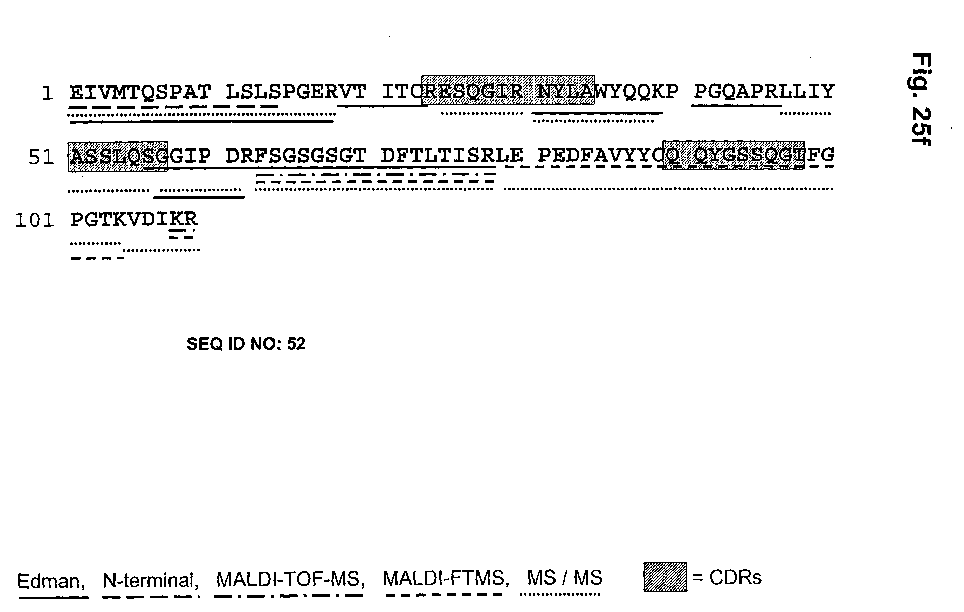

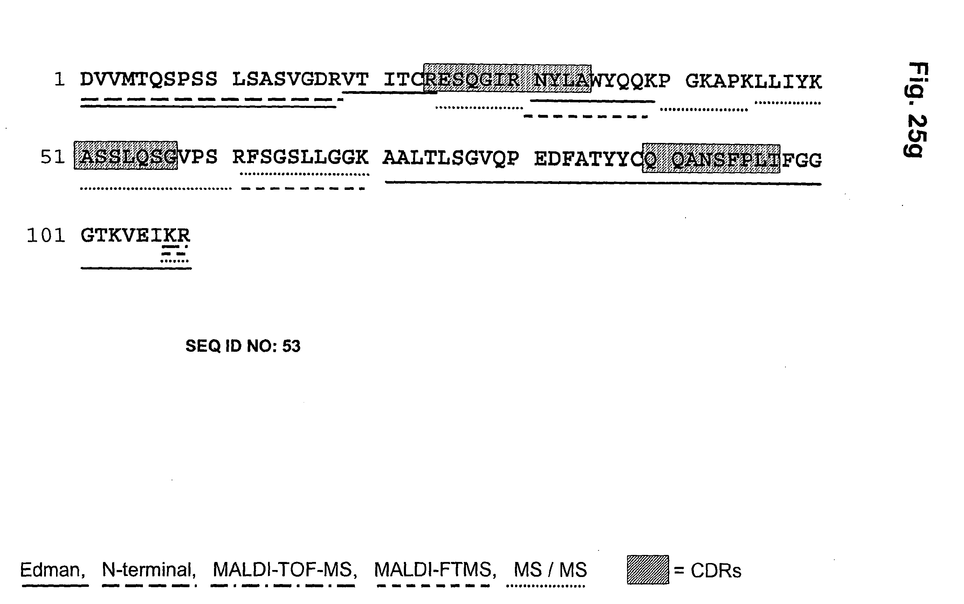

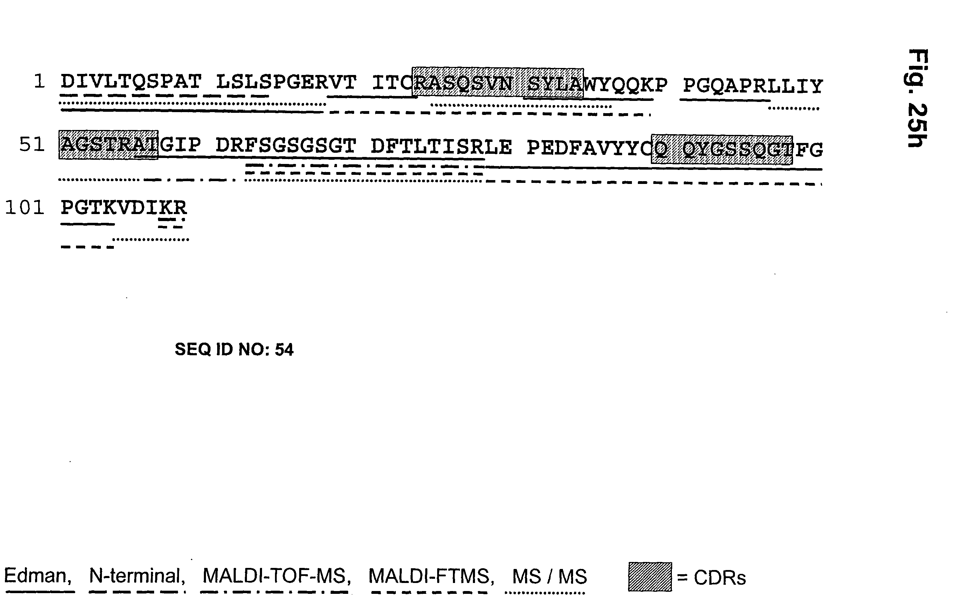

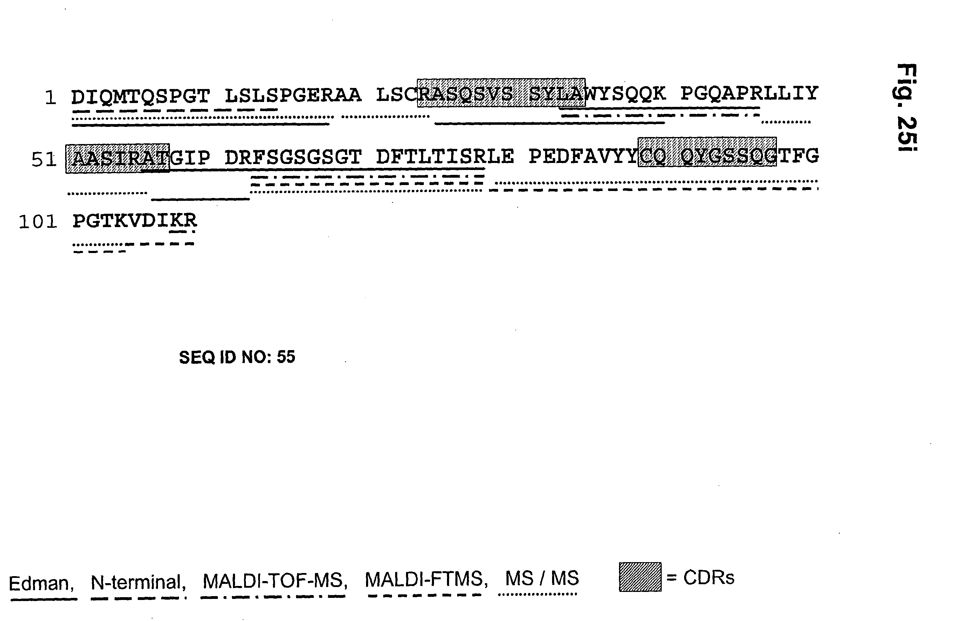

Amino acid sequences were determined for Aβ-autoantibodies isolated by affinity-purification from a) serum- IVIgG, and b) serum- IgGs isolated from two healthy adult individuals (m, 30 yrs). Sequences determinations for complete antibodies, heavy and light chains, (identified with disulfide-linkages as described above) are shown with assignments of structural details in Figures 29, 30 and 32. In the experimental details depicted in Figures 25 to 30, the different complementary methods used for completing the sequence determinations by complementary and overlapping partial sequences are shown by different underlining codes for (i), Edman N-terminal protein sequence determinations; and (ii - v), sequence determinations of proteolytic peptides isolated by HPLC using Edman sequencing, LC-MS/MS, MALDI-TOF-MS and high resolution MALDI-FTICR-MS, and MALDI-FTICR-MS of proteolytic peptide mixtures upon 2D-electrophoretic isolation. Furthermore, intradisulfide linkages of cystinyl residues and heavy chain-light chain-disulfide linkages identified at Cys-224 (HC-LC), Cys-230 and Cys-233 (HC), have been annotated in the heavy chain sequence. In addition to direct assignment of sequence positions of cysteine residues, disulfide linkages were confirmed by mass spectrometric molecular weight determinations from tryptic, non-reduced peptide mixtures (not shown), and subsequent DTT-reduced peptides, showing full agreement with homology comparison from predicted assignment of disulfide linkages from crystallographic data of a reference IgG1 structure (PDB 1IG, Brookhaven protein structure data base).

From the serum-IVIgG antibodies, sequences were identified for 12 heavy chain isoforms and 5 light chain isoforms of the respective variable regions; the light chain sequences comprised 4 kappa and 1 lambda chain sequences. For a major portion of the amino acid sequences, sequence data were corresponding with, and ascertaining each other by at least two complementary, overlapping partial sequence determinations. All antibody sequences were identified as IgG_G1 molecules.

Noteworthy results in the heavy and light chain sequences are the identification of several single amino acid variations in the *constant domains,* each of which was ascertained by several complementing mass spectrometric methods and by Edman sequencing of HLC-isolated tryptic peptides. The N-glycosylation site at N-301, was ascertained by Edman sequence determination of the tryptic peptide (297-304) (EEQYNSTYR); this peptide provided a blank in the sequencing cycle-5. After N-deglycosylation with PNGaseF, this peptide yielded the sequence determination, N-301; furthermore MALDI-FTICR-MS analysis of the deglycosylated peptide provided identification of the correct molecular mass. In addition a non-glycosylated Fc sequence variation was identified by mutation of N301A and by the presence of partially non-glycosylated N.

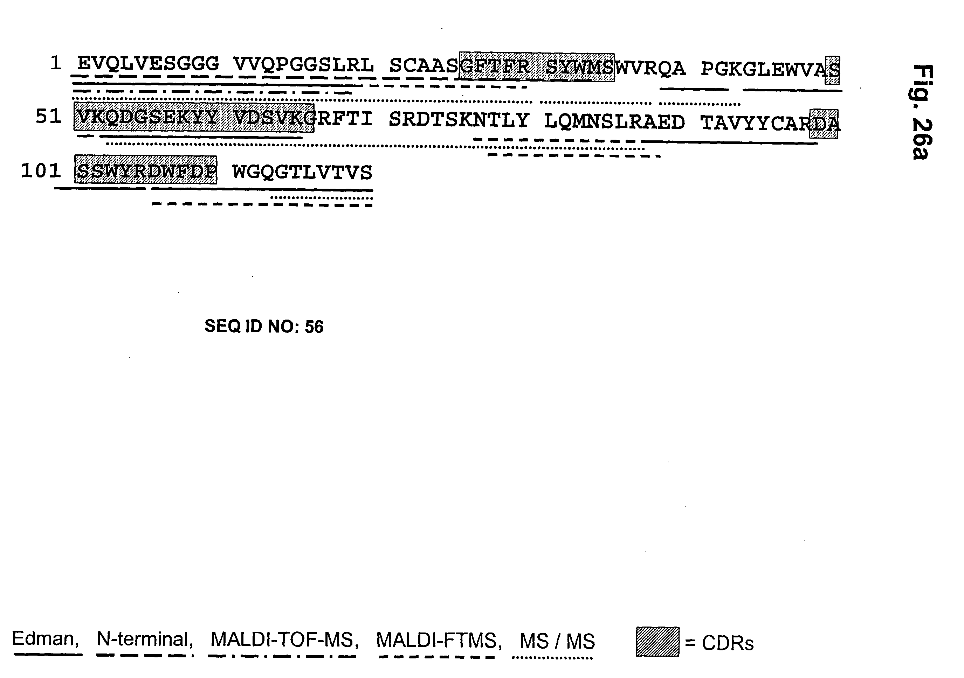

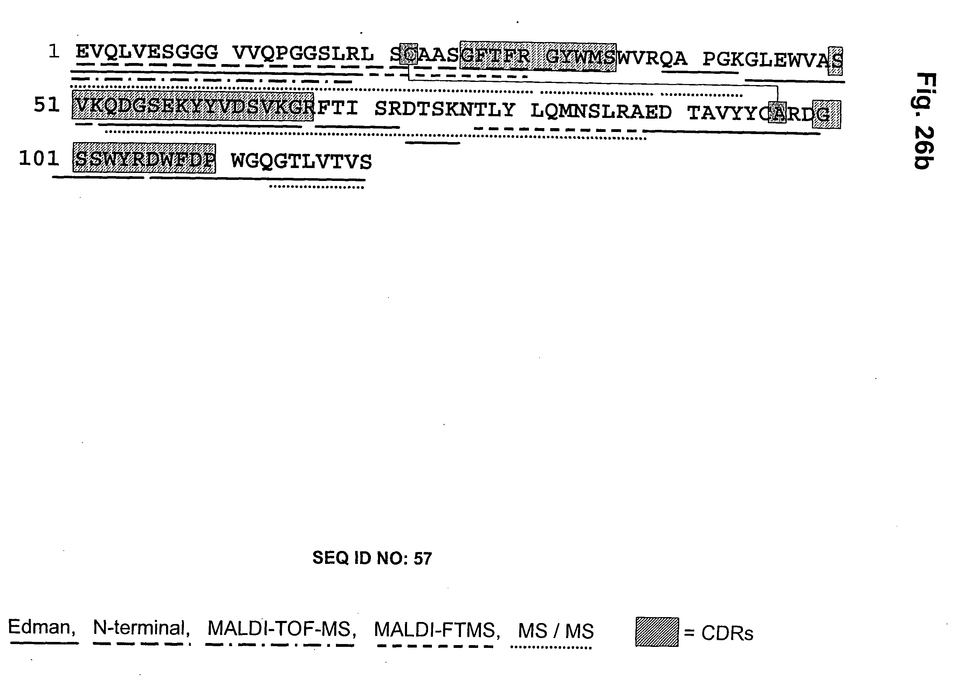

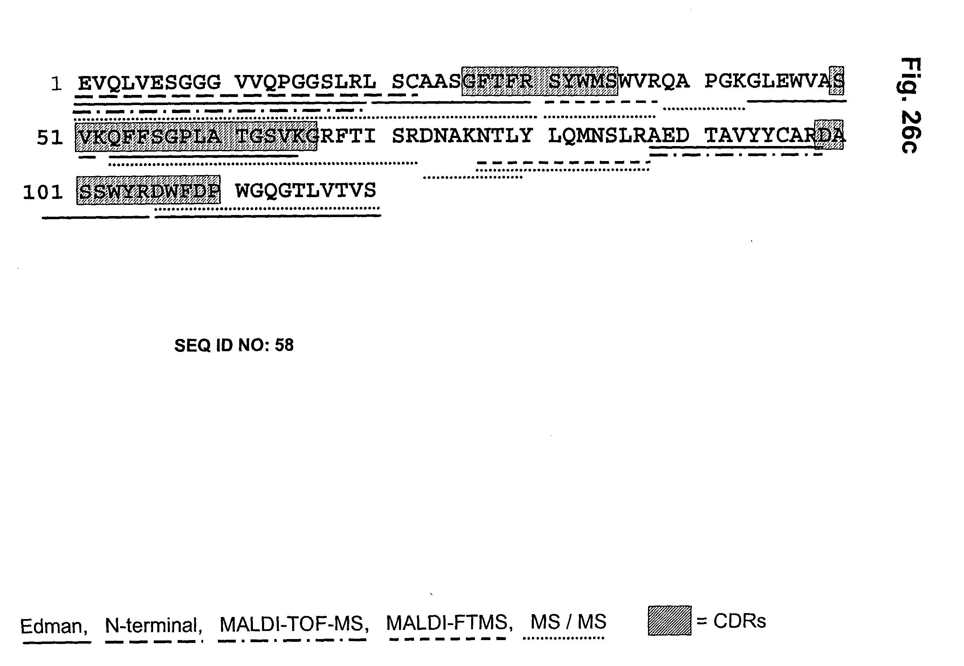

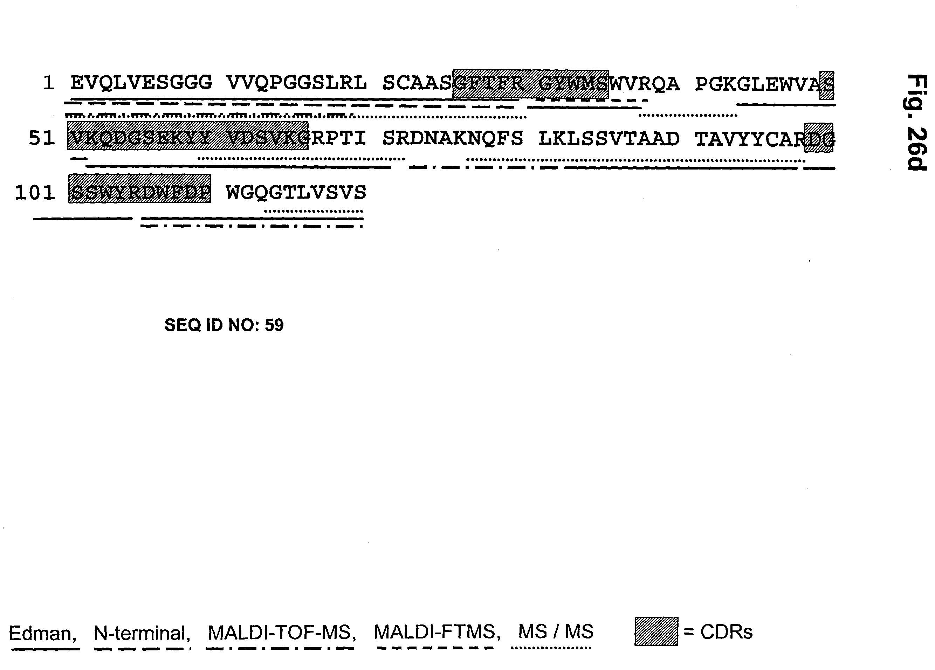

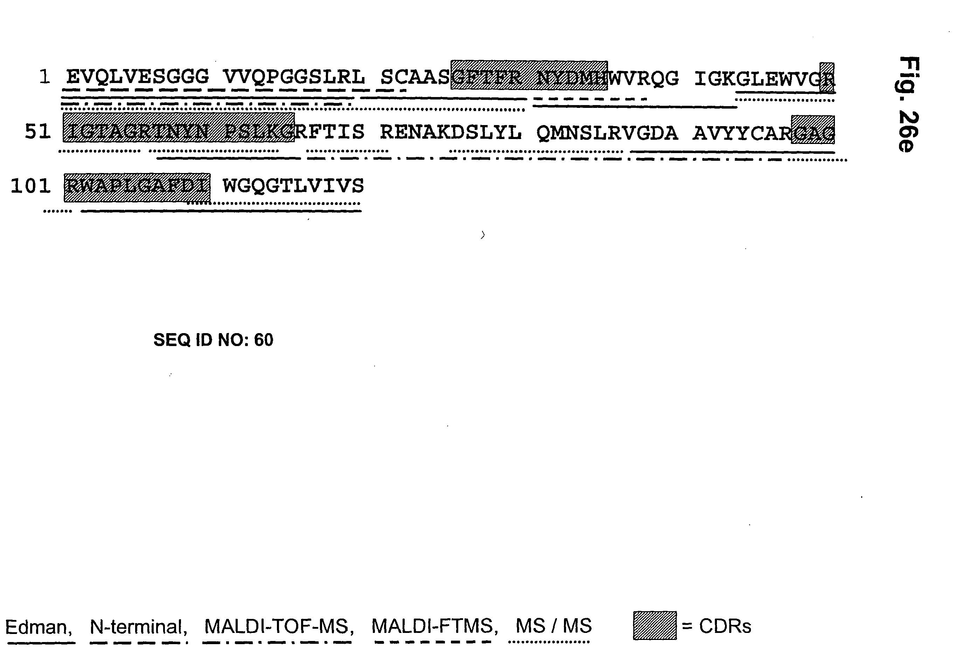

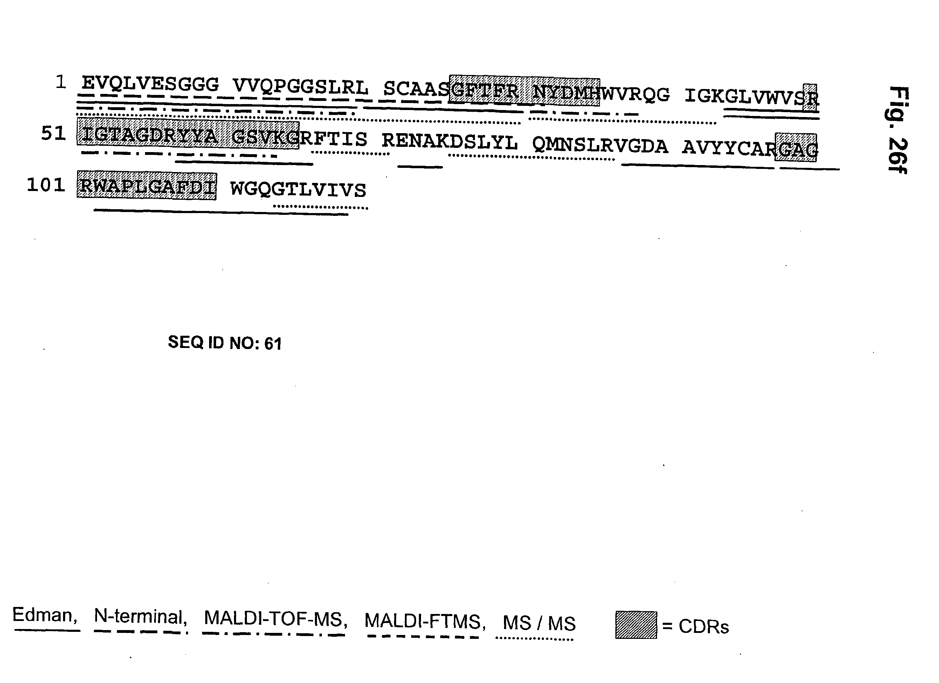

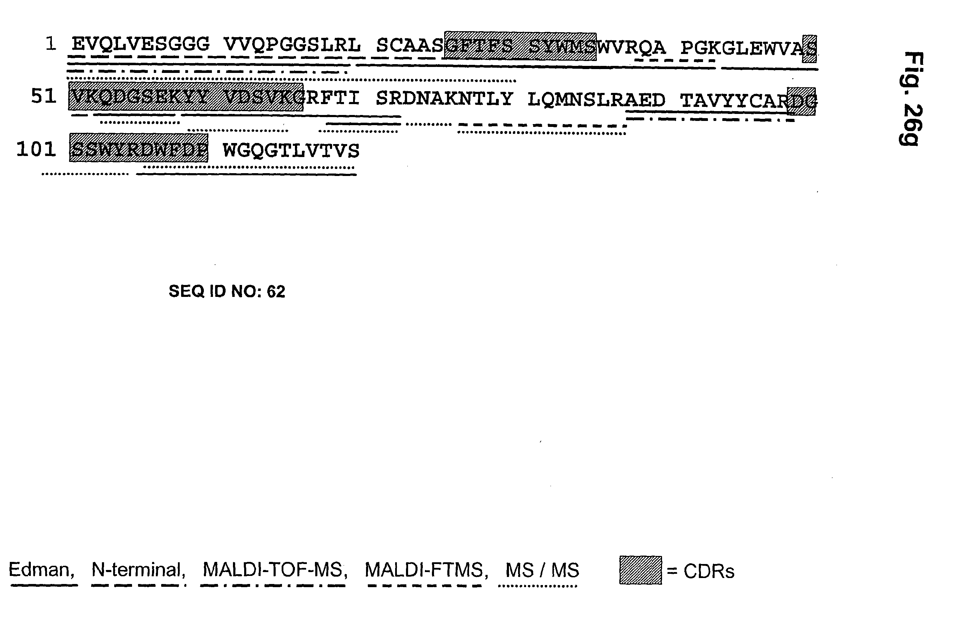

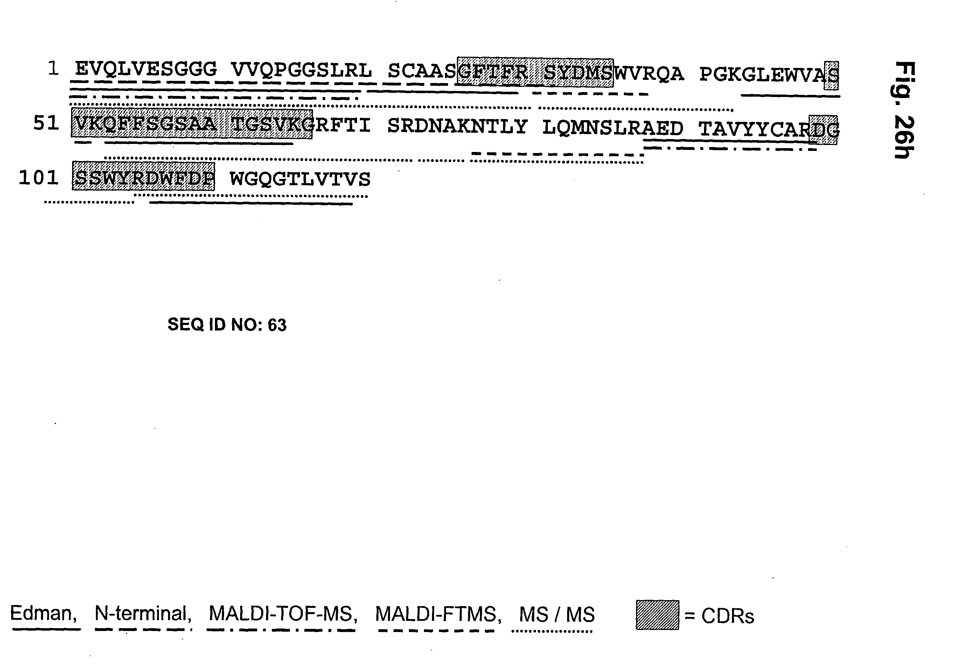

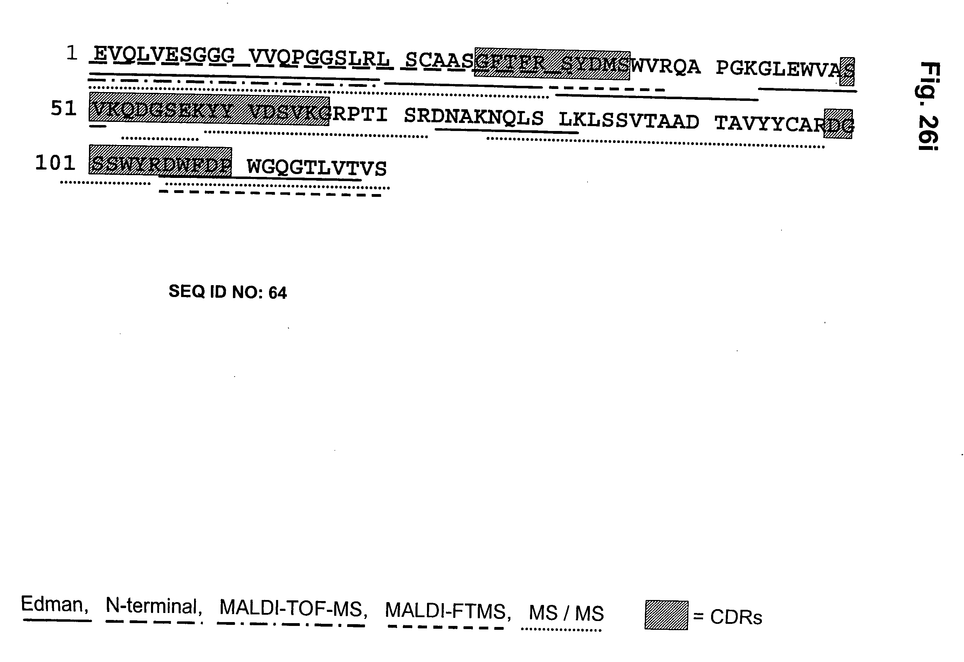

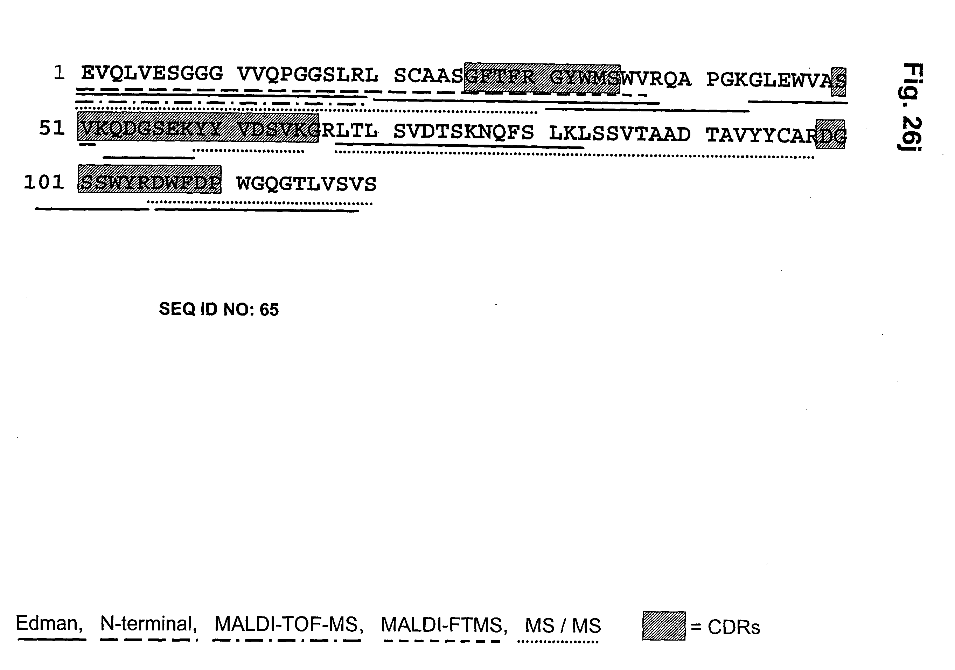

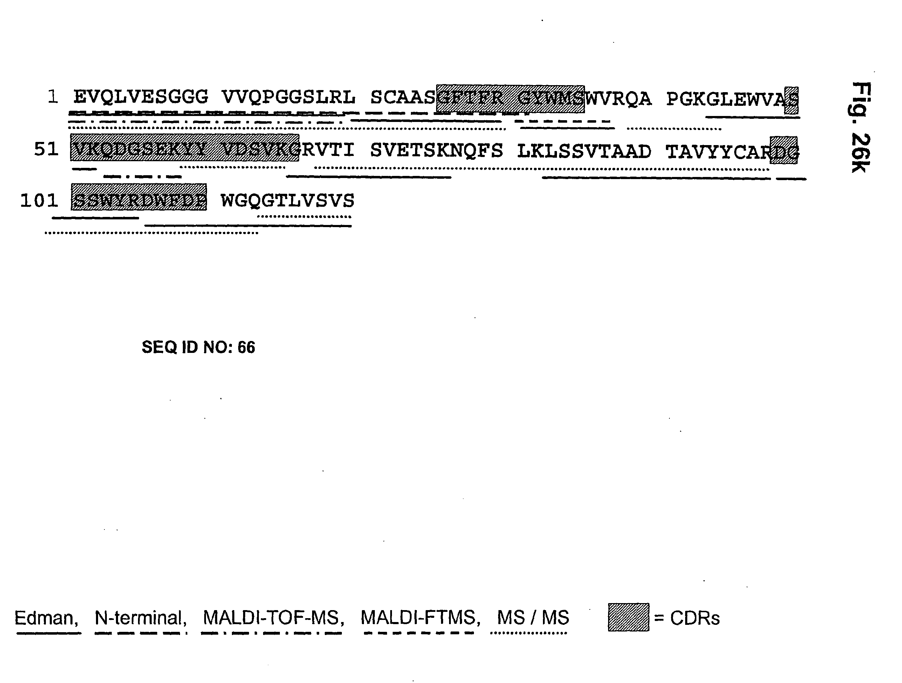

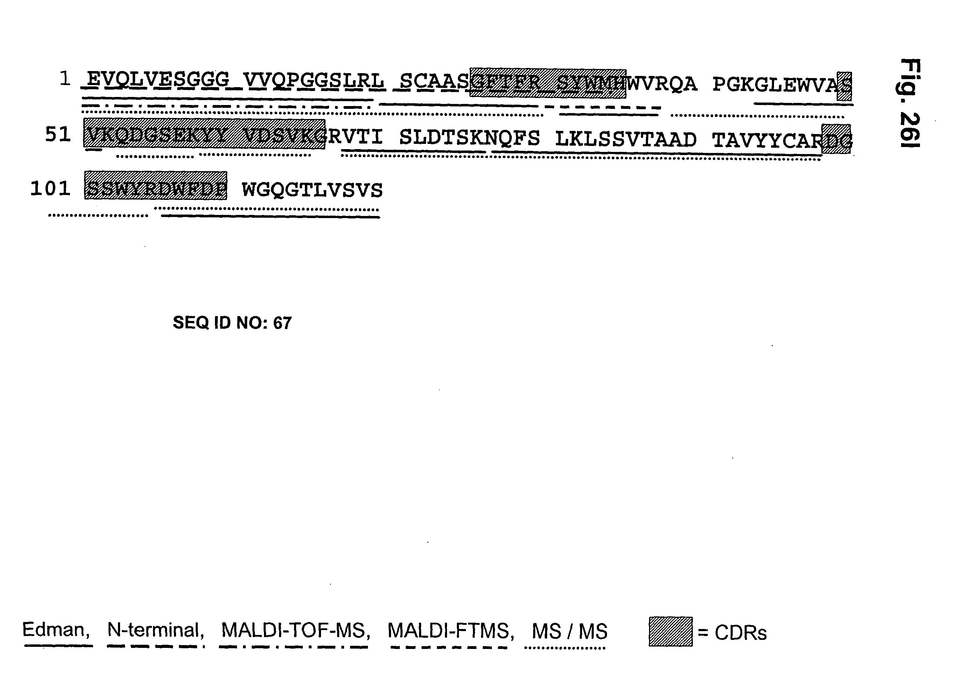

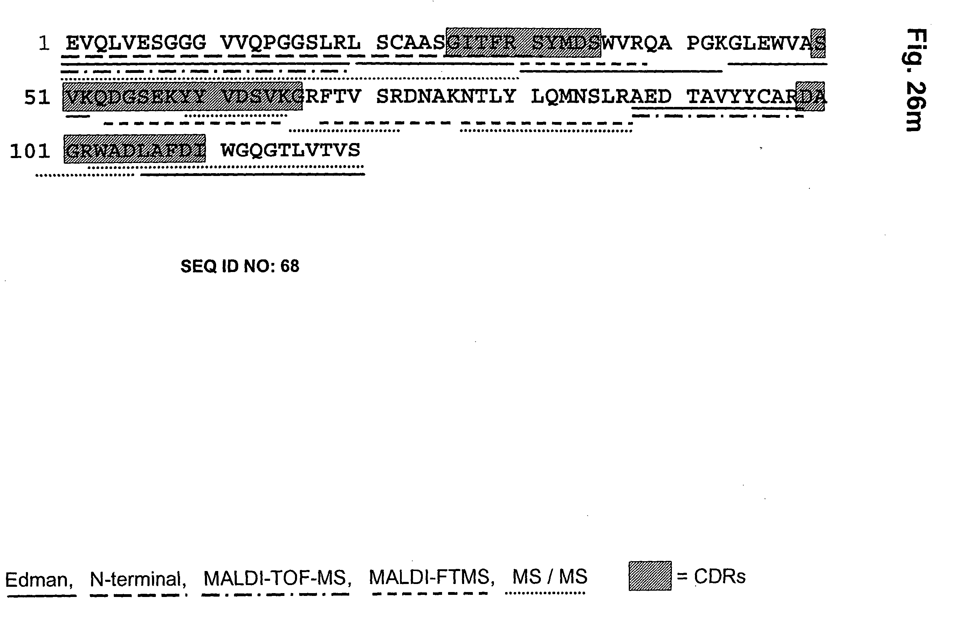

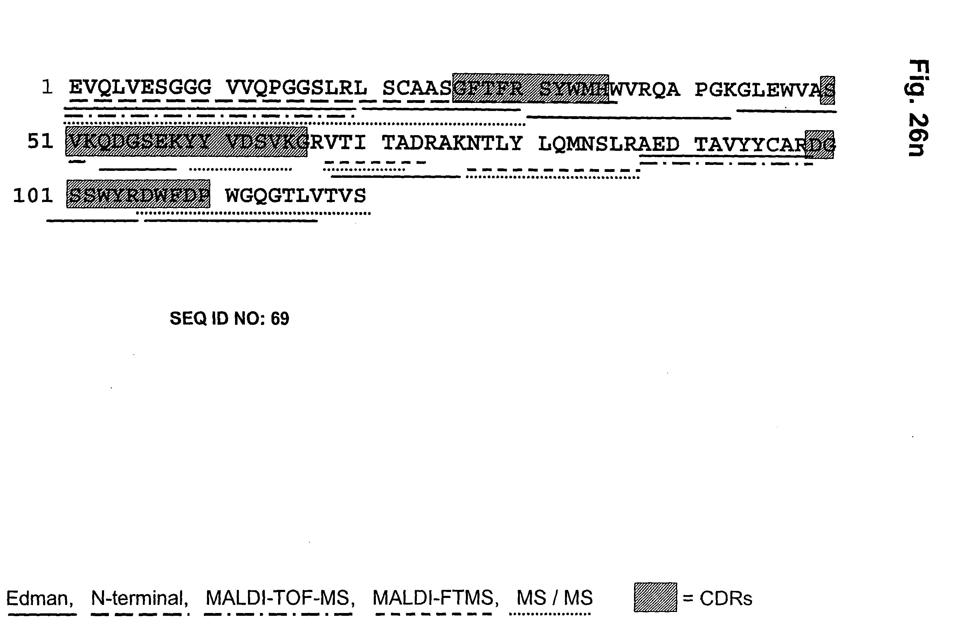

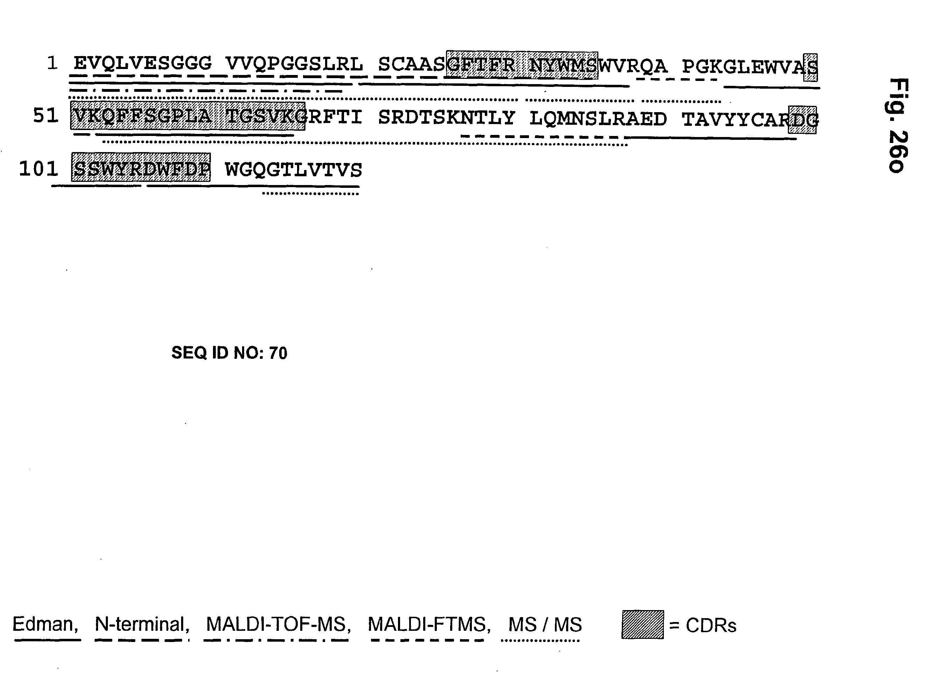

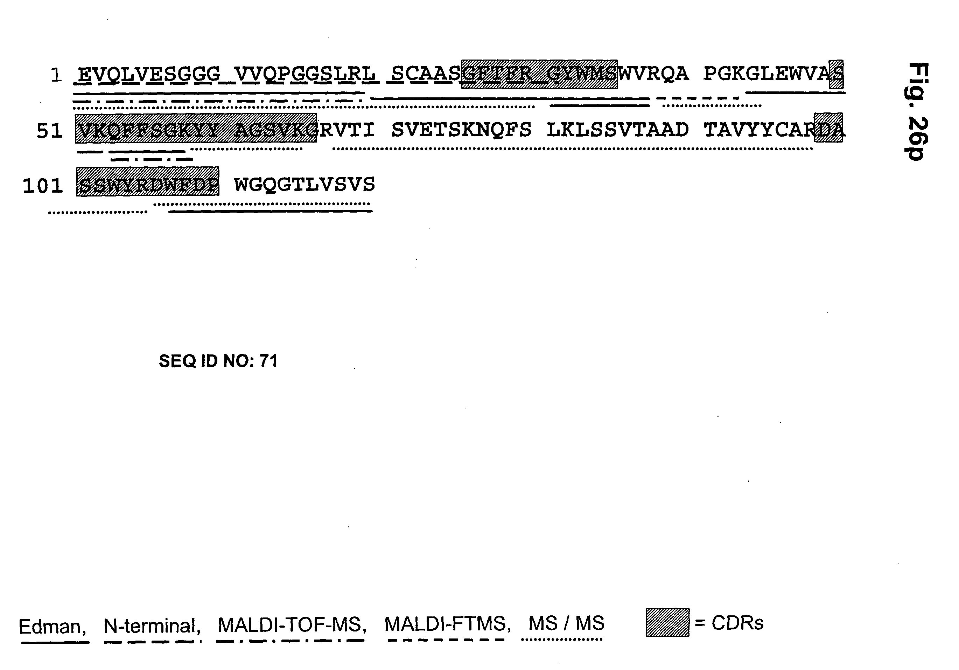

Variable sequences determined for light and heavy chains are summarised in Fig. 25 and 26, comprising 12 sequence variants (heavy chain) and 5 (light chain). Sequence variations in the heavy chains were found considerably more frequent than for light chains, except for the N-terminal sequences which showed several sequence variations for the light chains but complete homogeneity of the heavy chains. N-Terminal sequences were determined by a combination of direct Edman sequence analysis of proteins, Edman sequencing of tryptic N-terminal peptides, and LC-MS/MS sequencing of HPLC-isolated N-terminal peptides. A characteristic feature is the single uniform heavy chain N-terminal heavy chain (1-20), in contrast to the light chain N-terminal mutations. The N-terminal mutations of the light chains were confirmed by a blast homology search using the NCBI data base.

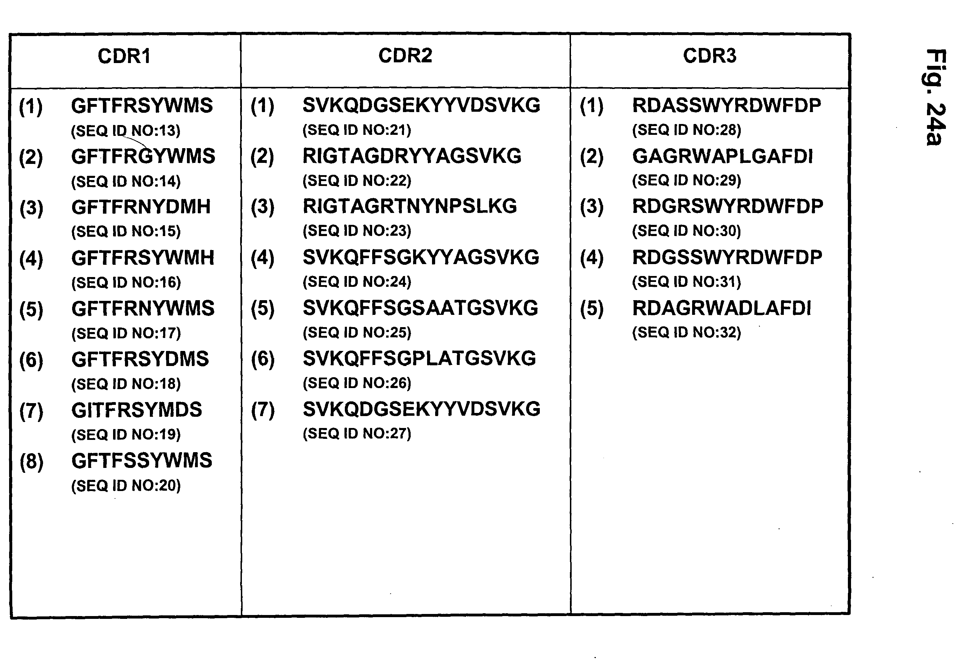

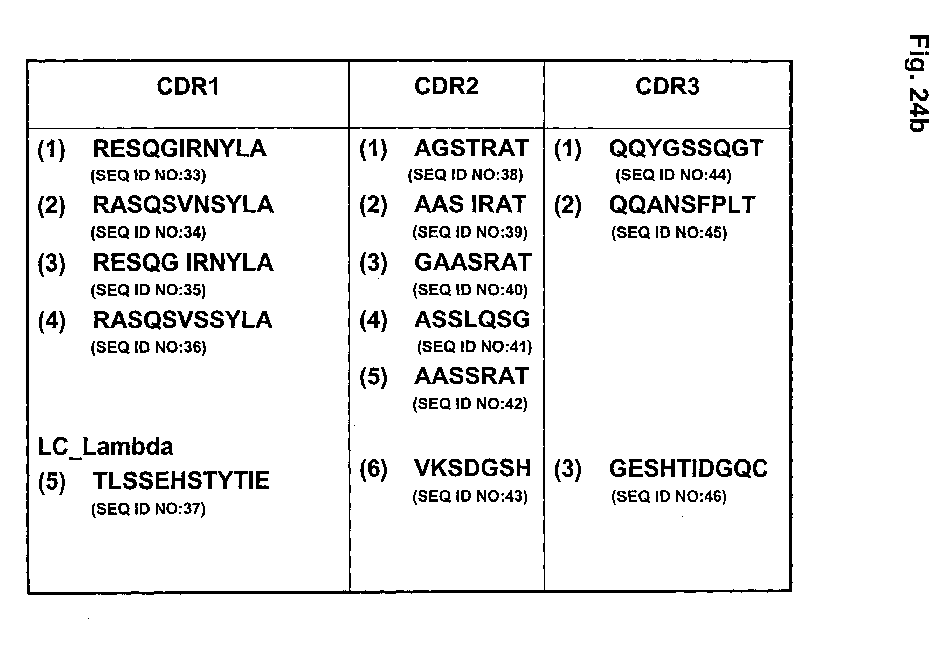

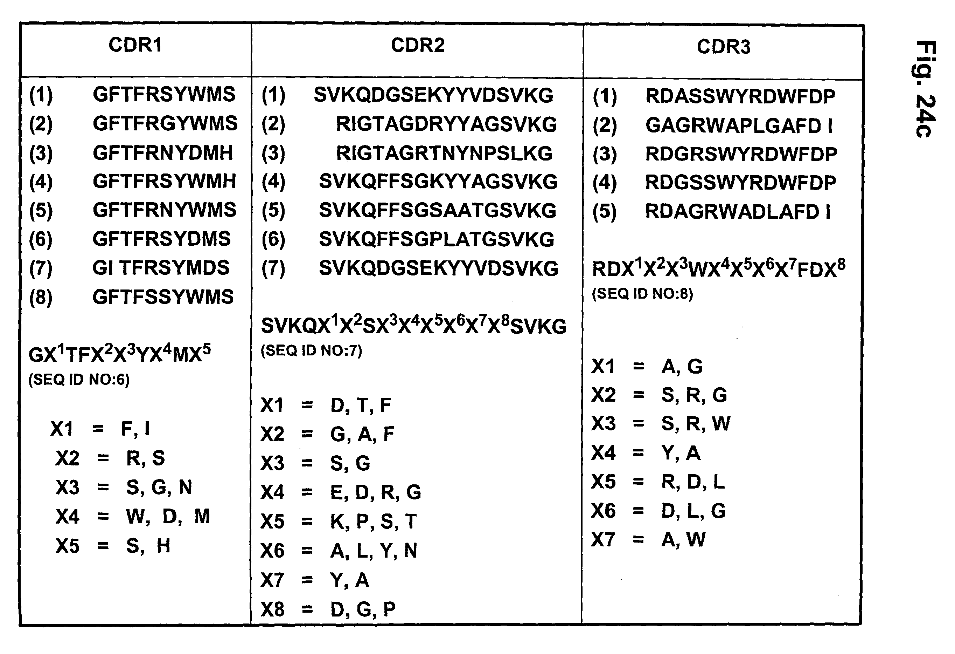

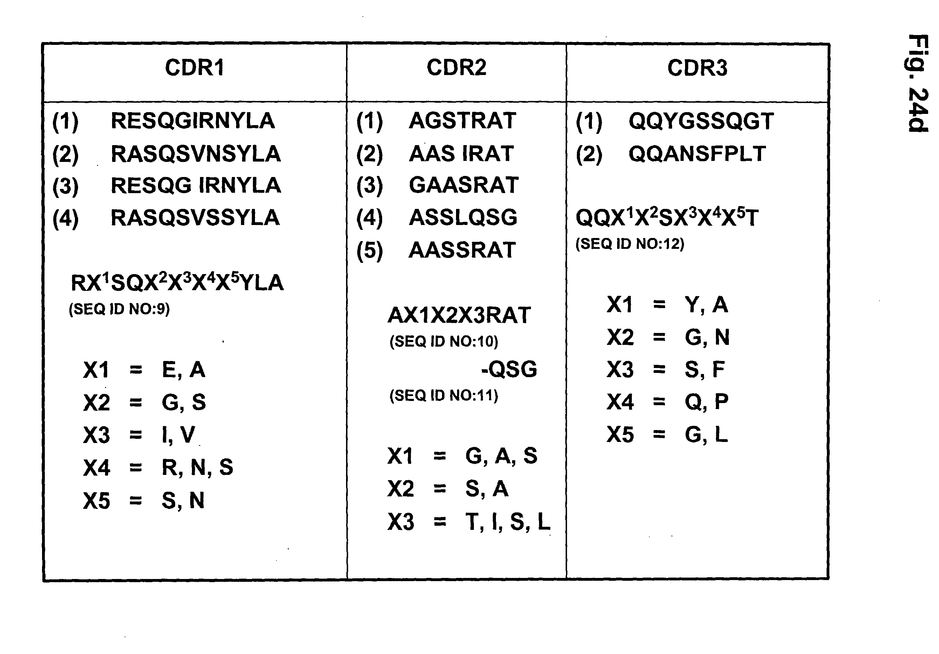

CDR Sequence domains determined for both heavy and light chain regions are summarised in Fig. 24 a to d. In agreement with mutations identified within the variable domains, a higher multiplicity was found for the heavy chains sequences with identifications of up to 7 CDR1, CDR2 and CDR3 sequences; while 4 and 5 sequences were determined for light chain CDR1 and CDR2 domains, and two CDR3 sequence variants. Using a stepwise examination of heavy and light chain CDRs, all CDR sequences were in agreement matching the Kabat rules (R. Kontermann, S. Dübel (eds.), Antibody Engineering; Springer Lab Manual Series; Springer, Heidelberg 2001; A. Galitsky, LM. Gelfand, A.E. Kister, Proc. Natl. Acad. Sci. USA 95, p. 5193-5198, 1998). The CDR sequences identified enabled the derivation of consensus sequences.

### 7) Detailed description of experimental procedures for sequence determinations

### Protein sample preparation for sequence determinations

Aβ-Autoantibodies isolated from serum-IVIgG were lyophilized and solubilized in denaturation buffer (6 M Urea; 50 mM Tris, pH=7.5) at a concentration of 1 µg/µL. Reduction of disulfide bridges was performed with DTT at a 10000x molar excess, for 2 hrs at 30 °C. Subsequent alkylation of free thiol groups was carried out with iodoacetamide at a 3000x molar excess by reaction for 1 hr at 20 °C in subdued light. The samples were subsequently lyophilized before separation of heavy and light chains by 1D-gel electrophoresis.

### N-Terminal Edman protein sequence analysis

Automated amino acid sequence analyses were performed with an Applied Biosystems 494 HT Procise Sequencer attached to a 140C Microgradient HPLC system, a 785A Programmable Absorbance Detector and a 610A data analysis system. All solvents and reagents used were of analytical ultragrade purity (supplied by Applied Biosystems Europe, Darmstadt, Germany).

The following reagents and materials (Applied Biosystems) were employed in all analyses: Blotting buffer: 25 mM Tris-HCl, 192 mM glycine, 0,1 % SDS, 20 % methanol; PVDF membrane: ProBlott, Applied Biosystems; Filter papers: GB005 (Schleicher & Schuell); Blotter: PeqLab PerfectBlue Tank-Elektroblotter Web M; staining solution: 0.1% Coomassie Blue R-250 in 50 % methanol; destaining solution: 50 % aqueous methanol.

The antibodies were reduced, alkylated and separated by 1D- SDS-PAGE into heavy and light chain components as described in 4.). Immediately after electrophoretic separation the fresh gel was equilibrated in transfer buffer for 10 min. The PVDF membrane (10 x 10 cm, the size of the gel) was wetted in methanol (analytical grade, Normapur) for 1 min and then equilibrated for 20 min in the transfer buffer. Two sheets of filter paper were cut to the dimensions of the gel (10 x 10 cm), and filter papers were soaked in the transfer buffer.

The blotting sandwich was assembled as follows: A wet filter paper was placed onto the anode (+) side of the blotting cassette. The equilibrated PVDF membrane was placed on top of the filter paper. The equilibrated gel was placed on top of the transfer membrane. The other wet filter paper was placed on top of the gel. Care was taken not to include air bubbles between the sandwich components. The cassette was then closed and immersed into the blotting tank.

The blotting was carried out at constant current 1 mA/cm² for 4 hours. After the protein transfer was completed, the PVDF membrane was washed twice for 15 min with MilliQ water to remove the SDS and glycine. The PVDF membrane was then washed with methanol for 1 min and then stained for 1 min. The stained membrane was washed with destaining solution until the protein spots were clearly visible from the background. The membrane was then allowed to dry in air. The protein spots were excised, placed in Eppendorf tubes and stored at 4 °C. Before sequence analysis the spots were washed with 100 % methanol until complete destaining. The protein spots were placed in the sequencer cartridge and sequenced using the standard *PL PVDF protein* method (pulsed liquid sequencing method for PVDF blotted proteins, Applied Biosystems).

### Proteolytic digestion of antibodies following gel electrophoretic separation

Heavy and light chains were separated by 1D-gel electrophoresis with 12 % separating gel and 5 % stacking gel and stained with colloidal Coomassie Blue as described in 4).

For in-gel proteolytic digestion and subsequent HPLC isolation and mass spectrometric analysis of the tryptic peptides, the gel bands were cut out and destained by addition of 60 % acetonitrile in MilliQ water for 20 min at 25°C. After removal of the supernatant and lyophilisation of the gel spot to dryness, 1 ml of a solution of 50 mM NH₄HCO₃ was added for rehydration and incubated for 20 min at 25°C. This procedure was repeated two times and the final rehydration was performed with the protease solution (12.5 ng/µl trypsin in 50 mM NH₄HCO₃) at 4°C for 45 min. The gel spots were then incubated for 12 hrs at 37°C in 1 ml 50 mM NH₄HCO₃ and protein fragments were eluted three times with 1 ml 60 % acetonitrile in water for 1 hr. The eluates were lyophilised to dryness and solubilised immediately prior to HPLC and MS analysis.

For protein identification/sequence and data base analyses following 2D-electrophoresis, sequence determinations by LC-MS/MS and Edman N-terminal sequence determinations of proteolytic peptides, the gel spots were excised, subjected to dehydration in acetonitrile, and following removal of acetonitrile *in vacuo* dried in a vacuum centrifuge. Sample preparation for proteolytic digestion was performed as described above, by reduction with a volume of 10 mM dithiotreitol (DTT) in 50 mM NH₄HCO₃ sufficient to cover the gel pieces, and protein was performed for 1 hr at 56 °C. After cooling to room temperature, the DTT-containing solution was replaced with the same volume of a solution of 55 mM iodoacetamide in 50 mM NH₄HCO₃. After 45 min incubation at room temperature in the dark with occasional shaking (vortexing), the gel pieces were washed for 10 min with 50-100 µL of 50 mM NH₄HCO₃, and dehydrated again by addition of the same volume of acetonitrile. The liquid phase was then removed and the gel pieces were completely dried in a vacuum centrifuge.

Excised gel pieces were digested with trypsin either manually or automatically using a DigestPro 96 robot (Intavis Bioanalytical Instruments, Langenfeld, Germany) according to literature procedures. For manual in-gel-digestion and subsequent mass spectrometric analysis, the spots were excised and destained by addition of 60 % acetonitrile in MilliQ water for 20 min at 25 °C. After removal of supernatant and lyophilisation of the gel spot, a solution of 50 mM NH₄HCO₃ was added for rehydration and incubated for 20 min at 25°C. This procedure was repeated two times, and final rehydration was then performed for 45 min with the protease solution (12.5 ng/µl trypsin in 50 mM NH₄HCO₃) at 4 °C. The gel spots were incubated for 12 h at 37°C in 50 mM NH₄HCO₃ and proteolytic peptides were eluted for 3-4 hrs with 60 % acetonitrile in water. The eluates were lyophilised to dryness and dissolved immediately before MALDI-MS analysis in 5 µl acetonitrile/0.1 % trifluoroacetic acid in water (2:1).

Automated in-gel-digestion for subsequent mass spectrometric analysis was performed with a DigestPro 96 robot (Intavis Bionalytical Instruments). The DigestPro 96 is a commercial digest robot system consisting of a Gilson 221XL robot, equipped with a module containing a temperature-regulated aluminium reactor block. The block can hold up to 96 protein samples and is mounted on rails so that it can be moved by the robot arm to either a washing or a sample collection position. Protein gel pieces were excised from the 2D-PAGE gels and loaded into a clean 96 well PCR plate which contained small holes pierced into the well bottoms. The plate was covered by a silicone membrane held in place by a lid and four mounting screws. The holes in the silicone membrane allow for reagent delivery by a specially designed dispensing needle. This needle has a second, outer channel which delivers nitrogen pressure to the reaction wells. Needle positioning allows either the delivery of liquid to the vial or the ejection from the reactor by 2.6-bar nitrogen pressure. The entire in-gel digestion process, as described below, was implemented on the robot platform, and was controlled by the DigestPro 96 software (version 4.02.; INTAVIS). Briefly, the gel pieces were washed four times with 50 µl of 50 mM NH₄HCO₃ and after each step dehydrated with 100 µl acetonitrile. After the last shrinking step, 50 µl of enzyme buffer (12.5 ng/µl trypsin in 50 mM NH₄HCO₃, pH ~ 8) were added to the tubes. The enzyme was drawn into the gel pieces for 30 min. Subsequently, 50 µl solution of 50 mM NH₄HCO₃ were added to cover the gel pieces, and after 6 hrs at 37 °C the peptides were extracted. The first extraction was performed with 50 µl NH₄HCO₃ followed by three extractions with 50 µl of 10 % formic acid; between extractions, the gel pieces were dehydrated with acetonitrile as described above. The collected extracts were then dried in a vacuum centrifuge and redissolved immediately before MS analysis, in either 5 µl MALDI-MS solution (acetonitrile:0.1 % trifluoroacetic acid in water, 2:1) or 5 µl ESI-MS solution (methanol:water:acetic acid, 50:48:2 (v/v/v)). For in-gel deglycosylation, the gel pieces were swollen in deglycosylation buffer, which was prepared by mixing 100 µL of a commercial N-Glycosidase F (PNGase F) preparation (Roche, Mannheim, Germany) with 100µL of 0.1 M ammonium bicarbonate buffer to provide a final enzyme concentration of 100 units mL⁻¹. If all liquid was taken up by the gel pieces, further digestion buffer (but without PNGase F) was added to the sample to keep the sample wet during overnight incubation at 37 °C. To avoid possible interference from PNGase F-related peptides in MALDI-MS analyses, the glycosidase was removed prior to proteolysis, by washing the gel pieces with 0.1 % SDS in 0.1 M ammonium bicarbonate (four times 250 µL for 1 hr each). All washing solutions were discarded and SDS was removed by incubation with 50:45:5 (v/v/v) methanol:water:acetic acid (30 min) and three times washing using 50 % acetonitrile in 0.1 M ammonium bicarbonate (30 min each). All washings were discarded, and the gel plugs then dried in a vacuum centrifuge. The same procedure was used for in-gel deglycosylation with EndoH glycosidase.

A ZipTip- cleanup procedure was then performed using ZipTip^{®}_{C18} pipette tips from Millipore (Eschborn, Germany). A ZipTip pipette tip is a microcolumn with a resin prepacked into the narrow end of a 10 µl pipette tip. ZipTip pipette tips contain C₁₈ or C₄ reversed-phase material for concentrating and purifying peptide and protein samples. ZipTip_{C18} pipette tips were applied for peptides and low molecular weight proteins, while ZipTip_{C4} pipette tips were applied for higher molecular weight proteins. The complete ZipTip procedure was carried out according to the instructions of the manufacturer. Briefly, it consists essentially of five steps: wetting; equilibration of the ZipTip pipette tip; binding of peptides and proteins to the pipette tip; washing; and elution.

### HPLC separation and isolation of proteolytic peptides

All analytical HPLC separations were performed with a BIO-RAD (Muenchen, Germany) 2700 HPLC system using a Vydac C₄ column (250 x 4.6 mm I.D.) with 5 µm silica (300 A pore size). Linear gradient elution (0 min 0 % solvent B; 5 min 0 % solvent B; 135 min 65 % solvent B, 150 min 100 % solvent B, 160 min 100 % B), with eluent A consisting of 0.1 % trifluoroacetic acid (TFA) in water, and eluent B of 0.1 % TFA in acetonitrile:water (80:20, v/v) at a flow rate of 1 mL/min. The proteolytic peptide samples, typically 50 µg-aliquots were dissolved in 200 µL of eluent A. Detection of peptides was generally performed at 220 nm using a BIO-RAD variable wavelength absorbance detector.

### N-terminal Edman sequence determinations of proteolytic peptides

Tryptic peptides were isolated by HPLC as described above, and lyophilised and stored at -20 °C prior to sequence analysis. The sample support used for sequence determinations consisted of a glass fibre filter (Applied Biosystems) which was treated with a 30 µL BioBrene^{™} Plus (Applied Biosystems) solution (100µg/µL Biobrene and 6,66 µg/µL NaCl in water), and precycled (3 cycles) using the standard *filter precycle* method. For sequence analyses the lyophilised HPLC fractions were reconstituted in 15 µL 0,1 % TFA, containing 20 % (v/v) acetonitrile in water. The reconstituted peptide solution was applied on the precycled glass fibre filter in aliquots of 5 µl to ensure a distribution as close to the centre of the glass fibre filter as possible, each application followed by drying under a stream of Ar for 1 min.

All sequence analyses were performed on an Applied Biosystems 494 HT Procise Sequencer/140C Microgradient System with 785A Programmable Absorbance Detector and 610A Data Analysis System as described above. All solvents and reagents used were from Applied Biosystems. The general method used for the analysis of proteolytic peptides was the standard *pulse-liquid* method.

### Sequence determinations by ESI-LC-MS/MS of proteolytic peptides

All sequence determinations of tryptic peptides isolated by HPLC (s. above) were performed with a Bruker Esquire-3000+ ion-trap LC-MS/MS system equipped with nano-ESI/LC ion source systems (Bruker Daltonics, Bremen, Germany). HPLC fractions of proteolytic peptides were collected in 1 ml Eppendorf cups and lyophilized to dryness and stored at -20 °C until LC/MS analysis. The HPLC fractions were dissolved in 16 µl of a solvent mixture containing 1 % formic acid in water:acetonitrile (9:1, v/v). The samples were sonicated for 5 min at 20 °C and centrifugated at 13 000 rpm/min for 3 min. The content of a sample was transferred into a 2 ml screw cap vial equipped with an internal microvial (0.1 ml) and placed in the LC/MS tray. A 3 µl aliquot of the sample was injected on the C-18 microcolumn by means of the automatic injection system, and an elution gradient listed in the table below (LC-MS gradient) was employed. The sample flows from the injection loop into the column and is then directed into the electrospray interface and through the ion optics into the ion trap. The total ion current (TIC) was recorded as a function of time, and is converted into the mass spectrum using the Data Analysis software (Bruker Daltonics). The most intensive ions to be used for MS/MS analysis were selected from the mass spectrum resulting from the first LC/MS run. For each precursor ion identified, a separate LC-MS/MS run was performed at identical gradient conditions (s. Table). Following start of the pumping system, the isolation and fragmentation of parent ions was switched on in the Esquire Control window, using the specification of precursor mass, isolation width and fragmentation amplitude. The total ion current (TIC) corresponding to the ion fragments was recorded as a function of time; if a single parent ion was subjected to fragmentation during the run, the TIC contains a single peak. The MS/MS spectrum of the precursor ion was generated by the Data Analysis software by averaging the pulses at half peak width. The m/z values of the fragments contained in the MS/MS spectrum and their intensities were exported into a data analysis file type (wearing the extension *mgf). The file was uploaded into the MS/MS Mascot search engine for performing the NCBInr data base search, using the following search parameters: taxonomy, Homo sapiens; allowed missed cleavages, - 1; peptide search tolerance, 2 Da; MS/MS tolerance, 0.8 Da; fixed modification, carbamidomethyl (cysteine); variable modification, Met-oxidation. The results displayed contain the mowse probaility score in form of a chart, providing the peptide sequence and the protein originating for each hit result. If the result for the fragment ions of a given precursor led to direct identification score of a peptide from immunoglobulin heavy- or light- chain, the peptide sequence obtained was taken to be a correct one. If no identification score was directly obtained for a given precursor and its MS/MS spectrum, the peptide sequence was ascertained by *de novo* sequencing, using the assignment function from the Data Analysis software. This function assigns the mass difference between two fragments into the mass for a specific amino acid. If the peptide sequence data obtained by the *de novo* procedure was identical with the sequence obtained by the NCBI database search, the sequence result was taken as correct. If the database search performed for a certain precursor ion did not provide any immunoglobulin peptide fragment, the corresponding precursor ion was assigned as unidentified.

**Table of LC-MS/MS gradient elution parameters.**

| Time (min) | Solvent A | Solvent B |
|---|---|---|
| 0 | 80 | 20 |
| 3 | 80 | 20 |
| 6 | 50 | 50 |
| 16 | 20 | 80 |
| 18 | 2 | 98 |
| 20 | 2 | 98 |
| 22 | 98 | 2 |
| 24 | 98 | 2 |

### MALDI-TOF mass spectrometry of proteolytic peptides

MALDI-TOF MS analysis was carried out with a Bruker Biflex linear TOF mass spectrometer (Bruker Daltonics, Bremen, Germany) equipped with a nitrogen UV laser (λ=337 nm), a 26-sample SCOUT source, a video system and a XMASS data system for spectra acquisition and instrument control. A saturated solution of α-cyano-4-hydroxy-cinnamic acid (HCCA) in acetonitrile: 0.1 % TFA in water (2:1 v/v) was used as the matrix. For all MALDI-MS analyses, 0.8 µL of matrix solution and 0.8 µL of the sample solution (proteolytic peptide mixture or tryptic peptides separated by HPLC) were mixed on the stainless steel MALDI target and allowed to dry. Acquisition of spectra was carried out at an acceleration voltage (V_{acc}) of 20 kV and a detector voltage of 1.5 kV. External calibration was carried out using the average masses of singly protonated ion signals of bovine insulin (5734.5 Da), bovine insulin B-chain oxidized (3496.9), human neurotensin (1673.9 Da), human angiotensin I (1297.5 Da), human bradykinin (1061.2) and human angiotensin II (1047.2 Da).

### MALDI-FT-ICR-MS of proteolytic peptides

MALDI-FTICR mass spectrometric analyses were performed with a Bruker APEX II FTICR instrument (Bruker Daltonics, Bremen, Germany) equipped with an actively shielded 7T superconducting magnet (Magnex, Oxford, UK), a cylindrical infinity ICR analyzer cell, and an external Scout 100 fully automated X-Y target stage MALDI source with pulsed collision gas. The pulsed nitrogen laser was operated at 337 nm.

Analyses of peptide samples were performed with a 100 mg/mL solution of 2,5-dihydroxybenzoic acid (DHB) in acetonitrile/0.1% TFA in water (2:1 v/v) used as the matrix. An aliquot of 0.5 µL of matrix solution and 0.5 µL of sample solution (tryptic peptide or peptide mixture) were mixed on the stainless steel MALDI target and allowed to dry. External calibration was carried out using the monoisotopic masses of singly protonated ion signals of bovine insulin (5730.609 Da), bovine insulin B-chain oxidized (3494.651), human neurotensin (1672.917 Da), human angiotensin I (1296.685 Da), human bradykinin (1060.569) and human angiotensin II (1046.542 Da). Acquisition and processing of spectra were performed with XMASS software (Bruker Daltonics, Bremen, Germany).

MALDI-FTICR-MS/MS analyses were performed with the Bruker ApexII FTICR-MS instrument equipped with SORI-CID (sustained-off-resonance-collision-induced - dissociation) dissociation, IRMPD instrumentation for fragmentation of peptide and protein ions (Damoc et al., 2003). Ions formed by MALDI ionization were trapped in the analyzer cell, and isolation of a precursor ion was performed by ejecting from the ICR cell all ions of higher and lower masses through the application of suitable excitation pulses, using the appropriate frequencies and amplitudes. The following experimental conditions were employed: correlated sweep attenuation: 8-10 dB, ejection safety belt: 500-1000 Hz. For SORI-CID, a low-amplitude rf- excitation was applied for 250 msec to the precursor ion at a frequency that is slightly off-resonance (500-1000 Hz) from the cyclotron frequency. The amplitude of the excitation was kept low so that the ion never went too far from the center of the cell. While this excitation was applied, the pressure was raised in the analyzer cell (10⁻⁸ mbar) by admitting a collision gas (argon) through a pulse valve for 20-80 msec. Under these conditions, the precursor ion underwent many low-energy collisions, which slowly activated the ion until it reached its threshold for dissociation.

For IRMPD (infrared-multiphoton-dissociation) experiments the mass-selected ions were photodissociated using a 25 W continuous wave CO₂ laser (10.6 µm, Synard, Mukilteo, WA, USA). The laser power was set to 50 % threshold and the laser irradiation time to 50-200 msec.

For protein identifications and sequence determinations (constant region sequences) of proteolytic peptide mixtures following 2D-gel electrophoresis, the following (publicly available) data base search engines were employed:
**Mascot -** Peptide mass fingerprint and MS/MS ion search from Matrix Science Ltd., London.
**ProFound -** Peptide mass fingerprint from Rockefeller and New York Universities.
**MS-Fit -** Peptide mass fingerprint from University of California, San Francisco (UCSF).
**MS-Tag -** MS/MS ion search from University of California, San Francisco (UCSF).

### Example 3: Determination of dissociation constants of antigen-antibody-complexes by ELISA

The K_{d} values were determined by a modification of the method of Kim et al. (1990).
1) For the indirect ELISA, microtiter plates were coated with 150 µL/well of streptavidin at 20 °C for 2 hrs. Wells were washed one time with 0.05 % (v/v) Tween- 20 detergent in phosphate-buffered saline (PBS) (Na₂HPO₄ 5 mM, NaCl 150 mM, pH 7.5). Biotinylated-(G)₅-Aβ(12-40) peptide at concentrations between 1 × 10⁻⁶ and 10⁻⁸ M were prepared in PBS and deposited in the wells at a volume of 100 µL/welL The wells were incubated for 2 hours at 20 °C temperature followed by a 4 times washing step and blocking with blocking buffer (BSA 5 % w/v, 0.05 % Tween-20 v/v in PBS) for 2 hours. Anti-Aβ(12-40) antibody was diluted to concentrations between 1.4× 10⁻⁷ and 10⁻⁹ M with blocking buffer and added at 100 µL/well. The microplate was incubated 20 °C for 2 hours and then washed with Covabuffer (0.15 M PBS, pH 7.2 containing 2M NaCl, 0.083 M MgSO₄ and 0.05% Tween-20). The wells were incubated with peroxidase-conjugated mouse anti-human IgG (1:5.000) for 45 min at 20 °C . Antibody binding was detected with a freshly prepared solution of 1,2-Phenylendiamine (OPD) containing 0.1 M citrate-phosphate, 0.1 % OPD and 0.006 % hydrogen peroxide. The enzyme reaction was monitored as a function of time at 450 nm, using an ELISA plate reader (Victor², Perkin Elmer Life/Analytical Sciences, Boston, MA). For each antibody and antigen concentration triplicate wells were prepared and measured. Direct proportionality was observed between absorbance and antibody concentration over a wide concentration range. This concentration range was used to select the initial concentration for K_{d} determinations. The initial concentration was selected to be within the linear region of the plot of optical density vs. antibody concentration.
2) For the determination of the K_{d} values the following conditions were applied. The antigen, Aβ(12-40) peptide at various concentrations (1×10⁻⁶ M to 4.8×10⁻¹⁰ M) was mixed with a constant concentration of antibody derived from the preliminary ELISA calibration. The incubation was performed in 5 % BSA, 0.05 % Tween-20 in PBS using polypropylene test tubes to minimize antibody loss by adsorption on the microreaction tube walls. After 2 hrs, 100 µl of each mixture was transferred and incubated for 30 min into the wells of a microtiter plate previously coated with biotinylated-(G)₅-Aβ(12-40) (1 µM) and blocked. The concentration of free antibody was then measured by indirect ELISA as described above. The K_{d} values were obtained by plotting the experimental data using the Sips coordinates.
   The following mean K_{d} values were determined for the Aβ-antibody complexes of Aβ(12-40) peptide:
   a) serum IVIG; purified: 8 x 10⁻⁹ M
   b) Serum-Ab, Ü-30-A: 14 x 10⁻⁹ M
   c) Serum-Ab, Ü-30-B: 18 x 10⁻⁹ M#

Since the range of binding/dissociation constants for the formation of Aβ-fibrils/aggregates has been estimated in the literature to be in the range of 10⁻⁶ M (determined), the binding of antibodies is determined to be specific. For IgG antibodies, typical K_{d}-values in the range of 10⁻⁸ to 10⁻⁹ M have been determined for a large variety of oligo- and polypeptide antigens and epitopes.

### Example 4: Use of isolated Aβ autoantibodies as medicament for inhibition of plaque formation etc.

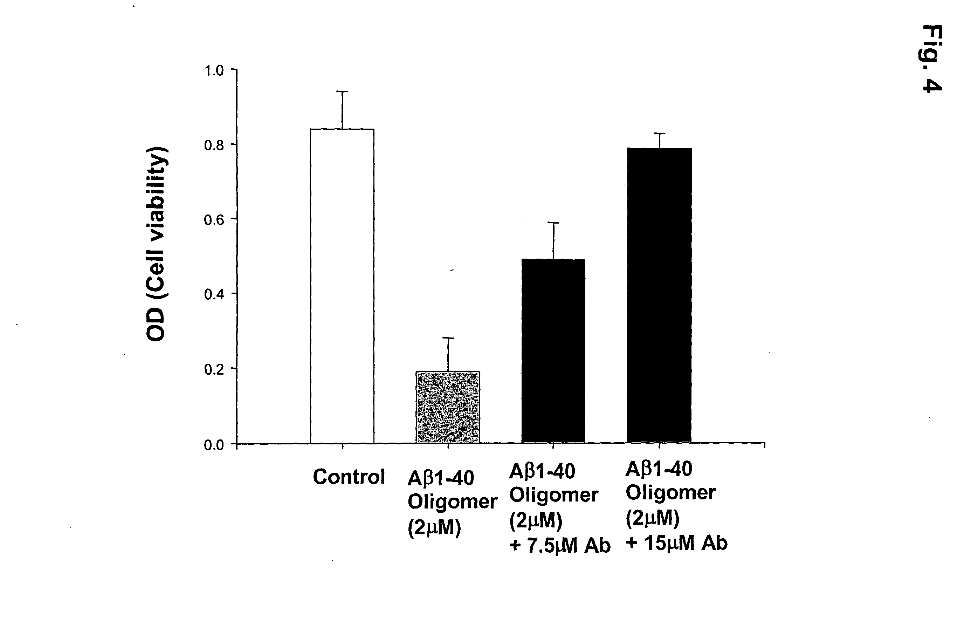

Human neuroblastoma cells (SH-Sy5y) were grown in RPMI 1640-Medium supplemented with 10 % fetal calf serum, 10 mM Hepes, 4 mM Glutamine and penicillin (200 units/ml), streptomycin (200 µg/ml). Cells were incubated at a density of 30.000 cells/well over night in a 96-well microtiter plate. After removal of medium, cells were washed with PBS, and toxic Aβ-oligomers (2 µM final concentration) were added at a volume of 100 µl fresh medium to 7.5 µM, or 15 µM Aβ-antboody, or without Aβ-autoantibody. MTT test was performed after 4-hrs incubation. Fig. 4 shows that the Aβ-mediated toxicity (grey bars) is almost completely antagonised by Aβ-autoantibodies (black bars).

## Claims

1. Polypeptide binding specifically to the C-terminal half of Aβ polypeptide as denoted in SEQ ID NO 2.

2. Polypeptide according to claim 1, wherein the polypeptide comprises one or more sequences selected from SEQ ID NOs: 6 to 12.

3. Polypeptide according to anyone of the preceding claims, wherein the polypeptide comprises one or more sequences selected from the group consisting of SEQ ID NOs: 13 to 20, SEQ ID NOs: 21 to 27, SEQ ID NOs:28 to 32; SEQ ID NOs: 33 to 37, SEQ ID NOs: 38 to 43, and SEQ ID NOs: 44 to 46.

4. Polypeptide according to claim 3, wherein the polypeptide comprises one sequence of each of said groups.

5. Polypeptide according to anyone of the preceding claims, wherein the polypeptide comprises a sequence selected from the group of SEQ ID NOs: 47 to 55 and/or a sequence selected from the group of SEQ ID NOs: 56 to 71.

6. Polypeptide according to anyone of the preceding claims, wherein the polypeptide is an antibody, in particular a monoclonal antibody, or a derivative thereof.

7. Polypeptide according to anyone of the preceding claims, wherein the polypeptide comprises a sequence selected from the group of SEQ ID NOs: 78 to 93 and/or a sequence selected from the group of SEQ ID NOs: 94 to 137.

8. Polypeptide according to anyone of the preceding claims, wherein the polypeptide is an antibody or derivative thereof comprising:
a)on the light chain as CDR1 SEQ ID NO:34, as CDR2 SEQ ID NO:38 and as CDR3 SEQ ID NO:44, and on the heavy chain as CDR1 SEQ ID NO:13, as CDR2 SEQ ID NO:21 and as CDR3 SEQ ID NO:28, or
b) on the light chain as CDR1 SEQ ID NO:33, as CDR2 SEQ ID NO:42 and as CDR3 SEQ ID NO:44, and on the heavy chain as CDR1 SEQ ID NO:14, as CDR2 SEQ ID NO:27 and as CDR3 SEQ ID NO:30.

9. Polypeptide according to anyone of the preceding claims for use in human and/or veterinary medicine.

10. Use of the polypeptide according to claim 9 for the manufacture of a medicament in order to treat and/or prevent the progression of Alzheimer's disease, Down's syndrome, Dementia with Lewy bodies, fronto-temporal dementia, cerebral amyloid angiopathy, and amyloidoses.

11. Use of a polypeptide comprising an amino acid sequence of an Aβ polypeptide, wherein the Aβ polypeptide has at least the sequence according to SEQ ID NO:2 and at most the sequence according to SEQ ID NO:4 for use in human and veterinary medicine, in particular for the manufacture of a medicament for the treatment and/or prevention of Alzheimer's disease, Down's syndrome, Dementia with Lewy bodies, fronto-temporal dementia, cerebral amyloid angiopathy, and amyloidoses.

12. Use of a polypeptide comprising an amino acid sequence of an Aβ polypeptide, wherein the Aβ polypeptide has at least the sequence according to SEQ ID NO:2 and at most the sequence according to SEQ ID NO:4 for isolation and separation of a polypeptide according to any one of claims 1 to 8 from a sample.

13. Method of separation of a polypeptide according to anyone of claims 1 to 7 from a sample, the method comprising the following steps:
a) incubating a polypeptide comprising an amino acid sequence of an Aβ peptide, wherein the Aβ peptide has at least the sequence according to SEQ ID NO:2, i.e. Aβ(21-37) and at most the sequence according to SEQ ID NO:4, i.e. Aβ(12-40), which polypeptide is immobilized on a carrier, with a sample,
b) separating said sample from the carrier.
c) separating polypeptides according to the invention bound to the polypeptide of step a) from the carrier.

14. Method of separation of a polypeptide according to anyone of claims 1 to 8 from a sample, the method comprising the following steps:
a) incubating a polypeptide, comprising an amino acid sequence of an Aβ peptide, wherein the Aβ peptide has at least the sequence according to SEQ ID NO:2 and at most the sequence according to SEQ ID NO:4, with a sample and subsequently
b) incubating the sample with a carrier having a binding affinity for the polypeptide of step a), and
c) separating said sample from the carrier, and,
d) separating polypeptides according to the invention bound to the polypeptide of step a) from the carrier.

15. Isolated cell producing a polypeptide according to anyone of claims 1 to 8.

16. Nucleic acid encoding a polypeptide according to anyone of claims 1 to 8.

17. Vector comprising the nucleic acid according to claim 16.

18. Host cell comprising the vector according to claim 17.


### Annex to the application documents - subsequently filed sequences listing

SEQUENCE LISTING

<110>  Rheinische Friedrich-Wilhelms-Universitaet Bonn
       Universitaet Konstanz

<120>  Prophylaxis and Therapy of Alzheimer's Disease and other
       neurodementing diseases

<130>  UBK-002 EP

<160>  144

<170>  PatentIn version 3.3


<210>  1
<211>  40
<212>  PRT
<213>  Homo sapiens

<400>  1

Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val
        35                  40


<210>  2
<211>  17
<212>  PRT
<213>  Homo sapiens

<400>  2

Ala Glu Asp Val Gly Ser Asn Lys Gly Ala Ile Ile Gly Leu Met Val
1               5                   10                  15
Gly


<210>  3
<211>  7
<212>  PRT
<213>  Homo sapiens

<400>  3

Phe Arg His Asp Ser Gly Tyr
1               5


<210>  4
<211>  29
<212>  PRT
<213>  Homo sapiens

<400>  4

Val His His Gln Lys Leu Val Phe Phe Ala Glu Asp Val Gly Ser Asn
1               5                   10                  15

Lys Gly Ala Ile Ile Gly Leu Met Val Gly Gly Val Val
            20                      25

<210>  5
<211>  20
<212>  PRT
<213>  Homo sapiens

<400>  5

Asp Ala Glu Phe Arg His Asp Ser Gly Tyr Glu Val His His Gln Lys
1               5                   10                  15
Leu Val Phe Phe
            20

<210>  6
<211>  10
<212>  PRT
<213>  Homo sapiens

<220>
<221>  misc_feature
<222>  (2)..(2)
<223>  Xaa can be Phe or Ile

<220>
<221>  misc_feature
<222>  (5)..(5)
<223>  Xaa can be Arg or Ser

<220>
<221>  misc_feature
<222>  (6)..(6)
<223>  Xaa can be Ser, Gly or Asn

<220>
<221>  misc_feature
<222>  (8)..(8)
<223>  Xaa can be Trp, Asp or Met

<220>
<221>  misc_feature
<222>  (10)..(10)
<223>  Xaa can be Ser or His

<400>  6

Gly Xaa Thr Phe Xaa Xaa Tyr Xaa Met Xaa
1               5                   10

<210>  7
<211>  17
<212>  PRT
<213>  Homo sapiens

<220>
<221>  misc_feature
<222>  (5)..(5)

<223>  Xaa can be Asp, Thr or Phe

<220>
<221>  misc_feature
<222>  (6)..(6)
<223>  Xaa can be Gly, Ala or Phe

<220>
<221>  misc_feature
<222>  (8)..(8)
<223>  Xaa can be Ser or Gly

<220>
<221>  misc_feature
<222>  (9)..(9)
<223>  Xaa can be Glu, Asp, Arg or Gly

<220>
<221>  misc_feature
<222>  (10)..(10)
<223>  Xaa can be Lys, Pro, Ser or Thr

<220>
<221>  misc_feature
<222>  (11)..(11)
<223>  Xaa can be Ala, Leu, Tyr or Asn

<220>
<221>  misc_feature
<222>  (12)..(12)
<223>  Xaa can be Tyr or Ala

<220>
<221>  misc_feature
<222>  (13)..(13)
<223>  Xaa can be Asp, Gly or Pro

<400>  7

Ser Val Lys Gln Xaa Xaa Ser Xaa Xaa Xaa Xaa Xaa Xaa Ser Val Lys
1               5                   10                  15
Gly


<210>  8
<211>  13
<212>  PRT
<213>  Homo sapiens


<220>
<221>  misc_feature
<222>  (3)..(3)
<223>  Xaa can be Ala or Gly

<220>
<221>  misc_feature
<222>  (4)..(4)
<223>  Xaa can be Ser, Arg or Gly

<220>
<221>  misc_feature

<222>  (5)..(5)
<223>  Xaa can be Ser, Arg or Trp

<220>
<221>  misc_feature
<222>  (7)..(7)
<223>  Xaa can be Tyr or Ala

<220>
<221>  misc_feature
<222>  (8)..(8)
<223>  Xaa can be Arg, Asp or Leu

<220>
<221>  misc_feature
<222>  (9)..(9)
<223>  Xaa can be Asp, Leu or Gly

<220>
<221>  misc_feature
<222>  (10)..(10)
<223>  Xaa can be Ala or Trp

<220>
<221>  misc_feature
<222>  (13)..(13)
<223>  Xaa can be Pro or Ile

<400>  8

Arg Asp Xaa Xaa Xaa Trp Xaa Xaa Xaa Xaa Phe Asp Xaa
1               5                   10


<210>  9
<211>  11
<212>  PRT
<213>  Homo sapiens

<220>
<221>  misc_feature
<222>  (2)..(2)
<223>  Xaa can be Glu or Ala

<220>
<221>  misc_feature
<222>  (5)..(5)
<223>  Xaa can be Gly or Ser

<220>
<221>  misc_feature
<222>  (6)..(6)
<223>  Xaa can be Ile or Val

<220>
<221>  misc_feature
<222>  (7)..(7)
<223>  Xaa can be Arg, Asn or Ser

<220>
<221>  misc_feature
<222>  (8)..(8)
<223>  Xaa can be Ser or Asn

<400>  9

Arg Xaa Ser Gln Xaa Xaa Xaa Xaa Tyr Leu Ala
1               5                   10


<210>  10
<211>  7
<212>  PRT
<213>  Homo sapiens


<220>
<221>  misc_feature
<222>  (2)..(2)
<223>  Xaa can be Gly, Ala or Ser

<220>
<221>  misc_feature
<222>  (3)..(3)
<223>  Xaa can be Ser or Ala

<220>
<221>  misc_feature
<222>  (4)..(4)
<223>  Xaa can be Thr, Ile, Ser or Leu

<400>  10

Ala Xaa Xaa Xaa Arg Ala Thr
1               5


<210>  11
<211>  7
<212>  PRT
<213>  Homo sapiens


<220>
<221>  misc_feature
<222>  (2)..(2)
<223>  Xaa can be Gly, Ala or Ser

<220>
<221>  misc_feature
<222>  (3)..(3)
<223>  Xaa can be Ser or Ala

<220>
<221>  misc_feature
<222>  (4)..(4)
<223>  Xaa can be Thr, Ile, Ser or Leu

<400>  11

Ala Xaa Xaa Xaa Gln Ser Gly
1               5


<210>  12
<211>  9

<212>  PRT
<213>  Homo sapiens


<220>
<221>  misc_feature
<222>  (3)..(3)
<223>  Xaa can be Tyr or Ala

<220>
<221>  misc_feature
<222>  (4)..(4)
<223>  Xaa can be Gly or Asn

<220>
<221>  misc_feature
<222>  (6)..(6)
<223>  Xaa can be Ser or Phe

<220>
<221>  misc_feature
<222>  (7)..(7)
<223>  Xaa can be Gln or Pro

<220>
<221>  misc_feature
<222>  (8)..(8)
<223>  Xaa can be Gly or Leu

<400>  12

Gln Gln Xaa Xaa Ser Xaa Xaa Xaa Thr
1               5


<210>  13
<211>  10
<212>  PRT
<213>  Homo sapiens

<400>  13

Gly Phe Thr Phe Arg Ser Tyr Trp Met Ser
1               5                   10


<210>  14
<211>  10
<212>  PRT
<213>  Homo sapiens

<400>  14

Gly Phe Thr Phe Arg Gly Tyr Trp Met Ser
1               5                   10


<210>  15
<211>  10
<212>  PRT
<213>  Homo sapiens

<400>  15

Gly Phe Thr Phe Arg Asn Tyr Asp Met His
1               5                   10


<210>  16
<211>  10
<212>  PRT
<213>  Homo sapiens

<400>  16

Gly Phe Thr Phe Arg Ser Tyr Trp Met His
1               5                   10


<210>  17
<211>  10
<212>  PRT
<213>  Homo sapiens

<400>  17

Gly Phe Thr Phe Arg Asn Tyr Trp Met Ser
1               5                   10


<210>  18
<211>  10
<212>  PRT
<213>  Homo sapiens

<400>  18

Gly Phe Thr Phe Arg Ser Tyr Asp Met Ser
1               5                   10


<210>  19
<211>  10
<212>  PRT
<213>  Homo sapiens

<400>  19

Gly Ile Thr Phe Arg Ser Tyr Met Asp Ser
1               5                   10


<210>  20
<211>  10
<212>  PRT
<213>  Homo sapiens

<400>  20

Gly Phe Thr Phe Ser Ser Tyr Trp Met Ser
1               5                   10


<210>  21
<211>  17
<212>  PRT

<213>  Homo sapiens

<400>  21

Ser Val Lys Gln Asp Gly Ser Glu Lys Tyr Tyr Val Asp Ser Val Lys
1               5                   10                  15
Gly

<210>  22
<211>  17
<212>  PRT
<213>  Homo sapiens

<400>  22

Arg Ile Gly Thr Ala Gly Asp Arg Tyr Tyr Ala Gly Ser Val Lys Gly
1               5                   10                  15
Val

<210>  23
<211>  16
<212>  PRT
<213>  Homo sapiens

<400>  23

Arg Ile Gly Thr Ala Gly Arg Thr Asn Tyr Asn Pro Ser Leu Lys Gly
1               5                   10                  15

<210>  24
<211>  17
<212>  PRT
<213>  Homo sapiens

<400>  24

Ser Val Lys Gln Phe Phe Ser Gly Lys Tyr Tyr Ala Gly Ser Val Lys
1               5                   10                  15
Gly

<210>  25
<211>  17
<212>  PRT
<213>  Homo sapiens

<400>  25

Ser Val Lys Gln Phe Phe Ser Gly Ser Ala Ala Thr Gly Ser Val Lys
1               5                   10                  15
Gly

<210>  26
<211>  17
<212>  PRT

<213>  Homo sapiens

<400>  26

Ser Val Lys Gln Phe Phe Ser Gly Pro Leu Ala Thr Gly Ser Val Lys
1               5                   10                  15
Gly

<210>  27
<211>  17
<212>  PRT
<213>  Homo sapiens

<400>  27

Ser Val Lys Gln Asp Gly Ser Glu Lys Tyr Tyr Val Asp Ser Val Lys
1               5                   10                  15
Gly

<210>  28
<211>  13
<212>  PRT
<213>  Homo sapiens

<400>  28

Arg Asp Ala Ser Ser Trp Tyr Arg Asp Trp Phe Asp Pro
1               5                   10

<210>  29
<211>  13
<212>  PRT
<213>  Homo sapiens

<400>  29

Gly Ala Gly Arg Trp Ala Pro Leu Gly Ala Phe Asp Ile
1               5                   10

<210>  30
<211>  13
<212>  PRT
<213>  Homo sapiens

<400>  30

Arg Asp Gly Arg Ser Trp Tyr Arg Asp Trp Phe Asp Pro
1               5                   10

<210>  31
<211>  13
<212>  PRT
<213>  Homo sapiens

<400>  31

Arg Asp Gly Ser Ser Trp Tyr Arg Asp Trp Phe Asp Pro
1               5                   10

<210>  32
<211>  13
<212>  PRT
<213>  Homo sapiens

<400>  32

Arg Asp Ala Gly Arg Trp Ala Asp Leu Ala Phe Asp Ile
1               5                   10

<210>  33
<211>  11
<212>  PRT
<213>  Homo sapiens

<400>  33

Arg Glu Ser Gln Gly Ile Arg Asn Tyr Leu Ala
1               5                   10

<210>  34
<211>  11
<212>  PRT
<213>  Homo sapiens

<400>  34

Arg Ala Ser Gln Ser Val Asn Ser Tyr Leu Ala
1               5                   10

<210>  35
<211>  11
<212>  PRT
<213>  Homo sapiens

<400>  35

Arg Glu Ser Gln Gly Ile Arg Asn Tyr Leu Ala
1               5                   10

<210>  36
<211>  11
<212>  PRT
<213>  Homo sapiens

<400>  36

Arg Ala Ser Gln Ser Val Ser Ser Tyr Leu Ala
1               5                   10

<210>  37
<211>  12
<212>  PRT
<213>  Homo sapiens

<400>  37

Thr Leu Ser Ser Glu His Ser Thr Tyr Thr Ile Glu
1               5                   10


<210>  38
<211>  7
<212>  PRT
<213>  Homo sapiens

<400>  38

Ala Gly Ser Thr Arg Ala Thr
1               5


<210>  39
<211>  7
<212>  PRT
<213>  Homo sapiens

<400>  39

Ala Ala Ser Ile Arg Ala Thr
1               5


<210>  40
<211>  7
<212>  PRT
<213>  Homo sapiens

<400>  40

Gly Ala Ala Ser Arg Ala Thr
1               5


<210>  41
<211>  7
<212>  PRT
<213>  Homo sapiens

<400>  41

Ala Ser Ser Leu Gln Ser Gly
1               5


<210>  42
<211>  7
<212>  PRT
<213>  Homo sapiens

<400>  42

Ala Ala Ser Ser Arg Ala Thr
1               5


<210>  43

<211>  7
<212>  PRT
<213>  Homo sapiens

<400>  43

Val Lys Ser Asp Gly Ser His
1               5


<210>  44
<211>  9
<212>  PRT
<213>  Homo sapiens

<400>  44

Gln Gln Tyr Gly Ser Ser Gln Gly Thr
1               5


<210>  45
<211>  9
<212>  PRT
<213>  Homo sapiens

<400>  45

Gln Gln Ala Asn Ser Phe Pro Leu Thr
1               5


<210>  46
<211>  10
<212>  PRT
<213>  Homo sapiens

<400>  46

Gly Glu Ser His Thr Ile Asp Gly Gln Cys
1               5                   10


<210>  47
<211>  109
<212>  PRT
<213>  Homo sapiens

<400>  47

Glu Ile Val Leu Thr Gln Ser Pro Ala Thr Leu Ser Leu Ser Pro Gly
1               5                   10                  15
Glu Arg Val Thr Ile Thr Cys Arg Ala Ser Gln Ser Val Asn Ser Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg

100                 105

<210>  48
<211>  109
<212>  PRT
<213>  Homo sapiens

<400>  48

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105

<210>  49
<211>  111
<212>  PRT
<213>  Homo sapiens

<400>  49

Leu Pro Val Leu Thr Gln Pro Pro Ser Ala Ser Ala Leu Leu Gly Ala
1               5                   10                  15
Ser Ile Lys Leu Thr Cys Thr Leu Ser Ser Glu His Ser Thr Tyr Thr
            20                  25                  30
Ile Glu Trp Tyr Gln Gln Arg Pro Gly Arg Ser Pro Gln Tyr Ile Met
        35                  40                  45
Lys Val Lys Ser Asp Gly Ser His Ser Lys Gly Asp Gly Ile Pro Asp
    50                  55                  60
Arg Phe Met Gly Ser Ser Ser Gly Ala Asp Arg Tyr Leu Thr Phe Ser
65                  70                  75                  80
Asn Leu Gln Ser Asp Asp Glu Ala Glu Tyr His Cys Gly Glu Ser His
                85                  90                  95
Thr Ile Asp Gly Gln Cys Trp Val Phe Gly Gly Gly Thr Lys Leu
            100                 105                 110

<210>  50
<211>  109
<212>  PRT
<213>  Homo sapiens

<400>  50

Asp Ile Gln Met Thr Gln Ser Pro Ala Thr Leu Ser Leu Ser Pro Gly
1               5                   10                  15
Glu Arg Ala Ala Leu Ser Cys Arg Ala Ser Gln Ser Val Asn Ser Tyr
            20                  25                  30
Leu Ala Trp Tyr Lys Gln Gln Lys Pro Gly Gln Ala Pro Arg Leu Leu
        35                  40                  45
Ile Tyr Gly Ala Ala Ser Arg Ala Thr Gly Ile Pro Ala Arg Phe Ser

    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105


<210>  51
<211>  109
<212>  PRT
<213>  Homo sapiens

<400>  51

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105


<210>  52
<211>  109
<212>  PRT
<213>  Homo sapiens

<400>  52

Glu Ile Val Met Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ser Ser Leu Gln Ser Gly Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105


<210>  53
<211>  108
<212>  PRT
<213>  Homo sapiens

<400>  53

Asp Val Val Met Thr Gln Ser Pro Ser Ser Leu Ser Ala Ser Val Gly

1               5                   10                  15
Asp Arg Val Thr Ile Thr Cys Arg Glu Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Lys Ala Ser Ser Leu Gln Ser Gly Val Pro Ser Arg Phe Ser Gly
    50                  55                  60
Ser Leu Leu Gly Gly Lys Ala Ala Leu Thr Leu Ser Gly Val Gln Pro
65                  70                  75                  80
Glu Asp Phe Ala Thr Tyr Tyr Cys Gln Gln Ala Asn Ser Phe Pro Leu
                85                  90                  95
Thr Phe Gly Gly Gly Thr Lys Val Glu Ile Lys Arg
            100                 105


<210>  54
<211>  109
<212>  PRT
<213>  Homo sapiens

<400>  54

Asp Ile Val Leu Thr Gln Ser Pro Ala Thr Leu Ser Leu Ser Pro Gly
1               5                   10                  15
Glu Arg Val Thr Ile Thr Cys Arg Ala Ser Gln Ser Val Asn Ser Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105


<210>  55
<211>  109
<212>  PRT
<213>  Homo sapiens

<400>  55

Asp Ile Gln Met Thr Gln Ser Pro Gly Thr Leu Ser Leu Ser Pro Gly
1               5                   10                  15
Glu Arg Ala Ala Leu Ser Cys Arg Ala Ser Gln Ser Val Ser Ser Tyr
            20                  25                  30
Leu Ala Trp Tyr Ser Gln Gln Lys Pro Gly Gln Ala Pro Arg Leu Leu
        35                  40                  45
Ile Tyr Ala Ala Ser Ile Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg
            100                 105


<210>  56
<211>  120

<212>  PRT
<213>  Homo sapiens

<400>  56

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser
        115                 120


<210>  57
<211>  120
<212>  PRT
<213>  Homo sapiens

<400>  57

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  58
<211>  120
<212>  PRT
<213>  Homo sapiens

<400>  58

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60

Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser
        115                 120

<210>  59
<211>  120
<212>  PRT
<213>  Homo sapiens

<400>  59

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser
        115                 120

<210>  60
<211>  120
<212>  PRT
<213>  Homo sapiens

<400>  60

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Glu Trp Val
        35                  40                  45
Gly Arg Ile Gly Thr Ala Gly Arg Thr Asn Tyr Asn Pro Ser Leu Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser
        115                 120

<210>  61
<211>  120
<212>  PRT

<213>  Homo sapiens

<400>  61

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Val Trp Val
        35                  40                  45
Ser Arg Ile Gly Thr Ala Gly Asp Arg Tyr Tyr Ala Gly Ser Val Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser
        115                 120


<210>  62
<211>  120
<212>  PRT
<213>  Homo sapiens

<400>  62

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  63
<211>  120
<212>  PRT
<213>  Homo sapiens

<400>  63

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Phe Phe Ser Gly Ser Ala Ala Thr Gly Ser Val
    50                  55                  60
Lys Gly Arg Phe Thr Ile Ser Arg Asp Asn Ala Lys Asn Thr Leu Tyr

65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  64
<211>  120
<212>  PRT
<213>  Homo sapiens

<400>  64

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Leu Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  65
<211>  120
<212>  PRT
<213>  Homo sapiens

<400>  65

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Leu Thr Leu Ser Val Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser
        115                 120


<210>  66
<211>  120
<212>  PRT
<213>  Homo sapiens

<400> 66

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser
        115                 120


<210>  67
<211>  120
<212>  PRT
<213>  Homo sapiens

<400>  67

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Leu Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser
        115                 120


<210>  68
<211>  120
<212>  PRT
<213>  Homo sapiens

<400>  68

Glu Val Gln Leu Val Glu Ser Gly Gly Gly Val Val Gln Pro Gly Gly
1               5                   10                  15
Ser Leu Arg Leu Ser Cys Ala Ala Ser Gly Ile Thr Phe Arg Ser Tyr
            20                  25                  30
Met Asp Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Val Ser Arg Asp Asn Ala Lys Asn Thr Leu Tyr
65                  70                  75                  80

Leu Gln Met Asn Ser Leu Arg Ala Glu Asp Thr Ala Val Tyr Tyr Cys
                85                  90                  95
Ala Arg Asp Ala Gly Arg Trp Ala Asp Leu Ala Phe Asp Ile Trp Gly
            100                 105                 110
Gln Gly Thr Leu Val Thr Val Ser
        115                 120


<210>  69
<211>  120
<212>  PRT
<213>  Homo sapiens

<400>  69

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Thr Ala Asp Arg Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  70
<211>  120
<212>  PRT
<213>  Homo sapiens

<400>  70

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser
        115                 120


<210>  71
<211>  120
<212>  PRT
<213>  Homo sapiens

<400> 71

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Lys Tyr Tyr Ala Gly Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Ser Val Ser
        115                 120


<210>  72
<211>  106
<212>  PRT
<213>  Homo sapiens

<400>  72

Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105


<210>  73
<211>  106
<212>  PRT
<213>  Homo sapiens

<400>  73

Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Leu Tyr Ala Cys Glu Val Thr His Gln Gly Leu Ser Ser Pro
                85                  90                  95
Val Thr Lys Ser Phe Asn Arg Gly Glu Cys

100                 105


<210>  74
<211>  109
<212>  PRT
<213>  Homo sapiens

<400>  74

Thr Val Leu Gly Gln Pro Lys Ala Ala Pro Ser Val Thr Leu Phe Pro
1               5                   10                  15
Pro Ser Ser Glu Glu Leu Gln Ala Asn Lys Ala Thr Leu Val Cys Leu
            20                  25                  30
Ile Ser Asp Phe Tyr Pro Gly Ala Val Thr Val Ala Trp Lys Ala Asp
        35                  40                  45
Ser Ser Pro Val Lys Ala Gly Val Glu Thr Thr Thr Pro Ser Lys Gln
    50                  55                  60
Ser Asn Asn Lys Tyr Ala Ala Ser Ser Tyr Leu Ser Leu Thr Pro Glu
65                  70                  75                  80
Gln Trp Lys Ser His Arg Ser Tyr Ser Cys Gln Val Thr His Glu Gly
                85                  90                  95
Ser Thr Val Glu Lys Thr Val Ala Pro Thr Glu Cys Ser
            100                 105


<210>  75
<211>  331
<212>  PRT
<213>  Homo sapiens

<400>  75

Ser Ala Ser Thr Lys Gly Pro Ser Val Phe Pro Leu Ala Pro Ser Ser
1               5                   10                  15
Lys Ser Thr Ser Gly Gly Thr Ala Ala Leu Gly Cys Leu Val Lys Asp
            20                  25                  30
Tyr Phe Pro Glu Pro Val Thr Val Ser Trp Asn Ser Gly Ala Leu Thr
        35                  40                  45
Ser Gly Val His Thr Phe Pro Ala Val Leu Gln Ser Ser Gly Leu Tyr
    50                  55                  60
Ser Leu Ser Ser Val Val Thr Val Pro Ser Ser Ser Leu Gly Thr Gln
65                  70                  75                  80
Thr Tyr Ile Cys Asn Val Asn His Lys Pro Ser Asn Thr Lys Val Asp
                85                  90                  95
Lys Lys Val Glu Pro Lys Ser Cys Asp Lys Thr His Thr Cys Pro Pro
            100                 105                 110
Cys Pro Ala Pro Glu Leu Leu Gly Gly Pro Ser Val Phe Leu Phe Pro
        115                 120                 125
Pro Lys Pro Lys Asp Thr Leu Met Ile Ser Arg Thr Pro Glu Val Thr
    130                 135                 140
Cys Val Val Val Asp Val Ser His Glu Asp Pro Glu Val Lys Phe Asn
145                 150                 155                 160
Trp Tyr Val Asp Gly Val Glu Val His Asn Ala Lys Thr Lys Pro Arg
                165                 170                 175
Glu Glu Gln Phe Asn Ser Thr Phe Arg Val Val Ser Val Leu Thr Val
            180                 185                 190
Leu His Gln Asp Trp Leu Asn Gly Lys Glu Tyr Lys Cys Lys Val Ser
        195                 200                 205
Asn Lys Ala Leu Pro Ala Pro Ile Glu Lys Thr Ile Ser Lys Ala Lys
    210                 215                 220
Gly Gln Pro Arg Glu Pro Gln Val Tyr Thr Leu Pro Pro Ser Arg Asp
225                 230                 235                 240

Glu Leu Thr Lys Asn Gln Val Ser Leu Thr Cys Leu Val Lys Gly Phe
                245                 250                 255
Tyr Pro Ser Asp Ile Ala Val Glu Trp Glu Ser Asn Gly Gln Pro Glu
            260                 265                 270
Asn Asn Tyr Lys Thr Thr Pro Pro Val Leu Asp Ser Asp Gly Ser Phe
        275                 280                 285
Phe Leu Tyr Ser Lys Leu Thr Val Asp Lys Ser Arg Trp Gln Gln Gly
    290                 295                 300
Asn Val Phe Ser Cys Ser Val Met His Glu Ala Leu His Asn His Tyr
305                 310                 315                 320
Thr Gln Lys Ser Leu Ser Leu Ser Pro Gly Lys
                325                 330

<210>  76
<211>  331
<212>  PRT
<213>  Homo sapiens

<400>  76

Ser Ala Ser Thr Lys Gly Pro Ser Val Phe Pro Leu Ala Pro Cys Ser
1               5                   10                  15
Arg Ser Thr Ser Glu Ser Thr Ala Ala Leu Gly Cys Leu Val Lys Asp
            20                  25                  30
Tyr Phe Pro Glu Pro Val Thr Val Ser Trp Asn Ser Gly Ala Leu Thr
        35                  40                  45
Ser Gly Val His Thr Phe Pro Ala Val Leu Gln Ser Ser Gly Leu Tyr
    50                  55                  60
Ser Leu Ser Ser Val Val Thr Val Pro Ser Ser Ser Leu Gly Thr Gln
65                  70                  75                  80
Thr Tyr Thr Cys Asn Val Asp His Lys Pro Ser Asn Thr Lys Val Asp
                85                  90                  95
Lys Lys Val Glu Pro Lys Ser Cys Asp Lys Thr His Thr Cys Pro Pro
            100                 105                 110
Cys Pro Ala Pro Glu Leu Leu Gly Gly Pro Ser Val Phe Leu Phe Pro
        115                 120                 125
Pro Lys Pro Lys Asp Thr Leu Met Ile Ser Arg Thr Pro Glu Val Thr
    130                 135                 140
Cys Val Val Val Asp Val Ser His Glu Asp Pro Glu Val Lys Phe Asn
145                 150                 155                 160
Trp Tyr Val Asp Gly Val Glu Val His Asn Ala Lys Thr Lys Pro Arg
                165                 170                 175
Glu Glu Gln Tyr Asn Ser Thr Tyr Arg Val Val Ser Val Leu Thr Val
            180                 185                 190
Val His Gln Asp Trp Leu Asn Gly Lys Glu Tyr Lys Cys Lys Val Ser
        195                 200                 205
Asn Lys Gly Leu Pro Ala Pro Ile Glu Lys Thr Ile Ser Lys Ala Lys
    210                 215                 220
Gly Gln Pro Arg Glu Pro Gln Val Tyr Thr Leu Pro Pro Ser Arg Glu
225                 230                 235                 240
Glu Met Thr Lys Asn Gln Val Thr Leu Thr Cys Leu Val Lys Gly Phe
                245                 250                 255
Tyr Pro Ser Asp Ile Ala Val Glu Trp Glu Ser Asn Gly Gln Pro Glu
            260                 265                 270
Asn Asn Tyr Lys Thr Thr Pro Pro Met Leu Asp Ser Asp Gly Ser Phe
        275                 280                 285
Phe Leu Tyr Ser Lys Leu Thr Val Asp Lys Ser Arg Trp Gln Gln Gly
    290                 295                 300
Asn Val Phe Ser Cys Ser Val Met His Glu Ala Leu His Asn His Tyr
305                 310                 315                 320
Thr Gln Lys Ser Leu Ser Leu Ser Pro Gly Lys
                325                 330

<210> 77
<211> 331
<212> PRT
<213> Homo sapiens

<400> 77

Ser Ala Ser Thr Lys Gly Pro Ser Val Phe Pro Leu Ala Pro Ser Ser
1               5                   10                  15
Lys Ser Thr Ser Gly Gly Thr Ala Ala Leu Gly Cys Leu Val Lys Asp
            20                  25                  30
Tyr Phe Pro Glu Pro Val Thr Val Ser Trp Asn Ser Gly Ala Leu Thr
        35                  40                  45
Ser Gly Val His Thr Phe Pro Ala Val Leu Gln Ser Ser Gly Leu Tyr
    50                  55                  60
Ser Leu Ser Ser Val Val Thr Val Pro Ser Ser Ser Leu Gly Thr Gln
65                  70                  75                  80
Thr Tyr Ile Cys Asn Val Asn His Lys Pro Ser Asn Thr Lys Val Asp
                85                  90                  95
Lys Lys Val Glu Pro Lys Ser Cys Asp Lys Thr His Thr Cys Pro Pro
            100                 105                 110
Cys Pro Ala Pro Glu Leu Leu Gly Gly Pro Ser Val Phe Leu Phe Pro
        115                 120                 125
Pro Lys Pro Lys Asp Thr Leu Met Ile Ser Arg Thr Pro Glu Val Thr
    130                 135                 140
Cys Val Val Val Asp Val Ser His Glu Asp Pro Glu Val Lys Phe Asn
145                 150                 155                 160
Trp Tyr Val Asp Gly Val Glu Val His Asn Ala Lys Thr Lys Pro Arg
                165                 170                 175
Glu Glu Gln Phe Ala Ser Thr Phe Arg Val Val Ser Val Leu Thr Val
            180                 185                 190
Leu His Gln Asp Trp Leu Asn Gly Lys Glu Tyr Lys Cys Lys Val Ser
        195                 200                 205
Asn Lys Gly Leu Pro Ala Pro Ile Glu Lys Thr Ile Ser Lys Ala Lys
    210                 215                 220
Gly Gln Pro Arg Glu Pro Gln Val Tyr Thr Leu Pro Pro Ser Arg Asp
225                 230                 235                 240
Glu Leu Thr Lys Asn Gln Val Ser Leu Thr Cys Leu Val Lys Gly Phe
                245                 250                 255
Tyr Pro Ser Asp Ile Ala Val Glu Trp Glu Ser Asn Gly Gln Pro Glu
            260                 265                 270
Asn Asn Tyr Lys Thr Thr Pro Pro Val Leu Asp Ser Asp Gly Ser Phe
        275                 280                 285
Phe Leu Tyr Ser Lys Leu Thr Val Asp Lys Ser Arg Trp Gln Gln Gly
    290                 295                 300
Asn Val Phe Ser Cys Ser Val Met His Glu Ala Leu His Asn His Tyr
305                 310                 315                 320
Thr Gln Lys Ser Leu Ser Leu Ser Pro Gly Lys
                325                 330


<210> 78
<211> 215
<212> PRT
<213> Homo sapiens

<400> 78

Glu Ile Val Leu Thr Gln Ser Pro Ala Thr Leu Ser Leu Ser Pro Gly
1               5                   10                  15
Glu Arg Val Thr Ile Thr Cys Arg Ala Ser Gln Ser Val Asn Ser Tyr

                20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  79
<211>  215
<212>  PRT
<213>  Homo sapiens

<400>  79

Glu Ile Val Leu Thr Gln Ser Pro Ala Thr Leu Ser Leu Ser Pro Gly
1               5                   10                  15
Glu Arg Val Thr Ile Thr Cys Arg Ala Ser Gln Ser Val Asn Ser Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  80
<211>  215
<212>  PRT
<213>  Homo sapiens

<400>  80

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  81
<211>  215
<212>  PRT
<213>  Homo sapiens

<400>  81

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  82
<211>  220
<212>  PRT
<213>  Homo sapiens

<400>  82

Leu Pro Val Leu Thr Gln Pro Pro Ser Ala Ser Ala Leu Leu Gly Ala
1               5                   10                  15
Ser Ile Lys Leu Thr Cys Thr Leu Ser Ser Glu His Ser Thr Tyr Thr
            20                  25                  30
Ile Glu Trp Tyr Gln Gln Arg Pro Gly Arg Ser Pro Gln Tyr Ile Met
        35                  40                  45
Lys Val Lys Ser Asp Gly Ser His Ser Lys Gly Asp Gly Ile Pro Asp
    50                  55                  60
Arg Phe Met Gly Ser Ser Ser Gly Ala Asp Arg Tyr Leu Thr Phe Ser
65                  70                  75                  80
Asn Leu Gln Ser Asp Asp Glu Ala Glu Tyr His Cys Gly Glu Ser His
                85                  90                  95
Thr Ile Asp Gly Gln Cys Trp Val Phe Gly Gly Gly Thr Lys Leu Thr
            100                 105                 110
Val Leu Gly Gln Pro Lys Ala Ala Pro Ser Val Thr Leu Phe Pro Pro
        115                 120                 125
Ser Ser Glu Glu Leu Gln Ala Asn Lys Ala Thr Leu Val Cys Leu Ile
    130                 135                 140
Ser Asp Phe Tyr Pro Gly Ala Val Thr Val Ala Trp Lys Ala Asp Ser
145                 150                 155                 160
Ser Pro Val Lys Ala Gly Val Glu Thr Thr Thr Pro Ser Lys Gln Ser
                165                 170                 175
Asn Asn Lys Tyr Ala Ala Ser Ser Tyr Leu Ser Leu Thr Pro Glu Gln
            180                 185                 190
Trp Lys Ser His Arg Ser Tyr Ser Cys Gln Val Thr His Glu Gly Ser
        195                 200                 205
Thr Val Glu Lys Thr Val Ala Pro Thr Glu Cys Ser
    210                 215                 220

<210>  83
<211>  215
<212>  PRT
<213>  Homo sapiens

<400>  83

Asp Ile Gln Met Thr Gln Ser Pro Ala Thr Leu Ser Leu Ser Pro Gly
1               5                   10                  15
Glu Arg Ala Ala Leu Ser Cys Arg Ala Ser Gln Ser Val Asn Ser Tyr
            20                  25                  30
Leu Ala Trp Tyr Lys Gln Gln Lys Pro Gly Gln Ala Pro Arg Leu Leu
        35                  40                  45
Ile Tyr Gly Ala Ala Ser Arg Ala Thr Gly Ile Pro Ala Arg Phe Ser

50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  84
<211>  215
<212>  PRT
<213>  Homo sapiens

<400>  84

Asp Ile Gln Met Thr Gln Ser Pro Ala Thr Leu Ser Leu Ser Pro Gly
1               5                   10                  15
Glu Arg Ala Ala Leu Ser Cys Arg Ala Ser Gln Ser Val Asn Ser Tyr
            20                  25                  30
Leu Ala Trp Tyr Lys Gln Gln Lys Pro Gly Gln Ala Pro Arg Leu Leu
        35                  40                  45
Ile Tyr Gly Ala Ala Ser Arg Ala Thr Gly Ile Pro Ala Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  85
<211>  215
<212>  PRT

<213>  Homo sapiens

<400>  85

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  86
<211>  215
<212>  PRT
<213>  Homo sapiens

<400>  86

Glu Ile Val Leu Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  87
<211>  215
<212>  PRT
<213>  Homo sapiens

<400>  87

Glu Ile Val Met Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ser Ser Leu Gln Ser Gly Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  88
<211>  215
<212>  PRT
<213>  Homo sapiens

<400>  88

Glu Ile Val Met Thr Gln Ser Pro Ala Thr Leu Ser Leu Ser Pro Gly
1               5                   10                  15
Glu Arg Val Thr Ile Thr Cys Arg Glu Ser Gln Gly Ile Arg Asn Tyr
            20                  25                  30
Leu Ala Trp Tyr Gln Gln Lys Pro Pro Gly Gln Ala Pro Arg Leu Leu
        35                  40                  45
Ile Tyr Ala Ser Ser Leu Gln Ser Gly Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln

                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  89
<211>  214
<212>  PRT
<213>  Homo sapiens

<400>  89

Asp Val Val Met Thr Gln Ser Pro Ser Ser Leu Ser Ala Ser Val Gly
1               5                   10                  15
Asp Arg Val Thr Ile Thr Cys Arg Glu Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Lys Ala Ser Ser Leu Gln Ser Gly Val Pro Ser Arg Phe Ser Gly
    50                  55                  60
Ser Leu Leu Gly Gly Lys Ala Ala Leu Thr Leu Ser Gly Val Gln Pro
65                  70                  75                  80
Glu Asp Phe Ala Thr Tyr Tyr Cys Gln Gln Ala Asn Ser Phe Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  90
<211>  214
<212>  PRT
<213>  Homo sapiens

<400>  90

Asp Val Val Met Thr Gln Ser Pro Ser Ser Leu Ser Ala Ser Val Gly
1               5                   10                  15
Asp Arg Val Thr Ile Thr Cys Arg Glu Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Lys Ala Ser Ser Leu Gln Ser Gly Val Pro Ser Arg Phe Ser Gly
    50                  55                  60
Ser Leu Leu Gly Gly Lys Ala Ala Leu Thr Leu Ser Gly Val Gln Pro
65                  70                  75                  80
Glu Asp Phe Ala Thr Tyr Tyr Cys Gln Gln Ala Asn Ser Phe Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Leu Tyr
            180                 185                 190
Ala Cys Glu Val Thr His Gln Gly Leu Ser Ser Pro Val Thr Lys Ser
        195                 200                 205
Phe Asn Arg Gly Glu Cys
    210


<210>  91
<211>  215
<212>  PRT
<213>  Homo sapiens

<400>  91

Asp Ile Val Leu Thr Gln Ser Pro Ala Thr Leu Ser Leu Ser Pro Gly
1               5                   10                  15
Glu Arg Val Thr Ile Thr Cys Arg Ala Ser Gln Ser Val Asn Ser Tyr
            20                  25                  30
Leu Ala Trp Tyr Gln Gln Lys Pro Pro Gly Gln Ala Pro Arg Leu Leu
        35                  40                  45
Ile Tyr Ala Gly Ser Thr Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205

Ser Phe Asn Arg Gly Glu Cys
    210                 215


<210>  92
<211>  215
<212>  PRT
<213>  Homo sapiens

<400>  92

Asp Ile Gln Met Thr Gln Ser Pro Gly Thr Leu Ser Leu Ser Pro Gly
1               5                   10                  15
Glu Arg Ala Ala Leu Ser Cys Arg Ala Ser Gln Ser Val Ser Ser Tyr
            20                  25                  30
Leu Ala Trp Tyr Ser Gln Gln Lys Pro Gly Gln Ala Pro Arg Leu Leu
        35                  40                  45
Ile Tyr Ala Ala Ser Ile Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210>  93
<211>  215
<212>  PRT
<213>  Homo sapiens

<400>  93

Asp Ile Gln Met Thr Gln Ser Pro Gly Thr Leu Ser Leu Ser Pro Gly
1               5                   10                  15
Glu Arg Ala Ala Leu Ser Cys Arg Ala Ser Gln Ser Val Ser Ser Tyr
            20                  25                  30
Leu Ala Trp Tyr Ser Gln Gln Lys Pro Gly Gln Ala Pro Arg Leu Leu
        35                  40                  45
Ile Tyr Ala Ala Ser Ile Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Gln
                85                  90                  95
Gly Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser

115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Leu
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  94
<211>  451
<212>  PRT
<213>  Homo sapiens

<400>  94

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile

                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 95
<211> 451
<212> PRT
<213> Homo sapiens

<400> 95

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr

290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  96
<211>  451
<212>  PRT
<213>  Homo sapiens

<400>  96

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His

260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  97
<211>  451
<212>  PRT
<213>  Homo sapiens

<400>  97

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly

225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  98
<211>  451
<212>  PRT
<213>  Homo sapiens

<400>  98

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His

195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  99
<211>  451
<212>  PRT
<213>  Homo sapiens

<400>  99

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala

                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  100
<211>  451
<212>  PRT
<213>  Homo sapiens

<400>  100

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala

130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  101
<211>  451
<212>  PRT
<213>  Homo sapiens

<400>  101

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly

                100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  102
<211>  451
<212>  PRT
<213>  Homo sapiens

<400>  102

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60
Lys Gly Arg Phe Thr Ile Ser Arg Asp Asn Ala Lys Asn Thr Leu Tyr

65                  70                  75                  80
Leu Gln Met Asn Ser Leu Arg Ala Glu Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  103
<211>  451
<212>  PRT
<213>  Homo sapiens

<400>  103

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val

35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  104
<211>  451
<212>  PRT
<213>  Homo sapiens

<400>  104

Glu Val Gln Leu Val Glu Ser Gly Gly Gly Val Val Gln Pro Gly Gly

1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  105
<211>  451
<212>  PRT
<213>  Homo sapiens

<400> 105

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 106
<211> 451
<212> PRT
<213> Homo sapiens

<400> 106

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Glu Trp Val
        35                  40                  45
Gly Arg Ile Gly Thr Ala Gly Arg Thr Asn Tyr Asn Pro Ser Leu Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445

Pro Gly Lys
    450


<210>  107
<211>  451
<212>  PRT
<213>  Homo sapiens

<400>  107

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Glu Trp Val
        35                  40                  45
Gly Arg Ile Gly Thr Ala Gly Arg Thr Asn Tyr Asn Pro Ser Leu Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  108
<211>  451
<212>  PRT
<213>  Homo sapiens

<400>  108

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Glu Trp Val
        35                  40                  45
Gly Arg Ile Gly Thr Ala Gly Arg Thr Asn Tyr Asn Pro Ser Leu Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  109
<211>  451
<212>  PRT
<213>  Homo sapiens

<400>  109

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Val Trp Val
        35                  40                  45
Ser Arg Ile Gly Thr Ala Gly Asp Arg Tyr Tyr Ala Gly Ser Val Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  110
<211>  451
<212>  PRT
<213>  Homo sapiens

<400>  110

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Val Trp Val
        35                  40                  45
Ser Arg Ile Gly Thr Ala Gly Asp Arg Tyr Tyr Ala Gly Ser Val Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320

Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  111
<211>  451
<212>  PRT
<213>  Homo sapiens

<400>  111

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Asp Met His Trp Val Arg Gln Gly Ile Gly Lys Gly Leu Val Trp Val
        35                  40                  45
Ser Arg Ile Gly Thr Ala Gly Asp Arg Tyr Tyr Ala Gly Ser Val Lys
    50                  55                  60
Gly Arg Phe Thr Ile Ser Arg Glu Asn Ala Lys Asp Ser Leu Tyr Leu
65                  70                  75                  80
Gln Met Asn Ser Leu Arg Val Gly Asp Ala Ala Val Tyr Tyr Cys Ala
                85                  90                  95
Arg Gly Ala Gly Arg Trp Ala Pro Leu Gly Ala Phe Asp Ile Trp Gly
            100                 105                 110
Gln Gly Thr Leu Val Ile Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285

His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  112
<211>  451
<212>  PRT
<213>  Homo sapiens

<400>  112

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  113
<211>  451
<212>  PRT
<213>  Homo sapiens

<400>  113

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  114
<211>  451
<212>  PRT
<213>  Homo sapiens

<400>  114

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  115
<211>  451
<212>  PRT
<213>  Homo sapiens

<400>  115

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Phe Phe Ser Gly Ser Ala Ala Thr Gly Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  116
<211>  451
<212>  PRT
<213>  Homo sapiens

<400>  116

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Phe Phe Ser Gly Ser Ala Ala Thr Gly Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  117
<211>  451
<212>  PRT
<213>  Homo sapiens

<400>  117

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Phe Phe Ser Gly Ser Ala Ala Thr Gly Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95

Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 118
<211> 451
<212> PRT
<213> Homo sapiens

<400> 118

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60

Lys Gly Arg Pro Thr Ile Ser Arg Asp Asn Ala Lys Asn Gln Leu Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  119
<211>  451
<212>  PRT
<213>  Homo sapiens

<400>  119

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30

Asp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Leu Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  120
<211>  451
<212>  PRT
<213>  Homo sapiens

<400>  120

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Asp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Pro Thr Ile Ser Arg Asp Asn Ala Lys Asn Gln Leu Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  121
<211>  451
<212>  PRT

<213> Homo sapiens

<400> 121

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Leu Thr Leu Ser Val Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 122
<211> 451
<212> PRT
<213> Homo sapiens

<400> 122

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Leu Thr Leu Ser Val Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser

        435                 440                 445
Pro Gly Lys
    450



<210>  123
<211>  451
<212>  PRT
<213>  Homo sapiens

<400>  123

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Leu Thr Leu Ser Val Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val

                    405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  124
<211>  451
<212>  PRT
<213>  Homo sapiens

<400>  124

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu

        370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  125
<211>  451
<212>  PRT
<213>  Homo sapiens

<400>  125

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val

340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  126
<211>  451
<212>  PRT
<213>  Homo sapiens

<400>  126

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly

305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  127
<211>  451
<212>  PRT
<213>  Homo sapiens

<400>  127

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Leu Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val

275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  128
<211>  451
<212>  PRT
<213>  Homo sapiens

<400>  128

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Leu Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met

                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 129
<211> 451
<212> PRT
<213> Homo sapiens

<400> 129

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Ser Leu Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys

210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  130
<211>  451
<212>  PRT
<213>  Homo sapiens

<400>  130

Glu Val Gln Leu Val Glu Ser Gly Gly Gly Val Val Gln Pro Gly Gly
1               5                   10                  15
Ser Leu Arg Leu Ser Cys Ala Ala Ser Gly Ile Thr Phe Arg Ser Tyr
            20                  25                  30
Met Asp Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Val Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Gly Arg Trp Ala Asp Leu Ala Phe Asp Ile Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val

180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  131
<211>  451
<212>  PRT
<213>  Homo sapiens

<400>  131

Glu Val Gln Leu Val Glu Ser Gly Gly Gly Val Val Gln Pro Gly Gly
1               5                   10                  15
Ser Leu Arg Leu Ser Cys Ala Ala Ser Gly Ile Thr Phe Arg Ser Tyr
            20                  25                  30
Met Asp Ser Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Phe Thr Val Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Gly Arg Trp Ala Asp Leu Ala Phe Asp Ile Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val

145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  132
<211>  451
<212>  PRT
<213>  Homo sapiens

<400>  132

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Thr Ala Asp Arg Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser

115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  133
<211>  451
<212>  PRT
<213>  Homo sapiens

<400>  133

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Ser Tyr
            20                  25                  30
Trp Met His Trp Val Arg Gln Ala Pro Gly Lys Gly Leu Glu Trp Val
        35                  40                  45
Ala Ser Val Lys Gln Asp Gly Ser Glu Lys Tyr Tyr Val Asp Ser Val
    50                  55                  60
Lys Gly Arg Val Thr Ile Thr Ala Asp Arg Ala Lys Asn Thr Leu Tyr
65                  70                  75                  80
Leu Gln Met Asn Ser Leu Arg Ala Glu Asp Thr Ala Val Tyr Tyr Cys

                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Thr Cys Asn Val Asp His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Thr
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 134
<211> 451
<212> PRT
<213> Homo sapiens

<400> 134

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val

50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450


<210>  135
<211>  451
<212>  PRT
<213>  Homo sapiens

<400>  135

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Asn Tyr

20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Pro Leu Ala Thr Gly Ser Val
    50                  55                  60
Lys Gly Arg Phe Thr Ile Ser Arg Asp Thr Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Arg Asp Trp Phe Asp Pro Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  136
<211>  451
<212>  PRT
<213>  Homo sapiens

<400>  136

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Lys Tyr Tyr Ala Gly Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210>  137
<211>  451

<212>  PRT
<213>  Homo sapiens

<400>  137

Glu Val Gln Leu Val Glu Ser Gly Gly Gly Val Val Gln Pro Gly Gly
1               5                   10                  15
Ser Leu Arg Leu Ser Cys Ala Ala Ser Gly Phe Thr Phe Arg Gly Tyr
            20                  25                  30
Trp Met Ser Trp Val Arg Gln Ala Pro Gly Lys Gly Leu Glu Trp Val
        35                  40                  45
Ala Ser Val Lys Gln Phe Phe Ser Gly Lys Tyr Tyr Ala Gly Ser Val
    50                  55                  60
Lys Gly Arg Val Thr Ile Ser Val Glu Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Ala Ser Ser Trp Tyr Arg Asp Trp Phe Asp Pro Trp Gly
            100                 105                 110
Gln Gly Thr Leu Val Ser Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Phe Ala Ser Thr Phe
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 138
<211> 18
<212> PRT
<213> Homo sapiens

<400> 138

Glu Ile Val Leu Thr Gln Ser Pro Ala Thr Leu Ser Leu Ser Pro Gly
1               5                   10                  15
Glu Arg

<210> 139
<211> 18
<212> PRT
<213> Homo sapiens

<400> 139

Glu Ile Val Met Thr Gln Ser Pro Ala Thr Leu Ser Leu Ser Pro Gly
1               5                   10                  15
Glu Arg

<210> 140
<211> 18
<212> PRT
<213> Homo sapiens

<400> 140

Asp Ile Gln Met Thr Gln Ser Pro Ala Thr Leu Ser Leu Ser Pro Gly
1               5                   10                  15
Glu Arg

<210> 141
<211> 18
<212> PRT
<213> Homo sapiens

<400> 141

Asp Val Val Met Thr Gln Ser Pro Ser Ser Leu Ser Ala Ser Val Gly
1               5                   10                  15
Asp Arg

<210> 142
<211> 18
<212> PRT
<213> Homo sapiens

<400> 142

Asp Ile Val Leu Thr Gln Ser Pro Ala Thr Leu Ser Leu Ser Pro Gly
1               5                   10                  15

Glu Arg

<210> 143
<211> 18
<212> PRT
<213> Homo sapiens

<400> 143

Asp Ile Gln Met Thr Gln Ser Pro Gly Thr Leu Ser Leu Ser Pro Gly
1               5                   10                  15
Glu Arg

<210> 144
<211> 18
<212> PRT
<213> Homo sapiens

<220>
<221> MISC_FEATURE
<222> (1)..(1)
<223> Xaa can be Glu or Asp

<220>
<221> MISC_FEATURE
<222> (2)..(2)
<223> Xaa can be Ile or Val

<220>
<221> MISC_FEATURE
<222> (3)..(3)
<223> Xaa can be  Val or Gln

<220>
<221> MISC_FEATURE
<222> (4)..(4)
<223> Xaa can be  Leu or Met

<220>
<221> MISC_FEATURE
<222> (9)..(9)
<223> Xaa can be  Ala, Gly or Ser

<220>
<221> MISC_FEATURE
<222> (10)..(10)
<223> Xaa can be  Thr or Ser

<220>
<221> MISC_FEATURE
<222> (13)..(13)
<223> Xaa can be Leu or Ala

<220>
<221> MISC_FEATURE
<222> (15)..(15)
<223> Xaa can be Pro or Val

<220>
<221>  MISC_FEATURE
<222>  (17)..(17)
<223>  Xaa can be Glu or Asp

<400>  144

Xaa Xaa Xaa Xaa Thr Gln Ser Pro Xaa Xaa Leu Ser Xaa Ser Xaa Gly
1               5                   10                  15
Xaa Arg